# EUROPEAN PATENT APPLICATION

(11) **EP 1 659 180 A2**
(43) Date of publication of application: **24.05.2006**
(21) Application number: 05110550.0
(22) Date of filing: 11.10.2000
(51) Int. Cl.: C12N 15/82, C07K 14/415, A01H 5/00

(54) **Flowering time modification**

(30) Priority: 12.10.1999 US 159464 P; 08.11.1999 US 164132 P; 17.11.1999 US 166228 P; 17.04.2000 US 197899 P; 22.08.2000 US 227439 P
(62) Divisional of application: 00978217.8
(71) Applicant: Mendel Biotechnology, Inc., Hayward, CA 94541 (US); Ratcliffe, Oliver, Oakland, CA 94606 (US); Heard, Jacqueline, San Mateo, CA 94402 (US); Samaha, Raymond, Capitola, CA 95010 (US); Creelman, Robert, Castro Valley, CA 94546 (US); Keddie, James, San Mateo, CA 94403 (US); Jiang, Cai-Zhong, Fremont, CA 94555 (US); Reuber, Lynne J., Fremont, CA 94538 (US); Riechmann, Jose Luis, Oakland, CA 94611 (US)
(72) Inventor: Ratcliffe, Oliver, Oakland, CA 94606 (US); Heard, Jaqueline, San Mateo, CA 94402 (US); Samaha, Raymond, Capitola, CA 95010 (US); Creelman, Robert, Castro Valley, CA 94546 (US); Keddie, James, San Mateo, CA 94403 (US); Jiang, Cai-Zhong, Fremont, CA 94555 (US); Reuber, Lynne, Fremont, CA 94538 (US); Riechmann, Jose Luis, Oakland, CA 94611 (US)
(74) Representative: von Kreisler Selting Werner

(57) **Abstract**

Recombinant polynucleotides and methods for modifying the flowering time of a plant are provided. Plants transformed with the recombinant polynucleotides may have flowering times that are accelerated, delayed or induced under specific conditions. Additionally, transformed plants may have altered vernalization requirements.

SEQUENCE LISTING

<110> Ratcliffe, Oliver
      Heard, Jacqueline
      Samaha, Raymond
      Creelman, Robert
      Keddie, James
      Jiang, Cai-zhong
      Reuber, Lynne
      Riechmann, Jose Luis

<120> Flowering Time Modification

<130> MBI-0021

<150> US 60/159,464
<151> 1999-10-12

<150> US 60/166,228
<151> 1999-11-17

<150> US 60/197,899
<151> 2000-04-17

<150> Plant Trait Modification III
<151> 2000-08-22

<150> US 60/164,132
<151> 1999-11-08

<160> 64

<170> PatentIn version 3.0

<210> 1
<211> 883
<212> DNA
<213> Arabidopsis thaliana

<220>
<221> CDS
<222> (31)..(621)
<223> G157

<400> 1
gggcataacc cttatcggag atttgaagcc atg gga aga aga aaa atc gag atc 54
Met Gly Arg Arg Lys Ile Glu Ile
1               5

aag cga atc gag aac aaa agc agt cga caa gtc act ttc tcc aaa cga 102
Lys Arg Ile Glu Asn Lys Ser Ser Arg Gln Val Thr Phe Ser Lys Arg
    10                  15                  20

cgc aat ggt ctc atc gac aaa gct cga caa ctt tcg att ctc tgt gaa 150
Arg Asn Gly Leu Ile Asp Lys Ala Arg Gln Leu Ser Ile Leu Cys Glu
25                  30                  35                  40

tcc tcc gtc gct gtt gtc gtc gta tct gcc tcc gga aaa ctc tat gac 198
Ser Ser Val Ala Val Val Val Val Ser Ala Ser Gly Lys Leu Tyr Asp
                45                  50                  55

tct tcc tcc ggt gac gac att tcc aag atc att gat cgt tat gaa ata 246

Ser Ser Ser Gly Asp Asp Ile Ser Lys Ile Ile Asp Arg Tyr Glu Ile
            60                  65                  70

caa cat gct gat gaa ctt aga gcc tta gat ctt gaa gaa aaa att cag        294
Gln His Ala Asp Glu Leu Arg Ala Leu Asp Leu Glu Glu Lys Ile Gln
        75                  80                  85

aat tat ctt cca cac aag gag tta cta gaa aca gtc caa agc aag ctt        342
Asn Tyr Leu Pro His Lys Glu Leu Leu Glu Thr Val Gln Ser Lys Leu
    90                  95                  100

gaa gaa cca aat gtc gat aat gta agt gta gat tct cta att tct ctg        390
Glu Glu Pro Asn Val Asp Asn Val Ser Val Asp Ser Leu Ile Ser Leu
105                 110                 115                 120

gag gaa caa ctt gag act gct ctg tcc gta agt aga gct agg aag gca        438
Glu Glu Gln Leu Glu Thr Ala Leu Ser Val Ser Arg Ala Arg Lys Ala
                125                 130                 135

gaa ctg atg atg gag tat atc gag tcc ctt aaa gaa aag gag aaa ttg        486
Glu Leu Met Met Glu Tyr Ile Glu Ser Leu Lys Glu Lys Glu Lys Leu
            140                 145                 150

ctg aga gaa gag aac cag gtt ctg gct agc cag atg gga aag aat acg        534
Leu Arg Glu Glu Asn Gln Val Leu Ala Ser Gln Met Gly Lys Asn Thr
        155                 160                 165

ttg ctg gca aca gat gat gag aga gga atg ttt ccg gga agt agc tcc        582
Leu Leu Ala Thr Asp Asp Glu Arg Gly Met Phe Pro Gly Ser Ser Ser
    170                 175                 180

ggc aac aaa ata ccg gag act ctc ccg ctg ctc aat tag ccaccatcat        631
Gly Asn Lys Ile Pro Glu Thr Leu Pro Leu Leu Asn
185                 190                 195

caacggctga gttttcacct taaactcaaa gcctgattca taattaagag aataaatttg        691

tatattataa aaagctgtgt aatctcaaac cttttatctt cctctagtgt ggaatttaag        751

gtcaaaaaga aaacgagaaa gtatggatca gtgttgtacc tccttcggag acaagatcag        811

agtttgtgtg tttgtgtctg aatgtacgga ttggattttt aaagttgtgc tttctttctt        871

caaaaaaaaa aa                                                          883

<210>  2
<211>  196
<212>  PRT
<213>  Arabidopsis thaliana

<400>  2

Met Gly Arg Arg Lys Ile Glu Ile Lys Arg Ile Glu Asn Lys Ser Ser
1               5                   10                  15

Arg Gln Val Thr Phe Ser Lys Arg Arg Asn Gly Leu Ile Asp Lys Ala
            20                  25                  30

Arg Gln Leu Ser Ile Leu Cys Glu Ser Ser Val Ala Val Val Val Val
        35                  40                  45

Ser Ala Ser Gly Lys Leu Tyr Asp Ser Ser Ser Gly Asp Asp Ile Ser
    50                  55                  60

Lys Ile Ile Asp Arg Tyr Glu Ile Gln His Ala Asp Glu Leu Arg Ala
65                  70                  75                  80

Leu Asp Leu Glu Glu Lys Ile Gln Asn Tyr Leu Pro His Lys Glu Leu
                85                  90                  95

Leu Glu Thr Val Gln Ser Lys Leu Glu Glu Pro Asn Val Asp Asn Val
            100                 105                 110

Ser Val Asp Ser Leu Ile Ser Leu Glu Glu Gln Leu Glu Thr Ala Leu
        115                 120                 125

Ser Val Ser Arg Ala Arg Lys Ala Glu Leu Met Met Glu Tyr Ile Glu
    130                 135                 140

Ser Leu Lys Glu Lys Glu Lys Leu Leu Arg Glu Glu Asn Gln Val Leu
145                 150                 155                 160

Ala Ser Gln Met Gly Lys Asn Thr Leu Leu Ala Thr Asp Asp Glu Arg
                165                 170                 175

Gly Met Phe Pro Gly Ser Ser Ser Gly Asn Lys Ile Pro Glu Thr Leu
            180                 185                 190

Pro Leu Leu Asn
        195

<210>  3
<211>  1196
<212>  DNA
<213>  Arabidopsis thaliana

<220>
<221>  CDS
<222>  (132)..(569)
<223>  G859

<400>  3
   aaaaaggaga gagagagaga gagagagaga gagagagaga gaaacgaaga aaaaaaaaga      60
   agcaaaaaac attgtgggtc tccggtgatt aggatcaaat tagggcacca gccttatcgg     120
   aggaagaagc c atg ggt aga aaa aaa gtc gag atc aag cga atc gag aac     170
                Met Gly Arg Lys Lys Val Glu Ile Lys Arg Ile Glu Asn
                1               5                   10
   aaa agt agt cga caa gtc act ttc tcc aaa cga cgc aat ggt ctc atc     218
   Lys Ser Ser Arg Gln Val Thr Phe Ser Lys Arg Arg Asn Gly Leu Ile
       15                  20                  25
   gag aaa gct cga caa ctt tca att ctc tgt gaa tct tcc atc gct gtt      266
   Glu Lys Ala Arg Gln Leu Ser Ile Leu Cys Glu Ser Ser Ile Ala Val
   30                  35                  40                  45
   ctc gtc gtc tcc ggc tcc gga aaa ctc tac aag tct gcc tcc ggt gac      314
   Leu Val Val Ser Gly Ser Gly Lys Leu Tyr Lys Ser Ala Ser Gly Asp
                   50                  55                  60
   aac atg tca aag atc att gat cgt tac gaa ata cat cat gct gat gaa      362
   Asn Met Ser Lys Ile Ile Asp Arg Tyr Glu Ile His His Ala Asp Glu
               65                  70                  75
   ctt gaa gcc tta gat ctt gca gaa aaa act cgg aat tat ctg cca ctc      410
   Leu Glu Ala Leu Asp Leu Ala Glu Lys Thr Arg Asn Tyr Leu Pro Leu
           80                  85                  90
   aaa gag tta cta gaa ata gtc caa agg tta gca caa aga cac ttt tat      458
   Lys Glu Leu Leu Glu Ile Val Gln Arg Leu Ala Gln Arg His Phe Tyr
       95                  100                 105
   ctc cct ctt ctt ctg atg aaa aat act ttt ttt ttt ctt ttc ttt tgg      506
   Leu Pro Leu Leu Leu Met Lys Asn Thr Phe Phe Phe Leu Phe Phe Trp
   110                 115                 120                 125
   cga att atg aat aca gca agc ttg aag aat caa atg tcg ata atg caa      554
   Arg Ile Met Asn Thr Ala Ser Leu Lys Asn Gln Met Ser Ile Met Gln
                   130                 135                 140
   gtg tgg ata ctt taa tttctctgga ggaacagctc gagactgctc tgtccgtaac      609
   Val Trp Ile Leu
               145
   tagagctagg aagacagaac taatgatggg ggaagtgaag tcccttcaaa aaacgcatgt      669
   caaagatcat tgatcgttat gaaatacatc atgctgatga acttaaagcc ttagatcttg      729
   cagaaaaaat tcggaattat cttccacaca aggagttact agaaatagtc caaagattct      789
   ctaatatcta tggaggaaca gctcgagact gctctgtcag taattagagc taagaagaca      849
   gaactaatga tggaggatat gaagtcactt caagaaaggg agaagttgct gatagaagag      909
   aaccagattc tggctagcca ggtggggaag aagacgtttc tggttataga aggtgacaga      969
   ggaatgtcat gggaaaatgg ctccggcaac aaagtacggg agactcttcc gctgctcaag     1029
   taatcaccat catcaacggc tgagctttca ccttaaactt acagcctgat tcagaagttt     1089
   ttacaaattt gtaaattata aaaagcttca taataatctc aaccttttta tcttcctcgc     1149
   gccaatgtgg aaattaaggt aaaccaaaaa aaaaaaaaaa aaaaaaa                   1196

<210>  4
<211>  145
<212>  PRT
<213>  Arabidopsis thaliana

<400>  4

Met Gly Arg Lys Lys Val Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Arg Leu Ala Gln Arg His Phe Tyr Leu Pro Leu
            100                 105                 110

Leu Leu Met Lys Asn Thr Phe Phe Phe Leu Phe Phe Trp Arg Ile Met
        115                 120                 125

Asn Thr Ala Ser Leu Lys Asn Gln Met Ser Ile Met Gln Val Trp Ile
    130                 135                 140

Leu
145

<210>  5
<211>  972
<212>  DNA
<213>  Arabidopsis thaliana

<220>
<221>  CDS
<222>  (132)..(569)
<223>  G859.1

<400>  5
aaaaaggaga gagagagaga gagagagaga gagagagaga gaaacgaaga aaaaaaaaga      60

agcaaaaaac attgtgggtc tccggtgatt aggatcaaat tagggcacca gccttatcgg     120

aggaagaagc c atg ggt aga aaa aaa gtc gag atc aag cga atc gag aac      170
             Met Gly Arg Lys Lys Val Glu Ile Lys Arg Ile Glu Asn
             1               5                   10

aaa agt agt cga caa gtc act ttc tcc aaa cga cgc aat ggt ctc atc      218
Lys Ser Ser Arg Gln Val Thr Phe Ser Lys Arg Arg Asn Gly Leu Ile
    15                  20                  25

gag aaa gct cga caa ctt tca att ctc tgt gaa tct tcc atc gct gtt 266
Glu Lys Ala Arg Gln Leu Ser Ile Leu Cys Glu Ser Ser Ile Ala Val
30                  35                  40                  45

ctc gtc gtc tcc ggc tcc gga aaa ctc tac aag tct gcc tcc ggt gac 314
Leu Val Val Ser Gly Ser Gly Lys Leu Tyr Lys Ser Ala Ser Gly Asp
                50                  55                  60

aac atg tca aag atc att gat cgt tac gaa ata cat cat gct gat gaa 362
Asn Met Ser Lys Ile Ile Asp Arg Tyr Glu Ile His His Ala Asp Glu
            65                  70                  75

ctt gaa gcc tta gat ctt gca gaa aaa act cgg aat tat ctg cca ctc 410
Leu Glu Ala Leu Asp Leu Ala Glu Lys Thr Arg Asn Tyr Leu Pro Leu
        80                  85                  90

aaa gag tta cta gaa ata gtc caa agg tta gca caa aga cac ttt tat 458
Lys Glu Leu Leu Glu Ile Val Gln Arg Leu Ala Gln Arg His Phe Tyr
    95                  100                 105

ctc cct ctt ctt ctg atg aaa aat act ttt ttt ttt ctt ttc ttt tgg 506
Leu Pro Leu Leu Leu Met Lys Asn Thr Phe Phe Phe Leu Phe Phe Trp
110                 115                 120                 125

cga att atg aat aca gca agc ttg aag aat caa atg tcg ata atg caa 554
Arg Ile Met Asn Thr Ala Ser Leu Lys Asn Gln Met Ser Ile Met Gln
                130                 135                 140

gtg tgg ata ctt taa tttctctgga ggaacagctc gagactgctc tgtccgtaac 609
Val Trp Ile Leu
            145

tagagctagg aagacagaac taatgatggg ggaagtgaag tcccttcaaa aaacggagaa 669

cttgctgaga gaagagaacc agactttggc tagccaggtg gggaagaaga cgtttctggt 729

tatagaaggt gacagaggaa tgtcatggga aaatggctcc ggcaacaaag tacgggagac 789

tcttccgctg ctcaagtaat caccatcatc aacggctgag ctttcacctt aaacttacag 849

cctgattcag aagtttttac aaatttgtaa attataaaaa gcttcataat aatctcaacc 909

tttttatctt cctcgcgcca atgtggaaat taaggtaaac caaaaaaaaa aaaaaaaaaa 969

aaa 972

<210> 6
<211> 145
<212> PRT
<213> Arabidopsis thaliana

<400> 6

Met Gly Arg Lys Lys Val Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Arg Leu Ala Gln Arg His Phe Tyr Leu Pro Leu
            100                 105                 110

Leu Leu Met Lys Asn Thr Phe Phe Phe Leu Phe Phe Trp Arg Ile Met
        115                 120                 125

Asn Thr Ala Ser Leu Lys Asn Gln Met Ser Ile Met Gln Val Trp Ile
    130                 135                 140

Leu
145

<210>  7
<211>  1036
<212>  DNA
<213>  Arabidopsis thaliana

<220>
<221>  CDS
<222>  (162)..(752)
<223>  G859.2

<400>  7
gatttgtcat tttttgtcta gccaaaaaaa aaaaaaaaaa aggagagaga gagagagaga      60

gagagagaga gaaacgaaga aaaaaaaaga agcaaaaaac attgtgggtc tccggtgatt     120

aggatcaaat tagggcacca gccttatcgg aggaagaagc c atg ggt aga aaa aaa     176
                                              Met Gly Arg Lys Lys
                                              1               5

gtc gag atc aag cga atc gag aac aaa agt agt cga caa gtc act ttc       224
Val Glu Ile Lys Arg Ile Glu Asn Lys Ser Ser Arg Gln Val Thr Phe
                10                  15                  20

tcc aaa cga cgc aat ggt ctc atc gag aaa gct cga caa ctt tca att       272
Ser Lys Arg Arg Asn Gly Leu Ile Glu Lys Ala Arg Gln Leu Ser Ile
            25                  30                  35

ctc tgt gaa tct tcc atc gct gtt ctc gtc gtc tcc ggc tcc gga aaa       320
Leu Cys Glu Ser Ser Ile Ala Val Leu Val Val Ser Gly Ser Gly Lys
        40                  45                  50

ctc tac aag tct gcc tcc ggt gac aac atg tca aag atc att gat cgt     368
Leu Tyr Lys Ser Ala Ser Gly Asp Asn Met Ser Lys Ile Ile Asp Arg
    55                  60                  65

tac gaa ata cat cat gct gat gaa ctt gaa gcc tta gat ctt gca gaa     416
Tyr Glu Ile His His Ala Asp Glu Leu Glu Ala Leu Asp Leu Ala Glu
70                  75                  80                  85

aaa act cgg aat tat ctg cca ctc aaa gag tta cta gaa ata gtc caa     464
Lys Thr Arg Asn Tyr Leu Pro Leu Lys Glu Leu Leu Glu Ile Val Gln
                90                  95                  100

agc aag ctt gaa gaa tca aat gtc gat aat gca agt gtg gat act tta     512
Ser Lys Leu Glu Glu Ser Asn Val Asp Asn Ala Ser Val Asp Thr Leu
            105                 110                 115

att tct ctg gag gaa cag ctc gag act gct ctg tcc gta act aga gct     560
Ile Ser Leu Glu Glu Gln Leu Glu Thr Ala Leu Ser Val Thr Arg Ala
        120                 125                 130

agg aag aca gaa cta atg atg ggg gaa gtg aag tcc ctt caa aaa acg     608
Arg Lys Thr Glu Leu Met Met Gly Glu Val Lys Ser Leu Gln Lys Thr
    135                 140                 145

gag aac ttg ctg aga gaa gag aac cag act ttg gct agc cag gtg ggg     656
Glu Asn Leu Leu Arg Glu Glu Asn Gln Thr Leu Ala Ser Gln Val Gly
150                 155                 160                 165

aag aag acg ttt ctg gtt ata gaa ggt gac aga gga atg tca tgg gaa     704
Lys Lys Thr Phe Leu Val Ile Glu Gly Asp Arg Gly Met Ser Trp Glu
                170                 175                 180

aat ggc tcc ggc aac aaa gta cgg gag act ctt ccg ctg ctc aag taa     752
Asn Gly Ser Gly Asn Lys Val Arg Glu Thr Leu Pro Leu Leu Lys
            185                 190                 195

tcaccatcat caacggctga gctttcacct taaacttaca gcctgattca gaagttttta     812

caaatttgta aattataaaa agcttcataa taatctcaac ctttttatct tcctcgcgcc     872

aatgtggaaa ttaaggttaa aaataaaata aaacagaagc tcatgcgaaa gaattgtaaa     932

actaagataa agctatagta gatctttatt gtaccttcgt agacgatata agatttattc     992

gtgtgtttgt cttcccctcn aaaaaaaaaa aaaaaaaaaa aaaa                    1036


<210>  8
<211>  196
<212>  PRT
<213>  Arabidopsis thaliana

<400>  8

Met Gly Arg Lys Lys Val Glu Ile Lys Arg Ile Glu Asn Lys Ser Ser
1               5                   10                  15


Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Ser Lys Leu Glu Glu Ser Asn Val Asp Asn Ala
            100                 105                 110

Ser Val Asp Thr Leu Ile Ser Leu Glu Glu Gln Leu Glu Thr Ala Leu
        115                 120                 125

Ser Val Thr Arg Ala Arg Lys Thr Glu Leu Met Met Gly Glu Val Lys
    130                 135                 140

Ser Leu Gln Lys Thr Glu Asn Leu Leu Arg Glu Glu Asn Gln Thr Leu
145                 150                 155                 160

Ala Ser Gln Val Gly Lys Lys Thr Phe Leu Val Ile Glu Gly Asp Arg
                165                 170                 175

Gly Met Ser Trp Glu Asn Gly Ser Gly Asn Lys Val Arg Glu Thr Leu
            180                 185                 190

Pro Leu Leu Lys
        195

<210> 9
<211> 1059
<212> DNA
<213> Arabidopsis thaliana

<220>
<221> CDS
<222> (219)..(809)
<223> G1842

<400> 9
actattacat gcctcttcct cgcttcaaaa cggcaccgtt tccacttgtt attatttttc 60

tctctatcgt ctaacaaaaa aaaaaactga cttgggattt tttttcattt gtctagccca 120

aaagaagaag atagaaacga agaaaaaaag caaacacatt ttgggtcccc ggtggttagg 180

atcaaattag ggcacaaacc ttatcggaga aagaagcc atg gga aga aga aaa gtc 236

Met Gly Arg Arg Lys Val
1               5

gag atc aag cga atc gag aac aaa agc agt cga caa gtc act ttc tcc     284
Glu Ile Lys Arg Ile Glu Asn Lys Ser Ser Arg Gln Val Thr Phe Ser
            10                  15                  20

aaa cga cgc aaa ggt ctc atc gaa aaa gct cga caa ctt tca att ctc     332
Lys Arg Arg Lys Gly Leu Ile Glu Lys Ala Arg Gln Leu Ser Ile Leu
        25                  30                  35

tgt gaa tct tcc atc gct gtt gtc gcc gtc tcc ggt tcc gga aaa ctc     380
Cys Glu Ser Ser Ile Ala Val Val Ala Val Ser Gly Ser Gly Lys Leu
    40                  45                  50

tac gac tct gcc tcc ggt gac aac atg tca aag atc att gat cgt tat     428
Tyr Asp Ser Ala Ser Gly Asp Asn Met Ser Lys Ile Ile Asp Arg Tyr
55                  60                  65                  70

gaa ata cat cat gct gat gaa ctt aaa gcc tta gat ctt gca gaa aaa     476
Glu Ile His His Ala Asp Glu Leu Lys Ala Leu Asp Leu Ala Glu Lys
                75                  80                  85

att cgg aat tat ctt cca cac aag gag tta cta gaa ata gtc caa agc     524
Ile Arg Asn Tyr Leu Pro His Lys Glu Leu Leu Glu Ile Val Gln Ser
            90                  95                  100

aag ctt gaa gaa tca aat gtc gat aat gta agt gta gat tct cta ata     572
Lys Leu Glu Glu Ser Asn Val Asp Asn Val Ser Val Asp Ser Leu Ile
        105                 110                 115

tct atg gag gaa cag ctc gag act gct ctg tca gta att aga gct aag     620
Ser Met Glu Glu Gln Leu Glu Thr Ala Leu Ser Val Ile Arg Ala Lys
    120                 125                 130

aag aca gaa cta atg atg gag gat atg aag tca ctt caa gaa agg gag     668
Lys Thr Glu Leu Met Met Glu Asp Met Lys Ser Leu Gln Glu Arg Glu
135                 140                 145                 150

aag ttg ctg ata gaa gag aac cag att ctg gct agc cag gtg ggg aag     716
Lys Leu Leu Ile Glu Glu Asn Gln Ile Leu Ala Ser Gln Val Gly Lys
                155                 160                 165

aag acg ttt ctg gtt ata gaa ggt gac aga gga atg tca cgg gaa aat     764
Lys Thr Phe Leu Val Ile Glu Gly Asp Arg Gly Met Ser Arg Glu Asn
            170                 175                 180

ggc tcc ggc aac aaa gta ccg gag act ctt tcg ctg ctc aag taa         809
Gly Ser Gly Asn Lys Val Pro Glu Thr Leu Ser Leu Leu Lys
        185                 190                 195

tcaccatcat caacggctga gctttcacca taaacttact cacagcctga ttcagaagct     869

tttacaaaat tgtaaattat aaaaagctgc ataataatct caaccttttt atcttcctcg     929

cgccaatgtg gaaataaagg taaaacaaaa cgaagctctt ttcttttatg cgaaagaatt     989

gtaaaactaa gataaagcta ccgatctttg ttgtacctta gtagacaaat atcagagttc    1049

ttgtgcttgt                                                           1059

<210>  10

<211>  196
<212>  PRT
<213>  Arabidopsis thaliana

<400>  10

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Ser Lys Leu Glu Glu Ser Asn Val Asp Asn Val
            100                 105                 110

Ser Val Asp Ser Leu Ile Ser Met Glu Glu Gln Leu Glu Thr Ala Leu
        115                 120                 125

Ser Val Ile Arg Ala Lys Lys Thr Glu Leu Met Met Glu Asp Met Lys
    130                 135                 140

Ser Leu Gln Glu Arg Glu Lys Leu Leu Ile Glu Glu Asn Gln Ile Leu
145                 150                 155                 160

Ala Ser Gln Val Gly Lys Lys Thr Phe Leu Val Ile Glu Gly Asp Arg
                165                 170                 175

Gly Met Ser Arg Glu Asn Gly Ser Gly Asn Lys Val Pro Glu Thr Leu
            180                 185                 190

Ser Leu Leu Lys
        195

<210>  11
<211>  880
<212>  DNA
<213>  Arabidopsis thaliana

<220>
<221> CDS
<222> (79)..(636)
<223> G1842.2

<400> 11
agaaaaaaag caaacacatt ttgggtcccc ggtggttagg atcaaattag ggcacaaacc 60

ttatcggaga aagaagcc atg gga aga aga aaa gtc gag atc aag cga atc 111
Met Gly Arg Arg Lys Val Glu Ile Lys Arg Ile
1               5                   10

gag aac aaa agc agt cga caa gtc act ttc tcc aaa cga cgc aaa ggt 159
Glu Asn Lys Ser Ser Arg Gln Val Thr Phe Ser Lys Arg Arg Lys Gly
            15                  20                  25

ctc atc gaa aaa gct cga caa ctt tca att ctc tgt gaa tct tcc atc 207
Leu Ile Glu Lys Ala Arg Gln Leu Ser Ile Leu Cys Glu Ser Ser Ile
        30                  35                  40

gct gtt gtc gcc gtc tcc ggt tcc gga aaa ctc tac gac tct gcc tcc 255
Ala Val Val Ala Val Ser Gly Ser Gly Lys Leu Tyr Asp Ser Ala Ser
    45                  50                  55

ggt gac aac atg tca aag atc att gat cgt tat gaa ata cat cat gct 303
Gly Asp Asn Met Ser Lys Ile Ile Asp Arg Tyr Glu Ile His His Ala
60                  65                  70                  75

gat gaa ctt aaa gcc tta gat ctt gca gaa aaa att cgg aat tat ctt 351
Asp Glu Leu Lys Ala Leu Asp Leu Ala Glu Lys Ile Arg Asn Tyr Leu
                80                  85                  90

cca cac aag gag tta cta gaa ata gtc caa agt gta gat tct cta ata 399
Pro His Lys Glu Leu Leu Glu Ile Val Gln Ser Val Asp Ser Leu Ile
            95                  100                 105

tct atg gag gaa cag ctc gag act gct ctg tca gta att aga gct aag 447
Ser Met Glu Glu Gln Leu Glu Thr Ala Leu Ser Val Ile Arg Ala Lys
        110                 115                 120

aag aca gaa cta atg atg gag gat atg aag tca ctt caa gaa agg gag 495
Lys Thr Glu Leu Met Met Glu Asp Met Lys Ser Leu Gln Glu Arg Glu
    125                 130                 135

aag ttg ctg ata gaa gag aac cag att ctg gct agc cag gtg ggg aag 543
Lys Leu Leu Ile Glu Glu Asn Gln Ile Leu Ala Ser Gln Val Gly Lys
140                 145                 150                 155

aag acg ttt ctg gtt ata gaa ggt gac aga gga atg tca cgg gaa aat 591
Lys Thr Phe Leu Val Ile Glu Gly Asp Arg Gly Met Ser Arg Glu Asn
                160                 165                 170

ggc tcc ggc aac aaa gta ccg gag act ctt tcg ctg ctc aag taa 636
Gly Ser Gly Asn Lys Val Pro Glu Thr Leu Ser Leu Leu Lys
            175                 180                 185

tcaccatcat caacggctga gctttcacca taaacttact cacagcctga ttcagaagct 696

tttacaaaat tgtaaattat aaaaagctgc ataataatct caaccttttt atcttcctcg 756

cgccaatgtg gaaataaagg taaaacaaaa cgaagctctt ttcttttatg cgaaagaatt 816

gtaaaactaa gataaagcta ccgatctttg ttgtacctta gtagacaaat atcagagttc     876

ttgt                                                                  880

<210>  12
<211>  185
<212>  PRT
<213>  Arabidopsis thaliana

<400>  12

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Ser Val Asp Ser Leu Ile Ser Met Glu Glu Gln
            100                 105                 110

Leu Glu Thr Ala Leu Ser Val Ile Arg Ala Lys Lys Thr Glu Leu Met
        115                 120                 125

Met Glu Asp Met Lys Ser Leu Gln Glu Arg Glu Lys Leu Leu Ile Glu
    130                 135                 140

Glu Asn Gln Ile Leu Ala Ser Gln Val Gly Lys Lys Thr Phe Leu Val
145                 150                 155                 160

Ile Glu Gly Asp Arg Gly Met Ser Arg Glu Asn Gly Ser Gly Asn Lys
                165                 170                 175

Val Pro Glu Thr Leu Ser Leu Leu Lys
            180                 185

<210>  13
<211>  978
<212>  DNA

<213>  Arabidopsis thaliana

<220>
<221>  CDS
<222>  (219)..(452)
<223>  G1842.6


<400>  13
actattacat gcctcttcct cgcttcaaaa cggcaccgtt tccacttgtt attatttttc      60

tctctatcgt ctaacaaaaa aaaaaactga cttgggattt tttttcattt gtctagccca     120

aaagaagaag atagaaacga agaaaaaaag caaacacatt ttgggtcccc ggtggttagg     180

atcaaattag ggcacaaacc ttatcggaga aagaagcc atg gga aga aga aaa gtc     236
                                          Met Gly Arg Arg Lys Val
                                          1               5

gag atc aag cga atc gag aac aaa agc agt cga caa gtc act ttc tcc       284
Glu Ile Lys Arg Ile Glu Asn Lys Ser Ser Arg Gln Val Thr Phe Ser
            10                  15                  20

aaa cga cgc aaa ggt ctc atc gaa aaa gct cga caa ctt tca att ctc       332
Lys Arg Arg Lys Gly Leu Ile Glu Lys Ala Arg Gln Leu Ser Ile Leu
        25                  30                  35

tgt gaa tct tcc atc gct gtt gtc gcc gtc tcc ggt tcc gga aaa ctc       380
Cys Glu Ser Ser Ile Ala Val Val Ala Val Ser Gly Ser Gly Lys Leu
    40                  45                  50

tac gac tct gcc tcc ggt gac aag atc ttg cag aaa aaa ttc gga att       428
Tyr Asp Ser Ala Ser Gly Asp Lys Ile Leu Gln Lys Lys Phe Gly Ile
55                  60                  65                  70

atc ttc cac aca agg agt tac tag aaatagtcca aagattctct aatatctatg      482
Ile Phe His Thr Arg Ser Tyr
                75

gaggaacagc tcgagactgc tctgtcagta attagagcta agaagacaga actaatgatg     542

gaggatatga agtcacttca agaaagggag aagttgctga tagaagagaa ccagattctg     602

gctagccagg tggggaagaa gacgtttctg gttatagaag gtgacagagg aatgtcacgg     662

gaaaatggct ccggcaacaa agtaccggag actctttcgc tgctcaagta atcaccatca     722

tcaacggctg agctttcacc ataaacttac tcacagcctg attcagaagc ttttacaaaa     782

ttgtaaatta taaaaagctg cataataatc tcaacctttt tatcttcctc gcgccaatgt     842

ggaaataaag gtaaaacaaa acgaagctct tttcttttat gcgaaagaat tgtaaaacta     902

agataaagct accgatcttt gttgtacctt agtagacaaa tatcagagtt cttgtgcttg     962

aaaaaaaaaa aaaaaa                                                     978


<210>  14
<211>  77
<212>  PRT
<213>  Arabidopsis thaliana

<400> 14

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Lys Ile Leu
    50                  55                  60

Gln Lys Lys Phe Gly Ile Ile Phe His Thr Arg Ser Tyr
65                  70                  75

<210> 15
<211> 876
<212> DNA
<213> Arabidopsis thaliana

<220>
<221> CDS
<222> (80)..(436)
<223> G1842.7

<400> 15
cagaaaaaaa gcaaacacat tttgggtccc cggtggttag gatcaaatta gggcacaaac      60

cttatcggag aaagaagcc atg gga aga aga aaa gtc gag atc aag cga atc      112
                     Met Gly Arg Arg Lys Val Glu Ile Lys Arg Ile
                     1               5                   10

gag aac aaa agc agt cga caa gtc act ttc tcc aaa cga cgc aaa ggt      160
Glu Asn Lys Ser Ser Arg Gln Val Thr Phe Ser Lys Arg Arg Lys Gly
            15                  20                  25

ctc atc gaa aaa gct cga caa ctt tca att ctc tgt gaa tct tcc atc      208
Leu Ile Glu Lys Ala Arg Gln Leu Ser Ile Leu Cys Glu Ser Ser Ile
        30                  35                  40

gct gtt gtc gcc gtc tcc ggt tcc gga aaa ctc tac gac tct gcc tcc      256
Ala Val Val Ala Val Ser Gly Ser Gly Lys Leu Tyr Asp Ser Ala Ser
    45                  50                  55

ggt gac aac atg tca aag atc att gat cgt tat gaa ata cat cat gct      304
Gly Asp Asn Met Ser Lys Ile Ile Asp Arg Tyr Glu Ile His His Ala
60                  65                  70                  75

gat gaa ctt aaa gcc tta gat ctt gca gaa aaa att cgg aat tat ctt      352
Asp Glu Leu Lys Ala Leu Asp Leu Ala Glu Lys Ile Arg Asn Tyr Leu
                80                  85                  90

cca cac aag gag tta cta gaa ata gtc caa aga ttc tct aat atc tat      400
Pro His Lys Glu Leu Leu Glu Ile Val Gln Arg Phe Ser Asn Ile Tyr
            95                  100                 105

gga gga aca gct cga gac tgc tct gtc agt aat tag agctaagaag 446
Gly Gly Thr Ala Arg Asp Cys Ser Val Ser Asn
        110                 115

acagaactaa tgatggagga tatgaagtca cttcaagaaa gggagaagtt gctgatagaa 506

gagaaccaga ttctggctag ccaggtgggg aagaagacgt ttctggttat agaaggtgac 566

agaggaatgt cacgggaaaa tggctccggc aacaaagtac cggagactct ttcgctgctc 626

aagtaatcac catcatcaac ggctgagctt tcaccataaa cttactcaca gcctgattca 686

gaagctttta caaaattgta aattataaaa agctgcataa taatctcaac ctttttatct 746

tcctcgcgcc aatgtggaaa taaaggtaaa acaaaacgaa gctcttttct tttatgcgaa 806

agaattgtaa aactaagata aagctaccga tctttgttgt accttagtag acaaatatca 866

gagttcttgt 876

<210> 16
<211> 118
<212> PRT
<213> Arabidopsis thaliana

<400> 16

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Arg Phe Ser Asn Ile Tyr Gly Gly Thr Ala Arg
            100                 105                 110

Asp Cys Ser Val Ser Asn
        115

<210> 17
<211> 818

<212>  DNA
<213>  Arabidopsis thaliana

<220>
<221>  CDS
<222>  (51)..(653)
<223>  G1843

<400>  17
cagacatcac aatcaaatta ggtcagaaga attagtcgga gaaaacagcc atg gga      56
                                                       Met Gly
                                                       1

aga aga aaa gta gag atc aaa cga att gag aac aaa agc tct cga caa     104
Arg Arg Lys Val Glu Ile Lys Arg Ile Glu Asn Lys Ser Ser Arg Gln
        5                   10                  15

gtt act ttc tgt aaa cga cga aat ggt ctc atg gag aaa gct cgt caa     152
Val Thr Phe Cys Lys Arg Arg Asn Gly Leu Met Glu Lys Ala Arg Gln
    20                  25                  30

ctc tca att ctt tgt gaa tcc tcc gtc gct ctt atc atc atc tct gcc     200
Leu Ser Ile Leu Cys Glu Ser Ser Val Ala Leu Ile Ile Ile Ser Ala
35                  40                  45                  50

acc gga aga ctc tac agc ttc tcc tca ggt gat agc atg gcc aag atc     248
Thr Gly Arg Leu Tyr Ser Phe Ser Ser Gly Asp Ser Met Ala Lys Ile
                55                  60                  65

ctc agt cgt tat gaa tta gaa cag gct gat gat ctt aaa acc ttg gat     296
Leu Ser Arg Tyr Glu Leu Glu Gln Ala Asp Asp Leu Lys Thr Leu Asp
            70                  75                  80

cta gaa gaa aaa act ctt aat tat ctt tcg cac aag gag ttg cta gaa     344
Leu Glu Glu Lys Thr Leu Asn Tyr Leu Ser His Lys Glu Leu Leu Glu
        85                  90                  95

aca atc caa tgc aag att gaa gaa gcg aaa agc gat aat gta agt ata     392
Thr Ile Gln Cys Lys Ile Glu Glu Ala Lys Ser Asp Asn Val Ser Ile
    100                 105                 110

gat tgt cta aag tcc ctg gaa gag cag ctc aag act gct ctg tct gta     440
Asp Cys Leu Lys Ser Leu Glu Glu Gln Leu Lys Thr Ala Leu Ser Val
115                 120                 125                 130

act aga gct agg aag aca gaa cta atg atg gag ctt gtg aag acc cat     488
Thr Arg Ala Arg Lys Thr Glu Leu Met Met Glu Leu Val Lys Thr His
                135                 140                 145

caa gag aag gag aag ctg ctg aga gag gag aac cag agt ttg act aac     536
Gln Glu Lys Glu Lys Leu Leu Arg Glu Glu Asn Gln Ser Leu Thr Asn
            150                 155                 160

cag ctt ata aag atg ggg aag atg aag aag tct gtg gaa gca gag gat     584
Gln Leu Ile Lys Met Gly Lys Met Lys Lys Ser Val Glu Ala Glu Asp
        165                 170                 175

gca aga gca atg tca ccg gaa agt agc tct gac aac aag cca ccg gag     632
Ala Arg Ala Met Ser Pro Glu Ser Ser Ser Asp Asn Lys Pro Pro Glu
    180                 185                 190

act ctc ctg ctt ctc aag taa ccaccatcac caacgactga ttcgaaaaat        683

Thr Leu Leu Leu Leu Lys
195                 200

aaaaattgta aaaattatga tttgtagttc ataaggaaag ctacatactg tatgttaaaa     743

atcctcttct tccccctgct acggaaaagt catccaagga gatgcatcaa ataaagtaat     803

tgatttttat tgtta                                                      818

<210>  18
<211>  200
<212>  PRT
<213>  Arabidopsis thaliana

<400>  18

Met Gly Arg Arg Lys Val Glu Ile Lys Arg Ile Glu Asn Lys Ser Ser
1               5                   10                  15

Arg Gln Val Thr Phe Cys Lys Arg Arg Asn Gly Leu Met Glu Lys Ala
            20                  25                  30

Arg Gln Leu Ser Ile Leu Cys Glu Ser Ser Val Ala Leu Ile Ile Ile
        35                  40                  45

Ser Ala Thr Gly Arg Leu Tyr Ser Phe Ser Ser Gly Asp Ser Met Ala
    50                  55                  60

Lys Ile Leu Ser Arg Tyr Glu Leu Glu Gln Ala Asp Asp Leu Lys Thr
65                  70                  75                  80

Leu Asp Leu Glu Glu Lys Thr Leu Asn Tyr Leu Ser His Lys Glu Leu
                85                  90                  95

Leu Glu Thr Ile Gln Cys Lys Ile Glu Glu Ala Lys Ser Asp Asn Val
            100                 105                 110

Ser Ile Asp Cys Leu Lys Ser Leu Glu Glu Gln Leu Lys Thr Ala Leu
        115                 120                 125

Ser Val Thr Arg Ala Arg Lys Thr Glu Leu Met Met Glu Leu Val Lys
    130                 135                 140

Thr His Gln Glu Lys Glu Lys Leu Leu Arg Glu Glu Asn Gln Ser Leu
145                 150                 155                 160

Thr Asn Gln Leu Ile Lys Met Gly Lys Met Lys Lys Ser Val Glu Ala
                165                 170                 175

Glu Asp Ala Arg Ala Met Ser Pro Glu Ser Ser Ser Asp Asn Lys Pro
            180                 185                 190

Pro Glu Thr Leu Leu Leu Leu Lys
        195                 200

<210>  19
<211>  834
<212>  DNA
<213>  Arabidopsis thaliana

<220>
<221>  CDS
<222>  (39)..(635)
<223>  G1844

<400>  19
agaaattagg ggattagatg tgtcggaaga gtgaagcc atg gga aga aga aga gta      56
                                          Met Gly Arg Arg Arg Val
                                          1               5

gag atc aaa cga att gag aac aaa agc agt aga caa gtc act ttc tgt      104
Glu Ile Lys Arg Ile Glu Asn Lys Ser Ser Arg Gln Val Thr Phe Cys
            10                  15                  20

aag aga cga aat ggt ctc atg gag aaa gct cgt caa ctc tca att ctc      152
Lys Arg Arg Asn Gly Leu Met Glu Lys Ala Arg Gln Leu Ser Ile Leu
        25                  30                  35

tgt gga tcc tcc gtc gct ctt ttc atc gtc tct tcc acc ggc aaa ctc      200
Cys Gly Ser Ser Val Ala Leu Phe Ile Val Ser Ser Thr Gly Lys Leu
    40                  45                  50

tac aac tcc tcc tcc ggc gac agc atg gcc aag atc atc agt cgt ttt      248
Tyr Asn Ser Ser Ser Gly Asp Ser Met Ala Lys Ile Ile Ser Arg Phe
55                  60                  65                  70

aaa ata caa caa gct gat gat cct gaa acc ttg gat ctt gaa gac aaa      296
Lys Ile Gln Gln Ala Asp Asp Pro Glu Thr Leu Asp Leu Glu Asp Lys
                75                  80                  85

act cag gat tat ctt tca cac aag gag tta cta gaa ata gtt caa aga      344
Thr Gln Asp Tyr Leu Ser His Lys Glu Leu Leu Glu Ile Val Gln Arg
            90                  95                  100

aag att gaa gaa gca aaa ggg gat aat gta agt ata gaa tct cta att      392
Lys Ile Glu Glu Ala Lys Gly Asp Asn Val Ser Ile Glu Ser Leu Ile
        105                 110                 115

tcc atg gaa gag cag ctc aag agt gct ctg tct gta att aga gct agg      440
Ser Met Glu Glu Gln Leu Lys Ser Ala Leu Ser Val Ile Arg Ala Arg
    120                 125                 130

aag aca gag tta ttg atg gag ctt gtg aag aac ctt cag gat aag gag      488
Lys Thr Glu Leu Leu Met Glu Leu Val Lys Asn Leu Gln Asp Lys Glu
135                 140                 145                 150

aag ttg ctg aaa gaa aag aac aag gtt cta gct agc gag gtg ggg aag      536
Lys Leu Leu Lys Glu Lys Asn Lys Val Leu Ala Ser Glu Val Gly Lys
                155                 160                 165

ctg aag aaa att ttg gaa aca ggg gat gaa aga gca gta atg tca ccg      584

Leu Lys Lys Ile Leu Glu Thr Gly Asp Glu Arg Ala Val Met Ser Pro
            170                 175                 180

gaa aat agc tct ggc cac agc cca ccg gag act ctc ccg ctt ctc aag        632
Glu Asn Ser Ser Gly His Ser Pro Pro Glu Thr Leu Pro Leu Leu Lys
        185                 190                 195

taa ccaccaatca tcaacggctg atttttcatc atcctgattc aaaaaaggta        685

aaaaaaattc atgtgtaaaa atcataaaga agctacatgt tttaaaatcc tcttctcccc        745

ctgcatacgg ataaatttat agaccaaaaa tataatgttt tccctcaaat aagatatcga        805

cctttgtgtt accttggaag acaggatca                                        834

<210>  20
<211>  198
<212>  PRT
<213>  Arabidopsis thaliana

<400>  20

Met Gly Arg Arg Arg Val Glu Ile Lys Arg Ile Glu Asn Lys Ser Ser
1               5                   10                  15

Arg Gln Val Thr Phe Cys Lys Arg Arg Asn Gly Leu Met Glu Lys Ala
            20                  25                  30

Arg Gln Leu Ser Ile Leu Cys Gly Ser Ser Val Ala Leu Phe Ile Val
        35                  40                  45

Ser Ser Thr Gly Lys Leu Tyr Asn Ser Ser Ser Gly Asp Ser Met Ala
    50                  55                  60

Lys Ile Ile Ser Arg Phe Lys Ile Gln Gln Ala Asp Asp Pro Glu Thr
65                  70                  75                  80

Leu Asp Leu Glu Asp Lys Thr Gln Asp Tyr Leu Ser His Lys Glu Leu
                85                  90                  95

Leu Glu Ile Val Gln Arg Lys Ile Glu Glu Ala Lys Gly Asp Asn Val
            100                 105                 110

Ser Ile Glu Ser Leu Ile Ser Met Glu Glu Gln Leu Lys Ser Ala Leu
        115                 120                 125

Ser Val Ile Arg Ala Arg Lys Thr Glu Leu Leu Met Glu Leu Val Lys
    130                 135                 140

Asn Leu Gln Asp Lys Glu Lys Leu Leu Lys Glu Lys Asn Lys Val Leu
145                 150                 155                 160

Ala Ser Glu Val Gly Lys Leu Lys Lys Ile Leu Glu Thr Gly Asp Glu
                165                 170                 175

Arg Ala Val Met Ser Pro Glu Asn Ser Ser Gly His Ser Pro Pro Glu
            180                 185                 190

Thr Leu Pro Leu Leu Lys
        195

<210>  21
<211>  753
<212>  DNA
<213>  Arabidopsis thaliana

<220>
<221>  CDS
<222>  (1)..(555)
<223>  G1844.2

<400>  21
atg gga aga aga aga gta gag atc aaa cga att gag aac aaa agc agt      48
Met Gly Arg Arg Arg Val Glu Ile Lys Arg Ile Glu Asn Lys Ser Ser
1               5                   10                  15

aga caa gtc act ttc tgt aag aga cga aat ggt ctc atg gag aaa gct      96
Arg Gln Val Thr Phe Cys Lys Arg Arg Asn Gly Leu Met Glu Lys Ala
            20                  25                  30

cgt caa ctc tca att ctc tgt gga tcc tcc gtc gct ctt ttc atc gtc     144
Arg Gln Leu Ser Ile Leu Cys Gly Ser Ser Val Ala Leu Phe Ile Val
        35                  40                  45

tct tcc acc ggc aaa ctc tac aac tcc tcc tcc ggc gac agc atg gcc     192
Ser Ser Thr Gly Lys Leu Tyr Asn Ser Ser Ser Gly Asp Ser Met Ala
    50                  55                  60

aag atc atc agt cgt ttt aaa ata caa caa gct gat gat cct gaa acc     240
Lys Ile Ile Ser Arg Phe Lys Ile Gln Gln Ala Asp Asp Pro Glu Thr
65                  70                  75                  80

ttg gat ctt gaa gac aaa act cag gat tat ctt tca cac aag gag tta     288
Leu Asp Leu Glu Asp Lys Thr Gln Asp Tyr Leu Ser His Lys Glu Leu
                85                  90                  95

cta gaa ata gtt caa aga aag att gaa gaa gca aaa ggg gat aat gta     336
Leu Glu Ile Val Gln Arg Lys Ile Glu Glu Ala Lys Gly Asp Asn Val
            100                 105                 110

agt ata gaa tct cta att tcc atg gaa gag cag ctc aag agt gct ctg     384
Ser Ile Glu Ser Leu Ile Ser Met Glu Glu Gln Leu Lys Ser Ala Leu
        115                 120                 125

tct gta att aga gct agg aag aca gag tta ttg atg gag ctt gtg aag     432
Ser Val Ile Arg Ala Arg Lys Thr Glu Leu Leu Met Glu Leu Val Lys
    130                 135                 140

aac ctt cag gat aag gtg ggg aag ctg aag aaa att ttg gaa aca ggg     480
Asn Leu Gln Asp Lys Val Gly Lys Leu Lys Lys Ile Leu Glu Thr Gly
145                 150                 155                 160

gat gaa aga gca gta atg tca ccg gaa aat agc tct ggc cac agc cca     528
Asp Glu Arg Ala Val Met Ser Pro Glu Asn Ser Ser Gly His Ser Pro
            165                 170                 175

ccg gag act ctc ccg ctt ctc aag taa ccaccaatca tcaacggctg     575
Pro Glu Thr Leu Pro Leu Leu Lys
        180

atttttcatc atcctgattc aaaaaaggta aaaaaaattc atgtgtaaaa atcataaaga     635

agctacatgt tttaaaatcc tcttctcccc ctgcatacgg ataaatttat agaccaaaaa     695

tataatgttt tccctcaaat aagatatcga cctttgtgtt accttggaag acaggatc     753

<210> 22
<211> 184
<212> PRT
<213> Arabidopsis thaliana

<400> 22
   Met Gly Arg Arg Arg Val Glu Ile Lys Arg Ile Glu Asn Lys Ser Ser
   1               5                   10                  15
   Arg Gln Val Thr Phe Cys Lys Arg Arg Asn Gly Leu Met Glu Lys Ala
               20                  25                  30
   Arg Gln Leu Ser Ile Leu Cys Gly Ser Ser Val Ala Leu Phe Ile Val
           35                  40                  45
   Ser Ser Thr Gly Lys Leu Tyr Asn Ser Ser Ser Gly Asp Ser Met Ala
       50                  55                  60
   Lys Ile Ile Ser Arg Phe Lys Ile Gln Gln Ala Asp Asp Pro Glu Thr
   65                  70                  75                  80
   Leu Asp Leu Glu Asp Lys Thr Gln Asp Tyr Leu Ser His Lys Glu Leu
                   85                  90                  95
   Leu Glu Ile Val Gln Arg Lys Ile Glu Glu Ala Lys Gly Asp Asn Val
               100                 105                 110
   Ser Ile Glu Ser Leu Ile Ser Met Glu Glu Gln Leu Lys Ser Ala Leu
           115                 120                 125
   Ser Val Ile Arg Ala Arg Lys Thr Glu Leu Leu Met Glu Leu Val Lys
       130                 135                 140
   Asn Leu Gln Asp Lys Val Gly Lys Leu Lys Lys Ile Leu Glu Thr Gly
   145                 150                 155                 160
   Asp Glu Arg Ala Val Met Ser Pro Glu Asn Ser Ser Gly His Ser Pro
                   165                 170                 175
   Pro Glu Thr Leu Pro Leu Leu Lys
               180

<210> 23
<211> 1134
<212> DNA
<213> Arabidopsis thaliana

<220>
<221> CDS
<222> (158)..(880)
<223> G861

<400> 23
   cttcttcctc ctcctccatc tcttctcttt actctctctt taatcatctc tcattcttga 60
   atcttgatcc atcaaaatca atcccgttct cgaaagatcc attaaaatca aaacctaagc 120
   tctctctctt gcttctaggg tttttttgtt cgttgtg atg gcg aga gaa aag att      175
                                            Met Ala Arg Glu Lys Ile
                                            1               5
   cag atc agg aag atc gac aac gca acg gcg aga caa gtg acg ttt tcg 223
   Gln Ile Arg Lys Ile Asp Asn Ala Thr Ala Arg Gln Val Thr Phe Ser
   10 15 20
   aaa cga aga aga ggg ctt ttc aag aaa gct gaa gaa ctc tcc gtt ctc 271
   Lys Arg Arg Arg Gly Leu Phe Lys Lys Ala Glu Glu Leu Ser Val Leu
   25 30 35
   tgc gac gcc gat gtc gct ctc atc atc ttc tct tcc acc gga aaa ctg 319
   Cys Asp Ala Asp Val Ala Leu Ile Ile Phe Ser Ser Thr Gly Lys Leu
   40 45 50
   ttc gag ttc tgt agc tcc agc atg aag gaa gtc cta gag agg cat aac 367
   Phe Glu Phe Cys Ser Ser Ser Met Lys Glu Val Leu Glu Arg His Asn
   55 60 65 70
   ttg cag tca aag aac ttg gag aag ctt gat cag cca tct ctt gag tta 415
   Leu Gln Ser Lys Asn Leu Glu Lys Leu Asp Gln Pro Ser Leu Glu Leu
   75 80 85
   cag ctg gtt gag aac agt gat cac gcc cga atg agt aaa gaa att gcg 463
   Gln Leu Val Glu Asn Ser Asp His Ala Arg Met Ser Lys Glu Ile Ala
   90 95 100
   gac aag agc cac cga cta agg caa atg aga gga gag gaa ctt caa gga 511
   Asp Lys Ser His Arg Leu Arg Gln Met Arg Gly Glu Glu Leu Gln Gly
   105 110 115
   ctt gac att gaa gag ctt cag cag cta gag aag gcc ctt gaa act ggt 559
   Leu Asp Ile Glu Glu Leu Gln Gln Leu Glu Lys Ala Leu Glu Thr Gly
   120 125 130
   ttg acg cgt gtg att gaa aca aag agt gac aag att atg agt gag atc 607
   Leu Thr Arg Val Ile Glu Thr Lys Ser Asp Lys Ile Met Ser Glu Ile
   135 140 145 150
   agc gaa ctt cag aaa aag gga atg caa ttg atg gat gag aac aag cgg     655
   Ser Glu Leu Gln Lys Lys Gly Met Gln Leu Met Asp Glu Asn Lys Arg
                   155                 160                 165
   ttg agg cag caa gga acg caa cta acg gaa gag aac gag cga ctt ggc     703
   Leu Arg Gln Gln Gly Thr Gln Leu Thr Glu Glu Asn Glu Arg Leu Gly
               170                 175                 180
   atg caa ata tgt aac aat gtg cat gca cac ggt ggt gct gaa tcg gag     751
   Met Gln Ile Cys Asn Asn Val His Ala His Gly Gly Ala Glu Ser Glu
           185                 190                 195
   aac gct gct gtg tac gag gaa gga cag tcg tcg gag tct att act aac     799
   Asn Ala Ala Val Tyr Glu Glu Gly Gln Ser Ser Glu Ser Ile Thr Asn
       200                 205                 210
   gcc gga aac tct acc gga gcg cct gtt gac tcc gag agc tcc gac act     847
   Ala Gly Asn Ser Thr Gly Ala Pro Val Asp Ser Glu Ser Ser Asp Thr
   215                 220                 225                 230
   tcc ctt agg ctc ggc tta ccg tat ggt ggt tag agatggaaca attcaaagaa   900
   Ser Leu Arg Leu Gly Leu Pro Tyr Gly Gly
                   235                 240
   gttgatggag tgaggagagt aatgtaaatc tttttaactc ggtagtaaca agagacaatg   960
   tctaagtagt gaattctcaa atgtttgtgt aagtttctgc ctatggaaga ggctttcatt  1020
   tttatgattt tcactatgta tgatctctct tcactgcatt tctggttagt aacggcttgt  1080
   caccgataaa ctttctcgtt atggaaagtt agaataaaaa aaaaaaaaaa aaaa        1134

<210>  24
<211>  240
<212>  PRT
<213>  Arabidopsis thaliana

<400>  24

Met Ala Arg Glu Lys Ile Gln Ile Arg Lys Ile Asp Asn Ala Thr Ala
1               5                   10                  15

Arg Gln Val Thr Phe Ser Lys Arg Arg Arg Gly Leu Phe Lys Lys Ala
            20                  25                  30

Glu Glu Leu Ser Val Leu Cys Asp Ala Asp Val Ala Leu Ile Ile Phe
        35                  40                  45

Ser Ser Thr Gly Lys Leu Phe Glu Phe Cys Ser Ser Ser Met Lys Glu
    50                  55                  60

Val Leu Glu Arg His Asn Leu Gln Ser Lys Asn Leu Glu Lys Leu Asp
65                  70                  75                  80

Gln Pro Ser Leu Glu Leu Gln Leu Val Glu Asn Ser Asp His Ala Arg
                85                  90                  95

Met Ser Lys Glu Ile Ala Asp Lys Ser His Arg Leu Arg Gln Met Arg
            100                 105                 110

Gly Glu Glu Leu Gln Gly Leu Asp Ile Glu Glu Leu Gln Gln Leu Glu
        115                 120                 125

Lys Ala Leu Glu Thr Gly Leu Thr Arg Val Ile Glu Thr Lys Ser Asp
    130                 135                 140

Lys Ile Met Ser Glu Ile Ser Glu Leu Gln Lys Lys Gly Met Gln Leu
145                 150                 155                 160

Met Asp Glu Asn Lys Arg Leu Arg Gln Gln Gly Thr Gln Leu Thr Glu
                165                 170                 175

Glu Asn Glu Arg Leu Gly Met Gln Ile Cys Asn Asn Val His Ala His
            180                 185                 190

Gly Gly Ala Glu Ser Glu Asn Ala Ala Val Tyr Glu Glu Gly Gln Ser
        195                 200                 205

Ser Glu Ser Ile Thr Asn Ala Gly Asn Ser Thr Gly Ala Pro Val Asp
    210                 215                 220

Ser Glu Ser Ser Asp Thr Ser Leu Arg Leu Gly Leu Pro Tyr Gly Gly
225                 230                 235                 240

<210> 25
<211> 1552
<212> DNA
<213> Arabidopsis thaliana

<220>
<221> CDS
<222> (193)..(825)
<223> G861.1

<400> 25
attctctctc tcncaaaatt tatttcctct ggcttcttct tcctcctcct ccatctcttc 60

tctttactct ctctttaatc atctctcatt cttgaatctt gatccatcaa aatcaatccc 120

gttctcgaaa gatccattaa aatcaaaacc taagctctct ctcttgcttc tagggttttt 180

ttgttcgttg tg atg gcg aga gaa aag att cag atc agg aag atc gac aac 231
Met Ala Arg Glu Lys Ile Gln Ile Arg Lys Ile Asp Asn
1               5                   10

gca acg gcg aga caa gtg acg ttt tcg aaa cga aga aga ggg ctt ttc 279
Ala Thr Ala Arg Gln Val Thr Phe Ser Lys Arg Arg Arg Gly Leu Phe
    15                  20                  25

aag aaa gct gaa gaa ctc tcc gtt ctc tgc gac gcc gat gtc gct ctc 327
Lys Lys Ala Glu Glu Leu Ser Val Leu Cys Asp Ala Asp Val Ala Leu
30                  35                  40                  45

atc atc ttc tct tcc acc gga aaa ctg ttc gag ttc tgt agc tcc agc 375
Ile Ile Phe Ser Ser Thr Gly Lys Leu Phe Glu Phe Cys Ser Ser Ser
                50                  55                  60

atg aag gaa gtc cta gag agg cat aac ttg cag tca aag aac ttg gag 423
Met Lys Glu Val Leu Glu Arg His Asn Leu Gln Ser Lys Asn Leu Glu
            65                  70                  75

aag ctt gat cag cca tct ctt gag tta cag ctg gtt gag aac agt gat 471
Lys Leu Asp Gln Pro Ser Leu Glu Leu Gln Leu Val Glu Asn Ser Asp
        80                  85                  90

cac gcc cga atg agt aaa gaa att gcg gac aag agc cac cga cta agg 519
His Ala Arg Met Ser Lys Glu Ile Ala Asp Lys Ser His Arg Leu Arg
    95                  100                 105

caa atg aga gga gag gaa ctt caa gga ctt gac att gaa gag ctt cag 567
Gln Met Arg Gly Glu Glu Leu Gln Gly Leu Asp Ile Glu Glu Leu Gln
110                 115                 120                 125

cag cta gag aag gcc ctt gaa act ggt ttg acg cgt gtg att gaa aca 615
Gln Leu Glu Lys Ala Leu Glu Thr Gly Leu Thr Arg Val Ile Glu Thr
                130                 135                 140

aag agt gac aag att atg agt gag atc agc gaa ctt cag aaa aag gga 663
Lys Ser Asp Lys Ile Met Ser Glu Ile Ser Glu Leu Gln Lys Lys Gly
            145                 150                 155

atg caa ttg atg gat gag aac aag cgg ttg agg cag caa gta tgt gtc 711
Met Gln Leu Met Asp Glu Asn Lys Arg Leu Arg Gln Gln Val Cys Val
        160                 165                 170

tta ccc tct ctg ttg ata aca aat ccc ttt ctt ttg tct acc att aac 759
Leu Pro Ser Leu Leu Ile Thr Asn Pro Phe Leu Leu Ser Thr Ile Asn
    175                 180                 185

gta cac act cct aaa ttt aat ccc cag ttg tct aca aca cat atg ttt 807
Val His Thr Pro Lys Phe Asn Pro Gln Leu Ser Thr Thr His Met Phe
190                 195                 200                 205

gat cat act gtg aga taa atgaataaac caagtgatat agcgcgattt 855
Asp His Thr Val Arg
                210

aaaaatgtct ttaaaactaa aggtaaccat gtagctagtt agtctctagg gtcctagagg 915

tctacgagtg tgcatgcatg gatttggtgc gtttttcttt tttcatcttc attttgtttt 975

ttgaaacaag gaaccataaa cgaatatata tctaattctt gtttgatata tagtttggtc 1035

gaggcttcat gtcaagattt gctcattcgt agttagttga tctctagaga aattcaaaac 1095

acatggtgcc actaaaaaca caaaatgcaa atacttagct agagaactta atgatatgtt 1155

ttgtcttgat ttttgcaggg aacgcaacta acggaagaga acgagcgact tggcatgcaa 1215

atatgtaaca atgtgcatgc acacggtggt gctgaatcgg agaacgctgc tgtgtacgag 1275

gaaggacagt cgtcggagtc tattactaac gccggaaact ctaccggagc gcctgttgac 1335

tccgagagct ccgacacttc ccttaggctc ggcttaccgt atggtggtta gagatggaac 1395

aattcaaaga agttgatgga gtgaggagag taatgtaaat ctttttaact cggtagtaac 1455

aagagacaat gtctaagtag tgaattctca aatgtttgtg taagtttctg cctatggaag 1515

aggctttcat ttttatgatt aaaaaaaaaa aaaaaaa 1552

<210> 26
<211> 210
<212> PRT
<213> Arabidopsis thaliana

<400> 26

Met Ala Arg Glu Lys Ile Gln Ile Arg Lys Ile Asp Asn Ala Thr Ala
1               5                   10                  15

Arg Gln Val Thr Phe Ser Lys Arg Arg Arg Gly Leu Phe Lys Lys Ala
            20                  25                  30

Glu Glu Leu Ser Val Leu Cys Asp Ala Asp Val Ala Leu Ile Ile Phe
        35                  40                  45

Ser Ser Thr Gly Lys Leu Phe Glu Phe Cys Ser Ser Ser Met Lys Glu
    50                  55                  60

Val Leu Glu Arg His Asn Leu Gln Ser Lys Asn Leu Glu Lys Leu Asp
65                  70                  75                  80

Gln Pro Ser Leu Glu Leu Gln Leu Val Glu Asn Ser Asp His Ala Arg
                85                  90                  95

Met Ser Lys Glu Ile Ala Asp Lys Ser His Arg Leu Arg Gln Met Arg
            100                 105                 110

Gly Glu Glu Leu Gln Gly Leu Asp Ile Glu Glu Leu Gln Gln Leu Glu
        115                 120                 125

Lys Ala Leu Glu Thr Gly Leu Thr Arg Val Ile Glu Thr Lys Ser Asp
    130                 135                 140

Lys Ile Met Ser Glu Ile Ser Glu Leu Gln Lys Lys Gly Met Gln Leu
145                 150                 155                 160

Met Asp Glu Asn Lys Arg Leu Arg Gln Gln Val Cys Val Leu Pro Ser
                165                 170                 175

Leu Leu Ile Thr Asn Pro Phe Leu Leu Ser Thr Ile Asn Val His Thr

180                 185                 190

Pro Lys Phe Asn Pro Gln Leu Ser Thr Thr His Met Phe Asp His Thr
        195                 200                 205

Val Arg
    210

<210>  27
<211>  943
<212>  DNA
<213>  Arabidopsis thaliana

<220>
<221>  CDS
<222>  (110)..(700)
<223>  G1759

<400>  27
cgagaaaagg aaaaaaaaaa atagaaagag aaaacgctta gtatctccgg cgacttgaac      60

ccaaacctga ggatcaaatt agggcacaaa gccctctcgg agagaagcc atg gga aga     118
                                                      Met Gly Arg
                                                      1

aaa aaa cta gaa atc aag cga att gag aac aaa agt agc cga caa gtc       166
Lys Lys Leu Glu Ile Lys Arg Ile Glu Asn Lys Ser Ser Arg Gln Val
    5                   10                  15

acc ttc tcc aaa cgt cgc aac ggt ctc atc gag aaa gct cgt cag ctt       214
Thr Phe Ser Lys Arg Arg Asn Gly Leu Ile Glu Lys Ala Arg Gln Leu
20                  25                  30                  35

tct gtt ctc tgt gac gca tcc gtc gct ctt ctc gtc gtc tcc gcc tcc       262
Ser Val Leu Cys Asp Ala Ser Val Ala Leu Leu Val Val Ser Ala Ser
                40                  45                  50

ggc aag ctc tac agc ttc tcc tcc ggc gat aac ctg gtc aag atc ctt       310
Gly Lys Leu Tyr Ser Phe Ser Ser Gly Asp Asn Leu Val Lys Ile Leu
            55                  60                  65

gat cga tat ggg aaa cag cat gct gat gat ctt aaa gcc ttg gat cat       358
Asp Arg Tyr Gly Lys Gln His Ala Asp Asp Leu Lys Ala Leu Asp His
        70                  75                  80

cag tca aaa gct ctg aac tat ggt tca cac tat gag cta ctt gaa ctt       406
Gln Ser Lys Ala Leu Asn Tyr Gly Ser His Tyr Glu Leu Leu Glu Leu
    85                  90                  95

gtg gat agc aag ctt gtg gga tca aat gtc aaa aat gtg agt atc gat       454
Val Asp Ser Lys Leu Val Gly Ser Asn Val Lys Asn Val Ser Ile Asp
100                 105                 110                 115

gct ctt gtt caa ctg gag gaa cac ctt gag act gcc ctc tcc gtg act       502
Ala Leu Val Gln Leu Glu Glu His Leu Glu Thr Ala Leu Ser Val Thr
                120                 125                 130

aga gcc aag aag acc gaa ctc atg ttg aag ctt gtt gag aat ctt aaa       550
Arg Ala Lys Lys Thr Glu Leu Met Leu Lys Leu Val Glu Asn Leu Lys

            135                 140                 145

gaa aag gag aaa atg ctg aaa gaa gag aac cag gtt ttg gct agc cag 598
Glu Lys Glu Lys Met Leu Lys Glu Glu Asn Gln Val Leu Ala Ser Gln
        150                 155                 160

atg gag aat aat cat cat gtg gga gca gaa gct gag atg gag atg tca 646
Met Glu Asn Asn His His Val Gly Ala Glu Ala Glu Met Glu Met Ser
    165                 170                 175

cct gct gga caa atc tcc gac aat ctt ccg gtg act ctc cca cta ctt 694
Pro Ala Gly Gln Ile Ser Asp Asn Leu Pro Val Thr Leu Pro Leu Leu
180                 185                 190                 195

aat tag ccaccttaaa tcggcggttg aaatcaaaat ccaaaacata tataattatg 750
Asn

aagaaaaaaa aaataagata tgtaattatt ccgctgataa gggcgagcgt ttgtatatct 810

taatactctc tctttggcca agagactttg tgtgtgatac ttaagtagac ggaactaagt 870

caatactatc tgttttaaga caaaaggttg atgaactttg taccttattc gtgtgagaaa 930

aaaaaaaaaa aaa 943

<210> 28
<211> 196
<212> PRT
<213> Arabidopsis thaliana

<400> 28

Met Gly Arg Lys Lys Leu Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Val Leu Cys Asp Ala Ser Val Ala Leu Leu Val Val
        35                  40                  45

Ser Ala Ser Gly Lys Leu Tyr Ser Phe Ser Ser Gly Asp Asn Leu Val
    50                  55                  60

Lys Ile Leu Asp Arg Tyr Gly Lys Gln His Ala Asp Asp Leu Lys Ala
65                  70                  75                  80

Leu Asp His Gln Ser Lys Ala Leu Asn Tyr Gly Ser His Tyr Glu Leu
                85                  90                  95

Leu Glu Leu Val Asp Ser Lys Leu Val Gly Ser Asn Val Lys Asn Val
            100                 105                 110

Ser Ile Asp Ala Leu Val Gln Leu Glu Glu His Leu Glu Thr Ala Leu

115                 120                 125

Ser Val Thr Arg Ala Lys Lys Thr Glu Leu Met Leu Lys Leu Val Glu
    130                 135                 140

Asn Leu Lys Glu Lys Glu Lys Met Leu Lys Glu Glu Asn Gln Val Leu
145                 150                 155                 160

Ala Ser Gln Met Glu Asn Asn His His Val Gly Ala Glu Ala Glu Met
                165                 170                 175

Glu Met Ser Pro Ala Gly Gln Ile Ser Asp Asn Leu Pro Val Thr Leu
            180                 185                 190

Pro Leu Leu Asn
        195

<210>  29
<211>  1171
<212>  DNA
<213>  Arabidopsis thaliana

<220>
<221>  CDS
<222>  (63)..(959)
<223>  G192

<400>  29
cttttttctc ttctctcctc agagattcga agctttttgt ctcccctgag taaccaaatt      60

ca atg gcc gac gat tgg gat ctc cac gcc gta gtc aga ggc tgc tca      107
   Met Ala Asp Asp Trp Asp Leu His Ala Val Val Arg Gly Cys Ser
   1               5                   10                  15

gcc gta agc tca tca gct act acc acc gta tat tcc ccc ggc gtt tca      155
Ala Val Ser Ser Ser Ala Thr Thr Thr Val Tyr Ser Pro Gly Val Ser
                20                  25                  30

tct cac aca aac cct ata ttc acc gtc gga cga caa agt aat gcc gtc      203
Ser His Thr Asn Pro Ile Phe Thr Val Gly Arg Gln Ser Asn Ala Val
            35                  40                  45

tcc ttc gga gag att cga gat ctc tac aca ccg ttc aca caa gaa tct      251
Ser Phe Gly Glu Ile Arg Asp Leu Tyr Thr Pro Phe Thr Gln Glu Ser
        50                  55                  60

gtc gtc tct tcg ttt tct tgt ata aac tac cca gaa gaa cct aga aag      299
Val Val Ser Ser Phe Ser Cys Ile Asn Tyr Pro Glu Glu Pro Arg Lys
    65                  70                  75

cca cag aac cag aaa cgt cct ctt tct ctc tct gct tct tcc ggt agc      347
Pro Gln Asn Gln Lys Arg Pro Leu Ser Leu Ser Ala Ser Ser Gly Ser
80                  85                  90                  95

gtc act agc aaa ccc agt ggc tcc aat acc tct aga tct aaa aga aga      395
Val Thr Ser Lys Pro Ser Gly Ser Asn Thr Ser Arg Ser Lys Arg Arg

                    100                 105                 110

aag ata cag cat aag aaa gtg tgc cat gta gca gca gaa gct tta aac      443
Lys Ile Gln His Lys Lys Val Cys His Val Ala Ala Glu Ala Leu Asn
            115                 120                 125

tcc gat gtc tgg gca tgg cga aag tac gga cag aaa ccc atc aaa ggt      491
Ser Asp Val Trp Ala Trp Arg Lys Tyr Gly Gln Lys Pro Ile Lys Gly
        130                 135                 140

tca cca tat cca aga gga tac tac aga tgt agt aca tca aaa ggt tgt      539
Ser Pro Tyr Pro Arg Gly Tyr Tyr Arg Cys Ser Thr Ser Lys Gly Cys
    145                 150                 155

tta gcc cgt aaa caa gtg gag cga aat aga tcc gac ccg aag atg ttt      587
Leu Ala Arg Lys Gln Val Glu Arg Asn Arg Ser Asp Pro Lys Met Phe
160                 165                 170                 175

atc gtc act tac acg gcg gag cat aat cat cca gct ccg aca cac cgt      635
Ile Val Thr Tyr Thr Ala Glu His Asn His Pro Ala Pro Thr His Arg
                180                 185                 190

aat tct ctc gcc gga agc aca cgt cag aaa cca tcc gat caa cag acg      683
Asn Ser Leu Ala Gly Ser Thr Arg Gln Lys Pro Ser Asp Gln Gln Thr
            195                 200                 205

agt aaa tct ccg acg acc act att gct act tat tca tcg tct ccg gtg      731
Ser Lys Ser Pro Thr Thr Thr Ile Ala Thr Tyr Ser Ser Ser Pro Val
        210                 215                 220

act tca gcc gac gaa ttt gtt ttg cct gtt gag gat cat cta gcg gtg      779
Thr Ser Ala Asp Glu Phe Val Leu Pro Val Glu Asp His Leu Ala Val
    225                 230                 235

gga gat ctt gac gga gaa gaa gat ctg tta tct ttg tcg gat acg gtg      827
Gly Asp Leu Asp Gly Glu Glu Asp Leu Leu Ser Leu Ser Asp Thr Val
240                 245                 250                 255

gtt agc gat gat ttc ttc gat ggg tta gag gaa ttc gca gcc gga gat      875
Val Ser Asp Asp Phe Phe Asp Gly Leu Glu Glu Phe Ala Ala Gly Asp
                260                 265                 270

agc ttt tcc ggg aac tcg gct ccg gcg agt ttt gat ctc tct tgg gtt      923
Ser Phe Ser Gly Asn Ser Ala Pro Ala Ser Phe Asp Leu Ser Trp Val
            275                 280                 285

gtg aac agt gcc gcc act acc acc gga gga ata tga ttagattacg           969
Val Asn Ser Ala Ala Thr Thr Thr Gly Gly Ile
        290                 295

acggcttaga atactcttat taggacagat ttataggatt aaggaattat tctcggagca   1029

tatgtaaaaa taggataaaa gaaaatgttc tttgttactt tttttcgggt tttcttccta   1089

ttgtttctaa acatcttaga aaaaatttaa ttgtatattc cttaagctcg atacatcttg   1149

ttttaaaaaa aaaaaaaaaa aa                                            1171

<210>  30
<211>  298
<212>  PRT
<213>  Arabidopsis thaliana

<400> 30

Met Ala Asp Asp Trp Asp Leu His Ala Val Val Arg Gly Cys Ser Ala
1               5                   10                  15

Val Ser Ser Ser Ala Thr Thr Thr Val Tyr Ser Pro Gly Val Ser Ser
            20                  25                  30

His Thr Asn Pro Ile Phe Thr Val Gly Arg Gln Ser Asn Ala Val Ser
        35                  40                  45

Phe Gly Glu Ile Arg Asp Leu Tyr Thr Pro Phe Thr Gln Glu Ser Val
    50                  55                  60

Val Ser Ser Phe Ser Cys Ile Asn Tyr Pro Glu Glu Pro Arg Lys Pro
65                  70                  75                  80

Gln Asn Gln Lys Arg Pro Leu Ser Leu Ser Ala Ser Ser Gly Ser Val
                85                  90                  95

Thr Ser Lys Pro Ser Gly Ser Asn Thr Ser Arg Ser Lys Arg Arg Lys
            100                 105                 110

Ile Gln His Lys Lys Val Cys His Val Ala Ala Glu Ala Leu Asn Ser
        115                 120                 125

Asp Val Trp Ala Trp Arg Lys Tyr Gly Gln Lys Pro Ile Lys Gly Ser
    130                 135                 140

Pro Tyr Pro Arg Gly Tyr Tyr Arg Cys Ser Thr Ser Lys Gly Cys Leu
145                 150                 155                 160

Ala Arg Lys Gln Val Glu Arg Asn Arg Ser Asp Pro Lys Met Phe Ile
                165                 170                 175

Val Thr Tyr Thr Ala Glu His Asn His Pro Ala Pro Thr His Arg Asn
            180                 185                 190

Ser Leu Ala Gly Ser Thr Arg Gln Lys Pro Ser Asp Gln Gln Thr Ser
        195                 200                 205

Lys Ser Pro Thr Thr Thr Ile Ala Thr Tyr Ser Ser Ser Pro Val Thr
    210                 215                 220

Ser Ala Asp Glu Phe Val Leu Pro Val Glu Asp His Leu Ala Val Gly
225                 230                 235                 240

Asp Leu Asp Gly Glu Glu Asp Leu Leu Ser Leu Ser Asp Thr Val Val
                245                 250                 255

Ser Asp Asp Phe Phe Asp Gly Leu Glu Glu Phe Ala Ala Gly Asp Ser
            260                 265                 270

Phe Ser Gly Asn Ser Ala Pro Ala Ser Phe Asp Leu Ser Trp Val Val
        275                 280                 285

Asn Ser Ala Ala Thr Thr Thr Gly Gly Ile
    290                 295

<210> 31
<211> 1139
<212> DNA
<213> Arabidopsis thaliana

<220>
<221> CDS
<222> (106)..(1032)
<223> G234

<400> 31
cacaacatca tacccaccaa catatataat cttgatcata gagagataaa cagaggccgc 60

tatcaagaac aagactaaga acaagacttc actaggagta caagt atg gga aga gca 117
Met Gly Arg Ala
1

ccg tgt tgt gac aaa gca aac gtg aag aaa ggg cct tgg tct cct gag 165
Pro Cys Cys Asp Lys Ala Asn Val Lys Lys Gly Pro Trp Ser Pro Glu
5                   10                  15                  20

gaa gat gca aaa ctc aaa tct tac att gaa aat agt ggc acc gga ggc 213
Glu Asp Ala Lys Leu Lys Ser Tyr Ile Glu Asn Ser Gly Thr Gly Gly
                25                  30                  35

aat tgg atc gct ttg cct caa aag att ggt tta aag aga tgt gga aag 261
Asn Trp Ile Ala Leu Pro Gln Lys Ile Gly Leu Lys Arg Cys Gly Lys
            40                  45                  50

agt tgc agg ctg agg tgg ctt aac tat ctt aga cca aac atc aaa cat 309
Ser Cys Arg Leu Arg Trp Leu Asn Tyr Leu Arg Pro Asn Ile Lys His
        55                  60                  65

ggt ggc ttc tct gag gaa gaa gaa aac atc att tgt agc ctt tac ctt 357
Gly Gly Phe Ser Glu Glu Glu Glu Asn Ile Ile Cys Ser Leu Tyr Leu
    70                  75                  80

aca att ggt agc agg tgg tct ata atc gct gct caa ttg ccg gga cga 405
Thr Ile Gly Ser Arg Trp Ser Ile Ile Ala Ala Gln Leu Pro Gly Arg
85                  90                  95                  100

aca gac aac gat ata aaa aac tat tgg aac acg agg ctc aag aag aaa 453
Thr Asp Asn Asp Ile Lys Asn Tyr Trp Asn Thr Arg Leu Lys Lys Lys
                105                 110                 115

ctc att aac aaa caa cgc aag gag ctt caa gaa gct tgt atg gag cag 501

Leu Ile Asn Lys Gln Arg Lys Glu Leu Gln Glu Ala Cys Met Glu Gln
            120                 125                 130

caa gag atg atg gtg atg atg aag aga caa cac caa caa caa caa atc      549
Gln Glu Met Met Val Met Met Lys Arg Gln His Gln Gln Gln Gln Ile
        135                 140                 145

caa act tct ttt atg atg aga caa gac caa aca atg ttc aca tgg cca      597
Gln Thr Ser Phe Met Met Arg Gln Asp Gln Thr Met Phe Thr Trp Pro
    150                 155                 160

cta cat cat cat aat gtt caa gtt cca gct ctt ttc aga atc aaa cca      645
Leu His His His Asn Val Gln Val Pro Ala Leu Phe Arg Ile Lys Pro
165                 170                 175                 180

act cgt ttt gcg acc aag aag atg tta agc cag tgc tca tca aga aca      693
Thr Arg Phe Ala Thr Lys Lys Met Leu Ser Gln Cys Ser Ser Arg Thr
                185                 190                 195

tgg tca aga tcg aag atc aag aac tgg aga aaa caa acc tca tca tca      741
Trp Ser Arg Ser Lys Ile Lys Asn Trp Arg Lys Gln Thr Ser Ser Ser
            200                 205                 210

tca aga ttc aat gac aac gct ttt gat cat ctc tct ttc tct caa ctc      789
Ser Arg Phe Asn Asp Asn Ala Phe Asp His Leu Ser Phe Ser Gln Leu
        215                 220                 225

ttg tta gat cct aat cat aac cac tta gga tca gga gag ggt ttc tcc      837
Leu Leu Asp Pro Asn His Asn His Leu Gly Ser Gly Glu Gly Phe Ser
    230                 235                 240

atg aac tct atc ttg agc gcc aac aca aac tct cca ttg ctt aac aca      885
Met Asn Ser Ile Leu Ser Ala Asn Thr Asn Ser Pro Leu Leu Asn Thr
245                 250                 255                 260

agt aat gat aat cag tgg ttc ggg aat ttc cag gcc gaa acc gta aac      933
Ser Asn Asp Asn Gln Trp Phe Gly Asn Phe Gln Ala Glu Thr Val Asn
                265                 270                 275

ttg ttc tca gga gcc tcc aca agt act tcg gca gat caa agc act ata      981
Leu Phe Ser Gly Ala Ser Thr Ser Thr Ser Ala Asp Gln Ser Thr Ile
            280                 285                 290

agt tgg gaa gac ata agc tct ctt gtt tat tct gat tca aag caa ttt     1029
Ser Trp Glu Asp Ile Ser Ser Leu Val Tyr Ser Asp Ser Lys Gln Phe
        295                 300                 305

ttt taattataat aatatattat tcttaagatg aaacgtacat cattattatt          1082
Phe

aattgggggt acgtaacgta tatatggaat aacgatctag tttgtttaaa tttaaaa      1139

<210>  32
<211>  309
<212>  PRT
<213>  Arabidopsis thaliana

<400>  32

Met Gly Arg Ala Pro Cys Cys Asp Lys Ala Asn Val Lys Lys Gly Pro
1               5                   10                  15

Trp Ser Pro Glu Glu Asp Ala Lys Leu Lys Ser Tyr Ile Glu Asn Ser
            20                  25                  30

Gly Thr Gly Gly Asn Trp Ile Ala Leu Pro Gln Lys Ile Gly Leu Lys
        35                  40                  45

Arg Cys Gly Lys Ser Cys Arg Leu Arg Trp Leu Asn Tyr Leu Arg Pro
    50                  55                  60

Asn Ile Lys His Gly Gly Phe Ser Glu Glu Glu Glu Asn Ile Ile Cys
65                  70                  75                  80

Ser Leu Tyr Leu Thr Ile Gly Ser Arg Trp Ser Ile Ile Ala Ala Gln
                85                  90                  95

Leu Pro Gly Arg Thr Asp Asn Asp Ile Lys Asn Tyr Trp Asn Thr Arg
            100                 105                 110

Leu Lys Lys Lys Leu Ile Asn Lys Gln Arg Lys Glu Leu Gln Glu Ala
        115                 120                 125

Cys Met Glu Gln Gln Glu Met Met Val Met Met Lys Arg Gln His Gln
    130                 135                 140

Gln Gln Gln Ile Gln Thr Ser Phe Met Met Arg Gln Asp Gln Thr Met
145                 150                 155                 160

Phe Thr Trp Pro Leu His His His Asn Val Gln Val Pro Ala Leu Phe
                165                 170                 175

Arg Ile Lys Pro Thr Arg Phe Ala Thr Lys Lys Met Leu Ser Gln Cys
            180                 185                 190

Ser Ser Arg Thr Trp Ser Arg Ser Lys Ile Lys Asn Trp Arg Lys Gln
        195                 200                 205

Thr Ser Ser Ser Ser Arg Phe Asn Asp Asn Ala Phe Asp His Leu Ser
    210                 215                 220

Phe Ser Gln Leu Leu Leu Asp Pro Asn His Asn His Leu Gly Ser Gly
225                 230                 235                 240

Glu Gly Phe Ser Met Asn Ser Ile Leu Ser Ala Asn Thr Asn Ser Pro
                245                 250                 255

Leu Leu Asn Thr Ser Asn Asp Asn Gln Trp Phe Gly Asn Phe Gln Ala

            260                 265                 270

Glu Thr Val Asn Leu Phe Ser Gly Ala Ser Thr Ser Thr Ser Ala Asp
        275                 280                 285

Gln Ser Thr Ile Ser Trp Glu Asp Ile Ser Ser Leu Val Tyr Ser Asp
    290                 295                 300

Ser Lys Gln Phe Phe
305

<210> 33
<211> 922
<212> DNA
<213> Arabidopsis thaliana

<220>
<221> CDS
<222> (54)..(647)
<223> G361

<400> 33
tctgtctctc tctctctctt tgtaaatata catatataga taagctcaca tat atg 56
Met
1

gcg act gaa aca tct tct ttg aag ctc ttc ggt ata aac cta ctt gaa 104
Ala Thr Glu Thr Ser Ser Leu Lys Leu Phe Gly Ile Asn Leu Leu Glu
            5                   10                  15

acg acg tcg gtt caa aac cag tca tcg gaa cca aga ccc gga tcc gga 152
Thr Thr Ser Val Gln Asn Gln Ser Ser Glu Pro Arg Pro Gly Ser Gly
        20                  25                  30

tca gga tcc gag tca cgt aag tac gag tgt caa tac tgt tgt aga gag 200
Ser Gly Ser Glu Ser Arg Lys Tyr Glu Cys Gln Tyr Cys Cys Arg Glu
    35                  40                  45

ttt gct aac tct caa gct ctt ggt ggt cac caa aac gct cac aag aaa 248
Phe Ala Asn Ser Gln Ala Leu Gly Gly His Gln Asn Ala His Lys Lys
50                  55                  60                  65

gag cgt cag ctt ctt aaa cgt gca cag atg tta gct act cgt ggt ttg 296
Glu Arg Gln Leu Leu Lys Arg Ala Gln Met Leu Ala Thr Arg Gly Leu
                70                  75                  80

cca cgt cat cat aat ttt cac cct cat acc aat ccg ctt ctc tcc gcc 344
Pro Arg His His Asn Phe His Pro His Thr Asn Pro Leu Leu Ser Ala
            85                  90                  95

ttc gcg ccg ctg cct cac ctc ctc tct cag ccg cat cct ccg ccg cat 392
Phe Ala Pro Leu Pro His Leu Leu Ser Gln Pro His Pro Pro Pro His
        100                 105                 110

atg atg ctc tct cct tct tct tcg agt tct aag tgg ctt tac ggt gaa 440
Met Met Leu Ser Pro Ser Ser Ser Ser Ser Lys Trp Leu Tyr Gly Glu
    115                 120                 125

cac atg tcg tca caa aac gcc gtt ggg tac ttt cat ggt gga agg gga 488
His Met Ser Ser Gln Asn Ala Val Gly Tyr Phe His Gly Gly Arg Gly
130                 135                 140                 145

ctt tac gga ggt ggc atg gag tct atg gcc gga gaa gta aag act cat 536
Leu Tyr Gly Gly Gly Met Glu Ser Met Ala Gly Glu Val Lys Thr His
                150                 155                 160

ggt ggt tct ttg ccg gag atg agg agg ttc gcc gga gat agt gat cgg 584
Gly Gly Ser Leu Pro Glu Met Arg Arg Phe Ala Gly Asp Ser Asp Arg
            165                 170                 175

agt agc gga att aag tta gag aat ggt att ggg ctg gac ctc cat tta 632
Ser Ser Gly Ile Lys Leu Glu Asn Gly Ile Gly Leu Asp Leu His Leu
        180                 185                 190

agc ctt ggg cca tga atgattataa ttttggccca gtaaagatct gtaaaatact 687
Ser Leu Gly Pro
    195

actaggattt catttttata gagtatgttt ttttccttaa tttcggttga aattggtgaa 747

tatttttatc tcttacttac caaatctcat atttctatgt atgcgtttgc tttcactttt 807

tttttttata taattcttct tgtaaaaaat gcaatgtgag ttttcttccc tatcattctg 867

tcaagctttg gttcaattat ttagtaatcg aataatatag gaatagtgtt gaaag 922

<210> 34
<211> 197
<212> PRT
<213> Arabidopsis thaliana

<400> 34

Met Ala Thr Glu Thr Ser Ser Leu Lys Leu Phe Gly Ile Asn Leu Leu
1               5                   10                  15

Glu Thr Thr Ser Val Gln Asn Gln Ser Ser Glu Pro Arg Pro Gly Ser
            20                  25                  30

Gly Ser Gly Ser Glu Ser Arg Lys Tyr Glu Cys Gln Tyr Cys Cys Arg
        35                  40                  45

Glu Phe Ala Asn Ser Gln Ala Leu Gly Gly His Gln Asn Ala His Lys
    50                  55                  60

Lys Glu Arg Gln Leu Leu Lys Arg Ala Gln Met Leu Ala Thr Arg Gly
65                  70                  75                  80

Leu Pro Arg His His Asn Phe His Pro His Thr Asn Pro Leu Leu Ser
                85                  90                  95

Ala Phe Ala Pro Leu Pro His Leu Leu Ser Gln Pro His Pro Pro Pro
            100                 105                 110

His Met Met Leu Ser Pro Ser Ser Ser Ser Ser Lys Trp Leu Tyr Gly
        115                 120                 125

Glu His Met Ser Ser Gln Asn Ala Val Gly Tyr Phe His Gly Gly Arg
    130                 135                 140

Gly Leu Tyr Gly Gly Gly Met Glu Ser Met Ala Gly Glu Val Lys Thr
145                 150                 155                 160

His Gly Gly Ser Leu Pro Glu Met Arg Arg Phe Ala Gly Asp Ser Asp
                165                 170                 175

Arg Ser Ser Gly Ile Lys Leu Glu Asn Gly Ile Gly Leu Asp Leu His
            180                 185                 190

Leu Ser Leu Gly Pro
        195

<210>  35
<211>  420
<212>  DNA
<213>  Arabidopsis thaliana

<220>
<221>  CDS
<222>  (1)..(420)
<223>  G486

<400>  35
atg aca gac gaa gat aga ttg ttg cca ata gcc aat gta ggg aga ctt      48
Met Thr Asp Glu Asp Arg Leu Leu Pro Ile Ala Asn Val Gly Arg Leu
1               5                   10                  15

atg aag caa atc cta cca tca aat gca aag atc tca aaa gaa gca aaa      96
Met Lys Gln Ile Leu Pro Ser Asn Ala Lys Ile Ser Lys Glu Ala Lys
            20                  25                  30

caa aca gtt caa gaa tgt gca aca gag ttc ata agc ttt gtt aca tgc      144
Gln Thr Val Gln Glu Cys Ala Thr Glu Phe Ile Ser Phe Val Thr Cys
        35                  40                  45

gaa gca tca gag aag tgc cac agg gag aat cgg aag acg gtg aat gga      192
Glu Ala Ser Glu Lys Cys His Arg Glu Asn Arg Lys Thr Val Asn Gly
    50                  55                  60

gac gac atc tgg tgg gct ctc agc act ctc ggc ctc gat aac tat gct      240
Asp Asp Ile Trp Trp Ala Leu Ser Thr Leu Gly Leu Asp Asn Tyr Ala
65                  70                  75                  80

gac gcc gtg ggt agg cat ctt cac aag tac cgt gaa gcc gag aga gaa      288
Asp Ala Val Gly Arg His Leu His Lys Tyr Arg Glu Ala Glu Arg Glu
                85                  90                  95

aga act gag cac aac aaa ggt agc aat gat agt ggg aat gag aaa gaa      336
Arg Thr Glu His Asn Lys Gly Ser Asn Asp Ser Gly Asn Glu Lys Glu

100                 105                 110

acc aac act aga agt gat gta cag aac caa tcg aca aaa ttt att aga        384
Thr Asn Thr Arg Ser Asp Val Gln Asn Gln Ser Thr Lys Phe Ile Arg
        115                 120                 125

gtt gtt gag aag gga agc agc tcc tcg gcc cgt tga                        420
Val Val Glu Lys Gly Ser Ser Ser Ser Ala Arg
    130                 135

<210>  36
<211>  139
<212>  PRT
<213>  Arabidopsis thaliana

<400>  36

Met Thr Asp Glu Asp Arg Leu Leu Pro Ile Ala Asn Val Gly Arg Leu
1               5                   10                  15

Met Lys Gln Ile Leu Pro Ser Asn Ala Lys Ile Ser Lys Glu Ala Lys
            20                  25                  30

Gln Thr Val Gln Glu Cys Ala Thr Glu Phe Ile Ser Phe Val Thr Cys
        35                  40                  45

Glu Ala Ser Glu Lys Cys His Arg Glu Asn Arg Lys Thr Val Asn Gly
    50                  55                  60

Asp Asp Ile Trp Trp Ala Leu Ser Thr Leu Gly Leu Asp Asn Tyr Ala
65                  70                  75                  80

Asp Ala Val Gly Arg His Leu His Lys Tyr Arg Glu Ala Glu Arg Glu
                85                  90                  95

Arg Thr Glu His Asn Lys Gly Ser Asn Asp Ser Gly Asn Glu Lys Glu
            100                 105                 110

Thr Asn Thr Arg Ser Asp Val Gln Asn Gln Ser Thr Lys Phe Ile Arg
        115                 120                 125

Val Val Glu Lys Gly Ser Ser Ser Ser Ala Arg
    130                 135

<210>  37
<211>  1707
<212>  DNA
<213>  Arabidopsis thaliana

<220>
<221>  CDS
<222>  (98)..(1444)
<223>  G748

<400> 37
   ccacgcgtcc gcactctccc aaatctctct tctttaacaa caaaaaaaaa atcacagaga 60
   catagagaga agaagacgga acagaggctc caaaaaa atg atg atg gag act aga 115
   Met Met Met Glu Thr Arg
   1 5
   gat cca gct att aag ctt ttc ggt atg aaa atc cct ttt ccg tcg gtt 163
   Asp Pro Ala Ile Lys Leu Phe Gly Met Lys Ile Pro Phe Pro Ser Val
   10 15 20
   ttt gaa tcg gca gtt acg gtg gag gat gac gaa gaa gat gac tgg agc 211
   Phe Glu Ser Ala Val Thr Val Glu Asp Asp Glu Glu Asp Asp Trp Ser
   25 30 35
   ggc gga gat gac aaa tca cca gag aag gta act cca gag tta tca gat 259
   Gly Gly Asp Asp Lys Ser Pro Glu Lys Val Thr Pro Glu Leu Ser Asp
   40 45 50
   aag aac aac aac aac tgt aac gac aac agt ttt aac aat tcg aaa ccc 307
   Lys Asn Asn Asn Asn Cys Asn Asp Asn Ser Phe Asn Asn Ser Lys Pro
   55 60 65 70
   gaa acc ttg gac aaa gag gaa gcg aca tca act gat cag ata gag agt 355
   Glu Thr Leu Asp Lys Glu Glu Ala Thr Ser Thr Asp Gln Ile Glu Ser
   75 80 85
   agt gac acg cct gag gat aat cag cag acg aca cct gat ggt aaa acc 403
   Ser Asp Thr Pro Glu Asp Asn Gln Gln Thr Thr Pro Asp Gly Lys Thr
   90 95 100
   cta aag aaa ccg act aag att cta ccg tgt ccg aga tgc aaa agc atg 451
   Leu Lys Lys Pro Thr Lys Ile Leu Pro Cys Pro Arg Cys Lys Ser Met
   105 110 115
   gag acc aag ttc tgt tat tac aac aac tac aac ata aac cag cct cgt 499
   Glu Thr Lys Phe Cys Tyr Tyr Asn Asn Tyr Asn Ile Asn Gln Pro Arg
   120 125 130
   cat ttc tgc aag gct tgt cag aga tat tgg act gct gga ggg act atg 547
   His Phe Cys Lys Ala Cys Gln Arg Tyr Trp Thr Ala Gly Gly Thr Met
   135 140 145 150
   agg aat gtt cct gtg ggg gca gga cgt cgt aag aac aaa agc tca tct 595
   Arg Asn Val Pro Val Gly Ala Gly Arg Arg Lys Asn Lys Ser Ser Ser
   155 160 165
   tct cat tac cgt cac atc act att tcc gag gct ctt gag gct gcg agg 643
   Ser His Tyr Arg His Ile Thr Ile Ser Glu Ala Leu Glu Ala Ala Arg
   170 175 180
   ctt gac ccg ggc tta cag gca aac aca agg gtc ttg agt ttt ggt ctc 691
   Leu Asp Pro Gly Leu Gln Ala Asn Thr Arg Val Leu Ser Phe Gly Leu
   185 190 195
   gaa gct cag cag cag cac gtt gct gct ccc atg aca cct gtt atg aag 739
   Glu Ala Gln Gln Gln His Val Ala Ala Pro Met Thr Pro Val Met Lys
   200 205 210
   cta caa gaa gat caa aag gtc tca aac ggt gct agg aac agg ttt cac 787
   Leu Gln Glu Asp Gln Lys Val Ser Asn Gly Ala Arg Asn Arg Phe His
   215                 220                 225                 230
   ggg tta gcg gat caa cgg ctt gta gct cgg gta gag aat gga gat gat      835
   Gly Leu Ala Asp Gln Arg Leu Val Ala Arg Val Glu Asn Gly Asp Asp
               235                 240                 245
   tgc tca agc gga tcc tct gtg acc acc tct aac aat cac tca gtg gat      883
   Cys Ser Ser Gly Ser Ser Val Thr Thr Ser Asn Asn His Ser Val Asp
           250                 255                 260
   gaa tca aga gca caa agc ggc agt gtt gtt gaa gca caa atg aac aac      931
   Glu Ser Arg Ala Gln Ser Gly Ser Val Val Glu Ala Gln Met Asn Asn
           265                 270                 275
   aac aac aac aat aac atg aat ggt tat gct tgc atc cca ggt gtt cca      979
   Asn Asn Asn Asn Asn Met Asn Gly Tyr Ala Cys Ile Pro Gly Val Pro
       280                 285                 290
   tgg cct tac acg tgg aat cca gcg atg cct cca cca ggt ttt tac ccg     1027
   Trp Pro Tyr Thr Trp Asn Pro Ala Met Pro Pro Pro Gly Phe Tyr Pro
   295                 300                 305                 310
   cct cca ggg tat cca atg ccg ttt tac cct tac tgg acc atc cca atg     1075
   Pro Pro Gly Tyr Pro Met Pro Phe Tyr Pro Tyr Trp Thr Ile Pro Met
                   315                 320                 325
   cta cca ccg cat caa tcc tca tcg cct ata agc caa aag tgt tca aat     1123
   Leu Pro Pro His Gln Ser Ser Ser Pro Ile Ser Gln Lys Cys Ser Asn
               330                 335                 340
   aca aac tct ccg act ctc gga aag cat ccg aga gat gaa gga tca tcg     1171
   Thr Asn Ser Pro Thr Leu Gly Lys His Pro Arg Asp Glu Gly Ser Ser
           345                 350                 355
   aaa aag gac aat gag aca gag cga aaa cag aag gcc ggg tgc gtt ctg     1219
   Lys Lys Asp Asn Glu Thr Glu Arg Lys Gln Lys Ala Gly Cys Val Leu
       360                 365                 370
   gtc ccg aaa acg ttg aga ata gat gat cct aac gaa gca gca aag agc     1267
   Val Pro Lys Thr Leu Arg Ile Asp Asp Pro Asn Glu Ala Ala Lys Ser
   375                 380                 385                 390
   tcg ata tgg aca aca ttg gga atc aag aac gag gcg atg tgc aaa gcc     1315
   Ser Ile Trp Thr Thr Leu Gly Ile Lys Asn Glu Ala Met Cys Lys Ala
                   395                 400                 405
   ggt ggt atg ttc aaa ggg ttt gat cat aag aca aag atg tat aac aac     1363
   Gly Gly Met Phe Lys Gly Phe Asp His Lys Thr Lys Met Tyr Asn Asn
               410                 415                 420
   gac aaa gct gag aac tcc cct gtt ctt tct gct aac cct gct gct cta     1411
   Asp Lys Ala Glu Asn Ser Pro Val Leu Ser Ala Asn Pro Ala Ala Leu
           425                 430                 435
   tca aga tca cac aat ttc cat gaa cag att tag agttacatat gtatatgtat   1464
   Ser Arg Ser His Asn Phe His Glu Gln Ile
       440                 445
   atatgtatga ttgattgtat gtatagatga tactggagaa tgatgagttt ttgagaatca   1524
   aactcttttc ttctttctag tgattgcctt tattccttta catgttttgg ttctctgtac   1584
   actatttgat ttaccttttt tactttcttt cttcatttgt caggaaatgt tggaagataa   1644
   cattaatggt aaaaagttgg tgtggaccgt tgttgcgttg gcatttcaaa aaaaaaaaaa 1704
   aaa 1707

<210> 38
<211> 448
<212> PRT
<213> Arabidopsis thaliana

<400> 38

Met Met Met Glu Thr Arg Asp Pro Ala Ile Lys Leu Phe Gly Met Lys
1               5                   10                  15

Ile Pro Phe Pro Ser Val Phe Glu Ser Ala Val Thr Val Glu Asp Asp
            20                  25                  30

Glu Glu Asp Asp Trp Ser Gly Gly Asp Asp Lys Ser Pro Glu Lys Val
        35                  40                  45

Thr Pro Glu Leu Ser Asp Lys Asn Asn Asn Asn Cys Asn Asp Asn Ser
    50                  55                  60

Phe Asn Asn Ser Lys Pro Glu Thr Leu Asp Lys Glu Glu Ala Thr Ser
65                  70                  75                  80

Thr Asp Gln Ile Glu Ser Ser Asp Thr Pro Glu Asp Asn Gln Gln Thr
                85                  90                  95

Thr Pro Asp Gly Lys Thr Leu Lys Lys Pro Thr Lys Ile Leu Pro Cys
            100                 105                 110

Pro Arg Cys Lys Ser Met Glu Thr Lys Phe Cys Tyr Tyr Asn Asn Tyr
        115                 120                 125

Asn Ile Asn Gln Pro Arg His Phe Cys Lys Ala Cys Gln Arg Tyr Trp
    130                 135                 140

Thr Ala Gly Gly Thr Met Arg Asn Val Pro Val Gly Ala Gly Arg Arg
145                 150                 155                 160

Lys Asn Lys Ser Ser Ser Ser His Tyr Arg His Ile Thr Ile Ser Glu
                165                 170                 175

Ala Leu Glu Ala Ala Arg Leu Asp Pro Gly Leu Gln Ala Asn Thr Arg
            180                 185                 190

Val Leu Ser Phe Gly Leu Glu Ala Gln Gln Gln His Val Ala Ala Pro
        195                 200                 205

Met Thr Pro Val Met Lys Leu Gln Glu Asp Gln Lys Val Ser Asn Gly
    210                 215                 220

Ala Arg Asn Arg Phe His Gly Leu Ala Asp Gln Arg Leu Val Ala Arg
225                 230                 235                 240

Val Glu Asn Gly Asp Asp Cys Ser Ser Gly Ser Ser Val Thr Thr Ser
                245                 250                 255

Asn Asn His Ser Val Asp Glu Ser Arg Ala Gln Ser Gly Ser Val Val
            260                 265                 270

Glu Ala Gln Met Asn Asn Asn Asn Asn Asn Asn Met Asn Gly Tyr Ala
        275                 280                 285

Cys Ile Pro Gly Val Pro Trp Pro Tyr Thr Trp Asn Pro Ala Met Pro
    290                 295                 300

Pro Pro Gly Phe Tyr Pro Pro Pro Gly Tyr Pro Met Pro Phe Tyr Pro
305                 310                 315                 320

Tyr Trp Thr Ile Pro Met Leu Pro Pro His Gln Ser Ser Ser Pro Ile
                325                 330                 335

Ser Gln Lys Cys Ser Asn Thr Asn Ser Pro Thr Leu Gly Lys His Pro
            340                 345                 350

Arg Asp Glu Gly Ser Ser Lys Lys Asp Asn Glu Thr Glu Arg Lys Gln
        355                 360                 365

Lys Ala Gly Cys Val Leu Val Pro Lys Thr Leu Arg Ile Asp Asp Pro
    370                 375                 380

Asn Glu Ala Ala Lys Ser Ser Ile Trp Thr Thr Leu Gly Ile Lys Asn
385                 390                 395                 400

Glu Ala Met Cys Lys Ala Gly Gly Met Phe Lys Gly Phe Asp His Lys
                405                 410                 415

Thr Lys Met Tyr Asn Asn Asp Lys Ala Glu Asn Ser Pro Val Leu Ser
            420                 425                 430

Ala Asn Pro Ala Ala Leu Ser Arg Ser His Asn Phe His Glu Gln Ile
        435                 440                 445

<210>  39

<211>  1095
<212>  DNA
<213>  Arabidopsis thaliana

<220>
<221>  CDS
<222>  (180)..(917)
<223>  G994


<400>  39
tgtatatata gttagttagt tgagataaac ttggttacca cttttgtgtg gtctttcttt      60

ttctttttct ccattttcca tttatcgacc ccttgggtgt agctaattac tttcgcgatt     120

ttcaaatcca ataaagtttt aatttgatga agcttttttt aaaccatata atataaata      179

atg ggt ggt cgt aaa cca tgt tgt gat gag gtt gga tta aga aag ggt      227
Met Gly Gly Arg Lys Pro Cys Cys Asp Glu Val Gly Leu Arg Lys Gly
1               5                   10                  15

cca tgg aca gtg gaa gaa gat ggg aaa cta gtt gat ttc tta agg gca      275
Pro Trp Thr Val Glu Glu Asp Gly Lys Leu Val Asp Phe Leu Arg Ala
            20                  25                  30

cgt ggc aac tgc ggt ggt ggt gga gga gga tgg tgc tgg aga gac gtg      323
Arg Gly Asn Cys Gly Gly Gly Gly Gly Gly Trp Cys Trp Arg Asp Val
        35                  40                  45

cca aaa ctg gcg ggg cta agg agg tgt ggc aaa agt tgc cgt ctc cgg      371
Pro Lys Leu Ala Gly Leu Arg Arg Cys Gly Lys Ser Cys Arg Leu Arg
    50                  55                  60

tgg act aat tat ctc cgg cca gat ctc aag aga ggt ctt ttt act gaa      419
Trp Thr Asn Tyr Leu Arg Pro Asp Leu Lys Arg Gly Leu Phe Thr Glu
65                  70                  75                  80

gaa gaa atc caa cta gtc att gat ctt cat gct cgc ctt ggc aat aga      467
Glu Glu Ile Gln Leu Val Ile Asp Leu His Ala Arg Leu Gly Asn Arg
                85                  90                  95

tgg tcg aag att gca gtg gag tta cca gga aga aca gac aac gat atc      515
Trp Ser Lys Ile Ala Val Glu Leu Pro Gly Arg Thr Asp Asn Asp Ile
            100                 105                 110

aaa aat tat tgg aac act cat ata aag agg aag ctt ata aga atg ggt      563
Lys Asn Tyr Trp Asn Thr His Ile Lys Arg Lys Leu Ile Arg Met Gly
        115                 120                 125

att gat cca aac aca cat cgt cga ttt gac caa caa aaa gtc aac gag      611
Ile Asp Pro Asn Thr His Arg Arg Phe Asp Gln Gln Lys Val Asn Glu
    130                 135                 140

gag gaa acg ata ttg gtc aac gat cca aag cct ctg tct gag acc gag      659
Glu Glu Thr Ile Leu Val Asn Asp Pro Lys Pro Leu Ser Glu Thr Glu
145                 150                 155                 160

gta tct gtt gct ttg aag aat gac acg tca gca gtg tta tca gga aat      707
Val Ser Val Ala Leu Lys Asn Asp Thr Ser Ala Val Leu Ser Gly Asn
                165                 170                 175

cta aac caa ttg gct gac gtg gac ggt gat gat cag ccg tgg agc ttt      755
Leu Asn Gln Leu Ala Asp Val Asp Gly Asp Asp Gln Pro Trp Ser Phe

180                 185                 190

cta atg gaa aat gac gaa gga gga ggt ggc gac gcc gcc gga gag ctt     803
Leu Met Glu Asn Asp Glu Gly Gly Gly Gly Asp Ala Ala Gly Glu Leu
        195                 200                 205

acg atg cta ttg tcc ggt gac att acg tca tca tgt tct tct tcg tca     851
Thr Met Leu Leu Ser Gly Asp Ile Thr Ser Ser Cys Ser Ser Ser Ser
    210                 215                 220

tct ttg tgg atg aag tat gga gaa ttc gga tac gaa gat tta gaa ctt     899
Ser Leu Trp Met Lys Tyr Gly Glu Phe Gly Tyr Glu Asp Leu Glu Leu
225                 230                 235                 240

gga tgt ttc gat gtt tag agattcaagt atgtttaatt aggccgtagg            947
Gly Cys Phe Asp Val
                245

ttgattaatc ataaggttca ttgacttcat tctagaattg tgtagttgga ccagtataaa   1007

gaatcaaagt tatgaaacat tgtaatttga tttccaaatt aatctaatga ataaatgtgc   1067

tttgcaaaaa aaaaaaaaaa aaaaaaaa                                      1095

<210>  40
<211>  245
<212>  PRT
<213>  Arabidopsis thaliana

<400>  40

Met Gly Gly Arg Lys Pro Cys Cys Asp Glu Val Gly Leu Arg Lys Gly
1               5                   10                  15

Pro Trp Thr Val Glu Glu Asp Gly Lys Leu Val Asp Phe Leu Arg Ala
            20                  25                  30

Arg Gly Asn Cys Gly Gly Gly Gly Gly Gly Trp Cys Trp Arg Asp Val
        35                  40                  45

Pro Lys Leu Ala Gly Leu Arg Arg Cys Gly Lys Ser Cys Arg Leu Arg
    50                  55                  60

Trp Thr Asn Tyr Leu Arg Pro Asp Leu Lys Arg Gly Leu Phe Thr Glu
65                  70                  75                  80

Glu Glu Ile Gln Leu Val Ile Asp Leu His Ala Arg Leu Gly Asn Arg
                85                  90                  95

Trp Ser Lys Ile Ala Val Glu Leu Pro Gly Arg Thr Asp Asn Asp Ile
            100                 105                 110

Lys Asn Tyr Trp Asn Thr His Ile Lys Arg Lys Leu Ile Arg Met Gly
        115                 120                 125

Ile Asp Pro Asn Thr His Arg Arg Phe Asp Gln Gln Lys Val Asn Glu
    130                 135                 140

Glu Glu Thr Ile Leu Val Asn Asp Pro Lys Pro Leu Ser Glu Thr Glu
145                 150                 155                 160

Val Ser Val Ala Leu Lys Asn Asp Thr Ser Ala Val Leu Ser Gly Asn
                165                 170                 175

Leu Asn Gln Leu Ala Asp Val Asp Gly Asp Asp Gln Pro Trp Ser Phe
            180                 185                 190

Leu Met Glu Asn Asp Glu Gly Gly Gly Gly Asp Ala Ala Gly Glu Leu
        195                 200                 205

Thr Met Leu Leu Ser Gly Asp Ile Thr Ser Ser Cys Ser Ser Ser Ser
    210                 215                 220

Ser Leu Trp Met Lys Tyr Gly Glu Phe Gly Tyr Glu Asp Leu Glu Leu
225                 230                 235                 240

Gly Cys Phe Asp Val
                245

<210>  41
<211>  965
<212>  DNA
<213>  Arabididopsis thaliana

<220>
<221>  CDS
<222>  (56)..(667)
<223>  G1335

<400>  41
   ttttttttta aaagatttag agagaaaagt gagttattaa gagattccaa tcaaa atg      58
                                                                Met
                                                                1
   agc gga gac aac ggc ggt ggt gag agg cgc aaa ggc tcc gtc aag tgg      106
   Ser Gly Asp Asn Gly Gly Gly Glu Arg Arg Lys Gly Ser Val Lys Trp
               5                   10                  15
   ttt gat acc cag aag ggt ttc ggc ttc atc act cct gac gac ggt ggc      154
   Phe Asp Thr Gln Lys Gly Phe Gly Phe Ile Thr Pro Asp Asp Gly Gly
           20                  25                  30
   gac gat ctc ttc gtt cac cag tcc tcc atc aga tct gag ggt ttc cgt      202
   Asp Asp Leu Phe Val His Gln Ser Ser Ile Arg Ser Glu Gly Phe Arg
       35                  40                  45
   agc ctc gct gcc gaa gaa gcc gta gag ttc gag gtt gag atc gac aac      250
   Ser Leu Ala Ala Glu Glu Ala Val Glu Phe Glu Val Glu Ile Asp Asn
   50                    55                    60                    65
   aac aac cgt ccc aag gcc atc gat gtt tct gga ccc gac ggc gct ccc     298
   Asn Asn Arg Pro Lys Ala Ile Asp Val Ser Gly Pro Asp Gly Ala Pro
                   70                  75                  80
   gtc caa gga aac agc ggt ggt ggt tca tct ggc gga cgc ggc ggt ttc     346
   Val Gln Gly Asn Ser Gly Gly Gly Ser Ser Gly Gly Arg Gly Gly Phe
               85                  90                  95
   ggt gga gga aga gga ggt gga cgc gga tct gga ggt gga tac ggc ggt     394
   Gly Gly Gly Arg Gly Gly Gly Arg Gly Ser Gly Gly Gly Tyr Gly Gly
           100                 105                 110
   ggc ggt ggt gga tac gga gga aga gga ggt ggt ggt cga gga ggc agc     442
   Gly Gly Gly Gly Tyr Gly Gly Arg Gly Gly Gly Gly Arg Gly Gly Ser
       115                 120                 125
   gac tgc tac aag tgt ggt gag ccc ggt cac atg gcg aga gac tgt tct     490
   Asp Cys Tyr Lys Cys Gly Glu Pro Gly His Met Ala Arg Asp Cys Ser
   130                 135                 140                 145
   gaa ggc ggt gga ggt tac gga gga ggc ggc ggt ggc tac gga ggt gga     538
   Glu Gly Gly Gly Gly Tyr Gly Gly Gly Gly Gly Gly Tyr Gly Gly Gly
                   150                 155                 160
   ggc gga tac ggc gga gga ggt ggt ggt tac gga ggt ggt ggc cgt gga     586
   Gly Gly Tyr Gly Gly Gly Gly Gly Gly Tyr Gly Gly Gly Gly Arg Gly
               165                 170                 175
   ggt ggt ggc ggc ggg gga agc tgc tac agc tgt ggc gag tcg gga cat     634
   Gly Gly Gly Gly Gly Gly Ser Cys Tyr Ser Cys Gly Glu Ser Gly His
           180                 185                 190
   ttc gcc agg gat tgc acc agc ggt gga cgt taa aaccaacgcc ggttacgcgg     687
   Phe Ala Arg Asp Cys Thr Ser Gly Gly Arg
       195                 200
   tggagaagag tgagttggtt atctcacaag tgatcggttc tttctcccgc cgccttctat     747
   ctctctatta tccacttttt gcttattatg atggatctct atctttgtta gttggttttt     807
   tcttgatggt ttcggattag gactcttctt ttggttttgc tacttatggt tggttttatt     867
   tatggtactt gtgatatggg tgaaatgctc tacttgttgc tctgtttcaa gtgttcataa     927
   tatgcgaaca aatattctgg gttttgtttc aaaaaaaa                              965

<210>  42
<211>  203
<212>  PRT
<213>  Arabididopsis thaliana

<400>  42

Met Ser Gly Asp Asn Gly Gly Gly Glu Arg Arg Lys Gly Ser Val Lys
1               5                   10                  15

Trp Phe Asp Thr Gln Lys Gly Phe Gly Phe Ile Thr Pro Asp Asp Gly
            20                  25                  30

Gly Asp Asp Leu Phe Val His Gln Ser Ser Ile Arg Ser Glu Gly Phe
        35                  40                  45

Arg Ser Leu Ala Ala Glu Glu Ala Val Glu Phe Glu Val Glu Ile Asp
    50                  55                  60

Asn Asn Asn Arg Pro Lys Ala Ile Asp Val Ser Gly Pro Asp Gly Ala
65                  70                  75                  80

Pro Val Gln Gly Asn Ser Gly Gly Gly Ser Ser Gly Gly Arg Gly Gly
                85                  90                  95

Phe Gly Gly Gly Arg Gly Gly Gly Arg Gly Ser Gly Gly Gly Tyr Gly
            100                 105                 110

Gly Gly Gly Gly Gly Tyr Gly Gly Arg Gly Gly Gly Gly Arg Gly Gly
        115                 120                 125

Ser Asp Cys Tyr Lys Cys Gly Glu Pro Gly His Met Ala Arg Asp Cys
    130                 135                 140

Ser Glu Gly Gly Gly Gly Tyr Gly Gly Gly Gly Gly Gly Tyr Gly Gly
145                 150                 155                 160

Gly Gly Gly Tyr Gly Gly Gly Gly Gly Gly Tyr Gly Gly Gly Gly Arg
                165                 170                 175

Gly Gly Gly Gly Gly Gly Gly Ser Cys Tyr Ser Cys Gly Glu Ser Gly
            180                 185                 190

His Phe Ala Arg Asp Cys Thr Ser Gly Gly Arg
        195                 200

<210>  43
<211>  1554
<212>  DNA
<213>  Arabidopsis thaliana

<220>
<221>  CDS
<222>  (137)..(1285)
<223>  G562

<400>  43
atttgaattt ctgggtttct ctctgtttaa gcttcttctt cttcatcttc tgcttacgtt      60

tcttcttcaa ggagctttcg gattcttgta gaaagagtca ttgttctctt gagtgggaaa     120

ccttgaaacc attcct atg gga aat agc agc gag gaa cca aag cct cct acc     172
                  Met Gly Asn Ser Ser Glu Glu Pro Lys Pro Pro Thr

                        1               5                   10

aaa tca gat aaa cca tct tca ccc ccg gtg gat caa aca aat gtt cat        220
Lys Ser Asp Lys Pro Ser Ser Pro Pro Val Asp Gln Thr Asn Val His
        15                  20                  25

gtc tac cct gat tgg gca gct atg cag gca tat tat ggt cca aga gta        268
Val Tyr Pro Asp Trp Ala Ala Met Gln Ala Tyr Tyr Gly Pro Arg Val
    30                  35                  40

gca atg cct cct tat tac aat tca gct atg gct gca tct ggt cat cct        316
Ala Met Pro Pro Tyr Tyr Asn Ser Ala Met Ala Ala Ser Gly His Pro
45                  50                  55                  60

cct cct cct tac atg tgg aat cct cag cat atg atg tca cca tct gga        364
Pro Pro Pro Tyr Met Trp Asn Pro Gln His Met Met Ser Pro Ser Gly
                65                  70                  75

gca ccc tat gct gct gtt tat cct cat gga gga gga gtt tac gct cat        412
Ala Pro Tyr Ala Ala Val Tyr Pro His Gly Gly Gly Val Tyr Ala His
            80                  85                  90

ccc ggt att ccc atg gga tca ctg cct caa ggt caa aag gat cca cct        460
Pro Gly Ile Pro Met Gly Ser Leu Pro Gln Gly Gln Lys Asp Pro Pro
        95                  100                 105

tta aca act ccg ggg acg ctt ttg agc atc gac act cct act aaa tct        508
Leu Thr Thr Pro Gly Thr Leu Leu Ser Ile Asp Thr Pro Thr Lys Ser
    110                 115                 120

aca ggg aac aca gac aat gga ttg atg aag aag ctg aaa gag ttt gat        556
Thr Gly Asn Thr Asp Asn Gly Leu Met Lys Lys Leu Lys Glu Phe Asp
125                 130                 135                 140

ggg ctt gct atg tct cta gga aat ggg aat cct gaa aat ggt gca gat        604
Gly Leu Ala Met Ser Leu Gly Asn Gly Asn Pro Glu Asn Gly Ala Asp
                145                 150                 155

gaa cat aaa cga tca cgg aac agc tca gaa act gat ggt tct act gat        652
Glu His Lys Arg Ser Arg Asn Ser Ser Glu Thr Asp Gly Ser Thr Asp
            160                 165                 170

gga agt gat ggg aat aca act ggg gca gat gaa ccg aaa ctt aaa aga        700
Gly Ser Asp Gly Asn Thr Thr Gly Ala Asp Glu Pro Lys Leu Lys Arg
        175                 180                 185

agt cga gag gga act cca aca aaa gat ggg aaa caa ttg gtt caa gct        748
Ser Arg Glu Gly Thr Pro Thr Lys Asp Gly Lys Gln Leu Val Gln Ala
    190                 195                 200

agc tca ttt cat tct gtt tct ccg tca agt ggt gat acc ggc gta aaa        796
Ser Ser Phe His Ser Val Ser Pro Ser Ser Gly Asp Thr Gly Val Lys
205                 210                 215                 220

ctc att caa gga tct gga gct ata ctc tct cct ggt gta agt gca aat        844
Leu Ile Gln Gly Ser Gly Ala Ile Leu Ser Pro Gly Val Ser Ala Asn
                225                 230                 235

tcc aac ccc ttc atg tca caa tct tta gcc atg gtt cct cct gaa act        892
Ser Asn Pro Phe Met Ser Gln Ser Leu Ala Met Val Pro Pro Glu Thr
            240                 245                 250

tgg ctt cag aac gag aga gaa ctg aaa cgg gag cga agg aaa cag tct        940

Trp Leu Gln Asn Glu Arg Glu Leu Lys Arg Glu Arg Arg Lys Gln Ser
        255                 260                 265

aat aga gaa tct gct aga agg tca aga tta agg aaa cag gcc gag aca 988
Asn Arg Glu Ser Ala Arg Arg Ser Arg Leu Arg Lys Gln Ala Glu Thr
    270                 275                 280

gaa gaa ctt gct agg aaa gtg gaa gcc ttg aca gcc gaa aac atg gca 1036
Glu Glu Leu Ala Arg Lys Val Glu Ala Leu Thr Ala Glu Asn Met Ala
285                 290                 295                 300

tta aga tct gaa cta aac caa ctt aat gag aaa tct gat aaa cta aga 1084
Leu Arg Ser Glu Leu Asn Gln Leu Asn Glu Lys Ser Asp Lys Leu Arg
                305                 310                 315

gga gca aat gca acc ttg ttg gac aaa ctg aaa tgc tcg gaa ccc gaa 1132
Gly Ala Asn Ala Thr Leu Leu Asp Lys Leu Lys Cys Ser Glu Pro Glu
            320                 325                 330

aag aga gtc ccc gca aat atg ttg tct aga gtt aag aac tca gga gct 1180
Lys Arg Val Pro Ala Asn Met Leu Ser Arg Val Lys Asn Ser Gly Ala
        335                 340                 345

gga gat aag aac aag aac caa gga gac aat gat tct aac tct aca agc 1228
Gly Asp Lys Asn Lys Asn Gln Gly Asp Asn Asp Ser Asn Ser Thr Ser
    350                 355                 360

aaa ttc cat caa ctg ctc gat acg aag cct cga gct aaa gca gta gct 1276
Lys Phe His Gln Leu Leu Asp Thr Lys Pro Arg Ala Lys Ala Val Ala
365                 370                 375                 380

gca ggc tga atcgatggta attcatgtcg atttctactt aatttgtcga 1325
Ala Gly

cataaacaaa gaaaataagt gctactaatt tcagaaaaac ttgatagata gatagtatag 1385

tagagagaga gagagagaga gaggtgtgat gattattgat ctataaattt tcggagagag 1445

agagggagaa agagaaactt ttcctccaga tgaaaatttg gtgttatggt ttgttactgt 1505

taatatagag aggcttttct ttttttataa aatggcttcc tttgttgca 1554

<210> 44
<211> 382
<212> PRT
<213> Arabidopsis thaliana

<400> 44

Met Gly Asn Ser Ser Glu Glu Pro Lys Pro Pro Thr Lys Ser Asp Lys
1               5                   10                  15

Pro Ser Ser Pro Pro Val Asp Gln Thr Asn Val His Val Tyr Pro Asp
            20                  25                  30

Trp Ala Ala Met Gln Ala Tyr Tyr Gly Pro Arg Val Ala Met Pro Pro
        35                  40                  45

Tyr Tyr Asn Ser Ala Met Ala Ala Ser Gly His Pro Pro Pro Pro Tyr
    50                  55                  60

Met Trp Asn Pro Gln His Met Met Ser Pro Ser Gly Ala Pro Tyr Ala
65                  70                  75                  80

Ala Val Tyr Pro His Gly Gly Gly Val Tyr Ala His Pro Gly Ile Pro
                85                  90                  95

Met Gly Ser Leu Pro Gln Gly Gln Lys Asp Pro Pro Leu Thr Thr Pro
            100                 105                 110

Gly Thr Leu Leu Ser Ile Asp Thr Pro Thr Lys Ser Thr Gly Asn Thr
        115                 120                 125

Asp Asn Gly Leu Met Lys Lys Leu Lys Glu Phe Asp Gly Leu Ala Met
    130                 135                 140

Ser Leu Gly Asn Gly Asn Pro Glu Asn Gly Ala Asp Glu His Lys Arg
145                 150                 155                 160

Ser Arg Asn Ser Ser Glu Thr Asp Gly Ser Thr Asp Gly Ser Asp Gly
                165                 170                 175

Asn Thr Thr Gly Ala Asp Glu Pro Lys Leu Lys Arg Ser Arg Glu Gly
            180                 185                 190

Thr Pro Thr Lys Asp Gly Lys Gln Leu Val Gln Ala Ser Ser Phe His
        195                 200                 205

Ser Val Ser Pro Ser Ser Gly Asp Thr Gly Val Lys Leu Ile Gln Gly
    210                 215                 220

Ser Gly Ala Ile Leu Ser Pro Gly Val Ser Ala Asn Ser Asn Pro Phe
225                 230                 235                 240

Met Ser Gln Ser Leu Ala Met Val Pro Pro Glu Thr Trp Leu Gln Asn
                245                 250                 255

Glu Arg Glu Leu Lys Arg Glu Arg Arg Lys Gln Ser Asn Arg Glu Ser
            260                 265                 270

Ala Arg Arg Ser Arg Leu Arg Lys Gln Ala Glu Thr Glu Glu Leu Ala
        275                 280                 285

Arg Lys Val Glu Ala Leu Thr Ala Glu Asn Met Ala Leu Arg Ser Glu
    290                 295                 300

Leu Asn Gln Leu Asn Glu Lys Ser Asp Lys Leu Arg Gly Ala Asn Ala
305                 310                 315                 320

Thr Leu Leu Asp Lys Leu Lys Cys Ser Glu Pro Glu Lys Arg Val Pro
                325                 330                 335

Ala Asn Met Leu Ser Arg Val Lys Asn Ser Gly Ala Gly Asp Lys Asn
            340                 345                 350

Lys Asn Gln Gly Asp Asn Asp Ser Asn Ser Thr Ser Lys Phe His Gln
        355                 360                 365

Leu Leu Asp Thr Lys Pro Arg Ala Lys Ala Val Ala Ala Gly
    370                 375                 380

<210>  45
<211>  513
<212>  DNA
<213>  Arabidopsis thaliana

<220>
<221>  CDS
<222>  (1)..(513)
<223>  G736

<400>  45
atg gcg act caa gat tct caa ggg att aaa ctc ttt ggc aaa act att      48
Met Ala Thr Gln Asp Ser Gln Gly Ile Lys Leu Phe Gly Lys Thr Ile
1               5                   10                  15

gca ttt aac act cga aca ata aaa aat gaa gaa gag aca cac ccg ccg      96
Ala Phe Asn Thr Arg Thr Ile Lys Asn Glu Glu Glu Thr His Pro Pro
            20                  25                  30

gag caa gaa gcc aca ata gcc gtt aga tca tca tca tca tcg gat ctg      144
Glu Gln Glu Ala Thr Ile Ala Val Arg Ser Ser Ser Ser Ser Asp Leu
        35                  40                  45

acg gcc gag aag cgt ccg gat aag atc ata gca tgt cca aga tgc aag      192
Thr Ala Glu Lys Arg Pro Asp Lys Ile Ile Ala Cys Pro Arg Cys Lys
    50                  55                  60

agc atg gag aca aag ttc tgt tac ttc aac aac tac aac ggt aat cag      240
Ser Met Glu Thr Lys Phe Cys Tyr Phe Asn Asn Tyr Asn Gly Asn Gln
65                  70                  75                  80

cct cga cac ttt tgt aaa ggc tgc cac cgt tac tgg acc gcc ggt ggt      288
Pro Arg His Phe Cys Lys Gly Cys His Arg Tyr Trp Thr Ala Gly Gly
                85                  90                  95

gca ctc cgg aac gtt ccc gtc ggc gcc ggt cgt cgg aag tcc aaa cca      336
Ala Leu Arg Asn Val Pro Val Gly Ala Gly Arg Arg Lys Ser Lys Pro
            100                 105                 110

cct ggt cgt gtc gtg gtt ggt atg ctt gga gat gga aat ggt gtt cgc      384
Pro Gly Arg Val Val Val Gly Met Leu Gly Asp Gly Asn Gly Val Arg

115                 120                 125

caa gtc gag ctt ata aat ggc ttg ctc gtt gag gag tgg cag cat gcc          432
Gln Val Glu Leu Ile Asn Gly Leu Leu Val Glu Glu Trp Gln His Ala
    130                 135                 140

gca gcc gca gct cac ggt agt ttc cgg cat gat ttt ccc atg aag cgg          480
Ala Ala Ala Ala His Gly Ser Phe Arg His Asp Phe Pro Met Lys Arg
145                 150                 155                 160

ctc cgg tgt tac tcc gac ggt caa tcg tgc tga                              513
Leu Arg Cys Tyr Ser Asp Gly Gln Ser Cys
                165                 170

<210>  46
<211>  170
<212>  PRT
<213>  Arabidopsis thaliana

<400>  46

Met Ala Thr Gln Asp Ser Gln Gly Ile Lys Leu Phe Gly Lys Thr Ile
1               5                   10                  15

Ala Phe Asn Thr Arg Thr Ile Lys Asn Glu Glu Glu Thr His Pro Pro
            20                  25                  30

Glu Gln Glu Ala Thr Ile Ala Val Arg Ser Ser Ser Ser Ser Asp Leu
        35                  40                  45

Thr Ala Glu Lys Arg Pro Asp Lys Ile Ile Ala Cys Pro Arg Cys Lys
    50                  55                  60

Ser Met Glu Thr Lys Phe Cys Tyr Phe Asn Asn Tyr Asn Gly Asn Gln
65                  70                  75                  80

Pro Arg His Phe Cys Lys Gly Cys His Arg Tyr Trp Thr Ala Gly Gly
                85                  90                  95

Ala Leu Arg Asn Val Pro Val Gly Ala Gly Arg Arg Lys Ser Lys Pro
            100                 105                 110

Pro Gly Arg Val Val Val Gly Met Leu Gly Asp Gly Asn Gly Val Arg
        115                 120                 125

Gln Val Glu Leu Ile Asn Gly Leu Leu Val Glu Glu Trp Gln His Ala
    130                 135                 140

Ala Ala Ala Ala His Gly Ser Phe Arg His Asp Phe Pro Met Lys Arg
145                 150                 155                 160

Leu Arg Cys Tyr Ser Asp Gly Gln Ser Cys

165                 170

<210>  47
<211>  974
<212>  DNA
<213>  Arabidopsis thaliana

<220>
<221>  CDS
<222>  (62)..(874)
<223>  G1073

<400>  47
ccccccgacc tgcctctaca gagacctgaa gattccagaa ccccacctga tcaaaaataa      60

c atg gaa ctt aac aga tct gaa gca gac gaa gca aag gcc gag acc act      109
  Met Glu Leu Asn Arg Ser Glu Ala Asp Glu Ala Lys Ala Glu Thr Thr
  1               5                   10                  15

ccc acc ggt gga gcc acc agc tca gcc aca gcc tct ggc tct tcc tcc      157
Pro Thr Gly Gly Ala Thr Ser Ser Ala Thr Ala Ser Gly Ser Ser Ser
            20                  25                  30

gga cgt cgt cca cgt ggt cgt cct gca ggt tcc aaa aac aaa ccc aaa      205
Gly Arg Arg Pro Arg Gly Arg Pro Ala Gly Ser Lys Asn Lys Pro Lys
        35                  40                  45

cct ccg acg att ata act aga gat agt cct aac gtc ctt aga tca cac      253
Pro Pro Thr Ile Ile Thr Arg Asp Ser Pro Asn Val Leu Arg Ser His
    50                  55                  60

gtt ctt gaa gtc acc tcc ggt tcg gac ata tcc gag gca gtc tcc acc      301
Val Leu Glu Val Thr Ser Gly Ser Asp Ile Ser Glu Ala Val Ser Thr
65                  70                  75                  80

tac gcc act cgt cgc ggc tgc ggc gtt tgc att ata agc ggc acg ggt      349
Tyr Ala Thr Arg Arg Gly Cys Gly Val Cys Ile Ile Ser Gly Thr Gly
                85                  90                  95

gcg gtc act aac gtc acg ata cgg caa cct gcg gct ccg gct ggt gga      397
Ala Val Thr Asn Val Thr Ile Arg Gln Pro Ala Ala Pro Ala Gly Gly
            100                 105                 110

ggt gtg att acc ctg cat ggt cgg ttt gac att ttg tct ttg acc ggt      445
Gly Val Ile Thr Leu His Gly Arg Phe Asp Ile Leu Ser Leu Thr Gly
        115                 120                 125

act gcg ctt cca ccg cct gca cca ccg gga gca gga ggt ttg acg gtg      493
Thr Ala Leu Pro Pro Pro Ala Pro Pro Gly Ala Gly Gly Leu Thr Val
    130                 135                 140

tat cta gcc gga ggt caa gga caa gtt gta gga ggg aat gtg gct ggt      541
Tyr Leu Ala Gly Gly Gln Gly Gln Val Val Gly Gly Asn Val Ala Gly
145                 150                 155                 160

tcg tta att gct tcg gga ccg gta gtg ttg atg gct gct tct ttt gca      589
Ser Leu Ile Ala Ser Gly Pro Val Val Leu Met Ala Ala Ser Phe Ala
                165                 170                 175

aac gca gtt tat gat agg tta ccg att gaa gag gaa gaa acc cca ccg      637
Asn Ala Val Tyr Asp Arg Leu Pro Ile Glu Glu Glu Glu Thr Pro Pro

180                 185                 190

ccg aga acc acc ggg gtg cag cag cag cag ccg gag gcg tct cag tcg      685
Pro Arg Thr Thr Gly Val Gln Gln Gln Gln Pro Glu Ala Ser Gln Ser
        195                 200                 205

tcg gag gtt acg ggg agt ggg gcc cag gcg tgt gag tca aac ctc caa      733
Ser Glu Val Thr Gly Ser Gly Ala Gln Ala Cys Glu Ser Asn Leu Gln
    210                 215                 220

ggt gga aat ggt gga gga ggt gtt gct ttc tac aat ctt gga atg aat      781
Gly Gly Asn Gly Gly Gly Gly Val Ala Phe Tyr Asn Leu Gly Met Asn
225                 230                 235                 240

atg aac aat ttt caa ttc tcc ggg gga gat att tac ggt atg agc ggc      829
Met Asn Asn Phe Gln Phe Ser Gly Gly Asp Ile Tyr Gly Met Ser Gly
                245                 250                 255

ggt agc gga gga ggt ggt ggc ggt gcg act aga ccc gcg ttt tag          874
Gly Ser Gly Gly Gly Gly Gly Gly Ala Thr Arg Pro Ala Phe
            260                 265                 270

agttttagcg ttttggtgac accttttgtt gcgtttgcgt gtttgacctc aaactactag    934

gctactagct atagcggttg cgaaatgcga atattaggtt                          974

<210>  48
<211>  270
<212>  PRT
<213>  Arabidopsis thaliana

<400>  48

Met Glu Leu Asn Arg Ser Glu Ala Asp Glu Ala Lys Ala Glu Thr Thr
1               5                   10                  15

Pro Thr Gly Gly Ala Thr Ser Ser Ala Thr Ala Ser Gly Ser Ser Ser
            20                  25                  30

Gly Arg Arg Pro Arg Gly Arg Pro Ala Gly Ser Lys Asn Lys Pro Lys
        35                  40                  45

Pro Pro Thr Ile Ile Thr Arg Asp Ser Pro Asn Val Leu Arg Ser His
    50                  55                  60

Val Leu Glu Val Thr Ser Gly Ser Asp Ile Ser Glu Ala Val Ser Thr
65                  70                  75                  80

Tyr Ala Thr Arg Arg Gly Cys Gly Val Cys Ile Ile Ser Gly Thr Gly
                85                  90                  95

Ala Val Thr Asn Val Thr Ile Arg Gln Pro Ala Ala Pro Ala Gly Gly
            100                 105                 110

Gly Val Ile Thr Leu His Gly Arg Phe Asp Ile Leu Ser Leu Thr Gly

115                 120                 125

Thr Ala Leu Pro Pro Pro Ala Pro Pro Gly Ala Gly Gly Leu Thr Val
    130                 135                 140

Tyr Leu Ala Gly Gly Gln Gly Gln Val Val Gly Gly Asn Val Ala Gly
145                 150                 155                 160

Ser Leu Ile Ala Ser Gly Pro Val Val Leu Met Ala Ala Ser Phe Ala
                165                 170                 175

Asn Ala Val Tyr Asp Arg Leu Pro Ile Glu Glu Glu Glu Thr Pro Pro
            180                 185                 190

Pro Arg Thr Thr Gly Val Gln Gln Gln Gln Pro Glu Ala Ser Gln Ser
        195                 200                 205

Ser Glu Val Thr Gly Ser Gly Ala Gln Ala Cys Glu Ser Asn Leu Gln
    210                 215                 220

Gly Gly Asn Gly Gly Gly Gly Val Ala Phe Tyr Asn Leu Gly Met Asn
225                 230                 235                 240

Met Asn Asn Phe Gln Phe Ser Gly Gly Asp Ile Tyr Gly Met Ser Gly
                245                 250                 255

Gly Ser Gly Gly Gly Gly Gly Gly Ala Thr Arg Pro Ala Phe
            260                 265                 270

<210>  49
<211>  1281
<212>  DNA
<213>  Arabidopsis thaliana

<220>
<221>  CDS
<222>  (8)..(904)
<223>  G1435

<400>  49
gtgaaac atg ggg aag gaa gtt atg gtg agc gat tac ggt gac gac gac        49
        Met Gly Lys Glu Val Met Val Ser Asp Tyr Gly Asp Asp Asp
        1               5                   10

gga gaa gac gcc ggc ggc ggc gat gaa tat agg att ccg gaa tgg gaa        97
Gly Glu Asp Ala Gly Gly Gly Asp Glu Tyr Arg Ile Pro Glu Trp Glu
15                  20                  25                  30

att ggt tta ccc aac gga gat gat ttg act ccg tta tct caa tat cta       145
Ile Gly Leu Pro Asn Gly Asp Asp Leu Thr Pro Leu Ser Gln Tyr Leu
                35                  40                  45

gtc ccg tcg att ctc gcg tta gct ttc agc atg atc cca gaa cga agc 193
Val Pro Ser Ile Leu Ala Leu Ala Phe Ser Met Ile Pro Glu Arg Ser
            50                  55                  60

cgt aca att cac gac gtc aat cgc gcg tcg caa atc acg ctc tct tcg 241
Arg Thr Ile His Asp Val Asn Arg Ala Ser Gln Ile Thr Leu Ser Ser
        65                  70                  75

ttg aga agc agt acc aat gct tcg tct gtg atg gag gag gtc gtg gat 289
Leu Arg Ser Ser Thr Asn Ala Ser Ser Val Met Glu Glu Val Val Asp
    80                  85                  90

cga gtt gaa tcg agt gtt cca gga tca gat ccg aag aaa cag aag aaa 337
Arg Val Glu Ser Ser Val Pro Gly Ser Asp Pro Lys Lys Gln Lys Lys
95                  100                 105                 110

tcg gat ggt ggt gaa gca gcg gcg gtg gag gat tcc acg gcg gag gaa 385
Ser Asp Gly Gly Glu Ala Ala Ala Val Glu Asp Ser Thr Ala Glu Glu
                115                 120                 125

gga gac tcc ggg cct gaa gac gcg tct ggg aag aca tcg aaa cga ccg 433
Gly Asp Ser Gly Pro Glu Asp Ala Ser Gly Lys Thr Ser Lys Arg Pro
            130                 135                 140

cgt tta gtg tgg aca ccg cag cta cac aag aga ttt gtg gac gtt gtg 481
Arg Leu Val Trp Thr Pro Gln Leu His Lys Arg Phe Val Asp Val Val
        145                 150                 155

gct cat cta ggg att aaa aac gca gtg ccg aag acg att atg cag ctg 529
Ala His Leu Gly Ile Lys Asn Ala Val Pro Lys Thr Ile Met Gln Leu
    160                 165                 170

atg aac gtg gaa gga ctt act cgt gag aac gtt gcg tct cat ttg cag 577
Met Asn Val Glu Gly Leu Thr Arg Glu Asn Val Ala Ser His Leu Gln
175                 180                 185                 190

aaa tat agg ctt tac ctt aaa cgg att caa gga ttg acg acg gaa gaa 625
Lys Tyr Arg Leu Tyr Leu Lys Arg Ile Gln Gly Leu Thr Thr Glu Glu
                195                 200                 205

gat cct tat tcg tcg tcg gat cag ctc ttc tct tca acg ccg gtt cct 673
Asp Pro Tyr Ser Ser Ser Asp Gln Leu Phe Ser Ser Thr Pro Val Pro
            210                 215                 220

cca cag agc ttt caa gac ggc gga gga agt aac gga aag ttg ggg gtt 721
Pro Gln Ser Phe Gln Asp Gly Gly Gly Ser Asn Gly Lys Leu Gly Val
        225                 230                 235

ccg gtt ccg gtt ccg tcg atg gtg cct att cca ggc tat ggg aat caa 769
Pro Val Pro Val Pro Ser Met Val Pro Ile Pro Gly Tyr Gly Asn Gln
    240                 245                 250

atg ggt atg caa gga tat tat caa cag tat agt aac cat ggc aat gaa 817
Met Gly Met Gln Gly Tyr Tyr Gln Gln Tyr Ser Asn His Gly Asn Glu
255                 260                 265                 270

tca aac caa tat atg atg cag cag aat aag ttt gga aca atg gtg aca 865
Ser Asn Gln Tyr Met Met Gln Gln Asn Lys Phe Gly Thr Met Val Thr
                275                 280                 285

tat cct tct gtt ggt ggt ggt gac gtg aat gac aag taa atggatctta 914
Tyr Pro Ser Val Gly Gly Gly Asp Val Asn Asp Lys
            290                 295

aaggtctata atttgctcta cagagagata ctggttcttg gcttatggtt tattttccca 974

cttcatgagg ttgttgtgac ttttaattct ccatgttttc cacacaagtc tttattgcct 1034

ttgtatagaa aatgatttcg agaaaatcac tgggaagctt ggtattgttg gaggatgaag 1094

ccttctatga atgatttagt ttcctactgt ctccattctt tatgaggtaa taaagccttc 1154

ttttgctcat cgcttgtagt cttcttaaat tcaagacagc gtcacatgtt tgttcggtta 1214

tgttaattgt ttctttcttt ggataatgaa gatagcatca ggtctcatgt ctcctcactt 1274

tgataaa 1281

<210> 50
<211> 298
<212> PRT
<213> Arabidopsis thaliana

<400> 50

Met Gly Lys Glu Val Met Val Ser Asp Tyr Gly Asp Asp Asp Gly Glu
1               5                   10                  15

Asp Ala Gly Gly Gly Asp Glu Tyr Arg Ile Pro Glu Trp Glu Ile Gly
            20                  25                  30

Leu Pro Asn Gly Asp Asp Leu Thr Pro Leu Ser Gln Tyr Leu Val Pro
        35                  40                  45

Ser Ile Leu Ala Leu Ala Phe Ser Met Ile Pro Glu Arg Ser Arg Thr
    50                  55                  60

Ile His Asp Val Asn Arg Ala Ser Gln Ile Thr Leu Ser Ser Leu Arg
65                  70                  75                  80

Ser Ser Thr Asn Ala Ser Ser Val Met Glu Glu Val Val Asp Arg Val
                85                  90                  95

Glu Ser Ser Val Pro Gly Ser Asp Pro Lys Lys Gln Lys Lys Ser Asp
            100                 105                 110

Gly Gly Glu Ala Ala Ala Val Glu Asp Ser Thr Ala Glu Glu Gly Asp
        115                 120                 125

Ser Gly Pro Glu Asp Ala Ser Gly Lys Thr Ser Lys Arg Pro Arg Leu
    130                 135                 140

Val Trp Thr Pro Gln Leu His Lys Arg Phe Val Asp Val Val Ala His
145                 150                 155                 160

Leu Gly Ile Lys Asn Ala Val Pro Lys Thr Ile Met Gln Leu Met Asn
                165                 170                 175

Val Glu Gly Leu Thr Arg Glu Asn Val Ala Ser His Leu Gln Lys Tyr
            180                 185                 190

Arg Leu Tyr Leu Lys Arg Ile Gln Gly Leu Thr Thr Glu Glu Asp Pro
        195                 200                 205

Tyr Ser Ser Ser Asp Gln Leu Phe Ser Ser Thr Pro Val Pro Pro Gln
    210                 215                 220

Ser Phe Gln Asp Gly Gly Gly Ser Asn Gly Lys Leu Gly Val Pro Val
225                 230                 235                 240

Pro Val Pro Ser Met Val Pro Ile Pro Gly Tyr Gly Asn Gln Met Gly
                245                 250                 255

Met Gln Gly Tyr Tyr Gln Gln Tyr Ser Asn His Gly Asn Glu Ser Asn
            260                 265                 270

Gln Tyr Met Met Gln Gln Asn Lys Phe Gly Thr Met Val Thr Tyr Pro
        275                 280                 285

Ser Val Gly Gly Gly Asp Val Asn Asp Lys
    290                 295

<210>  51
<211>  837
<212>  DNA
<213>  Arabidopsis thaliana

<220>
<221>  CDS
<222>  (54)..(629)
<223>  G180

<400>  51
gtaattacga tctacaacaa gtgacatcgt cgtcgacgac gattcaagag aat atg      56
                                                           Met
                                                           1

aac ttc ctc gtt cct ttt gaa gaa acc aat gtc tta acc ttt ttc tct      104
Asn Phe Leu Val Pro Phe Glu Glu Thr Asn Val Leu Thr Phe Phe Ser
            5                   10                  15

tct tct tct tcc tct tct ctt tct tct cct tct ttc ccc att cac aac      152
Ser Ser Ser Ser Ser Ser Leu Ser Ser Pro Ser Phe Pro Ile His Asn
        20                  25                  30

tct tcc tcc act act act act cat gca cct cta ggg ttt tct aat aat      200
Ser Ser Ser Thr Thr Thr Thr His Ala Pro Leu Gly Phe Ser Asn Asn
    35                  40                  45

ctt cag ggt gga gga ccc ttg gga tca aag gtg gtt aat gat gat cag     248
Leu Gln Gly Gly Gly Pro Leu Gly Ser Lys Val Val Asn Asp Asp Gln
50                  55                  60                  65

gag aat ttt gga ggt gga act aac aat gat gct cat tct aat tct tgg     296
Glu Asn Phe Gly Gly Gly Thr Asn Asn Asp Ala His Ser Asn Ser Trp
                70                  75                  80

tgg aga tca aat agt gga agt gga gat atg aag aac aaa gtg aag ata     344
Trp Arg Ser Asn Ser Gly Ser Gly Asp Met Lys Asn Lys Val Lys Ile
            85                  90                  95

agg agg aaa cta aga gag cca aga ttc tgt ttc caa acc aaa agc gat     392
Arg Arg Lys Leu Arg Glu Pro Arg Phe Cys Phe Gln Thr Lys Ser Asp
        100                 105                 110

gtt gat gtt ctt gac gat ggc tac aaa tgg cgt aaa tat ggt cag aaa     440
Val Asp Val Leu Asp Asp Gly Tyr Lys Trp Arg Lys Tyr Gly Gln Lys
    115                 120                 125

gtc gtc aag aac agc ctt cac ccc agg agt tat tac aga tgc aca cac     488
Val Val Lys Asn Ser Leu His Pro Arg Ser Tyr Tyr Arg Cys Thr His
130                 135                 140                 145

aac aac tgt agg gtg aaa aag aga gtg gag cga cta tcg gaa gat tgt     536
Asn Asn Cys Arg Val Lys Lys Arg Val Glu Arg Leu Ser Glu Asp Cys
                150                 155                 160

aga atg gtg att act act tac gaa ggt cgt cac aac cac att ccc tct     584
Arg Met Val Ile Thr Thr Tyr Glu Gly Arg His Asn His Ile Pro Ser
            165                 170                 175

gat gac tcc act tct cct gac cat gat tgt ctc tct tcc ttt taa         629
Asp Asp Ser Thr Ser Pro Asp His Asp Cys Leu Ser Ser Phe
        180                 185                 190

catctctttc tatatatcta tatatagaca gttatatgtg cacatataga tgtgtgatat     689

attgcatatt tgatattgca tgtgtttttc aagagtatgt catcagatgt tatgcatata     749

ttcttgactt gttgcttata gtatacatat gtaataatat atattgacat tggtagttca     809

tttctgttca aacaaaaaaa aaaaaaaa                                        837


<210>  52
<211>  191
<212>  PRT
<213>  Arabidopsis thaliana

<400>  52

Met Asn Phe Leu Val Pro Phe Glu Glu Thr Asn Val Leu Thr Phe Phe
1               5                   10                  15


Ser Ser Ser Ser Ser Ser Ser Leu Ser Ser Pro Ser Phe Pro Ile His
            20                  25                  30


Asn Ser Ser Ser Thr Thr Thr Thr His Ala Pro Leu Gly Phe Ser Asn
        35                  40                  45

Asn Leu Gln Gly Gly Gly Pro Leu Gly Ser Lys Val Val Asn Asp Asp
    50                  55                  60

Gln Glu Asn Phe Gly Gly Gly Thr Asn Asn Asp Ala His Ser Asn Ser
65                  70                  75                  80

Trp Trp Arg Ser Asn Ser Gly Ser Gly Asp Met Lys Asn Lys Val Lys
                85                  90                  95

Ile Arg Arg Lys Leu Arg Glu Pro Arg Phe Cys Phe Gln Thr Lys Ser
            100                 105                 110

Asp Val Asp Val Leu Asp Asp Gly Tyr Lys Trp Arg Lys Tyr Gly Gln
        115                 120                 125

Lys Val Val Lys Asn Ser Leu His Pro Arg Ser Tyr Tyr Arg Cys Thr
    130                 135                 140

His Asn Asn Cys Arg Val Lys Lys Arg Val Glu Arg Leu Ser Glu Asp
145                 150                 155                 160

Cys Arg Met Val Ile Thr Thr Tyr Glu Gly Arg His Asn His Ile Pro
                165                 170                 175

Ser Asp Asp Ser Thr Ser Pro Asp His Asp Cys Leu Ser Ser Phe
            180                 185                 190

<210>  53
<211>  1413
<212>  DNA
<213>  Arabidopsis thaliana

<220>
<221>  CDS
<222>  (121)..(1200)
<223>  G592

<400>  53
aagctattaa gatttggttt tctacaaatt tgttcttcct gaaacgtcac gagacagagc      60

ttacaagaag agaaaacaga ggaaatttcg ttgcattttt tttacatatt gattcgatta     120

atg gat tca aat aat cat ctc tac gac ccg aat ccc acc ggg tcg ggt      168
Met Asp Ser Asn Asn His Leu Tyr Asp Pro Asn Pro Thr Gly Ser Gly
1               5                   10                  15

ctt ctt cgt ttt aga tca gct ccg agc tct gtt ctc gcc gct ttt gtt      216
Leu Leu Arg Phe Arg Ser Ala Pro Ser Ser Val Leu Ala Ala Phe Val
            20                  25                  30

gac gac gac aag att ggt ttc gac tcc gat agg ttg ctt tca aga ttc      264

Asp Asp Asp Lys Ile Gly Phe Asp Ser Asp Arg Leu Leu Ser Arg Phe
        35                  40                  45

gtg acc tct aat ggc gtt aac gga gat ctg ggt tca cct aaa ttc gag 312
Val Thr Ser Asn Gly Val Asn Gly Asp Leu Gly Ser Pro Lys Phe Glu
    50                  55                  60

gat aag tct ccg gtt tcg tta acg aac acc tct gtt tca tac gcc gcc 360
Asp Lys Ser Pro Val Ser Leu Thr Asn Thr Ser Val Ser Tyr Ala Ala
65                  70                  75                  80

act ctg ccg cca ccg ccg cag ctt gag ccg tcg agt ttt ctg ggt ttg 408
Thr Leu Pro Pro Pro Pro Gln Leu Glu Pro Ser Ser Phe Leu Gly Leu
                85                  90                  95

ccg ccg cat tac ccg agg cag agt aaa ggg ata atg aac tcg gtt ggt 456
Pro Pro His Tyr Pro Arg Gln Ser Lys Gly Ile Met Asn Ser Val Gly
            100                 105                 110

ttg gat cag ttt ctc ggt atc aat aat cat cac acc aaa cca gtt gaa 504
Leu Asp Gln Phe Leu Gly Ile Asn Asn His His Thr Lys Pro Val Glu
        115                 120                 125

tct aat ctt ctc cgt caa agc agc tct cca gcc gga atg ttt act aat 552
Ser Asn Leu Leu Arg Gln Ser Ser Ser Pro Ala Gly Met Phe Thr Asn
    130                 135                 140

ctc tct gac caa aac ggt tat ggt tca atg agg aat ttg atg aat tac 600
Leu Ser Asp Gln Asn Gly Tyr Gly Ser Met Arg Asn Leu Met Asn Tyr
145                 150                 155                 160

gaa gaa gat gaa gag agt cca tct aat tcc aat gga tta aga cgc cat 648
Glu Glu Asp Glu Glu Ser Pro Ser Asn Ser Asn Gly Leu Arg Arg His
                165                 170                 175

tgc agt ctc tct tca agg cca cct tct tca ctt gga atg ctt tct caa 696
Cys Ser Leu Ser Ser Arg Pro Pro Ser Ser Leu Gly Met Leu Ser Gln
            180                 185                 190

ata cct gaa atc gca ccc gaa act aat ttt cca tat agc cat tgg aat 744
Ile Pro Glu Ile Ala Pro Glu Thr Asn Phe Pro Tyr Ser His Trp Asn
        195                 200                 205

gat cca tcc agc ttt att gat aac tta tcc tca ctt aaa aga gaa gcc 792
Asp Pro Ser Ser Phe Ile Asp Asn Leu Ser Ser Leu Lys Arg Glu Ala
    210                 215                 220

gag gac gat gga aaa ttg ttt ctc gga gct cag aac gga gag tcc ggg 840
Glu Asp Asp Gly Lys Leu Phe Leu Gly Ala Gln Asn Gly Glu Ser Gly
225                 230                 235                 240

aat cgt atg cag tta ctg tcg cat cat ttg agc cta cca aag tca tca 888
Asn Arg Met Gln Leu Leu Ser His His Leu Ser Leu Pro Lys Ser Ser
                245                 250                 255

tcg aca gcc tcg gac atg gtt tca gtg gat aag tat ctt cag cta caa 936
Ser Thr Ala Ser Asp Met Val Ser Val Asp Lys Tyr Leu Gln Leu Gln
            260                 265                 270

gat tct gtt cct tgt aaa atc aga gcc aaa cgt ggt tgc gct aca cat 984
Asp Ser Val Pro Cys Lys Ile Arg Ala Lys Arg Gly Cys Ala Thr His
        275                 280                 285

cct cga agc atc gct gaa cgg gta aga aga acg cgg ata agc gag cga     1032
Pro Arg Ser Ile Ala Glu Arg Val Arg Arg Thr Arg Ile Ser Glu Arg
    290                 295                 300

atg agg aag tta caa gag ctt gtt cct aac atg gac aag caa acc aac     1080
Met Arg Lys Leu Gln Glu Leu Val Pro Asn Met Asp Lys Gln Thr Asn
305                 310                 315                 320

act tcg gat atg ttg gat tta gct gtg gat tac atc aaa gat tta caa     1128
Thr Ser Asp Met Leu Asp Leu Ala Val Asp Tyr Ile Lys Asp Leu Gln
                325                 330                 335

aga cag tat aag att tta aac gac aac aga gct aac tgt aag tgt atg     1176
Arg Gln Tyr Lys Ile Leu Asn Asp Asn Arg Ala Asn Cys Lys Cys Met
            340                 345                 350

aac aag gag aag aag tca ata tag ggcgcaacaa agtgtgtagt agataggact     1230
Asn Lys Glu Lys Lys Ser Ile
        355

aaaaagcagg gagaaggaca agaaagaaac aatgtcatgt ctgaatattt tttagccgaa     1290

acagaccaaa ttgtctatgt aagctctcga gaaaagcatc tgcttccaac aaaattctaa     1350

gtaataaaat agtactcgat ttgttcttat ttcattatta caatgcagaa tctactaatc     1410

aaa                                                                   1413


<210>  54
<211>  359
<212>  PRT
<213>  Arabidopsis thaliana

<400>  54

Met Asp Ser Asn Asn His Leu Tyr Asp Pro Asn Pro Thr Gly Ser Gly
1               5                   10                  15

Leu Leu Arg Phe Arg Ser Ala Pro Ser Ser Val Leu Ala Ala Phe Val
            20                  25                  30

Asp Asp Asp Lys Ile Gly Phe Asp Ser Asp Arg Leu Leu Ser Arg Phe
        35                  40                  45

Val Thr Ser Asn Gly Val Asn Gly Asp Leu Gly Ser Pro Lys Phe Glu
    50                  55                  60

Asp Lys Ser Pro Val Ser Leu Thr Asn Thr Ser Val Ser Tyr Ala Ala
65                  70                  75                  80

Thr Leu Pro Pro Pro Pro Gln Leu Glu Pro Ser Ser Phe Leu Gly Leu
                85                  90                  95

Pro Pro His Tyr Pro Arg Gln Ser Lys Gly Ile Met Asn Ser Val Gly
            100                 105                 110

Leu Asp Gln Phe Leu Gly Ile Asn Asn His His Thr Lys Pro Val Glu
        115                 120                 125

Ser Asn Leu Leu Arg Gln Ser Ser Ser Pro Ala Gly Met Phe Thr Asn
    130                 135                 140

Leu Ser Asp Gln Asn Gly Tyr Gly Ser Met Arg Asn Leu Met Asn Tyr
145                 150                 155                 160

Glu Glu Asp Glu Glu Ser Pro Ser Asn Ser Asn Gly Leu Arg Arg His
                165                 170                 175

Cys Ser Leu Ser Ser Arg Pro Pro Ser Ser Leu Gly Met Leu Ser Gln
            180                 185                 190

Ile Pro Glu Ile Ala Pro Glu Thr Asn Phe Pro Tyr Ser His Trp Asn
        195                 200                 205

Asp Pro Ser Ser Phe Ile Asp Asn Leu Ser Ser Leu Lys Arg Glu Ala
    210                 215                 220

Glu Asp Asp Gly Lys Leu Phe Leu Gly Ala Gln Asn Gly Glu Ser Gly
225                 230                 235                 240

Asn Arg Met Gln Leu Leu Ser His His Leu Ser Leu Pro Lys Ser Ser
                245                 250                 255

Ser Thr Ala Ser Asp Met Val Ser Val Asp Lys Tyr Leu Gln Leu Gln
            260                 265                 270

Asp Ser Val Pro Cys Lys Ile Arg Ala Lys Arg Gly Cys Ala Thr His
        275                 280                 285

Pro Arg Ser Ile Ala Glu Arg Val Arg Arg Thr Arg Ile Ser Glu Arg
    290                 295                 300

Met Arg Lys Leu Gln Glu Leu Val Pro Asn Met Asp Lys Gln Thr Asn
305                 310                 315                 320

Thr Ser Asp Met Leu Asp Leu Ala Val Asp Tyr Ile Lys Asp Leu Gln
                325                 330                 335

Arg Gln Tyr Lys Ile Leu Asn Asp Asn Arg Ala Asn Cys Lys Cys Met
            340                 345                 350

Asn Lys Glu Lys Lys Ser Ile
        355

<210>  55
<211>  764
<212>  DNA
<213>  Arabidopsis thaliana

<220>
<221>  CDS
<222>  (15)..(725)
<223>  G208

<400>  55
cttcttcatt cacc atg gga aga tct cct tgt tgt gaa aaa gct cac aca      50
                Met Gly Arg Ser Pro Cys Cys Glu Lys Ala His Thr
                1               5                   10

aac aaa gga gct tgg act aaa gaa gaa gat caa cgt ctc gta gat tat      98
Asn Lys Gly Ala Trp Thr Lys Glu Glu Asp Gln Arg Leu Val Asp Tyr
        15                  20                  25

atc cgt aat cac ggt gaa ggt tgt tgg cgt tct ctt cct aaa tcc gct      146
Ile Arg Asn His Gly Glu Gly Cys Trp Arg Ser Leu Pro Lys Ser Ala
    30                  35                  40

gga ttg ttg cgt tgt ggt aaa agt tgt aga ttg aga tgg att aat tac      194
Gly Leu Leu Arg Cys Gly Lys Ser Cys Arg Leu Arg Trp Ile Asn Tyr
45                  50                  55                  60

ctt cgt cct gat ctt aaa cgt ggt aat ttt act gat gat gaa gat caa      242
Leu Arg Pro Asp Leu Lys Arg Gly Asn Phe Thr Asp Asp Glu Asp Gln
                65                  70                  75

atc atc atc aaa ctc cat agc tta ctc ggt aac aaa tgg tca ttg ata      290
Ile Ile Ile Lys Leu His Ser Leu Leu Gly Asn Lys Trp Ser Leu Ile
            80                  85                  90

gct gga aga tta cca gga aga aca gat aac gaa ata aag aat tat tgg      338
Ala Gly Arg Leu Pro Gly Arg Thr Asp Asn Glu Ile Lys Asn Tyr Trp
        95                  100                 105

aac act cat att aag agg aag ctt ctt agt cac ggt att gat cca caa      386
Asn Thr His Ile Lys Arg Lys Leu Leu Ser His Gly Ile Asp Pro Gln
    110                 115                 120

act cat cgt cag att aac gaa tcc aaa acg gtg tcg tct caa gtt gtt      434
Thr His Arg Gln Ile Asn Glu Ser Lys Thr Val Ser Ser Gln Val Val
125                 130                 135                 140

gtt cct att caa aac gat gcc gtt gag tat tct ttt tcc aat tta gcc      482
Val Pro Ile Gln Asn Asp Ala Val Glu Tyr Ser Phe Ser Asn Leu Ala
                145                 150                 155

gtt aaa ccg aag acg gaa aat tcc tcc gat aac gga gct tcg act agc      530
Val Lys Pro Lys Thr Glu Asn Ser Ser Asp Asn Gly Ala Ser Thr Ser
            160                 165                 170

ggc acg acg acg gac gag gat ctc cgg cag aat ggg gag tgt tat tat      578
Gly Thr Thr Thr Asp Glu Asp Leu Arg Gln Asn Gly Glu Cys Tyr Tyr
        175                 180                 185

agt gat aat tca gga cat ata aag ctg aat ttg gat tta act ctt ggg      626

Ser Asp Asn Ser Gly His Ile Lys Leu Asn Leu Asp Leu Thr Leu Gly
    190                 195                 200

ttt gga tcc tgg tcg ggt cgg ata gtc gga gtc ggg tca tcg gct gat        674
Phe Gly Ser Trp Ser Gly Arg Ile Val Gly Val Gly Ser Ser Ala Asp
205                 210                 215                 220

tct aaa ccg tgg tgc gac ccg gtg atg gag gcg cgt ttg tca ctg ttg        722
Ser Lys Pro Trp Cys Asp Pro Val Met Glu Ala Arg Leu Ser Leu Leu
                225                 230                 235

taa taatttgtca aaaaaatccc aaaaaatggg tttgttaaa                         764

<210>  56
<211>  236
<212>  PRT
<213>  Arabidopsis thaliana

<400>  56

Met Gly Arg Ser Pro Cys Cys Glu Lys Ala His Thr Asn Lys Gly Ala
1               5                   10                  15

Trp Thr Lys Glu Glu Asp Gln Arg Leu Val Asp Tyr Ile Arg Asn His
            20                  25                  30

Gly Glu Gly Cys Trp Arg Ser Leu Pro Lys Ser Ala Gly Leu Leu Arg
        35                  40                  45

Cys Gly Lys Ser Cys Arg Leu Arg Trp Ile Asn Tyr Leu Arg Pro Asp
    50                  55                  60

Leu Lys Arg Gly Asn Phe Thr Asp Asp Glu Asp Gln Ile Ile Ile Lys
65                  70                  75                  80

Leu His Ser Leu Leu Gly Asn Lys Trp Ser Leu Ile Ala Gly Arg Leu
                85                  90                  95

Pro Gly Arg Thr Asp Asn Glu Ile Lys Asn Tyr Trp Asn Thr His Ile
            100                 105                 110

Lys Arg Lys Leu Leu Ser His Gly Ile Asp Pro Gln Thr His Arg Gln
        115                 120                 125

Ile Asn Glu Ser Lys Thr Val Ser Ser Gln Val Val Val Pro Ile Gln
    130                 135                 140

Asn Asp Ala Val Glu Tyr Ser Phe Ser Asn Leu Ala Val Lys Pro Lys
145                 150                 155                 160

Thr Glu Asn Ser Ser Asp Asn Gly Ala Ser Thr Ser Gly Thr Thr Thr
                165                 170                 175

Asp Glu Asp Leu Arg Gln Asn Gly Glu Cys Tyr Tyr Ser Asp Asn Ser
            180                 185                 190

Gly His Ile Lys Leu Asn Leu Asp Leu Thr Leu Gly Phe Gly Ser Trp
        195                 200                 205

Ser Gly Arg Ile Val Gly Val Gly Ser Ser Ala Asp Ser Lys Pro Trp
    210                 215                 220

Cys Asp Pro Val Met Glu Ala Arg Leu Ser Leu Leu
225                 230                 235

<210>  57
<211>  28
<212>  DNA
<213>  synthetic oligonucleotide

<220>
<221>  misc_feature
<222>  ()..()
<223>  O25908

<400>  57
ggcataaccc ttatcggaga tttgaagc                                        28

<210>  58
<211>  28
<212>  DNA
<213>  synthetic oligonucleotide

<220>
<221>  misc_feature
<222>  ()..()
<223>  O25910

<400>  58
acacaaactc tgatcttgtc tccgaagg                                        28

<210>  59
<211>  30
<212>  DNA
<213>  synthetic oligonucleotide

<220>
<221>  misc_feature
<222>  ()..()
<223>  O28990

<400>  59
gcataaccct tatcggagat ttgaagccat                                      30

<210>  60
<211>  30
<212>  DNA
<213>  synthetic oligonucleotide

<220>
<221>  misc_feature
<222>  ()..()
<223>  O28991


<400>  60
aacattcctc tctcatcatc tgttgccagc                                        30


<210>  61
<211>  28
<212>  DNA
<213>  synthetic oligonucleotide

<220>
<221>  misc_feature
<222>  ()..()
<223>  O25907


<400>  61
aacgcttagt atctccggcg acttgaac                                          28


<210>  62
<211>  28
<212>  DNA
<213>  synthetic oligonucleotide

<220>
<221>  misc_feature
<222>  ()..()
<223>  O25909


<400>  62
ctcacacgaa taaggtacaa agttcatc                                          28


<210>  63
<211>  30
<212>  DNA
<213>  synthetic oligonucleotide

<400>  63
ttagtatctc cggcgacttg aacccaaacc                                        30


<210>  64
<211>  30
<212>  DNA
<213>  synthetic oligonucleotide

<220>
<221>  misc_feature
<222>  ()..()
<223>  O28985

<400> 64
agattctcaa caagcttcaa catgagttcg 30

### Annex to the application documents - subsequently filed sequences listing

SEQUENCE LISTING

<110> Ratcliffe, Oliver
      Heard, Jacqueline
      Samaha, Raymond
      Creelman, Robert
      Keddie, James
      Jiang, Cai-zhong
      Reuber, Lynne
      Riechmann, Jose Luis

<120> Flowering Time Modification

<130> MBI-0021

<140> EP 05110550.0
<141> 2000-10-11

<150> US 60/159,464
<151> 1999-10-12

<150> US 60/166,228
<151> 1999-11-17

<150> US 60/197,899
<151> 2000-04-17

<150> Plant Trait Modification III
<151> 2000-08-22

<150> US 60/164,132
<151> 1999-11-08

<160> 64

<170> PatentIn version 3.0

<210> 1
<211> 883
<212> DNA
<213> Arabidopsis thaliana

<220>
<221> CDS
<222> (31)..(621)
<223> G157

<400> 1
gggcataacc cttatcggag atttgaagcc atg gga aga aga aaa atc gag atc      54
                                 Met Gly Arg Arg Lys Ile Glu Ile
                                 1               5

aag cga atc gag aac aaa agc agt cga caa gtc act ttc tcc aaa cga      102
Lys Arg Ile Glu Asn Lys Ser Ser Arg Gln Val Thr Phe Ser Lys Arg
    10                  15                  20

cgc aat ggt ctc atc gac aaa gct cga caa ctt tcg att ctc tgt gaa      150
Arg Asn Gly Leu Ile Asp Lys Ala Arg Gln Leu Ser Ile Leu Cys Glu
25                  30                  35                  40

tcc tcc gtc gct gtt gtc gtc gta tct gcc tcc gga aaa ctc tat gac      198
Ser Ser Val Ala Val Val Val Val Ser Ala Ser Gly Lys Leu Tyr Asp

                45                  50                  55

tct tcc tcc ggt gac gac att tcc aag atc att gat cgt tat gaa ata 246
Ser Ser Ser Gly Asp Asp Ile Ser Lys Ile Ile Asp Arg Tyr Glu Ile
            60                  65                  70

caa cat gct gat gaa ctt aga gcc tta gat ctt gaa gaa aaa att cag 294
Gln His Ala Asp Glu Leu Arg Ala Leu Asp Leu Glu Glu Lys Ile Gln
        75                  80                  85

aat tat ctt cca cac aag gag tta cta gaa aca gtc caa agc aag ctt 342
Asn Tyr Leu Pro His Lys Glu Leu Leu Glu Thr Val Gln Ser Lys Leu
    90                  95                  100

gaa gaa cca aat gtc gat aat gta agt gta gat tct cta att tct ctg 390
Glu Glu Pro Asn Val Asp Asn Val Ser Val Asp Ser Leu Ile Ser Leu
105                 110                 115                 120

gag gaa caa ctt gag act gct ctg tcc gta agt aga gct agg aag gca 438
Glu Glu Gln Leu Glu Thr Ala Leu Ser Val Ser Arg Ala Arg Lys Ala
                125                 130                 135

gaa ctg atg atg gag tat atc gag tcc ctt aaa gaa aag gag aaa ttg 486
Glu Leu Met Met Glu Tyr Ile Glu Ser Leu Lys Glu Lys Glu Lys Leu
            140                 145                 150

ctg aga gaa gag aac cag gtt ctg gct agc cag atg gga aag aat acg 534
Leu Arg Glu Glu Asn Gln Val Leu Ala Ser Gln Met Gly Lys Asn Thr
        155                 160                 165

ttg ctg gca aca gat gat gag aga gga atg ttt ccg gga agt agc tcc 582
Leu Leu Ala Thr Asp Asp Glu Arg Gly Met Phe Pro Gly Ser Ser Ser
    170                 175                 180

ggc aac aaa ata ccg gag act ctc ccg ctg ctc aat tag ccaccatcat 631
Gly Asn Lys Ile Pro Glu Thr Leu Pro Leu Leu Asn
185                 190                 195

caacggctga gttttcacct taaactcaaa gcctgattca taattaagag aataaatttg 691

tatattataa aaagctgtgt aatctcaaac cttttatctt cctctagtgt ggaatttaag 751

gtcaaaaaga aaacgagaaa gtatggatca gtgttgtacc tccttcggag acaagatcag 811

agtttgtgtg tttgtgtctg aatgtacgga ttggattttt aaagttgtgc tttctttctt 871

caaaaaaaaa aa 883

<210> 2
<211> 196
<212> PRT
<213> Arabidopsis thaliana

<400> 2

Met Gly Arg Arg Lys Ile Glu Ile Lys Arg Ile Glu Asn Lys Ser Ser
1               5                   10                  15

Arg Gln Val Thr Phe Ser Lys Arg Arg Asn Gly Leu Ile Asp Lys Ala
            20                  25                  30

Arg Gln Leu Ser Ile Leu Cys Glu Ser Ser Val Ala Val Val Val Val
        35                  40                  45

Ser Ala Ser Gly Lys Leu Tyr Asp Ser Ser Ser Gly Asp Asp Ile Ser
    50                  55                  60

Lys Ile Ile Asp Arg Tyr Glu Ile Gln His Ala Asp Glu Leu Arg Ala
65                  70                  75                  80

Leu Asp Leu Glu Glu Lys Ile Gln Asn Tyr Leu Pro His Lys Glu Leu
                85                  90                  95

Leu Glu Thr Val Gln Ser Lys Leu Glu Glu Pro Asn Val Asp Asn Val
            100                 105                 110

Ser Val Asp Ser Leu Ile Ser Leu Glu Glu Gln Leu Glu Thr Ala Leu
        115                 120                 125

Ser Val Ser Arg Ala Arg Lys Ala Glu Leu Met Met Glu Tyr Ile Glu
    130                 135                 140

Ser Leu Lys Glu Lys Glu Lys Leu Leu Arg Glu Glu Asn Gln Val Leu
145                 150                 155                 160

Ala Ser Gln Met Gly Lys Asn Thr Leu Leu Ala Thr Asp Asp Glu Arg
                165                 170                 175

Gly Met Phe Pro Gly Ser Ser Ser Gly Asn Lys Ile Pro Glu Thr Leu
            180                 185                 190

Pro Leu Leu Asn
        195

<210>  3
<211>  1196
<212>  DNA
<213>  Arabidopsis thaliana

<220>
<221>  CDS
<222>  (132)..(569)
<223>  G859

<400>  3
aaaaaggaga gagagagaga gagagagaga gagagagaga gaaacgaaga aaaaaaaaga      60

agcaaaaaac attgtgggtc tccggtgatt aggatcaaat tagggcacca gccttatcgg     120

aggaagaagc c atg ggt aga aaa aaa gtc gag atc aag cga atc gag aac      170
             Met Gly Arg Lys Lys Val Glu Ile Lys Arg Ile Glu Asn

                1               5                   10

aaa agt agt cga caa gtc act ttc tcc aaa cga cgc aat ggt ctc atc      218
Lys Ser Ser Arg Gln Val Thr Phe Ser Lys Arg Arg Asn Gly Leu Ile
    15                  20                  25

gag aaa gct cga caa ctt tca att ctc tgt gaa tct tcc atc gct gtt      266
Glu Lys Ala Arg Gln Leu Ser Ile Leu Cys Glu Ser Ser Ile Ala Val
30                  35                  40                  45

ctc gtc gtc tcc ggc tcc gga aaa ctc tac aag tct gcc tcc ggt gac      314
Leu Val Val Ser Gly Ser Gly Lys Leu Tyr Lys Ser Ala Ser Gly Asp
                50                  55                  60

aac atg tca aag atc att gat cgt tac gaa ata cat cat gct gat gaa      362
Asn Met Ser Lys Ile Ile Asp Arg Tyr Glu Ile His His Ala Asp Glu
            65                  70                  75

ctt gaa gcc tta gat ctt gca gaa aaa act cgg aat tat ctg cca ctc      410
Leu Glu Ala Leu Asp Leu Ala Glu Lys Thr Arg Asn Tyr Leu Pro Leu
        80                  85                  90

aaa gag tta cta gaa ata gtc caa agg tta gca caa aga cac ttt tat      458
Lys Glu Leu Leu Glu Ile Val Gln Arg Leu Ala Gln Arg His Phe Tyr
    95                  100                 105

ctc cct ctt ctt ctg atg aaa aat act ttt ttt ttt ctt ttc ttt tgg      506
Leu Pro Leu Leu Leu Met Lys Asn Thr Phe Phe Phe Leu Phe Phe Trp
110                 115                 120                 125

cga att atg aat aca gca agc ttg aag aat caa atg tcg ata atg caa      554
Arg Ile Met Asn Thr Ala Ser Leu Lys Asn Gln Met Ser Ile Met Gln
                130                 135                 140

gtg tgg ata ctt taa tttctctgga ggaacagctc gagactgctc tgtccgtaac      609
Val Trp Ile Leu
            145

tagagctagg aagacagaac taatgatggg ggaagtgaag tcccttcaaa aaacgcatgt      669

caaagatcat tgatcgttat gaaatacatc atgctgatga acttaaagcc ttagatcttg      729

cagaaaaaat tcggaattat cttccacaca aggagttact agaaatagtc caaagattct      789

ctaatatcta tggaggaaca gctcgagact gctctgtcag taattagagc taagaagaca      849

gaactaatga tggaggatat gaagtcactt caagaaaggg agaagttgct gatagaagag      909

aaccagattc tggctagcca ggtggggaag aagacgtttc tggttataga aggtgacaga      969

ggaatgtcat gggaaaatgg ctccggcaac aaagtacggg agactcttcc gctgctcaag     1029

taatcaccat catcaacggc tgagctttca ccttaaactt acagcctgat tcagaagttt     1089

ttacaaattt gtaaattata aaaagcttca taataatctc aacctttta tcttcctcgc     1149

gccaatgtgg aaattaaggt aaaccaaaaa aaaaaaaaaa aaaaaaa                  1196

<210>  4
<211>  145
<212>  PRT
<213>  Arabidopsis thaliana

<400>  4

Met Gly Arg Lys Lys Val Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Arg Leu Ala Gln Arg His Phe Tyr Leu Pro Leu
            100                 105                 110

Leu Leu Met Lys Asn Thr Phe Phe Phe Leu Phe Phe Trp Arg Ile Met
        115                 120                 125

Asn Thr Ala Ser Leu Lys Asn Gln Met Ser Ile Met Gln Val Trp Ile
    130                 135                 140

Leu
145


<210>  5
<211>  972
<212>  DNA
<213>  Arabidopsis thaliana

<220>
<221>  CDS
<222>  (132)..(569)
<223>  G859.1

<400>  5
aaaaaggaga gagagagaga gagagagaga gagagagaga gaaacgaaga aaaaaaaaga      60

agcaaaaaac attgtgggtc tccggtgatt aggatcaaat tagggcacca gccttatcgg     120

aggaagaagc c atg ggt aga aaa aaa gtc gag atc aag cga atc gag aac     170
             Met Gly Arg Lys Lys Val Glu Ile Lys Arg Ile Glu Asn
             1               5                   10

aaa agt agt cga caa gtc act ttc tcc aaa cga cgc aat ggt ctc atc     218
Lys Ser Ser Arg Gln Val Thr Phe Ser Lys Arg Arg Asn Gly Leu Ile
    15                  20                  25

gag aaa gct cga caa ctt tca att ctc tgt gaa tct tcc atc gct gtt     266
Glu Lys Ala Arg Gln Leu Ser Ile Leu Cys Glu Ser Ser Ile Ala Val
30                  35                  40                  45

ctc gtc gtc tcc ggc tcc gga aaa ctc tac aag tct gcc tcc ggt gac     314
Leu Val Val Ser Gly Ser Gly Lys Leu Tyr Lys Ser Ala Ser Gly Asp
                50                  55                  60

aac atg tca aag atc att gat cgt tac gaa ata cat cat gct gat gaa     362
Asn Met Ser Lys Ile Ile Asp Arg Tyr Glu Ile His His Ala Asp Glu
            65                  70                  75

ctt gaa gcc tta gat ctt gca gaa aaa act cgg aat tat ctg cca ctc     410
Leu Glu Ala Leu Asp Leu Ala Glu Lys Thr Arg Asn Tyr Leu Pro Leu
        80                  85                  90

aaa gag tta cta gaa ata gtc caa agg tta gca caa aga cac ttt tat     458
Lys Glu Leu Leu Glu Ile Val Gln Arg Leu Ala Gln Arg His Phe Tyr
    95                  100                 105

ctc cct ctt ctt ctg atg aaa aat act ttt ttt ttt ctt ttc ttt tgg     506
Leu Pro Leu Leu Leu Met Lys Asn Thr Phe Phe Phe Leu Phe Phe Trp
110                 115                 120                 125

cga att atg aat aca gca agc ttg aag aat caa atg tcg ata atg caa     554
Arg Ile Met Asn Thr Ala Ser Leu Lys Asn Gln Met Ser Ile Met Gln
                130                 135                 140

gtg tgg ata ctt taa tttctctgga ggaacagctc gagactgctc tgtccgtaac     609
Val Trp Ile Leu
            145

tagagctagg aagacagaac taatgatggg ggaagtgaag tcccttcaaa aaacggagaa     669

cttgctgaga gaagagaacc agactttggc tagccaggtg gggaagaaga cgtttctggt     729

tatagaaggt gacagaggaa tgtcatggga aaatggctcc ggcaacaaag tacgggagac     789

tcttccgctg ctcaagtaat caccatcatc aacggctgag ctttcacctt aaacttacag     849

cctgattcag aagtttttac aaatttgtaa attataaaaa gcttcataat aatctcaacc     909

tttttatctt cctcgcgcca atgtggaaat taaggtaaac caaaaaaaaa aaaaaaaaaa     969

aaa                                                                   972

<210>  6
<211>  145
<212>  PRT
<213>  Arabidopsis thaliana

Met Gly Arg Lys Lys Val Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Arg Leu Ala Gln Arg His Phe Tyr Leu Pro Leu
            100                 105                 110

Leu Leu Met Lys Asn Thr Phe Phe Phe Leu Phe Phe Trp Arg Ile Met
        115                 120                 125

Asn Thr Ala Ser Leu Lys Asn Gln Met Ser Ile Met Gln Val Trp Ile
    130                 135                 140

Leu
145

<210> 7
<211> 1036
<213> Arabidopsis thaliana

<220>
<221> CDS
<222> (162)..(752)
<223> G859.2

<400> 7
gatttgtcat tttttgtcta gccaaaaaaa aaaaaaaaaa aggagagaga gagagagaga 60

gagagagaga gaaacgaaga aaaaaaaaga agcaaaaaac attgtgggtc tccggtgatt 120

aggatcaaat tagggcacca gccttatcgg aggaagaagc c atg ggt aga aaa aaa 176
Met Gly Arg Lys Lys
1               5

gtc gag atc aag cga atc gag aac aaa agt agt cga caa gtc act ttc 224
Val Glu Ile Lys Arg Ile Glu Asn Lys Ser Ser Arg Gln Val Thr Phe
                10                  15                  20

tcc aaa cga cgc aat ggt ctc atc gag aaa gct cga caa ctt tca att 272
Ser Lys Arg Arg Asn Gly Leu Ile Glu Lys Ala Arg Gln Leu Ser Ile
            25                  30                  35

ctc tgt gaa tct tcc atc gct gtt ctc gtc gtc tcc ggc tcc gga aaa 320
Leu Cys Glu Ser Ser Ile Ala Val Leu Val Val Ser Gly Ser Gly Lys

        40                  45                  50

ctc tac aag tct gcc tcc ggt gac aac atg tca aag atc att gat cgt 368
Leu Tyr Lys Ser Ala Ser Gly Asp Asn Met Ser Lys Ile Ile Asp Arg
    55                  60                  65

tac gaa ata cat cat gct gat gaa ctt gaa gcc tta gat ctt gca gaa 416
Tyr Glu Ile His His Ala Asp Glu Leu Glu Ala Leu Asp Leu Ala Glu
70                  75                  80                  85

aaa act cgg aat tat ctg cca ctc aaa gag tta cta gaa ata gtc caa 464
Lys Thr Arg Asn Tyr Leu Pro Leu Lys Glu Leu Leu Glu Ile Val Gln
                90                  95                  100

agc aag ctt gaa gaa tca aat gtc gat aat gca agt gtg gat act tta 512
Ser Lys Leu Glu Glu Ser Asn Val Asp Asn Ala Ser Val Asp Thr Leu
            105                 110                 115

att tct ctg gag gaa cag ctc gag act gct ctg tcc gta act aga gct 560
Ile Ser Leu Glu Glu Gln Leu Glu Thr Ala Leu Ser Val Thr Arg Ala
        120                 125                 130

agg aag aca gaa cta atg atg ggg gaa gtg aag tcc ctt caa aaa acg 608
Arg Lys Thr Glu Leu Met Met Gly Glu Val Lys Ser Leu Gln Lys Thr
    135                 140                 145

gag aac ttg ctg aga gaa gag aac cag act ttg gct agc cag gtg ggg 656
Glu Asn Leu Leu Arg Glu Glu Asn Gln Thr Leu Ala Ser Gln Val Gly
150                 155                 160                 165

aag aag acg ttt ctg gtt ata gaa ggt gac aga gga atg tca tgg gaa 704
Lys Lys Thr Phe Leu Val Ile Glu Gly Asp Arg Gly Met Ser Trp Glu
                170                 175                 180

aat ggc tcc ggc aac aaa gta cgg gag act ctt ccg ctg ctc aag taa 752
Asn Gly Ser Gly Asn Lys Val Arg Glu Thr Leu Pro Leu Leu Lys
            185                 190                 195

tcaccatcat caacggctga gctttcacct taaacttaca gcctgattca gaagttttta 812

caaatttgta aattataaaa agcttcataa taatctcaac ctttttatct tcctcgcgcc 872

aatgtggaaa ttaaggttaa aaataaaata aaacagaagc tcatgcgaaa gaattgtaaa 932

actaagataa agctatagta gatctttatt gtaccttcgt agacgatata agatttattc 992

gtgtgtttgt cttcccctcn aaaaaaaaaa aaaaaaaaaa aaaa 1036

<210> 8
<211> 196
<212> PRT
<213> Arabidopsis thaliana

<400> 8

Met Gly Arg Lys Lys Val Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Ile Leu Cys Glu Ser Ser Ile Ala Val Leu Val Val
        35                  40                  45

Ser Gly Ser Gly Lys Leu Tyr Lys Ser Ala Ser Gly Asp Asn Met Ser
    50                  55                  60

Lys Ile Ile Asp Arg Tyr Glu Ile His His Ala Asp Glu Leu Glu Ala
65                  70                  75                  80

Leu Asp Leu Ala Glu Lys Thr Arg Asn Tyr Leu Pro Leu Lys Glu Leu
                85                  90                  95

Leu Glu Ile Val Gln Ser Lys Leu Glu Glu Ser Asn Val Asp Asn Ala
            100                 105                 110

Ser Val Asp Thr Leu Ile Ser Leu Glu Glu Gln Leu Glu Thr Ala Leu
        115                 120                 125

Ser Val Thr Arg Ala Arg Lys Thr Glu Leu Met Met Gly Glu Val Lys
    130                 135                 140

Ser Leu Gln Lys Thr Glu Asn Leu Leu Arg Glu Glu Asn Gln Thr Leu
145                 150                 155                 160

Ala Ser Gln Val Gly Lys Lys Thr Phe Leu Val Ile Glu Gly Asp Arg
                165                 170                 175

Gly Met Ser Trp Glu Asn Gly Ser Gly Asn Lys Val Arg Glu Thr Leu
            180                 185                 190

Pro Leu Leu Lys
        195

<210> 9
<211> 1059
<212> DNA

<220>
<221> CDS
<222> (219)..(809)
<223> G1842

<400> 9
actattacat gcctcttcct cgcttcaaaa cggcaccgtt tccacttgtt attatttttc 60

tctctatcgt ctaacaaaaa aaaaaactga cttgggattt tttttcattt gtctagccca 120

aaagaagaag atagaaacga agaaaaaaag caaacacatt ttgggtcccc ggtggttagg 180

atcaaattag ggcacaaacc ttatcggaga aagaagcc atg gga aga aga aaa gtc 236

Met Gly Arg Arg Lys Val
1               5

gag atc aag cga atc gag aac aaa agc agt cga caa gtc act ttc tcc     284
Glu Ile Lys Arg Ile Glu Asn Lys Ser Ser Arg Gln Val Thr Phe Ser
            10                  15                  20

aaa cga cgc aaa ggt ctc atc gaa aaa gct cga caa ctt tca att ctc     332
Lys Arg Arg Lys Gly Leu Ile Glu Lys Ala Arg Gln Leu Ser Ile Leu
        25                  30                  35

tgt gaa tct tcc atc gct gtt gtc gcc gtc tcc ggt tcc gga aaa ctc     380
Cys Glu Ser Ser Ile Ala Val Val Ala Val Ser Gly Ser Gly Lys Leu
    40                  45                  50

tac gac tct gcc tcc ggt gac aac atg tca aag atc att gat cgt tat     428
Tyr Asp Ser Ala Ser Gly Asp Asn Met Ser Lys Ile Ile Asp Arg Tyr
55                  60                  65                  70

gaa ata cat cat gct gat gaa ctt aaa gcc tta gat ctt gca gaa aaa     476
Glu Ile His His Ala Asp Glu Leu Lys Ala Leu Asp Leu Ala Glu Lys
                75                  80                  85

att cgg aat tat ctt cca cac aag gag tta cta gaa ata gtc caa agc     524
Ile Arg Asn Tyr Leu Pro His Lys Glu Leu Leu Glu Ile Val Gln Ser
            90                  95                  100

aag ctt gaa gaa tca aat gtc gat aat gta agt gta gat tct cta ata     572
Lys Leu Glu Glu Ser Asn Val Asp Asn Val Ser Val Asp Ser Leu Ile
        105                 110                 115

tct atg gag gaa cag ctc gag act gct ctg tca gta att aga gct aag     620
Ser Met Glu Glu Gln Leu Glu Thr Ala Leu Ser Val Ile Arg Ala Lys
    120                 125                 130

aag aca gaa cta atg atg gag gat atg aag tca ctt caa gaa agg gag     668
Lys Thr Glu Leu Met Met Glu Asp Met Lys Ser Leu Gln Glu Arg Glu
135                 140                 145                 150

aag ttg ctg ata gaa gag aac cag att ctg gct agc cag gtg ggg aag     716
Lys Leu Leu Ile Glu Glu Asn Gln Ile Leu Ala Ser Gln Val Gly Lys
                155                 160                 165

aag acg ttt ctg gtt ata gaa ggt gac aga gga atg tca cgg gaa aat     764
Lys Thr Phe Leu Val Ile Glu Gly Asp Arg Gly Met Ser Arg Glu Asn
            170                 175                 180

ggc tcc ggc aac aaa gta ccg gag act ctt tcg ctg ctc aag taa         809
Gly Ser Gly Asn Lys Val Pro Glu Thr Leu Ser Leu Leu Lys
        185                 190                 195

tcaccatcat caacggctga gctttcacca taaacttact cacagcctga ttcagaagct     869

tttacaaaat tgtaaattat aaaaagctgc ataataatct caaccttttt atcttcctcg     929

cgccaatgtg gaaataaagg taaaacaaaa cgaagctctt ttcttttatg cgaaagaatt     989

gtaaaactaa gataaagcta ccgatctttg ttgtacctta gtagacaaat atcagagttc    1049

ttgtgcttgt                                                           1059

<210>  10

<211>  196
<212>  PRT
<213>  Arabidopsis thaliana

<400>  10

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Ser Lys Leu Glu Glu Ser Asn Val Asp Asn Val
            100                 105                 110

Ser Val Asp Ser Leu Ile Ser Met Glu Glu Gln Leu Glu Thr Ala Leu
        115                 120                 125

Ser Val Ile Arg Ala Lys Lys Thr Glu Leu Met Met Glu Asp Met Lys
    130                 135                 140

Ser Leu Gln Glu Arg Glu Lys Leu Leu Ile Glu Glu Asn Gln Ile Leu
145                 150                 155                 160

Ala Ser Gln Val Gly Lys Lys Thr Phe Leu Val Ile Glu Gly Asp Arg
                165                 170                 175

Gly Met Ser Arg Glu Asn Gly Ser Gly Asn Lys Val Pro Glu Thr Leu
            180                 185                 190

Ser Leu Leu Lys
        195


<210>  11
<211>  880
<212>  DNA
<213>  Arabidopsis thaliana

<220>
<221> CDS
<222> (79)..(636)
<223> G1842.2

<400> 11
agaaaaaaag caaacacatt ttgggtcccc ggtggttagg atcaaattag ggcacaaacc 60

ttatcggaga aagaagcc atg gga aga aga aaa gtc gag atc aag cga atc 111
Met Gly Arg Arg Lys Val Glu Ile Lys Arg Ile
1               5                   10

gag aac aaa agc agt cga caa gtc act ttc tcc aaa cga cgc aaa ggt 159
Glu Asn Lys Ser Ser Arg Gln Val Thr Phe Ser Lys Arg Arg Lys Gly
            15                  20                  25

ctc atc gaa aaa gct cga caa ctt tca att ctc tgt gaa tct tcc atc 207
Leu Ile Glu Lys Ala Arg Gln Leu Ser Ile Leu Cys Glu Ser Ser Ile
        30                  35                  40

gct gtt gtc gcc gtc tcc ggt tcc gga aaa ctc tac gac tct gcc tcc 255
Ala Val Val Ala Val Ser Gly Ser Gly Lys Leu Tyr Asp Ser Ala Ser
    45                  50                  55

ggt gac aac atg tca aag atc att gat cgt tat gaa ata cat cat gct 303
Gly Asp Asn Met Ser Lys Ile Ile Asp Arg Tyr Glu Ile His His Ala
60                  65                  70                  75

gat gaa ctt aaa gcc tta gat ctt gca gaa aaa att cgg aat tat ctt 351
Asp Glu Leu Lys Ala Leu Asp Leu Ala Glu Lys Ile Arg Asn Tyr Leu
                80                  85                  90

cca cac aag gag tta cta gaa ata gtc caa agt gta gat tct cta ata 399
Pro His Lys Glu Leu Leu Glu Ile Val Gln Ser Val Asp Ser Leu Ile
            95                  100                 105

tct atg gag gaa cag ctc gag act gct ctg tca gta att aga gct aag 447
Ser Met Glu Glu Gln Leu Glu Thr Ala Leu Ser Val Ile Arg Ala Lys
        110                 115                 120

aag aca gaa cta atg atg gag gat atg aag tca ctt caa gaa agg gag 495
Lys Thr Glu Leu Met Met Glu Asp Met Lys Ser Leu Gln Glu Arg Glu
    125                 130                 135

aag ttg ctg ata gaa gag aac cag att ctg gct agc cag gtg ggg aag 543
Lys Leu Leu Ile Glu Glu Asn Gln Ile Leu Ala Ser Gln Val Gly Lys
140                 145                 150                 155

aag acg ttt ctg gtt ata gaa ggt gac aga gga atg tca cgg gaa aat 591
Lys Thr Phe Leu Val Ile Glu Gly Asp Arg Gly Met Ser Arg Glu Asn
                160                 165                 170

ggc tcc ggc aac aaa gta ccg gag act ctt tcg ctg ctc aag taa 636
Gly Ser Gly Asn Lys Val Pro Glu Thr Leu Ser Leu Leu Lys
            175                 180                 185

tcaccatcat caacggctga gctttcacca taaacttact cacagcctga ttcagaagct 696

tttacaaaat tgtaaattat aaaaagctgc ataataatct caaccttttt atcttcctcg 756

cgccaatgtg gaaataaagg taaaacaaaa cgaagctctt ttcttttatg cgaaagaatt 816

gtaaaactaa gataaagcta ccgatctttg ttgtacctta gtagacaaat atcagagttc     876

ttgt                                                                  880

<210>  12
<211>  185
<212>  PRT
<213>  Arabidopsis thaliana

<400>  12

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Ser Val Asp Ser Leu Ile Ser Met Glu Glu Gln
            100                 105                 110

Leu Glu Thr Ala Leu Ser Val Ile Arg Ala Lys Lys Thr Glu Leu Met
        115                 120                 125

Met Glu Asp Met Lys Ser Leu Gln Glu Arg Glu Lys Leu Leu Ile Glu
    130                 135                 140

Glu Asn Gln Ile Leu Ala Ser Gln Val Gly Lys Lys Thr Phe Leu Val
145                 150                 155                 160

Ile Glu Gly Asp Arg Gly Met Ser Arg Glu Asn Gly Ser Gly Asn Lys
                165                 170                 175

Val Pro Glu Thr Leu Ser Leu Leu Lys
            180                 185

<210>  13
<211>  978
<212>  DNA

<213>  Arabidopsis thaliana

<220>
<221>  CDS
<222>  (219)..(452)
<223>  G1842.6


<400>  13
actattacat gcctcttcct cgcttcaaaa cggcaccgtt tccacttgtt attatttttc      60

tctctatcgt ctaacaaaaa aaaaaactga cttgggattt tttttcattt gtctagccca     120

aaagaagaag atagaaacga agaaaaaaag caaacacatt ttgggtcccc ggtggttagg     180

atcaaattag ggcacaaacc ttatcggaga aagaagcc atg gga aga aga aaa gtc     236
                                          Met Gly Arg Arg Lys Val
                                          1               5

gag atc aag cga atc gag aac aaa agc agt cga caa gtc act ttc tcc       284
Glu Ile Lys Arg Ile Glu Asn Lys Ser Ser Arg Gln Val Thr Phe Ser
            10                  15                  20

aaa cga cgc aaa ggt ctc atc gaa aaa gct cga caa ctt tca att ctc       332
Lys Arg Arg Lys Gly Leu Ile Glu Lys Ala Arg Gln Leu Ser Ile Leu
        25                  30                  35

tgt gaa tct tcc atc gct gtt gtc gcc gtc tcc ggt tcc gga aaa ctc       380
Cys Glu Ser Ser Ile Ala Val Val Ala Val Ser Gly Ser Gly Lys Leu
    40                  45                  50

tac gac tct gcc tcc ggt gac aag atc ttg cag aaa aaa ttc gga att       428
Tyr Asp Ser Ala Ser Gly Asp Lys Ile Leu Gln Lys Lys Phe Gly Ile
55                  60                  65                  70

atc ttc cac aca agg agt tac tag aaatagtcca aagattctct aatatctatg      482
Ile Phe His Thr Arg Ser Tyr
                75

gaggaacagc tcgagactgc tctgtcagta attagagcta agaagacaga actaatgatg     542

gaggatatga agtcacttca agaaagggag aagttgctga tagaagagaa ccagattctg     602

gctagccagg tggggaagaa gacgtttctg gttatagaag gtgacagagg aatgtcacgg     662

gaaaatggct ccggcaacaa agtaccggag actctttcgc tgctcaagta atcaccatca     722

tcaacggctg agctttcacc ataaacttac tcacagcctg attcagaagc ttttacaaaa     782

ttgtaaatta taaaaagctg cataataatc tcaacctttt tatcttcctc gcgccaatgt     842

ggaaataaag gtaaaacaaa acgaagctct tttcttttat gcgaaagaat tgtaaaacta     902

agataaagct accgatcttt gttgtacctt agtagacaaa tatcagagtt cttgtgcttg     962

aaaaaaaaaa aaaaaa                                                     978


<210>  14
<211>  77
<212>  PRT
<213>  Arabidopsis thaliana

<400> 14

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Lys Ile Leu
    50                  55                  60

Gln Lys Lys Phe Gly Ile Ile Phe His Thr Arg Ser Tyr
65                  70                  75

<210> 15
<211> 876
<212> DNA
<213> Arabidopsis thaliana

<220>
<221> CDS
<222> (80)..(436)
<223> G1842.7

<400> 15
cagaaaaaaa gcaaacacat tttgggtccc cggtggttag gatcaaatta gggcacaaac      60

cttatcggag aaagaagcc atg gga aga aga aaa gtc gag atc aag cga atc      112
                     1               5                   10

gag aac aaa agc agt cga caa gtc act ttc tcc aaa cga cgc aaa ggt      160
Glu Asn Lys Ser Ser Arg Gln Val Thr Phe Ser Lys Arg Arg Lys Gly
            15                  20                  25

ctc atc gaa aaa gct cga caa ctt tca att ctc tgt gaa tct tcc atc      208
Leu Ile Glu Lys Ala Arg Gln Leu Ser Ile Leu Cys Glu Ser Ser Ile
        30                  35                  40

gct gtt gtc gcc gtc tcc ggt tcc gga aaa ctc tac gac tct gcc tcc      256
Ala Val Val Ala Val Ser Gly Ser Gly Lys Leu Tyr Asp Ser Ala Ser
    45                  50                  55

ggt gac aac atg tca aag atc att gat cgt tat gaa ata cat cat gct      304
Gly Asp Asn Met Ser Lys Ile Ile Asp Arg Tyr Glu Ile His His Ala
60                  65                  70                  75

gat gaa ctt aaa gcc tta gat ctt gca gaa aaa att cgg aat tat ctt      352
Asp Glu Leu Lys Ala Leu Asp Leu Ala Glu Lys Ile Arg Asn Tyr Leu
                80                  85                  90

cca cac aag gag tta cta gaa ata gtc caa aga ttc tct aat atc tat      400
Pro His Lys Glu Leu Leu Glu Ile Val Gln Arg Phe Ser Asn Ile Tyr
            95                  100                 105

gga gga aca gct cga gac tgc tct gtc agt aat tag agctaagaag 446
Gly Gly Thr Ala Arg Asp Cys Ser Val Ser Asn
110 115

acagaactaa tgatggagga tatgaagtca cttcaagaaa gggagaagtt gctgatagaa 506

gagaaccaga ttctggctag ccaggtgggg aagaagacgt ttctggttat agaaggtgac 566

agaggaatgt cacgggaaaa tggctccggc aacaaagtac cggagactct ttcgctgctc 626

aagtaatcac catcatcaac ggctgagctt tcaccataaa cttactcaca gcctgattca 686

gaagctttta caaaattgta aattataaaa agctgcataa taatctcaac ctttttatct 746

tcctcgcgcc aatgtggaaa taaaggtaaa acaaaacgaa gctcttttct tttatgcgaa 806

agaattgtaa aactaagata aagctaccga tctttgttgt accttagtag acaaatatca 866

gagttcttgt 876

<210> 16
<211> 118
<212> PRT
<213> Arabidopsis thaliana

<400> 16

Met Gly Arg Arg Lys Val Glu Ile Lys Arg Ile Glu Asn Lys Ser Ser
1               5                   10                  15

Arg Gln Val Thr Phe Ser Lys Arg Arg Lys Gly Leu Ile Glu Lys Ala
            20                  25                  30

Arg Gln Leu Ser Ile Leu Cys Glu Ser Ser Ile Ala Val Val Ala Val
        35                  40                  45

Ser Gly Ser Gly Lys Leu Tyr Asp Ser Ala Ser Gly Asp Asn Met Ser
    50                  55                  60

Lys Ile Ile Asp Arg Tyr Glu Ile His His Ala Asp Glu Leu Lys Ala
65                  70                  75                  80

Leu Asp Leu Ala Glu Lys Ile Arg Asn Tyr Leu Pro His Lys Glu Leu
                85                  90                  95

Leu Glu Ile Val Gln Arg Phe Ser Asn Ile Tyr Gly Gly Thr Ala Arg
            100                 105                 110

Asp Cys Ser Val Ser Asn
        115

<210> 17
<211> 818
<212> DNA

<213>  Arabidopsis thaliana

<220>
<221>  CDS
<222>  (51)..(653)
<223>  G1843

<400>  17
cagacatcac aatcaaatta ggtcagaaga attagtcgga gaaaacagcc atg gga        56
                                                      Met Gly
                                                      1

aga aga aaa gta gag atc aaa cga att gag aac aaa agc tct cga caa      104
Arg Arg Lys Val Glu Ile Lys Arg Ile Glu Asn Lys Ser Ser Arg Gln
        5                   10                  15

gtt act ttc tgt aaa cga cga aat ggt ctc atg gag aaa gct cgt caa      152
Val Thr Phe Cys Lys Arg Arg Asn Gly Leu Met Glu Lys Ala Arg Gln
    20                  25                  30

ctc tca att ctt tgt gaa tcc tcc gtc gct ctt atc atc atc tct gcc      200
Leu Ser Ile Leu Cys Glu Ser Ser Val Ala Leu Ile Ile Ile Ser Ala
35                  40                  45                  50

acc gga aga ctc tac agc ttc tcc tca ggt gat agc atg gcc aag atc      248
Thr Gly Arg Leu Tyr Ser Phe Ser Ser Gly Asp Ser Met Ala Lys Ile
                55                  60                  65

ctc agt cgt tat gaa tta gaa cag gct gat gat ctt aaa acc ttg gat      296
Leu Ser Arg Tyr Glu Leu Glu Gln Ala Asp Asp Leu Lys Thr Leu Asp
            70                  75                  80

cta gaa gaa aaa act ctt aat tat ctt tcg cac aag gag ttg cta gaa      344
Leu Glu Glu Lys Thr Leu Asn Tyr Leu Ser His Lys Glu Leu Leu Glu
        85                  90                  95

aca atc caa tgc aag att gaa gaa gcg aaa agc gat aat gta agt ata      392
Thr Ile Gln Cys Lys Ile Glu Glu Ala Lys Ser Asp Asn Val Ser Ile
    100                 105                 110

gat tgt cta aag tcc ctg gaa gag cag ctc aag act gct ctg tct gta      440
Asp Cys Leu Lys Ser Leu Glu Glu Gln Leu Lys Thr Ala Leu Ser Val
115                 120                 125                 130

act aga gct agg aag aca gaa cta atg atg gag ctt gtg aag acc cat      488
Thr Arg Ala Arg Lys Thr Glu Leu Met Met Glu Leu Val Lys Thr His
                135                 140                 145

caa gag aag gag aag ctg ctg aga gag gag aac cag agt ttg act aac      536
Gln Glu Lys Glu Lys Leu Leu Arg Glu Glu Asn Gln Ser Leu Thr Asn
            150                 155                 160

cag ctt ata aag atg ggg aag atg aag aag tct gtg gaa gca gag gat      584
Gln Leu Ile Lys Met Gly Lys Met Lys Lys Ser Val Glu Ala Glu Asp
        165                 170                 175

gca aga gca atg tca ccg gaa agt agc tct gac aac aag cca ccg gag      632
Ala Arg Ala Met Ser Pro Glu Ser Ser Ser Asp Asn Lys Pro Pro Glu
    180                 185                 190

act ctc ctg ctt ctc aag taa ccaccatcac caacgactga ttcgaaaaat         683
Thr Leu Leu Leu Leu Lys

195                 200

aaaaattgta aaaattatga tttgtagttc ataaggaaag ctacatactg tatgttaaaa     743

atcctcttct tccccctgct acggaaaagt catccaagga gatgcatcaa ataaagtaat     803

tgatttttat tgtta                                                      818

<210>  18
<211>  200
<212>  PRT
<213>  Arabidopsis thaliana

<400>  18

Met Gly Arg Arg Lys Val Glu Ile Lys Arg Ile Glu Asn Lys Ser Ser
1               5                   10                  15

Arg Gln Val Thr Phe Cys Lys Arg Arg Asn Gly Leu Met Glu Lys Ala
            20                  25                  30

Arg Gln Leu Ser Ile Leu Cys Glu Ser Ser Val Ala Leu Ile Ile Ile
        35                  40                  45

Ser Ala Thr Gly Arg Leu Tyr Ser Phe Ser Ser Gly Asp Ser Met Ala
    50                  55                  60

Lys Ile Leu Ser Arg Tyr Glu Leu Glu Gln Ala Asp Asp Leu Lys Thr
65                  70                  75                  80

Leu Asp Leu Glu Glu Lys Thr Leu Asn Tyr Leu Ser His Lys Glu Leu
                85                  90                  95

Leu Glu Thr Ile Gln Cys Lys Ile Glu Glu Ala Lys Ser Asp Asn Val
            100                 105                 110

Ser Ile Asp Cys Leu Lys Ser Leu Glu Glu Gln Leu Lys Thr Ala Leu
        115                 120                 125

Ser Val Thr Arg Ala Arg Lys Thr Glu Leu Met Met Glu Leu Val Lys
    130                 135                 140

Thr His Gln Glu Lys Glu Lys Leu Leu Arg Glu Glu Asn Gln Ser Leu
145                 150                 155                 160

Thr Asn Gln Leu Ile Lys Met Gly Lys Met Lys Lys Ser Val Glu Ala
                165                 170                 175

Glu Asp Ala Arg Ala Met Ser Pro Glu Ser Ser Ser Asp Asn Lys Pro
            180                 185                 190

Pro Glu Thr Leu Leu Leu Leu Lys
        195                 200

<210>  19
<211>  834
<212>  DNA
<213>  Arabidopsis thaliana

<220>
<221>  CDS
<222>  (39)..(635)
<223>  G1844

<400>  19
agaaattagg ggattagatg tgtcggaaga gtgaagcc atg gga aga aga aga gta      56
                                          Met Gly Arg Arg Arg Val
                                          1               5

gag atc aaa cga att gag aac aaa agc agt aga caa gtc act ttc tgt      104
Glu Ile Lys Arg Ile Glu Asn Lys Ser Ser Arg Gln Val Thr Phe Cys
            10                  15                  20

aag aga cga aat ggt ctc atg gag aaa gct cgt caa ctc tca att ctc      152
Lys Arg Arg Asn Gly Leu Met Glu Lys Ala Arg Gln Leu Ser Ile Leu
        25                  30                  35

tgt gga tcc tcc gtc gct ctt ttc atc gtc tct tcc acc ggc aaa ctc      200
Cys Gly Ser Ser Val Ala Leu Phe Ile Val Ser Ser Thr Gly Lys Leu
    40                  45                  50

tac aac tcc tcc tcc ggc gac agc atg gcc aag atc atc agt cgt ttt      248
Tyr Asn Ser Ser Ser Gly Asp Ser Met Ala Lys Ile Ile Ser Arg Phe
55                  60                  65                  70

Lys Ile Gln Gln Ala Asp Asp Pro Glu Thr Leu Asp Leu Glu Asp Lys
                75                  80                  85

act cag gat tat ctt tca cac aag gag tta cta gaa ata gtt caa aga      344
Thr Gln Asp Tyr Leu Ser His Lys Glu Leu Leu Glu Ile Val Gln Arg
            90                  95                  100

aag att gaa gaa gca aaa ggg gat aat gta agt ata gaa tct cta att      392
Lys Ile Glu Glu Ala Lys Gly Asp Asn Val Ser Ile Glu Ser Leu Ile
        105                 110                 115

tcc atg gaa gag cag ctc aag agt gct ctg tct gta att aga gct agg      440
Ser Met Glu Glu Gln Leu Lys Ser Ala Leu Ser Val Ile Arg Ala Arg
    120                 125                 130

aag aca gag tta ttg atg gag ctt gtg aag aac ctt cag gat aag gag      488
Lys Thr Glu Leu Leu Met Glu Leu Val Lys Asn Leu Gln Asp Lys Glu
135                 140                 145                 150

aag ttg ctg aaa gaa aag aac aag gtt cta gct agc gag gtg ggg aag      536
Lys Leu Leu Lys Glu Lys Asn Lys Val Leu Ala Ser Glu Val Gly Lys
                155                 160                 165

ctg aag aaa att ttg gaa aca ggg gat gaa aga gca gta atg tca ccg      584
Leu Lys Lys Ile Leu Glu Thr Gly Asp Glu Arg Ala Val Met Ser Pro
            170                 175                 180

gaa aat agc tct ggc cac agc cca ccg gag act ctc ccg ctt ctc aag     632
Glu Asn Ser Ser Gly His Ser Pro Pro Glu Thr Leu Pro Leu Leu Lys
        185                 190                 195

taa ccaccaatca tcaacggctg atttttcatc atcctgattc aaaaaaggta     685

aaaaaaattc atgtgtaaaa atcataaaga agctacatgt tttaaaatcc tcttctcccc     745

ctgcatacgg ataaatttat agaccaaaaa tataatgttt tccctcaaat aagatatcga     805

cctttgtgtt accttggaag acaggatca     834

<210>  20
<211>  198
<212>  PRT
<213>  Arabidopsis thaliana

<400>  20

Met Gly Arg Arg Arg Val Glu Ile Lys Arg Ile Glu Asn Lys Ser Ser
1               5                   10                  15

Arg Gln Val Thr Phe Cys Lys Arg Arg Asn Gly Leu Met Glu Lys Ala
            20                  25                  30

Arg Gln Leu Ser Ile Leu Cys Gly Ser Ser Val Ala Leu Phe Ile Val
        35                  40                  45

Ser Ser Thr Gly Lys Leu Tyr Asn Ser Ser Ser Gly Asp Ser Met Ala
    50                  55                  60

Lys Ile Ile Ser Arg Phe Lys Ile Gln Gln Ala Asp Asp Pro Glu Thr
65                  70                  75                  80

Leu Asp Leu Glu Asp Lys Thr Gln Asp Tyr Leu Ser His Lys Glu Leu
                85                  90                  95

Leu Glu Ile Val Gln Arg Lys Ile Glu Glu Ala Lys Gly Asp Asn Val
            100                 105                 110

Ser Ile Glu Ser Leu Ile Ser Met Glu Glu Gln Leu Lys Ser Ala Leu
        115                 120                 125

Ser Val Ile Arg Ala Arg Lys Thr Glu Leu Leu Met Glu Leu Val Lys
    130                 135                 140

Asn Leu Gln Asp Lys Glu Lys Leu Leu Lys Glu Lys Asn Lys Val Leu
145                 150                 155                 160

Ala Ser Glu Val Gly Lys Leu Lys Lys Ile Leu Glu Thr Gly Asp Glu
                165                 170                 175

Arg Ala Val Met Ser Pro Glu Asn Ser Ser Gly His Ser Pro Pro Glu
            180                 185                 190

Thr Leu Pro Leu Leu Lys
        195

<210>  21
<211>  753
<212>  DNA
<213>  Arabidopsis thaliana

<220>
<221>  CDS
<222>  (1)..(555)
<223>  G1844.2

<400>  21
   atg gga aga aga aga gta gag atc aaa cga att gag aac aaa agc agt        48
   Met Gly Arg Arg Arg Val Glu Ile Lys Arg Ile Glu Asn Lys Ser Ser
   1               5                   10                  15
   aga caa gtc act ttc tgt aag aga cga aat ggt ctc atg gag aaa gct        96
   Arg Gln Val Thr Phe Cys Lys Arg Arg Asn Gly Leu Met Glu Lys Ala
               20                  25                  30
   cgt caa ctc tca att ctc tgt gga tcc tcc gtc gct ctt ttc atc gtc       144
   Arg Gln Leu Ser Ile Leu Cys Gly Ser Ser Val Ala Leu Phe Ile Val
           35                  40                  45
   tct tcc acc ggc aaa ctc tac aac tcc tcc tcc ggc gac agc atg gcc       192
   Ser Ser Thr Gly Lys Leu Tyr Asn Ser Ser Ser Gly Asp Ser Met Ala
       50                  55                  60
   aag atc atc agt cgt ttt aaa ata caa caa gct gat gat cct gaa acc       240
   Lys Ile Ile Ser Arg Phe Lys Ile Gln Gln Ala Asp Asp Pro Glu Thr
   65                  70                  75                  80
   ttg gat ctt gaa gac aaa act cag gat tat ctt tca cac aag gag tta       288
   Leu Asp Leu Glu Asp Lys Thr Gln Asp Tyr Leu Ser His Lys Glu Leu
                   85                  90                  95
   cta gaa ata gtt caa aga aag att gaa gaa gca aaa ggg gat aat gta       336
   Leu Glu Ile Val Gln Arg Lys Ile Glu Glu Ala Lys Gly Asp Asn Val
               100                 105                 110
   agt ata gaa tct cta att tcc atg gaa gag cag ctc aag agt gct ctg       384
   Ser Ile Glu Ser Leu Ile Ser Met Glu Glu Gln Leu Lys Ser Ala Leu
           115                 120                 125
   tct gta att aga gct agg aag aca gag tta ttg atg gag ctt gtg aag       432
   Ser Val Ile Arg Ala Arg Lys Thr Glu Leu Leu Met Glu Leu Val Lys
       130                 135                 140
   aac ctt cag gat aag gtg ggg aag ctg aag aaa att ttg gaa aca ggg       480
   Asn Leu Gln Asp Lys Val Gly Lys Leu Lys Lys Ile Leu Glu Thr Gly
   145                 150                 155                 160
   gat gaa aga gca gta atg tca ccg gaa aat agc tct ggc cac agc cca       528
   Asp Glu Arg Ala Val Met Ser Pro Glu Asn Ser Ser Gly His Ser Pro
                   165                 170                 175
   ccg gag act ctc ccg ctt ctc aag taa ccaccaatca tcaacggctg          575
   Pro Glu Thr Leu Pro Leu Leu Lys
               180
   atttttcatc atcctgattc aaaaaaggta aaaaaaattc atgtgtaaaa atcataaaga     635
   agctacatgt tttaaaatcc tcttctcccc ctgcatacgg ataaatttat agaccaaaaa  695
   tataatgttt tccctcaaat aagatatcga cctttgtgtt accttggaag acaggatc    753

<210>  22
<211>  184
<212>  PRT
<213>  Arabidopsis thaliana

<400>  22

Met Gly Arg Arg Arg Val Glu Ile Lys Arg Ile Glu Asn Lys Ser Ser
1               5                   10                  15

Arg Gln Val Thr Phe Cys Lys Arg Arg Asn Gly Leu Met Glu Lys Ala
            20                  25                  30

Arg Gln Leu Ser Ile Leu Cys Gly Ser Ser Val Ala Leu Phe Ile Val
        35                  40                  45

Ser Ser Thr Gly Lys Leu Tyr Asn Ser Ser Ser Gly Asp Ser Met Ala
    50                  55                  60

Lys Ile Ile Ser Arg Phe Lys Ile Gln Gln Ala Asp Asp Pro Glu Thr
65                  70                  75                  80

Leu Asp Leu Glu Asp Lys Thr Gln Asp Tyr Leu Ser His Lys Glu Leu
                85                  90                  95

Leu Glu Ile Val Gln Arg Lys Ile Glu Glu Ala Lys Gly Asp Asn Val
            100                 105                 110

Ser Ile Glu Ser Leu Ile Ser Met Glu Glu Gln Leu Lys Ser Ala Leu
        115                 120                 125

Ser Val Ile Arg Ala Arg Lys Thr Glu Leu Leu Met Glu Leu Val Lys
    130                 135                 140

Asn Leu Gln Asp Lys Val Gly Lys Leu Lys Lys Ile Leu Glu Thr Gly
145                 150                 155                 160

Asp Glu Arg Ala Val Met Ser Pro Glu Asn Ser Ser Gly His Ser Pro
                165                 170                 175

Pro Glu Thr Leu Pro Leu Leu Lys
            180

<210>  23
<211>  1134
<212>  DNA
<213>  Arabidopsis thaliana

<220>
<221>  CDS
<222>  (158)..(880)
<223>  G861

<400>  23
cttcttcctc ctcctccatc tcttctcttt actctctctt taatcatctc tcattcttga      60

atcttgatcc atcaaaatca atcccgttct cgaaagatcc attaaaatca aaacctaagc     120

tctctctctt gcttctaggg tttttttgtt cgttgtg atg gcg aga gaa aag att      175
                                         Met Ala Arg Glu Lys Ile
                                         1               5

cag atc agg aag atc gac aac gca acg gcg aga caa gtg acg ttt tcg      223
Gln Ile Arg Lys Ile Asp Asn Ala Thr Ala Arg Gln Val Thr Phe Ser
            10                  15                  20

aaa cga aga aga ggg ctt ttc aag aaa gct gaa gaa ctc tcc gtt ctc      271
Lys Arg Arg Arg Gly Leu Phe Lys Lys Ala Glu Glu Leu Ser Val Leu
        25                  30                  35

tgc gac gcc gat gtc gct ctc atc atc ttc tct tcc acc gga aaa ctg      319
Cys Asp Ala Asp Val Ala Leu Ile Ile Phe Ser Ser Thr Gly Lys Leu
    40                  45                  50

ttc gag ttc tgt agc tcc agc atg aag gaa gtc cta gag agg cat aac      367
Phe Glu Phe Cys Ser Ser Ser Met Lys Glu Val Leu Glu Arg His Asn
55                  60                  65                  70

ttg cag tca aag aac ttg gag aag ctt gat cag cca tct ctt gag tta      415
Leu Gln Ser Lys Asn Leu Glu Lys Leu Asp Gln Pro Ser Leu Glu Leu
                75                  80                  85

cag ctg gtt gag aac agt gat cac gcc cga atg agt aaa gaa att gcg      463
Gln Leu Val Glu Asn Ser Asp His Ala Arg Met Ser Lys Glu Ile Ala
            90                  95                  100

gac aag agc cac cga cta agg caa atg aga gga gag gaa ctt caa gga      511
Asp Lys Ser His Arg Leu Arg Gln Met Arg Gly Glu Glu Leu Gln Gly
        105                 110                 115

ctt gac att gaa gag ctt cag cag cta gag aag gcc ctt gaa act ggt      559
Leu Asp Ile Glu Glu Leu Gln Gln Leu Glu Lys Ala Leu Glu Thr Gly
    120                 125                 130

ttg acg cgt gtg att gaa aca aag agt gac aag att atg agt gag atc      607
Leu Thr Arg Val Ile Glu Thr Lys Ser Asp Lys Ile Met Ser Glu Ile
135                 140                 145                 150

agc gaa ctt cag aaa aag gga atg caa ttg atg gat gag aac aag cgg      655

Ser Glu Leu Gln Lys Lys Gly Met Gln Leu Met Asp Glu Asn Lys Arg
                155                 160                 165

ttg agg cag caa gga acg caa cta acg gaa gag aac gag cga ctt ggc 703
Leu Arg Gln Gln Gly Thr Gln Leu Thr Glu Glu Asn Glu Arg Leu Gly
            170                 175                 180

atg caa ata tgt aac aat gtg cat gca cac ggt ggt gct gaa tcg gag 751
Met Gln Ile Cys Asn Asn Val His Ala His Gly Gly Ala Glu Ser Glu
        185                 190                 195

aac gct gct gtg tac gag gaa gga cag tcg tcg gag tct att act aac 799
Asn Ala Ala Val Tyr Glu Glu Gly Gln Ser Ser Glu Ser Ile Thr Asn
    200                 205                 210

gcc gga aac tct acc gga gcg cct gtt gac tcc gag agc tcc gac act 847
Ala Gly Asn Ser Thr Gly Ala Pro Val Asp Ser Glu Ser Ser Asp Thr
215                 220                 225                 230

tcc ctt agg ctc ggc tta ccg tat ggt ggt tag agatggaaca attcaaagaa 900
Ser Leu Arg Leu Gly Leu Pro Tyr Gly Gly
                235                 240

gttgatggag tgaggagagt aatgtaaatc tttttaactc ggtagtaaca agagacaatg 960

tctaagtagt gaattctcaa atgtttgtgt aagtttctgc ctatggaaga ggctttcatt 1020

tttatgattt tcactatgta tgatctctct tcactgcatt tctggttagt aacggcttgt 1080

caccgataaa ctttctcgtt atggaaagtt agaataaaaa aaaaaaaaaa aaaa 1134

<210> 24
<211> 240
<212> PRT
<213> Arabidopsis thaliana

<400> 24

Met Ala Arg Glu Lys Ile Gln Ile Arg Lys Ile Asp Asn Ala Thr Ala
1               5                   10                  15

Arg Gln Val Thr Phe Ser Lys Arg Arg Arg Gly Leu Phe Lys Lys Ala
            20                  25                  30

Glu Glu Leu Ser Val Leu Cys Asp Ala Asp Val Ala Leu Ile Ile Phe
        35                  40                  45

Ser Ser Thr Gly Lys Leu Phe Glu Phe Cys Ser Ser Ser Met Lys Glu
    50                  55                  60

Val Leu Glu Arg His Asn Leu Gln Ser Lys Asn Leu Glu Lys Leu Asp
65                  70                  75                  80

Gln Pro Ser Leu Glu Leu Gln Leu Val Glu Asn Ser Asp His Ala Arg
                85                  90                  95

Met Ser Lys Glu Ile Ala Asp Lys Ser His Arg Leu Arg Gln Met Arg
            100                 105                 110

Gly Glu Glu Leu Gln Gly Leu Asp Ile Glu Glu Leu Gln Gln Leu Glu
        115                 120                 125

Lys Ala Leu Glu Thr Gly Leu Thr Arg Val Ile Glu Thr Lys Ser Asp
    130                 135                 140

Lys Ile Met Ser Glu Ile Ser Glu Leu Gln Lys Lys Gly Met Gln Leu
145                 150                 155                 160

Met Asp Glu Asn Lys Arg Leu Arg Gln Gln Gly Thr Gln Leu Thr Glu
                165                 170                 175

Glu Asn Glu Arg Leu Gly Met Gln Ile Cys Asn Asn Val His Ala His
            180                 185                 190

Gly Gly Ala Glu Ser Glu Asn Ala Ala Val Tyr Glu Glu Gly Gln Ser
        195                 200                 205

Ser Glu Ser Ile Thr Asn Ala Gly Asn Ser Thr Gly Ala Pro Val Asp
    210                 215                 220

Ser Glu Ser Ser Asp Thr Ser Leu Arg Leu Gly Leu Pro Tyr Gly Gly
225                 230                 235                 240

<210>  25
<211>  1552
<212>  DNA
<213>  Arabidopsis thaliana

<220>
<221>  CDS
<222>  (193)..(825)
<223>  G861.1

<400>  25
attctctctc tcncaaaatt tatttcctct ggcttcttct tcctcctcct ccatctcttc      60

tctttactct ctctttaatc atctctcatt cttgaatctt gatccatcaa aatcaatccc     120

gttctcgaaa gatccattaa aatcaaaacc taagctctct ctcttgcttc tagggttttt     180

ttgttcgttg tg atg gcg aga gaa aag att cag atc agg aag atc gac aac     231
              Met Ala Arg Glu Lys Ile Gln Ile Arg Lys Ile Asp Asn
              1               5                   10

gca acg gcg aga caa gtg acg ttt tcg aaa cga aga aga ggg ctt ttc     279
Ala Thr Ala Arg Gln Val Thr Phe Ser Lys Arg Arg Arg Gly Leu Phe
    15                  20                  25

aag aaa gct gaa gaa ctc tcc gtt ctc tgc gac gcc gat gtc gct ctc     327

Lys Lys Ala Glu Glu Leu Ser Val Leu Cys Asp Ala Asp Val Ala Leu
30                  35                  40                  45

atc atc ttc tct tcc acc gga aaa ctg ttc gag ttc tgt agc tcc agc 375
Ile Ile Phe Ser Ser Thr Gly Lys Leu Phe Glu Phe Cys Ser Ser Ser
                50                  55                  60

atg aag gaa gtc cta gag agg cat aac ttg cag tca aag aac ttg gag 423
Met Lys Glu Val Leu Glu Arg His Asn Leu Gln Ser Lys Asn Leu Glu
            65                  70                  75

aag ctt gat cag cca tct ctt gag tta cag ctg gtt gag aac agt gat 471
Lys Leu Asp Gln Pro Ser Leu Glu Leu Gln Leu Val Glu Asn Ser Asp
        80                  85                  90

cac gcc cga atg agt aaa gaa att gcg gac aag agc cac cga cta agg 519
His Ala Arg Met Ser Lys Glu Ile Ala Asp Lys Ser His Arg Leu Arg
    95                  100                 105

caa atg aga gga gag gaa ctt caa gga ctt gac att gaa gag ctt cag 567
Gln Met Arg Gly Glu Glu Leu Gln Gly Leu Asp Ile Glu Glu Leu Gln
110                 115                 120                 125

cag cta gag aag gcc ctt gaa act ggt ttg acg cgt gtg att gaa aca 615
Gln Leu Glu Lys Ala Leu Glu Thr Gly Leu Thr Arg Val Ile Glu Thr
                130                 135                 140

aag agt gac aag att atg agt gag atc agc gaa ctt cag aaa aag gga 663
Lys Ser Asp Lys Ile Met Ser Glu Ile Ser Glu Leu Gln Lys Lys Gly
            145                 150                 155

atg caa ttg atg gat gag aac aag cgg ttg agg cag caa gta tgt gtc 711
Met Gln Leu Met Asp Glu Asn Lys Arg Leu Arg Gln Gln Val Cys Val
        160                 165                 170

tta ccc tct ctg ttg ata aca aat ccc ttt ctt ttg tct acc att aac 759
Leu Pro Ser Leu Leu Ile Thr Asn Pro Phe Leu Leu Ser Thr Ile Asn
    175                 180                 185

gta cac act cct aaa ttt aat ccc cag ttg tct aca aca cat atg ttt 807
Val His Thr Pro Lys Phe Asn Pro Gln Leu Ser Thr Thr His Met Phe
190                 195                 200                 205

gat cat act gtg aga taa atgaataaac caagtgatat agcgcgattt 855
Asp His Thr Val Arg
                210

aaaaatgtct ttaaaactaa aggtaaccat gtagctagtt agtctctagg gtcctagagg 915

tctacgagtg tgcatgcatg gatttggtgc gtttttcttt tttcatcttc attttgtttt 975

ttgaaacaag gaaccataaa cgaatatata tctaattctt gtttgatata tagtttggtc 1035

gaggcttcat gtcaagattt gctcattcgt agttagttga tctctagaga aattcaaaac 1095

acatggtgcc actaaaaaca caaaatgcaa atacttagct agagaactta atgatatgtt 1155

ttgtcttgat tttgcagggg aacgcaacta acggaagaga acgagcgact tggcatgcaa 1215

atatgtaaca atgtgcatgc acacggtggt gctgaatcgg agaacgctgc tgtgtacgag 1275

gaaggacagt cgtcggagtc tattactaac gccggaaact ctaccggagc gcctgttgac 1335

tccgagagct ccgacacttc ccttaggctc ggcttaccgt atggtggtta gagatggaac 1395

aattcaaaga agttgatgga gtgaggagag taatgtaaat ctttttaact cggtagtaac 1455

aagagacaat gtctaagtag tgaattctca aatgtttgtg taagtttctg cctatggaag 1515

aggctttcat ttttatgatt aaaaaaaaaa aaaaaaa 1552

<210> 26
<211> 210
<212> PRT
<213> Arabidopsis thaliana

<400> 26

Met Ala Arg Glu Lys Ile Gln Ile Arg Lys Ile Asp Asn Ala Thr Ala
1               5                   10                  15

Arg Gln Val Thr Phe Ser Lys Arg Arg Arg Gly Leu Phe Lys Lys Ala
            20                  25                  30

Glu Glu Leu Ser Val Leu Cys Asp Ala Asp Val Ala Leu Ile Ile Phe
        35                  40                  45

Ser Ser Thr Gly Lys Leu Phe Glu Phe Cys Ser Ser Ser Met Lys Glu
    50                  55                  60

Val Leu Glu Arg His Asn Leu Gln Ser Lys Asn Leu Glu Lys Leu Asp
65                  70                  75                  80

Gln Pro Ser Leu Glu Leu Gln Leu Val Glu Asn Ser Asp His Ala Arg
                85                  90                  95

Met Ser Lys Glu Ile Ala Asp Lys Ser His Arg Leu Arg Gln Met Arg
            100                 105                 110

Gly Glu Glu Leu Gln Gly Leu Asp Ile Glu Glu Leu Gln Gln Leu Glu
        115                 120                 125

Lys Ala Leu Glu Thr Gly Leu Thr Arg Val Ile Glu Thr Lys Ser Asp
    130                 135                 140

Lys Ile Met Ser Glu Ile Ser Glu Leu Gln Lys Lys Gly Met Gln Leu
145                 150                 155                 160

Met Asp Glu Asn Lys Arg Leu Arg Gln Gln Val Cys Val Leu Pro Ser
                165                 170                 175

Leu Leu Ile Thr Asn Pro Phe Leu Leu Ser Thr Ile Asn Val His Thr
            180                 185                 190

Pro Lys Phe Asn Pro Gln Leu Ser Thr Thr His Met Phe Asp His Thr
        195                 200                 205

Val Arg
    210

<210> 27
<211> 943
<212> DNA
<213> Arabidopsis thaliana

<220>
<221> CDS
<222> (110)..(700)
<223> G1759

<400> 27
   cgagaaaagg aaaaaaaaaa atagaaagag aaaacgctta gtatctccgg cgacttgaac 60
   ccaaacctga ggatcaaatt agggcacaaa gccctctcgg agagaagcc atg gga aga     118
                                                         Met Gly Arg
                                                         1
   aaa aaa cta gaa atc aag cga att gag aac aaa agt agc cga caa gtc 166
   Lys Lys Leu Glu Ile Lys Arg Ile Glu Asn Lys Ser Ser Arg Gln Val
   5 10 15
   acc ttc tcc aaa cgt cgc aac ggt ctc atc gag aaa gct cgt cag ctt 214
   Thr Phe Ser Lys Arg Arg Asn Gly Leu Ile Glu Lys Ala Arg Gln Leu
   20 25 30 35
   tct gtt ctc tgt gac gca tcc gtc gct ctt ctc gtc gtc tcc gcc tcc 262
   Ser Val Leu Cys Asp Ala Ser Val Ala Leu Leu Val Val Ser Ala Ser
   40 45 50
   ggc aag ctc tac agc ttc tcc tcc ggc gat aac ctg gtc aag atc ctt 310
   Gly Lys Leu Tyr Ser Phe Ser Ser Gly Asp Asn Leu Val Lys Ile Leu
   55 60 65
   gat cga tat ggg aaa cag cat gct gat gat ctt aaa gcc ttg gat cat 358
   Asp Arg Tyr Gly Lys Gln His Ala Asp Asp Leu Lys Ala Leu Asp His
   70 75 80
   cag tca aaa gct ctg aac tat ggt tca cac tat gag cta ctt gaa ctt 406
   Gln Ser Lys Ala Leu Asn Tyr Gly Ser His Tyr Glu Leu Leu Glu Leu
   85 90 95
   gtg gat agc aag ctt gtg gga tca aat gtc aaa aat gtg agt atc gat 454
   Val Asp Ser Lys Leu Val Gly Ser Asn Val Lys Asn Val Ser Ile Asp
   100 105 110 115
   gct ctt gtt caa ctg gag gaa cac ctt gag act gcc ctc tcc gtg act 502
   Ala Leu Val Gln Leu Glu Glu His Leu Glu Thr Ala Leu Ser Val Thr
   120 125 130
   aga gcc aag aag acc gaa ctc atg ttg aag ctt gtt gag aat ctt aaa 550
   Arg Ala Lys Lys Thr Glu Leu Met Leu Lys Leu Val Glu Asn Leu Lys
   135 140 145
   gaa aag gag aaa atg ctg aaa gaa gag aac cag gtt ttg gct agc cag     598
   Glu Lys Glu Lys Met Leu Lys Glu Glu Asn Gln Val Leu Ala Ser Gln
           150                 155                 160
   atg gag aat aat cat cat gtg gga gca gaa gct gag atg gag atg tca     646
   Met Glu Asn Asn His His Val Gly Ala Glu Ala Glu Met Glu Met Ser
       165                 170                 175
   cct gct gga caa atc tcc gac aat ctt ccg gtg act ctc cca cta ctt     694
   Pro Ala Gly Gln Ile Ser Asp Asn Leu Pro Val Thr Leu Pro Leu Leu
   180                 185                 190                 195
   aat tag ccaccttaaa tcggcggttg aaatcaaaat ccaaaacata tataattatg     750
   Asn
   aagaaaaaaa aaataagata tgtaattatt ccgctgataa gggcgagcgt ttgtatatct     810
   taatactctc tctttggcca agagactttg tgtgtgatac ttaagtagac ggaactaagt     870
   caatactatc tgttttaaga caaaaggttg atgaactttg taccttattc gtgtgagaaa     930
   aaaaaaaaaa aaa                                                        943

<210>  28
<211>  196
<212>  PRT
<213>  Arabidopsis thaliana

<400>  28

Met Gly Arg Lys Lys Leu Glu Ile Lys Arg Ile Glu Asn Lys Ser Ser
1               5                   10                  15

Arg Gln Val Thr Phe Ser Lys Arg Arg Asn Gly Leu Ile Glu Lys Ala
            20                  25                  30

Arg Gln Leu Ser Val Leu Cys Asp Ala Ser Val Ala Leu Leu Val Val
        35                  40                  45

Ser Ala Ser Gly Lys Leu Tyr Ser Phe Ser Ser Gly Asp Asn Leu Val
    50                  55                  60

Lys Ile Leu Asp Arg Tyr Gly Lys Gln His Ala Asp Asp Leu Lys Ala
65                  70                  75                  80

Leu Asp His Gln Ser Lys Ala Leu Asn Tyr Gly Ser His Tyr Glu Leu
                85                  90                  95

Leu Glu Leu Val Asp Ser Lys Leu Val Gly Ser Asn Val Lys Asn Val
            100                 105                 110

Ser Ile Asp Ala Leu Val Gln Leu Glu Glu His Leu Glu Thr Ala Leu
        115                 120                 125

Ser Val Thr Arg Ala Lys Lys Thr Glu Leu Met Leu Lys Leu Val Glu
    130                 135                 140

Asn Leu Lys Glu Lys Glu Lys Met Leu Lys Glu Glu Asn Gln Val Leu
145                 150                 155                 160

Ala Ser Gln Met Glu Asn Asn His His Val Gly Ala Glu Ala Glu Met
                165                 170                 175

Glu Met Ser Pro Ala Gly Gln Ile Ser Asp Asn Leu Pro Val Thr Leu
            180                 185                 190

Pro Leu Leu Asn
        195

<210>  29
<211>  1171
<212>  DNA
<213>  Arabidopsis thaliana

<220>
<221>  CDS
<222>  (63)..(959)
<223>  G192

<400>  29
cttttttctc ttctctcctc agagattcga agctttttgt ctcccctgag taaccaaatt      60

ca atg gcc gac gat tgg gat ctc cac gcc gta gtc aga ggc tgc tca       107
   Met Ala Asp Asp Trp Asp Leu His Ala Val Val Arg Gly Cys Ser
   1               5                   10                  15

gcc gta agc tca tca gct act acc acc gta tat tcc ccc ggc gtt tca      155
Ala Val Ser Ser Ser Ala Thr Thr Thr Val Tyr Ser Pro Gly Val Ser
                20                  25                  30

tct cac aca aac cct ata ttc acc gtc gga cga caa agt aat gcc gtc      203
Ser His Thr Asn Pro Ile Phe Thr Val Gly Arg Gln Ser Asn Ala Val
            35                  40                  45

tcc ttc gga gag att cga gat ctc tac aca ccg ttc aca caa gaa tct      251
Ser Phe Gly Glu Ile Arg Asp Leu Tyr Thr Pro Phe Thr Gln Glu Ser
        50                  55                  60

gtc gtc tct tcg ttt tct tgt ata aac tac cca gaa gaa cct aga aag      299
Val Val Ser Ser Phe Ser Cys Ile Asn Tyr Pro Glu Glu Pro Arg Lys
    65                  70                  75

cca cag aac cag aaa cgt cct ctt tct ctc tct gct tct tcc ggt agc      347
Pro Gln Asn Gln Lys Arg Pro Leu Ser Leu Ser Ala Ser Ser Gly Ser
80                  85                  90                  95

gtc act agc aaa ccc agt ggc tcc aat acc tct aga tct aaa aga aga      395
Val Thr Ser Lys Pro Ser Gly Ser Asn Thr Ser Arg Ser Lys Arg Arg
                100                 105                 110

aag ata cag cat aag aaa gtg tgc cat gta gca gca gaa gct tta aac 443
Lys Ile Gln His Lys Lys Val Cys His Val Ala Ala Glu Ala Leu Asn
            115                 120                 125

tcc gat gtc tgg gca tgg cga aag tac gga cag aaa ccc atc aaa ggt 491
Ser Asp Val Trp Ala Trp Arg Lys Tyr Gly Gln Lys Pro Ile Lys Gly
        130                 135                 140

tca cca tat cca aga gga tac tac aga tgt agt aca tca aaa ggt tgt 539
Ser Pro Tyr Pro Arg Gly Tyr Tyr Arg Cys Ser Thr Ser Lys Gly Cys
    145                 150                 155

tta gcc cgt aaa caa gtg gag cga aat aga tcc gac ccg aag atg ttt 587
Leu Ala Arg Lys Gln Val Glu Arg Asn Arg Ser Asp Pro Lys Met Phe
160                 165                 170                 175

atc gtc act tac acg gcg gag cat aat cat cca gct ccg aca cac cgt 635
Ile Val Thr Tyr Thr Ala Glu His Asn His Pro Ala Pro Thr His Arg
                180                 185                 190

aat tct ctc gcc gga agc aca cgt cag aaa cca tcc gat caa cag acg 683
Asn Ser Leu Ala Gly Ser Thr Arg Gln Lys Pro Ser Asp Gln Gln Thr
            195                 200                 205

agt aaa tct ccg acg acc act att gct act tat tca tcg tct ccg gtg 731
Ser Lys Ser Pro Thr Thr Thr Ile Ala Thr Tyr Ser Ser Ser Pro Val
        210                 215                 220

act tca gcc gac gaa ttt gtt ttg cct gtt gag gat cat cta gcg gtg 779
Thr Ser Ala Asp Glu Phe Val Leu Pro Val Glu Asp His Leu Ala Val
    225                 230                 235

gga gat ctt gac gga gaa gaa gat ctg tta tct ttg tcg gat acg gtg 827
Gly Asp Leu Asp Gly Glu Glu Asp Leu Leu Ser Leu Ser Asp Thr Val
240                 245                 250                 255

gtt agc gat gat ttc ttc gat ggg tta gag gaa ttc gca gcc gga gat 875
Val Ser Asp Asp Phe Phe Asp Gly Leu Glu Glu Phe Ala Ala Gly Asp
                260                 265                 270

agc ttt tcc ggg aac tcg gct ccg gcg agt ttt gat ctc tct tgg gtt 923
Ser Phe Ser Gly Asn Ser Ala Pro Ala Ser Phe Asp Leu Ser Trp Val
            275                 280                 285

gtg aac agt gcc gcc act acc acc gga gga ata tga ttagattacg 969
Val Asn Ser Ala Ala Thr Thr Thr Gly Gly Ile
        290                 295

acggcttaga atactcttat taggacagat ttataggatt aaggaattat tctcggagca 1029

tatgtaaaaa taggataaaa gaaaatgttc tttgttactt tttttcgggt tttcttccta 1089

ttgtttctaa acatcttaga aaaaatttaa ttgtatattc cttaagctcg atacatcttg 1149

ttttaaaaaa aaaaaaaaaa aa 1171

<210> 30
<211> 298
<212> PRT
<213> Arabidopsis thaliana

<400> 30

Met Ala Asp Asp Trp Asp Leu His Ala Val Val Arg Gly Cys Ser Ala
1               5                   10                  15

Val Ser Ser Ser Ala Thr Thr Thr Val Tyr Ser Pro Gly Val Ser Ser
            20                  25                  30

His Thr Asn Pro Ile Phe Thr Val Gly Arg Gln Ser Asn Ala Val Ser
        35                  40                  45

Phe Gly Glu Ile Arg Asp Leu Tyr Thr Pro Phe Thr Gln Glu Ser Val
    50                  55                  60

Val Ser Ser Phe Ser Cys Ile Asn Tyr Pro Glu Glu Pro Arg Lys Pro
65                  70                  75                  80

Gln Asn Gln Lys Arg Pro Leu Ser Leu Ser Ala Ser Ser Gly Ser Val
                85                  90                  95

Thr Ser Lys Pro Ser Gly Ser Asn Thr Ser Arg Ser Lys Arg Arg Lys
            100                 105                 110

Ile Gln His Lys Lys Val Cys His Val Ala Ala Glu Ala Leu Asn Ser
        115                 120                 125

Asp Val Trp Ala Trp Arg Lys Tyr Gly Gln Lys Pro Ile Lys Gly Ser
    130                 135                 140

Pro Tyr Pro Arg Gly Tyr Tyr Arg Cys Ser Thr Ser Lys Gly Cys Leu
145                 150                 155                 160

Ala Arg Lys Gln Val Glu Arg Asn Arg Ser Asp Pro Lys Met Phe Ile
                165                 170                 175

Val Thr Tyr Thr Ala Glu His Asn His Pro Ala Pro Thr His Arg Asn
            180                 185                 190

Ser Leu Ala Gly Ser Thr Arg Gln Lys Pro Ser Asp Gln Gln Thr Ser
        195                 200                 205

Lys Ser Pro Thr Thr Thr Ile Ala Thr Tyr Ser Ser Ser Pro Val Thr
    210                 215                 220

Ser Ala Asp Glu Phe Val Leu Pro Val Glu Asp His Leu Ala Val Gly
225                 230                 235                 240

Asp Leu Asp Gly Glu Glu Asp Leu Leu Ser Leu Ser Asp Thr Val Val
                245                 250                 255

Ser Asp Asp Phe Phe Asp Gly Leu Glu Glu Phe Ala Ala Gly Asp Ser
            260                 265                 270

Phe Ser Gly Asn Ser Ala Pro Ala Ser Phe Asp Leu Ser Trp Val Val
        275                 280                 285

Asn Ser Ala Ala Thr Thr Thr Gly Gly Ile
    290                 295

<210> 31
<211> 1139
<212> DNA
<213> Arabidopsis thaliana

<220>
<221> CDS
<222> (106)..(1032)
<223> G234

<400> 31
cacaacatca tacccaccaa catatataat cttgatcata gagagataaa cagaggccgc 60

tatcaagaac aagactaaga acaagacttc actaggagta caagt atg gga aga gca 117
Met Gly Arg Ala
1

ccg tgt tgt gac aaa gca aac gtg aag aaa ggg cct tgg tct cct gag 165
Pro Cys Cys Asp Lys Ala Asn Val Lys Lys Gly Pro Trp Ser Pro Glu
5                   10                  15                  20

gaa gat gca aaa ctc aaa tct tac att gaa aat agt ggc acc gga ggc 213
Glu Asp Ala Lys Leu Lys Ser Tyr Ile Glu Asn Ser Gly Thr Gly Gly
                25                  30                  35

aat tgg atc gct ttg cct caa aag att ggt tta aag aga tgt gga aag 261
Asn Trp Ile Ala Leu Pro Gln Lys Ile Gly Leu Lys Arg Cys Gly Lys
            40                  45                  50

agt tgc agg ctg agg tgg ctt aac tat ctt aga cca aac atc aaa cat 309
Ser Cys Arg Leu Arg Trp Leu Asn Tyr Leu Arg Pro Asn Ile Lys His
        55                  60                  65

ggt ggc ttc tct gag gaa gaa gaa aac atc att tgt agc ctt tac ctt 357
Gly Gly Phe Ser Glu Glu Glu Glu Asn Ile Ile Cys Ser Leu Tyr Leu
    70                  75                  80

aca att ggt agc agg tgg tct ata atc gct gct caa ttg ccg gga cga 405
Thr Ile Gly Ser Arg Trp Ser Ile Ile Ala Ala Gln Leu Pro Gly Arg
85                  90                  95                  100

aca gac aac gat ata aaa aac tat tgg aac acg agg ctc aag aag aaa 453
Thr Asp Asn Asp Ile Lys Asn Tyr Trp Asn Thr Arg Leu Lys Lys Lys
                105                 110                 115

ctc att aac aaa caa cgc aag gag ctt caa gaa gct tgt atg gag cag 501
Leu Ile Asn Lys Gln Arg Lys Glu Leu Gln Glu Ala Cys Met Glu Gln
            120                 125                 130

caa gag atg atg gtg atg atg aag aga caa cac caa caa caa caa atc 549
Gln Glu Met Met Val Met Met Lys Arg Gln His Gln Gln Gln Gln Ile
        135                 140                 145

caa act tct ttt atg atg aga caa gac caa aca atg ttc aca tgg cca 597
Gln Thr Ser Phe Met Met Arg Gln Asp Gln Thr Met Phe Thr Trp Pro
    150                 155                 160

cta cat cat cat aat gtt caa gtt cca gct ctt ttc aga atc aaa cca 645
Leu His His His Asn Val Gln Val Pro Ala Leu Phe Arg Ile Lys Pro
165                 170                 175                 180

act cgt ttt gcg acc aag aag atg tta agc cag tgc tca tca aga aca 693
Thr Arg Phe Ala Thr Lys Lys Met Leu Ser Gln Cys Ser Ser Arg Thr
                185                 190                 195

tgg tca aga tcg aag atc aag aac tgg aga aaa caa acc tca tca tca 741
Trp Ser Arg Ser Lys Ile Lys Asn Trp Arg Lys Gln Thr Ser Ser Ser
            200                 205                 210

tca aga ttc aat gac aac gct ttt gat cat ctc tct ttc tct caa ctc 789
Ser Arg Phe Asn Asp Asn Ala Phe Asp His Leu Ser Phe Ser Gln Leu
        215                 220                 225

ttg tta gat cct aat cat aac cac tta gga tca gga gag ggt ttc tcc 837
Leu Leu Asp Pro Asn His Asn His Leu Gly Ser Gly Glu Gly Phe Ser
    230                 235                 240

atg aac tct atc ttg agc gcc aac aca aac tct cca ttg ctt aac aca 885
Met Asn Ser Ile Leu Ser Ala Asn Thr Asn Ser Pro Leu Leu Asn Thr
245                 250                 255                 260

agt aat gat aat cag tgg ttc ggg aat ttc cag gcc gaa acc gta aac 933
Ser Asn Asp Asn Gln Trp Phe Gly Asn Phe Gln Ala Glu Thr Val Asn
                265                 270                 275

ttg ttc tca gga gcc tcc aca agt act tcg gca gat caa agc act ata 981
Leu Phe Ser Gly Ala Ser Thr Ser Thr Ser Ala Asp Gln Ser Thr Ile
            280                 285                 290

agt tgg gaa gac ata agc tct ctt gtt tat tct gat tca aag caa ttt 1029
Ser Trp Glu Asp Ile Ser Ser Leu Val Tyr Ser Asp Ser Lys Gln Phe
        295                 300                 305

ttt taattataat aatatattat tcttaagatg aaacgtacat cattattatt 1082
Phe

aattgggggt acgtaacgta tatatggaat aacgatctag tttgtttaaa tttaaaa 1139

<210> 32
<211> 309
<212> PRT
<213> Arabidopsis thaliana

<400> 32

Met Gly Arg Ala Pro Cys Cys Asp Lys Ala Asn Val Lys Lys Gly Pro
1               5                   10                  15

Trp Ser Pro Glu Glu Asp Ala Lys Leu Lys Ser Tyr Ile Glu Asn Ser
        20                  25                  30

Gly Thr Gly Gly Asn Trp Ile Ala Leu Pro Gln Lys Ile Gly Leu Lys
        35                  40                  45

Arg Cys Gly Lys Ser Cys Arg Leu Arg Trp Leu Asn Tyr Leu Arg Pro
    50                  55                  60

Asn Ile Lys His Gly Gly Phe Ser Glu Glu Glu Glu Asn Ile Ile Cys
65                  70                  75                  80

Ser Leu Tyr Leu Thr Ile Gly Ser Arg Trp Ser Ile Ile Ala Ala Gln
                85                  90                  95

Leu Pro Gly Arg Thr Asp Asn Asp Ile Lys Asn Tyr Trp Asn Thr Arg
            100                 105                 110

Leu Lys Lys Lys Leu Ile Asn Lys Gln Arg Lys Glu Leu Gln Glu Ala
        115                 120                 125

Cys Met Glu Gln Gln Glu Met Met Val Met Met Lys Arg Gln His Gln
    130                 135                 140

Gln Gln Gln Ile Gln Thr Ser Phe Met Met Arg Gln Asp Gln Thr Met
145                 150                 155                 160

Phe Thr Trp Pro Leu His His His Asn Val Gln Val Pro Ala Leu Phe
                165                 170                 175

Arg Ile Lys Pro Thr Arg Phe Ala Thr Lys Lys Met Leu Ser Gln Cys
            180                 185                 190

Ser Ser Arg Thr Trp Ser Arg Ser Lys Ile Lys Asn Trp Arg Lys Gln
        195                 200                 205

Thr Ser Ser Ser Ser Arg Phe Asn Asp Asn Ala Phe Asp His Leu Ser
    210                 215                 220

Phe Ser Gln Leu Leu Leu Asp Pro Asn His Asn His Leu Gly Ser Gly
225                 230                 235                 240

Glu Gly Phe Ser Met Asn Ser Ile Leu Ser Ala Asn Thr Asn Ser Pro
                245                 250                 255

Leu Leu Asn Thr Ser Asn Asp Asn Gln Trp Phe Gly Asn Phe Gln Ala
            260                 265                 270

Glu Thr Val Asn Leu Phe Ser Gly Ala Ser Thr Ser Thr Ser Ala Asp
        275                 280                 285

Gln Ser Thr Ile Ser Trp Glu Asp Ile Ser Ser Leu Val Tyr Ser Asp
    290                 295                 300

Ser Lys Gln Phe Phe
305

<210>  33
<211>  922
<212>  DNA
<213>  Arabidopsis thaliana

<220>
<221>  CDS
<222>  (54)..(647)
<223>  G361

<400>  33
tctgtctctc tctctctctt tgtaaatata catatataga taagctcaca tat atg      56
                                                           Met
                                                           1

gcg act gaa aca tct tct ttg aag ctc ttc ggt ata aac cta ctt gaa      104
Ala Thr Glu Thr Ser Ser Leu Lys Leu Phe Gly Ile Asn Leu Leu Glu
            5                   10                  15

acg acg tcg gtt caa aac cag tca tcg gaa cca aga ccc gga tcc gga      152
Thr Thr Ser Val Gln Asn Gln Ser Ser Glu Pro Arg Pro Gly Ser Gly
        20                  25                  30

tca gga tcc gag tca cgt aag tac gag tgt caa tac tgt tgt aga gag      200
Ser Gly Ser Glu Ser Arg Lys Tyr Glu Cys Gln Tyr Cys Cys Arg Glu
    35                  40                  45

ttt gct aac tct caa gct ctt ggt ggt cac caa aac gct cac aag aaa      248
Phe Ala Asn Ser Gln Ala Leu Gly Gly His Gln Asn Ala His Lys Lys
50                  55                  60                  65

gag cgt cag ctt ctt aaa cgt gca cag atg tta gct act cgt ggt ttg      296
Glu Arg Gln Leu Leu Lys Arg Ala Gln Met Leu Ala Thr Arg Gly Leu
                70                  75                  80

cca cgt cat cat aat ttt cac cct cat acc aat ccg ctt ctc tcc gcc      344
Pro Arg His His Asn Phe His Pro His Thr Asn Pro Leu Leu Ser Ala
            85                  90                  95

ttc gcg ccg ctg cct cac ctc ctc tct cag ccg cat cct ccg ccg cat      392
Phe Ala Pro Leu Pro His Leu Leu Ser Gln Pro His Pro Pro Pro His
        100                 105                 110

atg atg ctc tct cct tct tct tcg agt tct aag tgg ctt tac ggt gaa      440
Met Met Leu Ser Pro Ser Ser Ser Ser Ser Lys Trp Leu Tyr Gly Glu
    115                 120                 125

cac atg tcg tca caa aac gcc gtt ggg tac ttt cat ggt gga agg gga      488
His Met Ser Ser Gln Asn Ala Val Gly Tyr Phe His Gly Gly Arg Gly

130                 135                 140                 145

ctt tac gga ggt ggc atg gag tct atg gcc gga gaa gta aag act cat 536
Leu Tyr Gly Gly Gly Met Glu Ser Met Ala Gly Glu Val Lys Thr His
                150                 155                 160

ggt ggt tct ttg ccg gag atg agg agg ttc gcc gga gat agt gat cgg 584
Gly Gly Ser Leu Pro Glu Met Arg Arg Phe Ala Gly Asp Ser Asp Arg
            165                 170                 175

agt agc gga att aag tta gag aat ggt att ggg ctg gac ctc cat tta 632
Ser Ser Gly Ile Lys Leu Glu Asn Gly Ile Gly Leu Asp Leu His Leu
        180                 185                 190

agc ctt ggg cca tga atgattataa ttttggccca gtaaagatct gtaaaatact 687
Ser Leu Gly Pro
    195

actaggattt catttttata gagtatgttt ttttccttaa tttcggttga aattggtgaa 747

tatttttatc tcttacttac caaatctcat atttctatgt atgcgtttgc tttcactttt 807

tttttttata taattcttct tgtaaaaaat gcaatgtgag ttttcttccc tatcattctg 867

tcaagctttg gttcaattat ttagtaatcg aataatatag gaatagtgtt gaaag 922

<210> 34
<211> 197
<212> PRT
<213> Arabidopsis thaliana

<400> 34

Met Ala Thr Glu Thr Ser Ser Leu Lys Leu Phe Gly Ile Asn Leu Leu
1               5                   10                  15

Glu Thr Thr Ser Val Gln Asn Gln Ser Ser Glu Pro Arg Pro Gly Ser
            20                  25                  30

Gly Ser Gly Ser Glu Ser Arg Lys Tyr Glu Cys Gln Tyr Cys Cys Arg
        35                  40                  45

Glu Phe Ala Asn Ser Gln Ala Leu Gly Gly His Gln Asn Ala His Lys
    50                  55                  60

Lys Glu Arg Gln Leu Leu Lys Arg Ala Gln Met Leu Ala Thr Arg Gly
65                  70                  75                  80

Leu Pro Arg His His Asn Phe His Pro His Thr Asn Pro Leu Leu Ser
                85                  90                  95

Ala Phe Ala Pro Leu Pro His Leu Leu Ser Gln Pro His Pro Pro Pro
            100                 105                 110

His Met Met Leu Ser Pro Ser Ser Ser Ser Ser Lys Trp Leu Tyr Gly

115                 120                 125

Glu His Met Ser Ser Gln Asn Ala Val Gly Tyr Phe His Gly Gly Arg
    130                 135                 140

Gly Leu Tyr Gly Gly Gly Met Glu Ser Met Ala Gly Glu Val Lys Thr
145                 150                 155                 160

His Gly Gly Ser Leu Pro Glu Met Arg Arg Phe Ala Gly Asp Ser Asp
                165                 170                 175

Arg Ser Ser Gly Ile Lys Leu Glu Asn Gly Ile Gly Leu Asp Leu His
            180                 185                 190

Leu Ser Leu Gly Pro
        195

<210>  35
<211>  420
<212>  DNA
<213>  Arabidopsis thaliana

<220>
<221>  CDS
<222>  (1)..(420)
<223>  G486

<400>  35
atg aca gac gaa gat aga ttg ttg cca ata gcc aat gta ggg aga ctt        48
Met Thr Asp Glu Asp Arg Leu Leu Pro Ile Ala Asn Val Gly Arg Leu
1               5                   10                  15

atg aag caa atc cta cca tca aat gca aag atc tca aaa gaa gca aaa        96
Met Lys Gln Ile Leu Pro Ser Asn Ala Lys Ile Ser Lys Glu Ala Lys
            20                  25                  30

caa aca gtt caa gaa tgt gca aca gag ttc ata agc ttt gtt aca tgc        144
Gln Thr Val Gln Glu Cys Ala Thr Glu Phe Ile Ser Phe Val Thr Cys
        35                  40                  45

gaa gca tca gag aag tgc cac agg gag aat cgg aag acg gtg aat gga        192
Glu Ala Ser Glu Lys Cys His Arg Glu Asn Arg Lys Thr Val Asn Gly
    50                  55                  60

gac gac atc tgg tgg gct ctc agc act ctc ggc ctc gat aac tat gct        240
Asp Asp Ile Trp Trp Ala Leu Ser Thr Leu Gly Leu Asp Asn Tyr Ala
65                  70                  75                  80

gac gcc gtg ggt agg cat ctt cac aag tac cgt gaa gcc gag aga gaa        288
Asp Ala Val Gly Arg His Leu His Lys Tyr Arg Glu Ala Glu Arg Glu
                85                  90                  95

aga act gag cac aac aaa ggt agc aat gat agt ggg aat gag aaa gaa        336
Arg Thr Glu His Asn Lys Gly Ser Asn Asp Ser Gly Asn Glu Lys Glu
            100                 105                 110

acc aac act aga agt gat gta cag aac caa tcg aca aaa ttt att aga     384
Thr Asn Thr Arg Ser Asp Val Gln Asn Gln Ser Thr Lys Phe Ile Arg
        115                 120                 125

gtt gtt gag aag gga agc agc tcc tcg gcc cgt tga                     420
Val Val Glu Lys Gly Ser Ser Ser Ser Ala Arg
    130                 135

<210>  36
<211>  139
<212>  PRT
<213>  Arabidopsis thaliana

<400>  36

Met Thr Asp Glu Asp Arg Leu Leu Pro Ile Ala Asn Val Gly Arg Leu
1               5                   10                  15

Met Lys Gln Ile Leu Pro Ser Asn Ala Lys Ile Ser Lys Glu Ala Lys
            20                  25                  30

Gln Thr Val Gln Glu Cys Ala Thr Glu Phe Ile Ser Phe Val Thr Cys
        35                  40                  45

Glu Ala Ser Glu Lys Cys His Arg Glu Asn Arg Lys Thr Val Asn Gly
    50                  55                  60

Asp Asp Ile Trp Trp Ala Leu Ser Thr Leu Gly Leu Asp Asn Tyr Ala
65                  70                  75                  80

Asp Ala Val Gly Arg His Leu His Lys Tyr Arg Glu Ala Glu Arg Glu
                85                  90                  95

Arg Thr Glu His Asn Lys Gly Ser Asn Asp Ser Gly Asn Glu Lys Glu
            100                 105                 110

Thr Asn Thr Arg Ser Asp Val Gln Asn Gln Ser Thr Lys Phe Ile Arg
        115                 120                 125

Val Val Glu Lys Gly Ser Ser Ser Ser Ala Arg
    130                 135

<210>  37
<211>  1707
<212>  DNA
<213>  Arabidopsis thaliana

<220>
<221>  CDS
<222>  (98)..(1444)
<223>  G748

<400> 37
ccacgcgtcc gcactctccc aaatctctct tctttaacaa caaaaaaaaa atcacagaga 60

catagagaga agaagacgga acagaggctc caaaaaa atg atg atg gag act aga 115
Met Met Met Glu Thr Arg
1               5

gat cca gct att aag ctt ttc ggt atg aaa atc cct ttt ccg tcg gtt 163
Asp Pro Ala Ile Lys Leu Phe Gly Met Lys Ile Pro Phe Pro Ser Val
            10                  15                  20

ttt gaa tcg gca gtt acg gtg gag gat gac gaa gaa gat gac tgg agc 211
Phe Glu Ser Ala Val Thr Val Glu Asp Asp Glu Glu Asp Asp Trp Ser
        25                  30                  35

ggc gga gat gac aaa tca cca gag aag gta act cca gag tta tca gat 259
Gly Gly Asp Asp Lys Ser Pro Glu Lys Val Thr Pro Glu Leu Ser Asp
    40                  45                  50

aag aac aac aac aac tgt aac gac aac agt ttt aac aat tcg aaa ccc 307
Lys Asn Asn Asn Asn Cys Asn Asp Asn Ser Phe Asn Asn Ser Lys Pro
55                  60                  65                  70

gaa acc ttg gac aaa gag gaa gcg aca tca act gat cag ata gag agt 355
Glu Thr Leu Asp Lys Glu Glu Ala Thr Ser Thr Asp Gln Ile Glu Ser
                75                  80                  85

agt gac acg cct gag gat aat cag cag acg aca cct gat ggt aaa acc 403
Ser Asp Thr Pro Glu Asp Asn Gln Gln Thr Thr Pro Asp Gly Lys Thr
            90                  95                  100

cta aag aaa ccg act aag att cta ccg tgt ccg aga tgc aaa agc atg 451
Leu Lys Lys Pro Thr Lys Ile Leu Pro Cys Pro Arg Cys Lys Ser Met
        105                 110                 115

gag acc aag ttc tgt tat tac aac aac tac aac ata aac cag cct cgt 499
Glu Thr Lys Phe Cys Tyr Tyr Asn Asn Tyr Asn Ile Asn Gln Pro Arg
    120                 125                 130

cat ttc tgc aag gct tgt cag aga tat tgg act gct gga ggg act atg 547
His Phe Cys Lys Ala Cys Gln Arg Tyr Trp Thr Ala Gly Gly Thr Met
135                 140                 145                 150

agg aat gtt cct gtg ggg gca gga cgt cgt aag aac aaa agc tca tct 595
Arg Asn Val Pro Val Gly Ala Gly Arg Arg Lys Asn Lys Ser Ser Ser
                155                 160                 165

tct cat tac cgt cac atc act att tcc gag gct ctt gag gct gcg agg 643
Ser His Tyr Arg His Ile Thr Ile Ser Glu Ala Leu Glu Ala Ala Arg
            170                 175                 180

ctt gac ccg ggc tta cag gca aac aca agg gtc ttg agt ttt ggt ctc 691
Leu Asp Pro Gly Leu Gln Ala Asn Thr Arg Val Leu Ser Phe Gly Leu
        185                 190                 195

gaa gct cag cag cag cac gtt gct gct ccc atg aca cct gtt atg aag 739
Glu Ala Gln Gln Gln His Val Ala Ala Pro Met Thr Pro Val Met Lys
    200                 205                 210

cta caa gaa gat caa aag gtc tca aac ggt gct agg aac agg ttt cac 787
Leu Gln Glu Asp Gln Lys Val Ser Asn Gly Ala Arg Asn Arg Phe His
215                 220                 225                 230

ggg tta gcg gat caa cgg ctt gta gct cgg gta gag aat gga gat gat 835
Gly Leu Ala Asp Gln Arg Leu Val Ala Arg Val Glu Asn Gly Asp Asp
                235                 240                 245

tgc tca agc gga tcc tct gtg acc acc tct aac aat cac tca gtg gat 883
Cys Ser Ser Gly Ser Ser Val Thr Thr Ser Asn Asn His Ser Val Asp
            250                 255                 260

gaa tca aga gca caa agc ggc agt gtt gtt gaa gca caa atg aac aac 931
Glu Ser Arg Ala Gln Ser Gly Ser Val Val Glu Ala Gln Met Asn Asn
        265                 270                 275

aac aac aac aat aac atg aat ggt tat gct tgc atc cca ggt gtt cca 979
Asn Asn Asn Asn Asn Met Asn Gly Tyr Ala Cys Ile Pro Gly Val Pro
    280                 285                 290

tgg cct tac acg tgg aat cca gcg atg cct cca cca ggt ttt tac ccg 1027
Trp Pro Tyr Thr Trp Asn Pro Ala Met Pro Pro Pro Gly Phe Tyr Pro
295                 300                 305                 310

cct cca ggg tat cca atg ccg ttt tac cct tac tgg acc atc cca atg 1075
Pro Pro Gly Tyr Pro Met Pro Phe Tyr Pro Tyr Trp Thr Ile Pro Met
                315                 320                 325

cta cca ccg cat caa tcc tca tcg cct ata agc caa aag tgt tca aat 1123
Leu Pro Pro His Gln Ser Ser Ser Pro Ile Ser Gln Lys Cys Ser Asn
            330                 335                 340

aca aac tct ccg act ctc gga aag cat ccg aga gat gaa gga tca tcg 1171
Thr Asn Ser Pro Thr Leu Gly Lys His Pro Arg Asp Glu Gly Ser Ser
        345                 350                 355

aaa aag gac aat gag aca gag cga aaa cag aag gcc ggg tgc gtt ctg 1219
Lys Lys Asp Asn Glu Thr Glu Arg Lys Gln Lys Ala Gly Cys Val Leu
    360                 365                 370

gtc ccg aaa acg ttg aga ata gat gat cct aac gaa gca gca aag agc 1267
Val Pro Lys Thr Leu Arg Ile Asp Asp Pro Asn Glu Ala Ala Lys Ser
375                 380                 385                 390

tcg ata tgg aca aca ttg gga atc aag aac gag gcg atg tgc aaa gcc 1315
Ser Ile Trp Thr Thr Leu Gly Ile Lys Asn Glu Ala Met Cys Lys Ala
                395                 400                 405

ggt ggt atg ttc aaa ggg ttt gat cat aag aca aag atg tat aac aac 1363
Gly Gly Met Phe Lys Gly Phe Asp His Lys Thr Lys Met Tyr Asn Asn
            410                 415                 420

gac aaa gct gag aac tcc cct gtt ctt tct gct aac cct gct gct cta 1411
Asp Lys Ala Glu Asn Ser Pro Val Leu Ser Ala Asn Pro Ala Ala Leu
        425                 430                 435

tca aga tca cac aat ttc cat gaa cag att tag agttacatat gtatatgtat 1464
Ser Arg Ser His Asn Phe His Glu Gln Ile
    440                 445

atatgtatga ttgattgtat gtatagatga tactggagaa tgatgagttt ttgagaatca 1524

aactcttttc ttctttctag tgattgcctt tattccttta catgttttgg ttctctgtac 1584

actatttgat ttaccttttt tactttcttt cttcatttgt caggaaatgt tggaagataa 1644

cattaatggt aaaaagttgg tgtggaccgt tgttgcgttg gcatttcaaa aaaaaaaaaa 1704

aaa                                                                 1707

<210>  38
<211>  448
<212>  PRT
<213>  Arabidopsis thaliana

<400>  38

Met Met Met Glu Thr Arg Asp Pro Ala Ile Lys Leu Phe Gly Met Lys
1               5                   10                  15

Ile Pro Phe Pro Ser Val Phe Glu Ser Ala Val Thr Val Glu Asp Asp
            20                  25                  30

Glu Glu Asp Asp Trp Ser Gly Gly Asp Asp Lys Ser Pro Glu Lys Val
        35                  40                  45

Thr Pro Glu Leu Ser Asp Lys Asn Asn Asn Asn Cys Asn Asp Asn Ser
    50                  55                  60

Phe Asn Asn Ser Lys Pro Glu Thr Leu Asp Lys Glu Glu Ala Thr Ser
65                  70                  75                  80

Thr Asp Gln Ile Glu Ser Ser Asp Thr Pro Glu Asp Asn Gln Gln Thr
                85                  90                  95

Thr Pro Asp Gly Lys Thr Leu Lys Lys Pro Thr Lys Ile Leu Pro Cys
            100                 105                 110

Pro Arg Cys Lys Ser Met Glu Thr Lys Phe Cys Tyr Tyr Asn Asn Tyr
        115                 120                 125

Asn Ile Asn Gln Pro Arg His Phe Cys Lys Ala Cys Gln Arg Tyr Trp
    130                 135                 140

Thr Ala Gly Gly Thr Met Arg Asn Val Pro Val Gly Ala Gly Arg Arg
145                 150                 155                 160

Lys Asn Lys Ser Ser Ser Ser His Tyr Arg His Ile Thr Ile Ser Glu
                165                 170                 175

Ala Leu Glu Ala Ala Arg Leu Asp Pro Gly Leu Gln Ala Asn Thr Arg
            180                 185                 190

Val Leu Ser Phe Gly Leu Glu Ala Gln Gln Gln His Val Ala Ala Pro
        195                 200                 205

Met Thr Pro Val Met Lys Leu Gln Glu Asp Gln Lys Val Ser Asn Gly
    210                 215                 220

Ala Arg Asn Arg Phe His Gly Leu Ala Asp Gln Arg Leu Val Ala Arg
225                 230                 235                 240

Val Glu Asn Gly Asp Asp Cys Ser Ser Gly Ser Ser Val Thr Thr Ser
                245                 250                 255

Asn Asn His Ser Val Asp Glu Ser Arg Ala Gln Ser Gly Ser Val Val
            260                 265                 270

Glu Ala Gln Met Asn Asn Asn Asn Asn Asn Asn Met Asn Gly Tyr Ala
        275                 280                 285

Cys Ile Pro Gly Val Pro Trp Pro Tyr Thr Trp Asn Pro Ala Met Pro
    290                 295                 300

Pro Pro Gly Phe Tyr Pro Pro Pro Gly Tyr Pro Met Pro Phe Tyr Pro
305                 310                 315                 320

Tyr Trp Thr Ile Pro Met Leu Pro Pro His Gln Ser Ser Ser Pro Ile
                325                 330                 335

Ser Gln Lys Cys Ser Asn Thr Asn Ser Pro Thr Leu Gly Lys His Pro
            340                 345                 350

Arg Asp Glu Gly Ser Ser Lys Lys Asp Asn Glu Thr Glu Arg Lys Gln
        355                 360                 365

Lys Ala Gly Cys Val Leu Val Pro Lys Thr Leu Arg Ile Asp Asp Pro
    370                 375                 380

Asn Glu Ala Ala Lys Ser Ser Ile Trp Thr Thr Leu Gly Ile Lys Asn
385                 390                 395                 400

Glu Ala Met Cys Lys Ala Gly Gly Met Phe Lys Gly Phe Asp His Lys
                405                 410                 415

Thr Lys Met Tyr Asn Asn Asp Lys Ala Glu Asn Ser Pro Val Leu Ser
            420                 425                 430

Ala Asn Pro Ala Ala Leu Ser Arg Ser His Asn Phe His Glu Gln Ile
        435                 440                 445

<210>  39
<211>  1095
<212>  DNA

<213>  Arabidopsis thaliana

<220>
<221>  CDS
<222>  (180)..(917)
<223>  G994


<400>  39
tgtatatata gttagttagt tgagataaac ttggttacca cttttgtgtg gtctttcttt      60

ttctttttct ccattttcca tttatcgacc ccttgggtgt agctaattac tttcgcgatt     120

ttcaaatcca ataaagtttt aatttgatga agcttttttt aaaccatata atataaata      179

atg ggt ggt cgt aaa cca tgt tgt gat gag gtt gga tta aga aag ggt      227
Met Gly Gly Arg Lys Pro Cys Cys Asp Glu Val Gly Leu Arg Lys Gly
1               5                   10                  15

cca tgg aca gtg gaa gaa gat ggg aaa cta gtt gat ttc tta agg gca      275
Pro Trp Thr Val Glu Glu Asp Gly Lys Leu Val Asp Phe Leu Arg Ala
            20                  25                  30

cgt ggc aac tgc ggt ggt ggt gga gga gga tgg tgc tgg aga gac gtg      323
Arg Gly Asn Cys Gly Gly Gly Gly Gly Gly Trp Cys Trp Arg Asp Val
        35                  40                  45

cca aaa ctg gcg ggg cta agg agg tgt ggc aaa agt tgc cgt ctc cgg      371
Pro Lys Leu Ala Gly Leu Arg Arg Cys Gly Lys Ser Cys Arg Leu Arg
    50                  55                  60

tgg act aat tat ctc cgg cca gat ctc aag aga ggt ctt ttt act gaa      419
Trp Thr Asn Tyr Leu Arg Pro Asp Leu Lys Arg Gly Leu Phe Thr Glu
65                  70                  75                  80

gaa gaa atc caa cta gtc att gat ctt cat gct cgc ctt ggc aat aga      467
Glu Glu Ile Gln Leu Val Ile Asp Leu His Ala Arg Leu Gly Asn Arg
                85                  90                  95

tgg tcg aag att gca gtg gag tta cca gga aga aca gac aac gat atc      515
Trp Ser Lys Ile Ala Val Glu Leu Pro Gly Arg Thr Asp Asn Asp Ile
            100                 105                 110

aaa aat tat tgg aac act cat ata aag agg aag ctt ata aga atg ggt      563
Lys Asn Tyr Trp Asn Thr His Ile Lys Arg Lys Leu Ile Arg Met Gly
        115                 120                 125

att gat cca aac aca cat cgt cga ttt gac caa caa aaa gtc aac gag      611
Ile Asp Pro Asn Thr His Arg Arg Phe Asp Gln Gln Lys Val Asn Glu
    130                 135                 140

gag gaa acg ata ttg gtc aac gat cca aag cct ctg tct gag acc gag      659
Glu Glu Thr Ile Leu Val Asn Asp Pro Lys Pro Leu Ser Glu Thr Glu
145                 150                 155                 160

gta tct gtt gct ttg aag aat gac acg tca gca gtg tta tca gga aat      707
Val Ser Val Ala Leu Lys Asn Asp Thr Ser Ala Val Leu Ser Gly Asn
                165                 170                 175

cta aac caa ttg gct gac gtg gac ggt gat gat cag ccg tgg agc ttt      755
Leu Asn Gln Leu Ala Asp Val Asp Gly Asp Asp Gln Pro Trp Ser Phe
            180                 185                 190

cta atg gaa aat gac gaa gga gga ggt ggc gac gcc gcc gga gag ctt 803
Leu Met Glu Asn Asp Glu Gly Gly Gly Gly Asp Ala Ala Gly Glu Leu
        195                 200                 205

acg atg cta ttg tcc ggt gac att acg tca tca tgt tct tct tcg tca 851
Thr Met Leu Leu Ser Gly Asp Ile Thr Ser Ser Cys Ser Ser Ser Ser
    210                 215                 220

tct ttg tgg atg aag tat gga gaa ttc gga tac gaa gat tta gaa ctt 899
Ser Leu Trp Met Lys Tyr Gly Glu Phe Gly Tyr Glu Asp Leu Glu Leu
225                 230                 235                 240

gga tgt ttc gat gtt tag agattcaagt atgtttaatt aggccgtagg 947
Gly Cys Phe Asp Val
                245

ttgattaatc ataaggttca ttgacttcat tctagaattg tgtagttgga ccagtataaa 1007

gaatcaaagt tatgaaacat tgtaatttga tttccaaatt aatctaatga ataaatgtgc 1067

tttgcaaaaa aaaaaaaaaa aaaaaaaa 1095

<210> 40
<211> 245
<212> PRT
<213> Arabidopsis thaliana

<400> 40

Met Gly Gly Arg Lys Pro Cys Cys Asp Glu Val Gly Leu Arg Lys Gly
1               5                   10                  15

Pro Trp Thr Val Glu Glu Asp Gly Lys Leu Val Asp Phe Leu Arg Ala
            20                  25                  30

Arg Gly Asn Cys Gly Gly Gly Gly Gly Gly Trp Cys Trp Arg Asp Val
        35                  40                  45

Pro Lys Leu Ala Gly Leu Arg Arg Cys Gly Lys Ser Cys Arg Leu Arg
    50                  55                  60

Trp Thr Asn Tyr Leu Arg Pro Asp Leu Lys Arg Gly Leu Phe Thr Glu
65                  70                  75                  80

Glu Glu Ile Gln Leu Val Ile Asp Leu His Ala Arg Leu Gly Asn Arg
                85                  90                  95

Trp Ser Lys Ile Ala Val Glu Leu Pro Gly Arg Thr Asp Asn Asp Ile
            100                 105                 110

Lys Asn Tyr Trp Asn Thr His Ile Lys Arg Lys Leu Ile Arg Met Gly
        115                 120                 125

Ile Asp Pro Asn Thr His Arg Arg Phe Asp Gln Gln Lys Val Asn Glu

130                 135                 140

Glu Glu Thr Ile Leu Val Asn Asp Pro Lys Pro Leu Ser Glu Thr Glu
145                 150                 155                 160

Val Ser Val Ala Leu Lys Asn Asp Thr Ser Ala Val Leu Ser Gly Asn
                165                 170                 175

Leu Asn Gln Leu Ala Asp Val Asp Gly Asp Asp Gln Pro Trp Ser Phe
            180                 185                 190

Leu Met Glu Asn Asp Glu Gly Gly Gly Gly Asp Ala Ala Gly Glu Leu
        195                 200                 205

Thr Met Leu Leu Ser Gly Asp Ile Thr Ser Ser Cys Ser Ser Ser Ser
    210                 215                 220

Ser Leu Trp Met Lys Tyr Gly Glu Phe Gly Tyr Glu Asp Leu Glu Leu
225                 230                 235                 240

Gly Cys Phe Asp Val
                245

<210>  41
<211>  965
<212>  DNA
<213>  Arabididopsis thaliana

<220>
<221>  CDS
<222>  (56)..(667)
<223>  G1335

<400>  41
ttttttttta aaagatttag agagaaaagt gagttattaa gagattccaa tcaaa atg      58
                                                             Met
                                                             1

agc gga gac aac ggc ggt ggt gag agg cgc aaa ggc tcc gtc aag tgg      106
Ser Gly Asp Asn Gly Gly Gly Glu Arg Arg Lys Gly Ser Val Lys Trp
            5                   10                  15

ttt gat acc cag aag ggt ttc ggc ttc atc act cct gac gac ggt ggc      154
Phe Asp Thr Gln Lys Gly Phe Gly Phe Ile Thr Pro Asp Asp Gly Gly
        20                  25                  30

gac gat ctc ttc gtt cac cag tcc tcc atc aga tct gag ggt ttc cgt      202
Asp Asp Leu Phe Val His Gln Ser Ser Ile Arg Ser Glu Gly Phe Arg
    35                  40                  45

agc ctc gct gcc gaa gaa gcc gta gag ttc gag gtt gag atc gac aac      250
Ser Leu Ala Ala Glu Glu Ala Val Glu Phe Glu Val Glu Ile Asp Asn
50                  55                  60                  65

aac aac cgt ccc aag gcc atc gat gtt tct gga ccc gac ggc gct ccc     298
Asn Asn Arg Pro Lys Ala Ile Asp Val Ser Gly Pro Asp Gly Ala Pro
                70                  75                  80

gtc caa gga aac agc ggt ggt ggt tca tct ggc gga cgc ggc ggt ttc     346
Val Gln Gly Asn Ser Gly Gly Gly Ser Ser Gly Gly Arg Gly Gly Phe
            85                  90                  95

ggt gga gga aga gga ggt gga cgc gga tct gga ggt gga tac ggc ggt     394
Gly Gly Gly Arg Gly Gly Gly Arg Gly Ser Gly Gly Gly Tyr Gly Gly
        100                 105                 110

ggc ggt ggt gga tac gga gga aga gga ggt ggt ggt cga gga ggc agc     442
Gly Gly Gly Gly Tyr Gly Gly Arg Gly Gly Gly Gly Arg Gly Gly Ser
    115                 120                 125

gac tgc tac aag tgt ggt gag ccc ggt cac atg gcg aga gac tgt tct     490
Asp Cys Tyr Lys Cys Gly Glu Pro Gly His Met Ala Arg Asp Cys Ser
130                 135                 140                 145

gaa ggc ggt gga ggt tac gga gga ggc ggc ggt ggc tac gga ggt gga     538
Glu Gly Gly Gly Gly Tyr Gly Gly Gly Gly Gly Gly Tyr Gly Gly Gly
                150                 155                 160

ggc gga tac ggc gga gga ggt ggt ggt tac gga ggt ggt ggc cgt gga     586
Gly Gly Tyr Gly Gly Gly Gly Gly Gly Tyr Gly Gly Gly Gly Arg Gly
            165                 170                 175

ggt ggt ggc ggc ggg gga agc tgc tac agc tgt ggc gag tcg gga cat     634
Gly Gly Gly Gly Gly Gly Ser Cys Tyr Ser Cys Gly Glu Ser Gly His
        180                 185                 190

ttc gcc agg gat tgc acc agc ggt gga cgt taa aaccaacgcc ggttacgcgg     687
Phe Ala Arg Asp Cys Thr Ser Gly Gly Arg
    195                 200

tggagaagag tgagttggtt atctcacaag tgatcggttc tttctcccgc cgccttctat     747

ctctctatta tccacttttt gcttattatg atggatctct atctttgtta gttggttttt     807

tcttgatggt ttcggattag gactcttctt ttggttttgc tacttatggt tggttttatt     867

tatggtactt gtgatatggg tgaaatgctc tacttgttgc tctgtttcaa gtgttcataa     927

tatgcgaaca aatattctgg gttttgtttc aaaaaaaa                             965

<210>  42
<211>  203
<212>  PRT
<213>  Arabididopsis thaliana

<400>  42

Met Ser Gly Asp Asn Gly Gly Gly Glu Arg Arg Lys Gly Ser Val Lys
1               5                   10                  15

Trp Phe Asp Thr Gln Lys Gly Phe Gly Phe Ile Thr Pro Asp Asp Gly
            20                  25                  30

Gly Asp Asp Leu Phe Val His Gln Ser Ser Ile Arg Ser Glu Gly Phe

35                  40                  45

Arg Ser Leu Ala Ala Glu Glu Ala Val Glu Phe Glu Val Glu Ile Asp
    50                  55                  60

Asn Asn Asn Arg Pro Lys Ala Ile Asp Val Ser Gly Pro Asp Gly Ala
65                  70                  75                  80

Pro Val Gln Gly Asn Ser Gly Gly Gly Ser Ser Gly Gly Arg Gly Gly
                85                  90                  95

Phe Gly Gly Gly Arg Gly Gly Gly Arg Gly Ser Gly Gly Gly Tyr Gly
            100                 105                 110

Gly Gly Gly Gly Gly Tyr Gly Gly Arg Gly Gly Gly Gly Arg Gly Gly
        115                 120                 125

Ser Asp Cys Tyr Lys Cys Gly Glu Pro Gly His Met Ala Arg Asp Cys
    130                 135                 140

Ser Glu Gly Gly Gly Gly Tyr Gly Gly Gly Gly Gly Gly Tyr Gly Gly
145                 150                 155                 160

Gly Gly Gly Tyr Gly Gly Gly Gly Gly Gly Tyr Gly Gly Gly Gly Arg
                165                 170                 175

Gly Gly Gly Gly Gly Gly Gly Ser Cys Tyr Ser Cys Gly Glu Ser Gly
            180                 185                 190

His Phe Ala Arg Asp Cys Thr Ser Gly Gly Arg
        195                 200

<210>  43
<211>  1554
<212>  DNA
<213>  Arabidopsis thaliana

<220>
<221>  CDS
<222>  (137)..(1285)
<223>  G562

<400>  43
atttgaattt ctgggtttct ctctgtttaa gcttcttctt cttcatcttc tgcttacgtt      60

tcttcttcaa ggagctttcg gattcttgta gaaagagtca ttgttctctt gagtgggaaa     120

ccttgaaacc attcct atg gga aat agc agc gag gaa cca aag cct cct acc     172
                  Met Gly Asn Ser Ser Glu Glu Pro Lys Pro Pro Thr
                  1               5                   10

aaa tca gat aaa cca tct tca ccc ccg gtg gat caa aca aat gtt cat 220
Lys Ser Asp Lys Pro Ser Ser Pro Pro Val Asp Gln Thr Asn Val His
        15                  20                  25

gtc tac cct gat tgg gca gct atg cag gca tat tat ggt cca aga gta 268
Val Tyr Pro Asp Trp Ala Ala Met Gln Ala Tyr Tyr Gly Pro Arg Val
    30                  35                  40

gca atg cct cct tat tac aat tca gct atg gct gca tct ggt cat cct 316
Ala Met Pro Pro Tyr Tyr Asn Ser Ala Met Ala Ala Ser Gly His Pro
45                  50                  55                  60

cct cct cct tac atg tgg aat cct cag cat atg atg tca cca tct gga 364
Pro Pro Pro Tyr Met Trp Asn Pro Gln His Met Met Ser Pro Ser Gly
                65                  70                  75

gca ccc tat gct gct gtt tat cct cat gga gga gga gtt tac gct cat 412
Ala Pro Tyr Ala Ala Val Tyr Pro His Gly Gly Gly Val Tyr Ala His
            80                  85                  90

ccc ggt att ccc atg gga tca ctg cct caa ggt caa aag gat cca cct 460
Pro Gly Ile Pro Met Gly Ser Leu Pro Gln Gly Gln Lys Asp Pro Pro
        95                  100                 105

tta aca act ccg ggg acg ctt ttg agc atc gac act cct act aaa tct 508
Leu Thr Thr Pro Gly Thr Leu Leu Ser Ile Asp Thr Pro Thr Lys Ser
    110                 115                 120

aca ggg aac aca gac aat gga ttg atg aag aag ctg aaa gag ttt gat 556
Thr Gly Asn Thr Asp Asn Gly Leu Met Lys Lys Leu Lys Glu Phe Asp
125                 130                 135                 140

ggg ctt gct atg tct cta gga aat ggg aat cct gaa aat ggt gca gat 604
Gly Leu Ala Met Ser Leu Gly Asn Gly Asn Pro Glu Asn Gly Ala Asp
                145                 150                 155

gaa cat aaa cga tca cgg aac agc tca gaa act gat ggt tct act gat 652
Glu His Lys Arg Ser Arg Asn Ser Ser Glu Thr Asp Gly Ser Thr Asp
            160                 165                 170

gga agt gat ggg aat aca act ggg gca gat gaa ccg aaa ctt aaa aga 700
Gly Ser Asp Gly Asn Thr Thr Gly Ala Asp Glu Pro Lys Leu Lys Arg
        175                 180                 185

agt cga gag gga act cca aca aaa gat ggg aaa caa ttg gtt caa gct 748
Ser Arg Glu Gly Thr Pro Thr Lys Asp Gly Lys Gln Leu Val Gln Ala
    190                 195                 200

agc tca ttt cat tct gtt tct ccg tca agt ggt gat acc ggc gta aaa 796
Ser Ser Phe His Ser Val Ser Pro Ser Ser Gly Asp Thr Gly Val Lys
205                 210                 215                 220

ctc att caa gga tct gga gct ata ctc tct cct ggt gta agt gca aat 844
Leu Ile Gln Gly Ser Gly Ala Ile Leu Ser Pro Gly Val Ser Ala Asn
                225                 230                 235

tcc aac ccc ttc atg tca caa tct tta gcc atg gtt cct cct gaa act 892
Ser Asn Pro Phe Met Ser Gln Ser Leu Ala Met Val Pro Pro Glu Thr
            240                 245                 250

tgg ctt cag aac gag aga gaa ctg aaa cgg gag cga agg aaa cag tct 940
Trp Leu Gln Asn Glu Arg Glu Leu Lys Arg Glu Arg Arg Lys Gln Ser
        255                 260                 265

aat aga gaa tct gct aga agg tca aga tta agg aaa cag gcc gag aca 988
Asn Arg Glu Ser Ala Arg Arg Ser Arg Leu Arg Lys Gln Ala Glu Thr
    270                 275                 280

gaa gaa ctt gct agg aaa gtg gaa gcc ttg aca gcc gaa aac atg gca 1036
Glu Glu Leu Ala Arg Lys Val Glu Ala Leu Thr Ala Glu Asn Met Ala
285                 290                 295                 300

tta aga tct gaa cta aac caa ctt aat gag aaa tct gat aaa cta aga 1084
Leu Arg Ser Glu Leu Asn Gln Leu Asn Glu Lys Ser Asp Lys Leu Arg
                305                 310                 315

gga gca aat gca acc ttg ttg gac aaa ctg aaa tgc tcg gaa ccc gaa 1132
Gly Ala Asn Ala Thr Leu Leu Asp Lys Leu Lys Cys Ser Glu Pro Glu
            320                 325                 330

aag aga gtc ccc gca aat atg ttg tct aga gtt aag aac tca gga gct 1180
Lys Arg Val Pro Ala Asn Met Leu Ser Arg Val Lys Asn Ser Gly Ala
        335                 340                 345

gga gat aag aac aag aac caa gga gac aat gat tct aac tct aca agc 1228
Gly Asp Lys Asn Lys Asn Gln Gly Asp Asn Asp Ser Asn Ser Thr Ser
    350                 355                 360

aaa ttc cat caa ctg ctc gat acg aag cct cga gct aaa gca gta gct 1276
Lys Phe His Gln Leu Leu Asp Thr Lys Pro Arg Ala Lys Ala Val Ala
365                 370                 375                 380

gca ggc tga atcgatggta attcatgtcg atttctactt aatttgtcga 1325
Ala Gly

cataaacaaa gaaaataagt gctactaatt tcagaaaaac ttgatagata gatagtatag 1385

tagagagaga gagagagaga gaggtgtgat gattattgat ctataaattt tcggagagag 1445

agagggagaa agagaaactt ttcctccaga tgaaaatttg gtgttatggt ttgttactgt 1505

taatatagag aggcttttct ttttttataa aatggcttcc tttgttgca 1554

<210> 44
<211> 382
<212> PRT
<213> Arabidopsis thaliana

<400> 44

Met Gly Asn Ser Ser Glu Glu Pro Lys Pro Pro Thr Lys Ser Asp Lys
1               5                   10                  15

Pro Ser Ser Pro Pro Val Asp Gln Thr Asn Val His Val Tyr Pro Asp
            20                  25                  30

Trp Ala Ala Met Gln Ala Tyr Tyr Gly Pro Arg Val Ala Met Pro Pro
        35                  40                  45

Tyr Tyr Asn Ser Ala Met Ala Ala Ser Gly His Pro Pro Pro Pro Tyr
    50                  55                  60

Met Trp Asn Pro Gln His Met Met Ser Pro Ser Gly Ala Pro Tyr Ala
65                  70                  75                  80

Ala Val Tyr Pro His Gly Gly Gly Val Tyr Ala His Pro Gly Ile Pro
                85                  90                  95

Met Gly Ser Leu Pro Gln Gly Gln Lys Asp Pro Pro Leu Thr Thr Pro
            100                 105                 110

Gly Thr Leu Leu Ser Ile Asp Thr Pro Thr Lys Ser Thr Gly Asn Thr
        115                 120                 125

Asp Asn Gly Leu Met Lys Lys Leu Lys Glu Phe Asp Gly Leu Ala Met
    130                 135                 140

Ser Leu Gly Asn Gly Asn Pro Glu Asn Gly Ala Asp Glu His Lys Arg
145                 150                 155                 160

Ser Arg Asn Ser Ser Glu Thr Asp Gly Ser Thr Asp Gly Ser Asp Gly
                165                 170                 175

Asn Thr Thr Gly Ala Asp Glu Pro Lys Leu Lys Arg Ser Arg Glu Gly
            180                 185                 190

Thr Pro Thr Lys Asp Gly Lys Gln Leu Val Gln Ala Ser Ser Phe His
        195                 200                 205

Ser Val Ser Pro Ser Ser Gly Asp Thr Gly Val Lys Leu Ile Gln Gly
    210                 215                 220

Ser Gly Ala Ile Leu Ser Pro Gly Val Ser Ala Asn Ser Asn Pro Phe
225                 230                 235                 240

Met Ser Gln Ser Leu Ala Met Val Pro Pro Glu Thr Trp Leu Gln Asn
                245                 250                 255

Glu Arg Glu Leu Lys Arg Glu Arg Arg Lys Gln Ser Asn Arg Glu Ser
            260                 265                 270

Ala Arg Arg Ser Arg Leu Arg Lys Gln Ala Glu Thr Glu Glu Leu Ala
        275                 280                 285

Arg Lys Val Glu Ala Leu Thr Ala Glu Asn Met Ala Leu Arg Ser Glu
    290                 295                 300

Leu Asn Gln Leu Asn Glu Lys Ser Asp Lys Leu Arg Gly Ala Asn Ala

305                 310                 315                 320

Thr Leu Leu Asp Lys Leu Lys Cys Ser Glu Pro Glu Lys Arg Val Pro
                325                 330                 335

Ala Asn Met Leu Ser Arg Val Lys Asn Ser Gly Ala Gly Asp Lys Asn
            340                 345                 350

Lys Asn Gln Gly Asp Asn Asp Ser Asn Ser Thr Ser Lys Phe His Gln
        355                 360                 365

Leu Leu Asp Thr Lys Pro Arg Ala Lys Ala Val Ala Ala Gly
    370                 375                 380

<210>  45
<211>  513
<212>  DNA
<213>  Arabidopsis thaliana

<220>
<221>  CDS
<222>  (1)..(513)
<223>  G736

<400>  45
atg gcg act caa gat tct caa ggg att aaa ctc ttt ggc aaa act att      48
Met Ala Thr Gln Asp Ser Gln Gly Ile Lys Leu Phe Gly Lys Thr Ile
1               5                   10                  15

gca ttt aac act cga aca ata aaa aat gaa gaa gag aca cac ccg ccg      96
Ala Phe Asn Thr Arg Thr Ile Lys Asn Glu Glu Glu Thr His Pro Pro
            20                  25                  30

gag caa gaa gcc aca ata gcc gtt aga tca tca tca tca tcg gat ctg      144
Glu Gln Glu Ala Thr Ile Ala Val Arg Ser Ser Ser Ser Ser Asp Leu
        35                  40                  45

acg gcc gag aag cgt ccg gat aag atc ata gca tgt cca aga tgc aag      192
Thr Ala Glu Lys Arg Pro Asp Lys Ile Ile Ala Cys Pro Arg Cys Lys
    50                  55                  60

agc atg gag aca aag ttc tgt tac ttc aac aac tac aac ggt aat cag      240
Ser Met Glu Thr Lys Phe Cys Tyr Phe Asn Asn Tyr Asn Gly Asn Gln
65                  70                  75                  80

cct cga cac ttt tgt aaa ggc tgc cac cgt tac tgg acc gcc ggt ggt      288
Pro Arg His Phe Cys Lys Gly Cys His Arg Tyr Trp Thr Ala Gly Gly
                85                  90                  95

gca ctc cgg aac gtt ccc gtc ggc gcc ggt cgt cgg aag tcc aaa cca      336
Ala Leu Arg Asn Val Pro Val Gly Ala Gly Arg Arg Lys Ser Lys Pro
            100                 105                 110

cct ggt cgt gtc gtg gtt ggt atg ctt gga gat gga aat ggt gtt cgc      384
Pro Gly Arg Val Val Val Gly Met Leu Gly Asp Gly Asn Gly Val Arg
        115                 120                 125

caa gtc gag ctt ata aat ggc ttg ctc gtt gag gag tgg cag cat gcc 432
Gln Val Glu Leu Ile Asn Gly Leu Leu Val Glu Glu Trp Gln His Ala
    130                 135                 140

gca gcc gca gct cac ggt agt ttc cgg cat gat ttt ccc atg aag cgg 480
Ala Ala Ala Ala His Gly Ser Phe Arg His Asp Phe Pro Met Lys Arg
145                 150                 155                 160

ctc cgg tgt tac tcc gac ggt caa tcg tgc tga 513
Leu Arg Cys Tyr Ser Asp Gly Gln Ser Cys
                165                 170

<210> 46
<211> 170
<212> PRT
<213> Arabidopsis thaliana

<400> 46

Met Ala Thr Gln Asp Ser Gln Gly Ile Lys Leu Phe Gly Lys Thr Ile
1               5                   10                  15

Ala Phe Asn Thr Arg Thr Ile Lys Asn Glu Glu Glu Thr His Pro Pro
            20                  25                  30

Glu Gln Glu Ala Thr Ile Ala Val Arg Ser Ser Ser Ser Ser Asp Leu
        35                  40                  45

Thr Ala Glu Lys Arg Pro Asp Lys Ile Ile Ala Cys Pro Arg Cys Lys
    50                  55                  60

Ser Met Glu Thr Lys Phe Cys Tyr Phe Asn Asn Tyr Asn Gly Asn Gln
65                  70                  75                  80

Pro Arg His Phe Cys Lys Gly Cys His Arg Tyr Trp Thr Ala Gly Gly
                85                  90                  95

Ala Leu Arg Asn Val Pro Val Gly Ala Gly Arg Arg Lys Ser Lys Pro
            100                 105                 110

Pro Gly Arg Val Val Val Gly Met Leu Gly Asp Gly Asn Gly Val Arg
        115                 120                 125

Gln Val Glu Leu Ile Asn Gly Leu Leu Val Glu Glu Trp Gln His Ala
    130                 135                 140

Ala Ala Ala Ala His Gly Ser Phe Arg His Asp Phe Pro Met Lys Arg
145                 150                 155                 160

Leu Arg Cys Tyr Ser Asp Gly Gln Ser Cys
                165                 170

<210>  47
<211>  974
<212>  DNA
<213>  Arabidopsis thaliana

<220>
<221>  CDS
<222>  (62)..(874)
<223>  G1073


<400>  47
cccccgacc tgcctctaca gagacctgaa gattccagaa ccccacctga tcaaaaataa      60

c atg gaa ctt aac aga tct gaa gca gac gaa gca aag gcc gag acc act     109
  Met Glu Leu Asn Arg Ser Glu Ala Asp Glu Ala Lys Ala Glu Thr Thr
  1               5                   10                  15

ccc acc ggt gga gcc acc agc tca gcc aca gcc tct ggc tct tcc tcc       157
Pro Thr Gly Gly Ala Thr Ser Ser Ala Thr Ala Ser Gly Ser Ser Ser
            20                  25                  30

gga cgt cgt cca cgt ggt cgt cct gca ggt tcc aaa aac aaa ccc aaa       205
Gly Arg Arg Pro Arg Gly Arg Pro Ala Gly Ser Lys Asn Lys Pro Lys
        35                  40                  45

cct ccg acg att ata act aga gat agt cct aac gtc ctt aga tca cac       253
Pro Pro Thr Ile Ile Thr Arg Asp Ser Pro Asn Val Leu Arg Ser His
    50                  55                  60

gtt ctt gaa gtc acc tcc ggt tcg gac ata tcc gag gca gtc tcc acc       301
Val Leu Glu Val Thr Ser Gly Ser Asp Ile Ser Glu Ala Val Ser Thr
65                  70                  75                  80

tac gcc act cgt cgc ggc tgc ggc gtt tgc att ata agc ggc acg ggt       349
Tyr Ala Thr Arg Arg Gly Cys Gly Val Cys Ile Ile Ser Gly Thr Gly
                85                  90                  95

gcg gtc act aac gtc acg ata cgg caa cct gcg gct ccg gct ggt gga       397
Ala Val Thr Asn Val Thr Ile Arg Gln Pro Ala Ala Pro Ala Gly Gly
            100                 105                 110

ggt gtg att acc ctg cat ggt cgg ttt gac att ttg tct ttg acc ggt       445
Gly Val Ile Thr Leu His Gly Arg Phe Asp Ile Leu Ser Leu Thr Gly
        115                 120                 125

act gcg ctt cca ccg cct gca cca ccg gga gca gga ggt ttg acg gtg       493
Thr Ala Leu Pro Pro Pro Ala Pro Pro Gly Ala Gly Gly Leu Thr Val
    130                 135                 140

tat cta gcc gga ggt caa gga caa gtt gta gga ggg aat gtg gct ggt       541
Tyr Leu Ala Gly Gly Gln Gly Gln Val Val Gly Gly Asn Val Ala Gly
145                 150                 155                 160

tcg tta att gct tcg gga ccg gta gtg ttg atg gct gct tct ttt gca       589
Ser Leu Ile Ala Ser Gly Pro Val Val Leu Met Ala Ala Ser Phe Ala
                165                 170                 175

aac gca gtt tat gat agg tta ccg att gaa gag gaa gaa acc cca ccg       637
Asn Ala Val Tyr Asp Arg Leu Pro Ile Glu Glu Glu Glu Thr Pro Pro
            180                 185                 190

ccg aga acc acc ggg gtg cag cag cag cag ccg gag gcg tct cag tcg     685
Pro Arg Thr Thr Gly Val Gln Gln Gln Gln Pro Glu Ala Ser Gln Ser
        195                 200                 205

tcg gag gtt acg ggg agt ggg gcc cag gcg tgt gag tca aac ctc caa     733
Ser Glu Val Thr Gly Ser Gly Ala Gln Ala Cys Glu Ser Asn Leu Gln
    210                 215                 220

ggt gga aat ggt gga gga ggt gtt gct ttc tac aat ctt gga atg aat     781
Gly Gly Asn Gly Gly Gly Gly Val Ala Phe Tyr Asn Leu Gly Met Asn
225                 230                 235                 240

atg aac aat ttt caa ttc tcc ggg gga gat att tac ggt atg agc ggc     829
Met Asn Asn Phe Gln Phe Ser Gly Gly Asp Ile Tyr Gly Met Ser Gly
                245                 250                 255

ggt agc gga gga ggt ggt ggc ggt gcg act aga ccc gcg ttt tag         874
Gly Ser Gly Gly Gly Gly Gly Gly Ala Thr Arg Pro Ala Phe
            260                 265                 270

agttttagcg ttttggtgac accttttgtt gcgtttgcgt gtttgacctc aaactactag   934

gctactagct atagcggttg cgaaatgcga atattaggtt                         974

<210>  48
<211>  270
<212>  PRT
<213>  Arabidopsis thaliana

<400>  48

Met Glu Leu Asn Arg Ser Glu Ala Asp Glu Ala Lys Ala Glu Thr Thr
1               5                   10                  15

Pro Thr Gly Gly Ala Thr Ser Ser Ala Thr Ala Ser Gly Ser Ser Ser
            20                  25                  30

Gly Arg Arg Pro Arg Gly Arg Pro Ala Gly Ser Lys Asn Lys Pro Lys
        35                  40                  45

Pro Pro Thr Ile Ile Thr Arg Asp Ser Pro Asn Val Leu Arg Ser His
    50                  55                  60

Val Leu Glu Val Thr Ser Gly Ser Asp Ile Ser Glu Ala Val Ser Thr
65                  70                  75                  80

Tyr Ala Thr Arg Arg Gly Cys Gly Val Cys Ile Ile Ser Gly Thr Gly
                85                  90                  95

Ala Val Thr Asn Val Thr Ile Arg Gln Pro Ala Ala Pro Ala Gly Gly
            100                 105                 110

Gly Val Ile Thr Leu His Gly Arg Phe Asp Ile Leu Ser Leu Thr Gly
        115                 120                 125

Thr Ala Leu Pro Pro Pro Ala Pro Pro Gly Ala Gly Gly Leu Thr Val
    130                 135                 140

Tyr Leu Ala Gly Gly Gln Gly Gln Val Val Gly Gly Asn Val Ala Gly
145                 150                 155                 160

Ser Leu Ile Ala Ser Gly Pro Val Val Leu Met Ala Ala Ser Phe Ala
                165                 170                 175

Asn Ala Val Tyr Asp Arg Leu Pro Ile Glu Glu Glu Glu Thr Pro Pro
            180                 185                 190

Pro Arg Thr Thr Gly Val Gln Gln Gln Gln Pro Glu Ala Ser Gln Ser
        195                 200                 205

Ser Glu Val Thr Gly Ser Gly Ala Gln Ala Cys Glu Ser Asn Leu Gln
    210                 215                 220

Gly Gly Asn Gly Gly Gly Gly Val Ala Phe Tyr Asn Leu Gly Met Asn
225                 230                 235                 240

Met Asn Asn Phe Gln Phe Ser Gly Gly Asp Ile Tyr Gly Met Ser Gly
                245                 250                 255

Gly Ser Gly Gly Gly Gly Gly Gly Ala Thr Arg Pro Ala Phe
            260                 265                 270

<210>  49
<211>  1281
<212>  DNA
<213>  Arabidopsis thaliana

<220>
<221>  CDS
<222>  (8)..(904)
<223>  G1435

<400>  49
gtgaaac atg ggg aag gaa gtt atg gtg agc gat tac ggt gac gac gac        49
        Met Gly Lys Glu Val Met Val Ser Asp Tyr Gly Asp Asp Asp
        1               5                   10

gga gaa gac gcc ggc ggc ggc gat gaa tat agg att ccg gaa tgg gaa        97
Gly Glu Asp Ala Gly Gly Gly Asp Glu Tyr Arg Ile Pro Glu Trp Glu
15                  20                  25                  30

att ggt tta ccc aac gga gat gat ttg act ccg tta tct caa tat cta       145
Ile Gly Leu Pro Asn Gly Asp Asp Leu Thr Pro Leu Ser Gln Tyr Leu
                35                  40                  45

gtc ccg tcg att ctc gcg tta gct ttc agc atg atc cca gaa cga agc       193
Val Pro Ser Ile Leu Ala Leu Ala Phe Ser Met Ile Pro Glu Arg Ser

            50                  55                  60

cgt aca att cac gac gtc aat cgc gcg tcg caa atc acg ctc tct tcg 241
Arg Thr Ile His Asp Val Asn Arg Ala Ser Gln Ile Thr Leu Ser Ser
        65                  70                  75

ttg aga agc agt acc aat gct tcg tct gtg atg gag gag gtc gtg gat 289
Leu Arg Ser Ser Thr Asn Ala Ser Ser Val Met Glu Glu Val Val Asp
    80                  85                  90

cga gtt gaa tcg agt gtt cca gga tca gat ccg aag aaa cag aag aaa 337
Arg Val Glu Ser Ser Val Pro Gly Ser Asp Pro Lys Lys Gln Lys Lys
95                  100                 105                 110

tcg gat ggt ggt gaa gca gcg gcg gtg gag gat tcc acg gcg gag gaa 385
Ser Asp Gly Gly Glu Ala Ala Ala Val Glu Asp Ser Thr Ala Glu Glu
                115                 120                 125

gga gac tcc ggg cct gaa gac gcg tct ggg aag aca tcg aaa cga ccg 433
Gly Asp Ser Gly Pro Glu Asp Ala Ser Gly Lys Thr Ser Lys Arg Pro
            130                 135                 140

cgt tta gtg tgg aca ccg cag cta cac aag aga ttt gtg gac gtt gtg 481
Arg Leu Val Trp Thr Pro Gln Leu His Lys Arg Phe Val Asp Val Val
        145                 150                 155

gct cat cta ggg att aaa aac gca gtg ccg aag acg att atg cag ctg 529
Ala His Leu Gly Ile Lys Asn Ala Val Pro Lys Thr Ile Met Gln Leu
    160                 165                 170

atg aac gtg gaa gga ctt act cgt gag aac gtt gcg tct cat ttg cag 577
Met Asn Val Glu Gly Leu Thr Arg Glu Asn Val Ala Ser His Leu Gln
175                 180                 185                 190

aaa tat agg ctt tac ctt aaa cgg att caa gga ttg acg acg gaa gaa 625
Lys Tyr Arg Leu Tyr Leu Lys Arg Ile Gln Gly Leu Thr Thr Glu Glu
                195                 200                 205

gat cct tat tcg tcg tcg gat cag ctc ttc tct tca acg ccg gtt cct 673
Asp Pro Tyr Ser Ser Ser Asp Gln Leu Phe Ser Ser Thr Pro Val Pro
            210                 215                 220

cca cag agc ttt caa gac ggc gga gga agt aac gga aag ttg ggg gtt 721
Pro Gln Ser Phe Gln Asp Gly Gly Gly Ser Asn Gly Lys Leu Gly Val
        225                 230                 235

ccg gtt ccg gtt ccg tcg atg gtg cct att cca ggc tat ggg aat caa 769
Pro Val Pro Val Pro Ser Met Val Pro Ile Pro Gly Tyr Gly Asn Gln
    240                 245                 250

atg ggt atg caa gga tat tat caa cag tat agt aac cat ggc aat gaa 817
Met Gly Met Gln Gly Tyr Tyr Gln Gln Tyr Ser Asn His Gly Asn Glu
255                 260                 265                 270

tca aac caa tat atg atg cag cag aat aag ttt gga aca atg gtg aca 865
Ser Asn Gln Tyr Met Met Gln Gln Asn Lys Phe Gly Thr Met Val Thr
                275                 280                 285

tat cct tct gtt ggt ggt ggt gac gtg aat gac aag taa atggatctta 914
Tyr Pro Ser Val Gly Gly Gly Asp Val Asn Asp Lys
            290                 295

aaggtctata atttgctcta cagagagata ctggttcttg gcttatggtt tattttccca 974

cttcatgagg ttgttgtgac ttttaattct ccatgttttc cacacaagtc tttattgcct 1034

ttgtatagaa aatgatttcg agaaaatcac tgggaagctt ggtattgttg gaggatgaag 1094

ccttctatga atgatttagt ttcctactgt ctccattctt tatgaggtaa taaagccttc 1154

ttttgctcat cgcttgtagt cttcttaaat tcaagacagc gtcacatgtt tgttcggtta 1214

tgttaattgt ttctttcttt ggataatgaa gatagcatca ggtctcatgt ctcctcactt 1274

tgataaa 1281

<210> 50
<211> 298
<212> PRT
<213> Arabidopsis thaliana

<400> 50

Met Gly Lys Glu Val Met Val Ser Asp Tyr Gly Asp Asp Asp Gly Glu
1               5                   10                  15

Asp Ala Gly Gly Gly Asp Glu Tyr Arg Ile Pro Glu Trp Glu Ile Gly
            20                  25                  30

Leu Pro Asn Gly Asp Asp Leu Thr Pro Leu Ser Gln Tyr Leu Val Pro
        35                  40                  45

Ser Ile Leu Ala Leu Ala Phe Ser Met Ile Pro Glu Arg Ser Arg Thr
    50                  55                  60

Ile His Asp Val Asn Arg Ala Ser Gln Ile Thr Leu Ser Ser Leu Arg
65                  70                  75                  80

Ser Ser Thr Asn Ala Ser Ser Val Met Glu Glu Val Val Asp Arg Val
                85                  90                  95

Glu Ser Ser Val Pro Gly Ser Asp Pro Lys Lys Gln Lys Lys Ser Asp
            100                 105                 110

Gly Gly Glu Ala Ala Ala Val Glu Asp Ser Thr Ala Glu Glu Gly Asp
        115                 120                 125

Ser Gly Pro Glu Asp Ala Ser Gly Lys Thr Ser Lys Arg Pro Arg Leu
    130                 135                 140

Val Trp Thr Pro Gln Leu His Lys Arg Phe Val Asp Val Val Ala His
145                 150                 155                 160

Leu Gly Ile Lys Asn Ala Val Pro Lys Thr Ile Met Gln Leu Met Asn
                165                 170                 175

Val Glu Gly Leu Thr Arg Glu Asn Val Ala Ser His Leu Gln Lys Tyr
            180                 185                 190

Arg Leu Tyr Leu Lys Arg Ile Gln Gly Leu Thr Thr Glu Glu Asp Pro
        195                 200                 205

Tyr Ser Ser Ser Asp Gln Leu Phe Ser Ser Thr Pro Val Pro Pro Gln
    210                 215                 220

Ser Phe Gln Asp Gly Gly Gly Ser Asn Gly Lys Leu Gly Val Pro Val
225                 230                 235                 240

Pro Val Pro Ser Met Val Pro Ile Pro Gly Tyr Gly Asn Gln Met Gly
                245                 250                 255

Met Gln Gly Tyr Tyr Gln Gln Tyr Ser Asn His Gly Asn Glu Ser Asn
            260                 265                 270

Gln Tyr Met Met Gln Gln Asn Lys Phe Gly Thr Met Val Thr Tyr Pro
        275                 280                 285

Ser Val Gly Gly Gly Asp Val Asn Asp Lys
    290                 295

<210>  51
<211>  837
<212>  DNA
<213>  Arabidopsis thaliana

<220>
<221>  CDS
<222>  (54)..(629)
<223>  G180

<400>  51
gtaattacga tctacaacaa gtgacatcgt cgtcgacgac gattcaagag aat atg        56
                                                           Met
                                                           1

aac ttc ctc gtt cct ttt gaa gaa acc aat gtc tta acc ttt ttc tct      104
Asn Phe Leu Val Pro Phe Glu Glu Thr Asn Val Leu Thr Phe Phe Ser
            5                   10                  15

tct tct tct tcc tct tct ctt tct tct cct tct ttc ccc att cac aac      152
Ser Ser Ser Ser Ser Ser Leu Ser Ser Pro Ser Phe Pro Ile His Asn
        20                  25                  30

tct tcc tcc act act act act cat gca cct cta ggg ttt tct aat aat      200
Ser Ser Ser Thr Thr Thr Thr His Ala Pro Leu Gly Phe Ser Asn Asn
    35                  40                  45

ctt cag ggt gga gga ccc ttg gga tca aag gtg gtt aat gat gat cag      248

Leu Gln Gly Gly Gly Pro Leu Gly Ser Lys Val Val Asn Asp Asp Gln
50                  55                  60                  65

gag aat ttt gga ggt gga act aac aat gat gct cat tct aat tct tgg      296
Glu Asn Phe Gly Gly Gly Thr Asn Asn Asp Ala His Ser Asn Ser Trp
                70                  75                  80

tgg aga tca aat agt gga agt gga gat atg aag aac aaa gtg aag ata      344
Trp Arg Ser Asn Ser Gly Ser Gly Asp Met Lys Asn Lys Val Lys Ile
            85                  90                  95

agg agg aaa cta aga gag cca aga ttc tgt ttc caa acc aaa agc gat      392
Arg Arg Lys Leu Arg Glu Pro Arg Phe Cys Phe Gln Thr Lys Ser Asp
        100                 105                 110

gtt gat gtt ctt gac gat ggc tac aaa tgg cgt aaa tat ggt cag aaa      440
Val Asp Val Leu Asp Asp Gly Tyr Lys Trp Arg Lys Tyr Gly Gln Lys
    115                 120                 125

gtc gtc aag aac agc ctt cac ccc agg agt tat tac aga tgc aca cac      488
Val Val Lys Asn Ser Leu His Pro Arg Ser Tyr Tyr Arg Cys Thr His
130                 135                 140                 145

aac aac tgt agg gtg aaa aag aga gtg gag cga cta tcg gaa gat tgt      536
Asn Asn Cys Arg Val Lys Lys Arg Val Glu Arg Leu Ser Glu Asp Cys
                150                 155                 160

aga atg gtg att act act tac gaa ggt cgt cac aac cac att ccc tct      584
Arg Met Val Ile Thr Thr Tyr Glu Gly Arg His Asn His Ile Pro Ser
            165                 170                 175

gat gac tcc act tct cct gac cat gat tgt ctc tct tcc ttt taa          629
Asp Asp Ser Thr Ser Pro Asp His Asp Cys Leu Ser Ser Phe
        180                 185                 190

catctctttc tatatatcta tatatagaca gttatatgtg cacatataga tgtgtgatat    689

attgcatatt tgatattgca tgtgtttttc aagagtatgt catcagatgt tatgcatata    749

ttcttgactt gttgcttata gtatacatat gtaataatat atattgacat tggtagttca    809

tttctgttca aacaaaaaaa aaaaaaaa                                       837

<210>  52
<211>  191
<212>  PRT
<213>  Arabidopsis thaliana

<400>  52

Met Asn Phe Leu Val Pro Phe Glu Glu Thr Asn Val Leu Thr Phe Phe
1               5                   10                  15

Ser Ser Ser Ser Ser Ser Ser Leu Ser Ser Pro Ser Phe Pro Ile His
            20                  25                  30

Asn Ser Ser Ser Thr Thr Thr Thr His Ala Pro Leu Gly Phe Ser Asn
        35                  40                  45

Asn Leu Gln Gly Gly Gly Pro Leu Gly Ser Lys Val Val Asn Asp Asp
50                  55                  60

Gln Glu Asn Phe Gly Gly Gly Thr Asn Asn Asp Ala His Ser Asn Ser
65                  70                  75                  80

Trp Trp Arg Ser Asn Ser Gly Ser Gly Asp Met Lys Asn Lys Val Lys
                85                  90                  95

Ile Arg Arg Lys Leu Arg Glu Pro Arg Phe Cys Phe Gln Thr Lys Ser
            100                 105                 110

Asp Val Asp Val Leu Asp Asp Gly Tyr Lys Trp Arg Lys Tyr Gly Gln
        115                 120                 125

Lys Val Val Lys Asn Ser Leu His Pro Arg Ser Tyr Tyr Arg Cys Thr
    130                 135                 140

His Asn Asn Cys Arg Val Lys Lys Arg Val Glu Arg Leu Ser Glu Asp
145                 150                 155                 160

Cys Arg Met Val Ile Thr Thr Tyr Glu Gly Arg His Asn His Ile Pro
                165                 170                 175

Ser Asp Asp Ser Thr Ser Pro Asp His Asp Cys Leu Ser Ser Phe
            180                 185                 190

<210>  53
<211>  1413
<212>  DNA
<213>  Arabidopsis thaliana

<220>
<221>  CDS
<222>  (121)..(1200)
<223>  G592

<400>  53
aagctattaa gatttggttt tctacaaatt tgttcttcct gaaacgtcac gagacagagc      60

ttacaagaag agaaaacaga ggaaatttcg ttgcattttt tttacatatt gattcgatta     120

atg gat tca aat aat cat ctc tac gac ccg aat ccc acc ggg tcg ggt       168
Met Asp Ser Asn Asn His Leu Tyr Asp Pro Asn Pro Thr Gly Ser Gly
1               5                   10                  15

ctt ctt cgt ttt aga tca gct ccg agc tct gtt ctc gcc gct ttt gtt       216
Leu Leu Arg Phe Arg Ser Ala Pro Ser Ser Val Leu Ala Ala Phe Val
            20                  25                  30

gac gac gac aag att ggt ttc gac tcc gat agg ttg ctt tca aga ttc       264
Asp Asp Asp Lys Ile Gly Phe Asp Ser Asp Arg Leu Leu Ser Arg Phe
        35                  40                  45

gtg acc tct aat ggc gtt aac gga gat ctg ggt tca cct aaa ttc gag 312
Val Thr Ser Asn Gly Val Asn Gly Asp Leu Gly Ser Pro Lys Phe Glu
    50                  55                  60

gat aag tct ccg gtt tcg tta acg aac acc tct gtt tca tac gcc gcc 360
Asp Lys Ser Pro Val Ser Leu Thr Asn Thr Ser Val Ser Tyr Ala Ala
65                  70                  75                  80

act ctg ccg cca ccg ccg cag ctt gag ccg tcg agt ttt ctg ggt ttg 408
Thr Leu Pro Pro Pro Pro Gln Leu Glu Pro Ser Ser Phe Leu Gly Leu
                85                  90                  95

ccg ccg cat tac ccg agg cag agt aaa ggg ata atg aac tcg gtt ggt 456
Pro Pro His Tyr Pro Arg Gln Ser Lys Gly Ile Met Asn Ser Val Gly
            100                 105                 110

ttg gat cag ttt ctc ggt atc aat aat cat cac acc aaa cca gtt gaa 504
Leu Asp Gln Phe Leu Gly Ile Asn Asn His His Thr Lys Pro Val Glu
        115                 120                 125

tct aat ctt ctc cgt caa agc agc tct cca gcc gga atg ttt act aat 552
Ser Asn Leu Leu Arg Gln Ser Ser Ser Pro Ala Gly Met Phe Thr Asn
    130                 135                 140

ctc tct gac caa aac ggt tat ggt tca atg agg aat ttg atg aat tac 600
Leu Ser Asp Gln Asn Gly Tyr Gly Ser Met Arg Asn Leu Met Asn Tyr
145                 150                 155                 160

gaa gaa gat gaa gag agt cca tct aat tcc aat gga tta aga cgc cat 648
Glu Glu Asp Glu Glu Ser Pro Ser Asn Ser Asn Gly Leu Arg Arg His
                165                 170                 175

tgc agt ctc tct tca agg cca cct tct tca ctt gga atg ctt tct caa 696
Cys Ser Leu Ser Ser Arg Pro Pro Ser Ser Leu Gly Met Leu Ser Gln
            180                 185                 190

ata cct gaa atc gca ccc gaa act aat ttt cca tat agc cat tgg aat 744
Ile Pro Glu Ile Ala Pro Glu Thr Asn Phe Pro Tyr Ser His Trp Asn
        195                 200                 205

gat cca tcc agc ttt att gat aac tta tcc tca ctt aaa aga gaa gcc 792
Asp Pro Ser Ser Phe Ile Asp Asn Leu Ser Ser Leu Lys Arg Glu Ala
    210                 215                 220

gag gac gat gga aaa ttg ttt ctc gga gct cag aac gga gag tcc ggg 840
Glu Asp Asp Gly Lys Leu Phe Leu Gly Ala Gln Asn Gly Glu Ser Gly
225                 230                 235                 240

aat cgt atg cag tta ctg tcg cat cat ttg agc cta cca aag tca tca 888
Asn Arg Met Gln Leu Leu Ser His His Leu Ser Leu Pro Lys Ser Ser
                245                 250                 255

tcg aca gcc tcg gac atg gtt tca gtg gat aag tat ctt cag cta caa 936
Ser Thr Ala Ser Asp Met Val Ser Val Asp Lys Tyr Leu Gln Leu Gln
            260                 265                 270

gat tct gtt cct tgt aaa atc aga gcc aaa cgt ggt tgc gct aca cat 984
Asp Ser Val Pro Cys Lys Ile Arg Ala Lys Arg Gly Cys Ala Thr His
        275                 280                 285

cct cga agc atc gct gaa cgg gta aga aga acg cgg ata agc gag cga 1032
Pro Arg Ser Ile Ala Glu Arg Val Arg Arg Thr Arg Ile Ser Glu Arg

290                 295                 300

atg agg aag tta caa gag ctt gtt cct aac atg gac aag caa acc aac      1080
Met Arg Lys Leu Gln Glu Leu Val Pro Asn Met Asp Lys Gln Thr Asn
305                 310                 315                 320

act tcg gat atg ttg gat tta gct gtg gat tac atc aaa gat tta caa      1128
Thr Ser Asp Met Leu Asp Leu Ala Val Asp Tyr Ile Lys Asp Leu Gln
                325                 330                 335

aga cag tat aag att tta aac gac aac aga gct aac tgt aag tgt atg      1176
Arg Gln Tyr Lys Ile Leu Asn Asp Asn Arg Ala Asn Cys Lys Cys Met
            340                 345                 350

aac aag gag aag aag tca ata tag ggcgcaacaa agtgtgtagt agataggact     1230
Asn Lys Glu Lys Lys Ser Ile
        355

aaaaagcagg gagaaggaca agaaagaaac aatgtcatgt ctgaatattt tttagccgaa    1290

acagaccaaa ttgtctatgt aagctctcga gaaaagcatc tgcttccaac aaaattctaa    1350

gtaataaaat agtactcgat ttgttcttat ttcattatta caatgcagaa tctactaatc    1410

aaa                                                                  1413


<210>  54
<211>  359
<212>  PRT
<213>  Arabidopsis thaliana

<400>  54

Met Asp Ser Asn Asn His Leu Tyr Asp Pro Asn Pro Thr Gly Ser Gly
1               5                   10                  15

Leu Leu Arg Phe Arg Ser Ala Pro Ser Ser Val Leu Ala Ala Phe Val
            20                  25                  30

Asp Asp Asp Lys Ile Gly Phe Asp Ser Asp Arg Leu Leu Ser Arg Phe
        35                  40                  45

Val Thr Ser Asn Gly Val Asn Gly Asp Leu Gly Ser Pro Lys Phe Glu
    50                  55                  60

Asp Lys Ser Pro Val Ser Leu Thr Asn Thr Ser Val Ser Tyr Ala Ala
65                  70                  75                  80

Thr Leu Pro Pro Pro Pro Gln Leu Glu Pro Ser Ser Phe Leu Gly Leu
                85                  90                  95

Pro Pro His Tyr Pro Arg Gln Ser Lys Gly Ile Met Asn Ser Val Gly
            100                 105                 110

Leu Asp Gln Phe Leu Gly Ile Asn Asn His His Thr Lys Pro Val Glu

        115                 120                 125

Ser Asn Leu Leu Arg Gln Ser Ser Ser Pro Ala Gly Met Phe Thr Asn
    130                 135                 140

Leu Ser Asp Gln Asn Gly Tyr Gly Ser Met Arg Asn Leu Met Asn Tyr
145                 150                 155                 160

Glu Glu Asp Glu Glu Ser Pro Ser Asn Ser Asn Gly Leu Arg Arg His
                165                 170                 175

Cys Ser Leu Ser Ser Arg Pro Pro Ser Ser Leu Gly Met Leu Ser Gln
            180                 185                 190

Ile Pro Glu Ile Ala Pro Glu Thr Asn Phe Pro Tyr Ser His Trp Asn
        195                 200                 205

Asp Pro Ser Ser Phe Ile Asp Asn Leu Ser Ser Leu Lys Arg Glu Ala
    210                 215                 220

Glu Asp Asp Gly Lys Leu Phe Leu Gly Ala Gln Asn Gly Glu Ser Gly
225                 230                 235                 240

Asn Arg Met Gln Leu Leu Ser His His Leu Ser Leu Pro Lys Ser Ser
                245                 250                 255

Ser Thr Ala Ser Asp Met Val Ser Val Asp Lys Tyr Leu Gln Leu Gln
            260                 265                 270

Asp Ser Val Pro Cys Lys Ile Arg Ala Lys Arg Gly Cys Ala Thr His
        275                 280                 285

Pro Arg Ser Ile Ala Glu Arg Val Arg Arg Thr Arg Ile Ser Glu Arg
    290                 295                 300

Met Arg Lys Leu Gln Glu Leu Val Pro Asn Met Asp Lys Gln Thr Asn
305                 310                 315                 320

Thr Ser Asp Met Leu Asp Leu Ala Val Asp Tyr Ile Lys Asp Leu Gln
                325                 330                 335

Arg Gln Tyr Lys Ile Leu Asn Asp Asn Arg Ala Asn Cys Lys Cys Met
            340                 345                 350

Asn Lys Glu Lys Lys Ser Ile
        355

<210> 55
<211> 764
<212> DNA
<213> Arabidopsis thaliana

<220>
<221> CDS
<222> (15)..(725)
<223> G208

<400> 55
cttcttcatt cacc atg gga aga tct cct tgt tgt gaa aaa gct cac aca 50
Met Gly Arg Ser Pro Cys Cys Glu Lys Ala His Thr
1               5                   10

aac aaa gga gct tgg act aaa gaa gaa gat caa cgt ctc gta gat tat 98
Asn Lys Gly Ala Trp Thr Lys Glu Glu Asp Gln Arg Leu Val Asp Tyr
        15                  20                  25

atc cgt aat cac ggt gaa ggt tgt tgg cgt tct ctt cct aaa tcc gct 146
Ile Arg Asn His Gly Glu Gly Cys Trp Arg Ser Leu Pro Lys Ser Ala
    30                  35                  40

gga ttg ttg cgt tgt ggt aaa agt tgt aga ttg aga tgg att aat tac 194
Gly Leu Leu Arg Cys Gly Lys Ser Cys Arg Leu Arg Trp Ile Asn Tyr
45                  50                  55                  60

ctt cgt cct gat ctt aaa cgt ggt aat ttt act gat gat gaa gat caa 242
Leu Arg Pro Asp Leu Lys Arg Gly Asn Phe Thr Asp Asp Glu Asp Gln
                65                  70                  75

atc atc atc aaa ctc cat agc tta ctc ggt aac aaa tgg tca ttg ata 290
Ile Ile Ile Lys Leu His Ser Leu Leu Gly Asn Lys Trp Ser Leu Ile
            80                  85                  90

gct gga aga tta cca gga aga aca gat aac gaa ata aag aat tat tgg 338
Ala Gly Arg Leu Pro Gly Arg Thr Asp Asn Glu Ile Lys Asn Tyr Trp
        95                  100                 105

aac act cat att aag agg aag ctt ctt agt cac ggt att gat cca caa 386
Asn Thr His Ile Lys Arg Lys Leu Leu Ser His Gly Ile Asp Pro Gln
    110                 115                 120

act cat cgt cag att aac gaa tcc aaa acg gtg tcg tct caa gtt gtt 434
Thr His Arg Gln Ile Asn Glu Ser Lys Thr Val Ser Ser Gln Val Val
125                 130                 135                 140

gtt cct att caa aac gat gcc gtt gag tat tct ttt tcc aat tta gcc 482
Val Pro Ile Gln Asn Asp Ala Val Glu Tyr Ser Phe Ser Asn Leu Ala
                145                 150                 155

gtt aaa ccg aag acg gaa aat tcc tcc gat aac gga gct tcg act agc 530
Val Lys Pro Lys Thr Glu Asn Ser Ser Asp Asn Gly Ala Ser Thr Ser
            160                 165                 170

ggc acg acg acg gac gag gat ctc cgg cag aat ggg gag tgt tat tat 578
Gly Thr Thr Thr Asp Glu Asp Leu Arg Gln Asn Gly Glu Cys Tyr Tyr
        175                 180                 185

agt gat aat tca gga cat ata aag ctg aat ttg gat tta act ctt ggg 626
Ser Asp Asn Ser Gly His Ile Lys Leu Asn Leu Asp Leu Thr Leu Gly
    190                 195                 200

ttt gga tcc tgg tcg ggt cgg ata gtc gga gtc ggg tca tcg gct gat 674
Phe Gly Ser Trp Ser Gly Arg Ile Val Gly Val Gly Ser Ser Ala Asp
205                 210                 215                 220

tct aaa ccg tgg tgc gac ccg gtg atg gag gcg cgt ttg tca ctg ttg 722
Ser Lys Pro Trp Cys Asp Pro Val Met Glu Ala Arg Leu Ser Leu Leu
                225                 230                 235

taa taatttgtca aaaaaatccc aaaaaatggg tttgttaaa 764

<210> 56
<211> 236
<212> PRT
<213> Arabidopsis thaliana

<400> 56

Met Gly Arg Ser Pro Cys Cys Glu Lys Ala His Thr Asn Lys Gly Ala
1               5                   10                  15

Trp Thr Lys Glu Glu Asp Gln Arg Leu Val Asp Tyr Ile Arg Asn His
            20                  25                  30

Gly Glu Gly Cys Trp Arg Ser Leu Pro Lys Ser Ala Gly Leu Leu Arg
        35                  40                  45

Cys Gly Lys Ser Cys Arg Leu Arg Trp Ile Asn Tyr Leu Arg Pro Asp
    50                  55                  60

Leu Lys Arg Gly Asn Phe Thr Asp Asp Glu Asp Gln Ile Ile Ile Lys
65                  70                  75                  80

Leu His Ser Leu Leu Gly Asn Lys Trp Ser Leu Ile Ala Gly Arg Leu
                85                  90                  95

Pro Gly Arg Thr Asp Asn Glu Ile Lys Asn Tyr Trp Asn Thr His Ile
            100                 105                 110

Lys Arg Lys Leu Leu Ser His Gly Ile Asp Pro Gln Thr His Arg Gln
        115                 120                 125

Ile Asn Glu Ser Lys Thr Val Ser Ser Gln Val Val Val Pro Ile Gln
    130                 135                 140

Asn Asp Ala Val Glu Tyr Ser Phe Ser Asn Leu Ala Val Lys Pro Lys
145                 150                 155                 160

Thr Glu Asn Ser Ser Asp Asn Gly Ala Ser Thr Ser Gly Thr Thr Thr
                165                 170                 175

Asp Glu Asp Leu Arg Gln Asn Gly Glu Cys Tyr Tyr Ser Asp Asn Ser
            180                 185                 190

Gly His Ile Lys Leu Asn Leu Asp Leu Thr Leu Gly Phe Gly Ser Trp
        195                 200                 205

Ser Gly Arg Ile Val Gly Val Gly Ser Ser Ala Asp Ser Lys Pro Trp
    210                 215                 220

Cys Asp Pro Val Met Glu Ala Arg Leu Ser Leu Leu
225                 230                 235

<210>  57
<211>  28
<212>  DNA
<213>  artificial sequence

<220>
<223>  description of artificial sequence: primer

<400>  57
ggcataaccc ttatcggaga tttgaagc                                          28

<210>  58
<211>  28
<212>  DNA
<213>  artificial sequence

<220>
<223>  description of artificial sequence: primer

<400>  58
acacaaactc tgatcttgtc tccgaagg                                          28

<210>  59
<211>  30
<212>  DNA
<213>  artificial sequence

<220>
<223>  description of artificial sequence: primer

<400>  59
gcataaccct tatcggagat ttgaagccat                                        30

<210>  60
<211>  30
<212>  DNA
<213>  artificial sequence

<220>
<223>  description of artificial sequence: primer

<400>  60
aacattcctc tctcatcatc tgttgccagc                                        30


<210>  61
<211>  28
<212>  DNA
<213>  artificial sequence

<220>
<223>  description of artificial sequence: primer


<400>  61
aacgcttagt atctccggcg acttgaac                                          28


<210>  62
<211>  28
<212>  DNA
<213>  artificial sequence

<220>
<223>  description of artificial sequence: primer


<400>  62
ctcacacgaa taaggtacaa agttcatc                                          28


<210>  63
<211>  30
<212>  DNA
<213>  artificial sequence

<220>
<223>  description of artificial sequence: primer


<400>  63
ttagtatctc cggcgacttg aacccaaacc                                        30


<210>  64
<211>  30
<212>  DNA
<213>  artificial sequence

<220>
<223>  description of artificial sequence: primer


<400>  64
agattctcaa caagcttcaa catgagttcg                                        30

## Description

The present invention claims priority in part from US Provisional Application Serial Nos. 60/159,464 filed October 12, 1999; 60/164,132 filed November 8, 1999; 60/166,228 filed November 17, 1999; 60/197,899 filed April 17, 2000; and Plant Trait Modification III, filed August 22, 2000.

### FIELD OF THE INVENTION

This invention is in the field of plant molecular biology and relates to compositions and methods for modifying a plant's flowering time or vernalization requirements.

### BACKGROUND OF THE INVENTION

In order to maximize reproductive success, plants have evolved complex mechanisms to ensure that flowering occurs under favorable conditions. Analysis of late flowering mutants and ecotypes in *Arabidopsis* has revealed that such mechanisms are based upon several genetic pathways which may contain 80 or more genes (Martinez-Zapater and Somerville, (1990) *Plant Physiol.* 92:770-776; Koornneef et al. (1991) *Mol. Gen. Genet.* 229:57-66; EM Meyerwitz and CR Somerville Eds (1994) *Arabidopsis* pp 403-433 Cold Spring Harbor Laboratory Press, New York). Together these loci co-ordinate flowering time with environmental variables (e.g. day-length, temperature, light quality, and nutrient availability) and with the developmental stage of the plant.

Arabidopsis flowers rapidly when grown under long day conditions of 16 hours or continuous light, but flowers much later under short day conditions of 8 or 10 hours light. Genes regulating this response constitute the photoperiod pathway and were identified by mutations that cause late flowering under long day conditions but which do not alter flowering in short day conditions. Examples from this group, which promote flowering in response to long days, include *CONSTANS* (CO), *GIGANTEA (G*/*), FT, FWA, FE, FD,* and *FHA.* A second group of genes, which includes *LUMINIDEPENDENS (LD), FCA, FVE, FY,* and *FPA,* form an autonomous pathway that is active under all day-length conditions. Mutants for this second class of genes flower later than wild type controls irrespective of the day length conditions (Koornneef *et al.* (1991) *Mol. Gen. Genet.* 229:57-66; EM Meyerwitz and CR Somerville Eds (1994) *Arabidopsis* pp 403-433 Cold Spring Harbor Laboratory Press, New York).

In addition to differing in their response to day-length, mutants from the photoperiod and autonomous pathways show a differential response to prolonged cold (vernalization) treatments (Vince-Prue, (1975) *Vernalization.* In Photoperiodism in Plants pp 263-291, McGraw Hill, London) Through a vernalization response, *Arabidopsis* ecotypes from Northern latitudes, such as Stockholm, are adapted to flower in the spring following exposure to cold winter conditions. This avoids flowering in the late summer when seed maturation might be curtailed by the onset of winter conditions (Reeves and Coupland, (2000) *Curr. Opin. Plant Biol* 3:37-42). When these ecotypes are grown in the laboratory they flower late, but will flower much earlier if subjected to a cold period of 4-6 weeks during seed germination. In a comparable manner, mutants from the autonomous pathway exhibit a very marked reduction in flowering time when subjected to vernalization. In contrast, mutants from the photoperiod pathway only show a minor response to cold treatments (Chandler *et al.,* (1996) *Plant J.* 10:637-644; Koornneef *et al.,* (1998) *Genetics* 148:885-892). Thus, vernalization can overcome the requirement for the autonomous pathway conditions (Reeves and Coupland, (2000) *Curr. Opin. Plant Biol* 3:37-42).

Two *Arabidopsis* genes, *FLOWERING LOCUS* C, *FLC* (also known as *FLOWERING LOCUS F, FLF)* and *FRIGIDA (FRI),* act in conjunction to repress flowering in the absence of a vernalization treatment (Napp-Zinn, K. (1957) *Indukt. Abstammungs. Verebungsl.* 88:253-285; Napp-Zinn K. (1985) *CRC Handbook* of *Flowering,* Vol. 1, A. H. Halevy, pp 492-503; Clarke and Dean (1994) *Mol. Gen. Genet.* 248:81-89; Koornneef. et al., (1994) *Plant Journal* 6:911-919; Lee et al., (1994) *Plant Journal* 6:903-909.) Dominant functional alleles of FLC and *FRI* are found together in Northern European *Arabidopsis* ecotypes such as Pitztal and Stockholm. These ecotypes are extremely late flowering when non-vernalized. The widely used laboratory ecotype Columbia contains functional alleles at only one of these two loci and flower slightly later than strains such as Landsberg *erecta* which possess functional alleles of neither gene. The *FRIGIDA* protein sequence has not yet been published. However, the *FLC* gene has recently been cloned and shown to encode a MADS box protein (Sheldon C. et al., 1999, *Plant Cell* 11:445-458; Michaels S. and Amasino, R., 1999, *Plant Cell* 11:949-956). Dominant alleles and overexpression of *FLC* have been reported to delay flowering, while null *flc* mutants are early flowering (Lee *et al.,* (1994) *Plant J.* 6:903-909; Michaels and Amasino, (1999) *Plant Cell* 11:949-956; Sheldon *et al.,* (1999) *Proc. Natl. Acad. Sci.* 97:3753-3758). Thus, *FLC* acts to prevent premature flowering.

We have discovered transcription factors that regulate flowering time or vernalization requirements of plants. These transcription factors could therefore be useful to manipulate flowering characteristics of a plant.

### SUMMARY OF THE INVENTION

In one aspect, the present invention relates to a transgenic plant comprising a recombinant polynucleotide. The recombinant polynucleotide comprises a nucleotide sequence encoding a polypeptide comprising at least 6 consecutive amino acids of a sequence selected from the group consisting of SEQ ID Nos. 2N, where N=1-28 but excluding SEQ ID No. 28, and the presence of the recombinant polynucleotide alters the flowering time or vernalization requirements of the transgenic plant when compared with the same trait of another plant lacking the recombinant polynucleotide.

In one embodiment, the nucleotide sequence encodes a polypeptide comprising a conserved domain such as 1) a localization domain, 2) an activation domain, 3) a repression domain, 4) an oligomerization domain or 5) a DNA binding domain of SEQ ID Nos. 2N, where N=1-28. In another embodiment, the recombinant polynucleotide encodes a polypeptide comprising a conserved domain having greater than an 84% sequence identity to a sequence selected from the group consisting of SEQ ID Nos. 2N, where N=1-28. In a further embodiment, the nucleotide sequence further comprises a promoter operably linked to the nucleotide sequence. The promoter may be a constitutive or inducible or tissue-active.

In a second aspect, the present invention relates to a method for altering a plant's flowering time or vernalization requirements. The method comprises (a) transforming a plant with a recombinant polynucleotide comprising a nucleotide sequence encoding a polypeptide comprising at least 6 consecutive amino acids of a sequence selected from the group consisting of SEQ ID Nos. 2N, where N=1-28; (b) selecting transformed plants; and (c) identifying a transformed plant with the desired trait.

In one embodiment, the nucleotide sequence encodes a polypeptide comprising a conserved domain such as 1) a localization domain, 2) an activation domain, 3) a repression domain, 4) an oligomerization domain or 5) a DNA binding domain domain of SEQ ID Nos. 2N, where N=1-28 but excluding SEQ ID No. 28. In another embodiment, the recombinant polynucleotide encodes a polypeptide comprising a conserved domain having greater than an 84% sequence identity to a sequence selected from the group consisting of SEQ ID Nos. 2N, where N=1-28. In a further embodiment, the nucleotide sequence further comprises a promoter operably linked to the nucleotide sequence. The promoter may be a constitutive or inducible or tissue-active.

In a third aspect, the present invention relates to another method for altering a plant trait associated with flowering time or the plant's vernalization requirements. The method comprises (a) transforming the plant with a recombinant polynucleotide comprising a nucleotide sequence comprising at least 18 consecutive nucleotides of a sequence selected from the group consisting of SEQ ID Nos. 2N-1, where N= 1-28 but excluding SEQ ID No. 27; and (b) selecting said transformed plant.

In yet another aspect, the present invention is yet another method for altering a plant's flowering time or vernalization requirements. The method comprises (a) providing a database sequence; (b) comparing the database sequence with a polypeptide selected from SEQ ID Nos. 2N, where N= 1-28; (c) selecting a database sequence that meets selected sequence criteria; and (d) transforming said database sequence in the plant. Alternatively, the database sequence can be compared with a polynucleotide selected from SEQ ID Nos. 2N-1, where N= 1-28.

### BRIEF DESCRIPTION OF THE FIGURES

Figure 1 provides a table of exemplary polynucleotide and polypeptide sequences of the invention. The table includes from left to right for each sequence: the SEQ ID No., the internal code reference number, whether the sequence is a polynucleotide or polypeptide sequence, and identification of any conserved domains for the polypeptide sequences.
Figure 2 provides a table of sequences that are homologous to the sequences provided in the Sequence Listing. The table includes from left to right: the SEQ ID No., the internal code reference number, the unique Genbank sequence ID No. (NID), the probability that the comparison was generated by chance (P-value), and the species from which the homologous gene was identified.

### DETAILED DESCRIPTION OF THE INVENTION

### DEFINITIONS

A "recombinant polynucleotide" is a nucleotide sequence comprising a gene coding sequence or a fragment thereof (comprising at least 18 consecutive nucleotides, preferably at least 30 consecutive nucleotides, and more preferably at least 50 consecutive nucleotides). Additionally, the polynucleotide may comprise a promoter, an intron, an enhancer region, a polyadenylation site, a translation initiation site, 5' or 3' untranslated regions, a reporter gene, a selectable marker or the like. The polynucleotide may comprise single stranded or double stranded DNA or RNA. The polynucleotide may comprise modified bases or a modified backbone. The polynucleotide may be genomic, a transcript (such as an mRNA) or a processed nucleotide sequence (such as a cDNA). The polynucleotide may comprise a sequence in either sense or antisense orientations.

A "recombinant polynucleotide" is a polynucleotide that is not in its native state, e.g., the polynucleotide is comprised of a nucleotide sequence not found in nature or the polynucleotide is separated from nucleotide sequences with which it typically is in proximity or is next to nucleotide sequences with which it typically is not in proximity.

A "recombinant polypeptide" is a polypeptide derived from the translation of a recombinant polynucleotide or is more enriched in a cell than the polypeptide in its natural state in a wild type cell, e.g. more than 5% enriched, more than 10% enriched or more than 20% enriched and is not the result of a natural response of a wild type plant or is separated from other components with which it is typically associated with in a cell.

A "transgenic plant" may refer to a plant that contains genetic material not normally found in a wild type plant of the same species, or in a naturally occurring variety or in a cultivar, and which has been introduced into the plant by human manipulation. A transgenic plant is a plant that may contain an expression vector or cassette. The expression cassette comprises a gene coding sequence and allows for the expression of the gene coding sequence. The expression cassette may be introduced into a plant by transformation or by breeding after transformation of a parent plant.

A transgenic plant refers to a whole plant as well as to a plant part, such as seed, fruit, leaf, or root, plant tissue, plant cells, protoplasts or any other plant material, and progeny thereof.

The phrase "altered expression" in reference to polynucleotide or polypeptide expression refers to an expression pattern in the transgenic plant that is different from the expression pattern in the wild type plant or a reference; for example, by expression in a cell type other than a cell type in which the sequence is expressed in the wild type plant, or by expression at a time other than at the time the sequence is expressed in the wild type plant, or by a response to different inducible agents, such as hormones or environmental signals, or at different expression levels (either higher or lower) compared with those found in a wild type plant. The term also refers to lowering the levels of expression to below the detection level or completely abolishing expression. The resulting expression pattern may be transient or stable, constitutive or inducible.

A "transcription factor" (TF) refers to a polynucleotide or polypeptide that controls the expression of a gene or genes either directly by binding to one or more nucleotide sequences associated with a gene coding sequence or indirectly by affecting the level or activity of other polypeptides that do bind directly or indirectly to one or more nucleotide sequences associated with a gene coding sequence. A TF, in this definition, includes any polypeptide that can activate or repress transcription of a single gene or a number of genes. This polypeptide group includes, but is not limited to, DNA binding proteins, protein kinases, protein phosphatases, GTP-binding proteins and receptors.

The transcription factor sequence may comprise a whole coding sequence or a fragment or domain of a coding sequence. A "fragment or domain", as referred to polypeptides, may be a portion of a polypeptide which performs at least one biological function of the intact polypeptide in substantially the same manner or to a similar extent as does the intact polypeptide. A fragment may comprise, for example, a DNA binding domain that binds to a specific DNA promoter region, an activation domain or a domain for protein-protein interactions. Fragments may vary in size from as few as 6 amino acids to the length of the intact polypeptide, but are preferably at least 30 amino acids in length and more preferably at least 60 amino acids in length. In reference to a nucleotide sequence "a fragment" refers to any sequence of at least consecutive 18 nucleotides, preferably at least 30 nucleotides, more preferably at least 50, of any of the sequences provided herein.

Exemplary polynucleotides and polypeptides comprise a sequence provided in the Sequence Listing as SEQ ID No. 1: G157 (cDNA); SEQ ID No. 2: G157 (protein); SEQ ID No. 3: G859 (cDNA); SEQ ID No. 4: G859 (protein); SEQ ID No. 5: G859.1 (cDNA); SEQ ID No. 6: G859.1 (protein); SEQ ID No. 7: G859.2 (cDNA); SEQ ID No. 8: G859.2 (protein); SEQ ID No. 9: G1842 (cDNA); SEQ ID No. 10: G1842 (protein); SEQ ID No. 11: G1842.2 (cDNA); SEQ ID No. 12: G1842.2 (protein); SEQ ID No. 13: G1842.6 (cDNA); SEQ ID No. 14: G1842.6 (protein); SEQ ID No. 15: G1842.7 (cDNA); SEQ ID No. 16: G1842.7 (protein); SEQ ID No. 17: G1843 (cDNA); SEQ ID No. 18: G1843 (protein); SEQ ID No. 19: G1844 (cDNA); SEQ ID No. 20: G1844 (protein); SEQ ID No. 21: G1844.2 (cDNA); SEQ ID No. 22: G1844.2 (protein); SEQ ID No. 23: G861 (cDNA); SEQ ID No. 24: G861 (protein); SEQ ID No. 25: G861.1 (cDNA); SEQ ID No. 26: G861.1 (protein); SEQ ID No. 27: G1759 (cDNA); SEQ ID No. 28: G1759 (protein); SEQ ID No. 29: G192 (cDNA); SEQ ID No. 30: G192 (protein); SEQ ID No. 31: G234 (cDNA); SEQ ID No. 32: G234 (protein); SEQ ID No. 33: G361 (cDNA); SEQ ID No. 34: G361 (protein); SEQ ID No. 35: G486 (cDNA); SEQ ID No. 36: G486 (protein); SEQ ID No. 37: G748 (cDNA); SEQ ID No. 38: G748 (protein); SEQ ID No. 39: G994 (cDNA); SEQ ID No. 40: G994 (protein); SEQ ID No. 41: G1335 (cDNA); SEQ ID No. 42: G1335 (protein); SEQ ID No. 43: G562 (cDNA); SEQ ID No. 44: G562 (protein); SEQ ID No. 45: G736 (cDNA); SEQ ID No. 46: G736 (protein); SEQ ID No. 47: G1073 (cDNA); SEQ ID No. 48: G1073 (protein); SEQ ID No. 49: G1435 (cDNA); SEQ ID No. 50: G1435 (protein); SEQ ID No. 51: G180 (cDNA); SEQ ID No. 52: G180 (protein); SEQ ID No. 53: G592 (cDNA); SEQ ID No. 54: G592 (protein); SEQ ID No. 55: G208 (cDNA); and SEQ ID No. 56: G208 (protein).

A "conserved domain" refers to a polynucleotide or polypeptide fragment that is more conserved at a sequence level than other fragments when the polynucleotide or polypeptide is compared with homologous genes or proteins from other plants. The conserved domain may be 1) a localization domain, 2) an activation domain, 3) a repression domain, 4) a dimerization or oligomerization domain, 5) a DNA binding domain or any combination thereof. For MADS proteins, the conserved domain is typically a DNA-binding domain.

A nucleotide sequence is "operably linked" when it is placed into a functional relationship with another nucleotide sequence. For example, a promoter or enhancer is operably linked to a gene coding sequence if the presence of the promoter or enhancer increases the level of expression of the gene coding sequence.

"Trait" refers to a physiological, morphological, biochemical or physical characteristic of a plant or particular plant material or cell. This characteristic may be visible to the human eye, such as seed or plant size, or be measured by biochemical techniques, such as the protein, starch or oil content of seed or leaves or by the observation of the expression level of genes by employing Northerns, RT PCR, microarray gene expression assays or reporter gene expression systems or be measured by agricultural observations such as stress tolerance, yield or disease resistance.

"Trait modification" refers to a detectable difference in a characteristic in a transgenic plant with modified expression of a polynucleotide or polypeptide of the present invention relative to a plant not doing so, such as a wild type plant. The trait modification may entail at least a 5% increase or decrease in an observed trait (difference), at least a 10% difference, at least a 20% difference, at least a 30%, at least a 50%, at least a 70%, at least a 100% or a greater difference. It is known that there may be a natural variation in the modified trait. Therefore, the trait modification observed entails a change in the normal distribution of the trait in transgenic plants compared with the distribution observed in wild type plant.

Trait modifications of particular interest include those to seed (embryo), fruit, root, flower, leaf, stem, shoot, seedling or the like, including: enhanced tolerance to environmental conditions including freezing, chilling, heat, drought, water saturation, radiation and ozone; enhanced resistance to microbial, fungal or viral diseases; resistance to nematodes, decreased herbicide sensitivity, enhanced tolerance of heavy metals (or enhanced ability to take up heavy metals), enhanced growth under poor photoconditions (e.g., low light and/or short day length), or changes in expression levels of genes of interest. Other phenotypes that may be modified relate to the production of plant metabolites, such as variations in the production of taxol, tocopherol, tocotrienol, sterols, phytosterols, vitamins, wax monomers, anti-oxidants, amino acids, lignins, cellulose, tannins, prenyllipids (such as chlorophylls and carotenoids), glucosinolates, and terpenoids, enhanced or compositionally altered protein or oil production (especially in seeds), or modified sugar (insoluble or soluble) and/or starch composition. Physical plant characteristics that may be modified include cell development (such as the number of trichomes), fruit and seed size and number, yields of plant parts such as stems, leaves and roots, the stability of the seeds during storage, characteristics of the seed pod (e.g., susceptibility to shattering), root hair length and quantity, internode distances, or the quality of seed coat. Plant growth characteristics that may be modified include growth rate, germination rate of seeds, vigor of plants and seedlings, leaf and flower senescence, male sterility, apomixis, flowering time, flower abscission, rate of nitrogen uptake, biomass or transpiration characteristics, as well as plant architecture characteristics such as apical dominance, branching patterns, number of organs, organ identity, organ shape or size.

Of particular interest are traits relating to modified vernalization requirements or flowering time characteristics, such as changes in flowering time in response to day-length, in response to temperature, in response to light quality, nutrient availability, and development stage of the plant, and the like.

### 1. The Sequences

We have discovered particular plant transcription factors (TFs) that can be employed to modify the flowering time of a plant. Therefore, the flowering time of plants can be either decreased, increased, or made inducible under specific conditions using the TFs of this invention. Additionally, the transcription factors can be used to modify the vernalization requirements of the plant.

The plant transcription factors may belong to one of the following transcription factor families: the AP2 (APETALA2) domain transcription factor family (Riechmann and Meyerowitz (1998) *Biol. Chem.* 379:633-646); the MYB transcription factor family (Martin and Paz-Ares, (1997) *Trends Genet.* 13:67-73); the MADS domain transcription factor family (Riechmann and Meyerowitz (1997) *Biol. Chem.* 378:1079-1101); the WRKY protein family (Ishiguro and Nakamura (1994) *Mol. Gen. Genet.* 244:563-571); the ankyrin-repeat protein family (Zhang et al. (1992) *Plant Cell* 4:1575-1588); the zinc finger protein (Z) family (Klug and Schwabe (1995) *FASEB J.* 9: 597-604); the homeobox (HB) protein family (Duboule (1994) *Guidebook to the Homeobox Genes,* Oxford University Press); the CAAT-element binding proteins (Forsburg and Guarente (1989) *Genes Dev.* 3:1166-1178); the squamosa promoter binding proteins (SPB) (Klein et al. (1996) *Mol. Gen. Genet.* 1996 250:7-16); the NAM protein family (Souer et al. (1996) Cell 85:159-170); the IAA/AUX proteins (Rouse et al. (1998) *Science* 279:1371-1373); the HLH/MYC protein family (Littlewood et al. (1994) *Prot. Profile* 1:639-709); the DNA-binding protein (DBP) family (Tucker et al. (1994) *EMBO J.* 13:2994-3002); the bZIP family of transcription factors (Foster et al. (1994) *FASEB J.* 8:192-200); the Box P-binding protein (the BPF-1 ) family (da Costa e Silva et al. (1993) *Plant J.* 4:125-135); the high mobility group (HMG) family (Bustin and Reeves (1996) *Prog. Nucl. Acids Res. Mol. Biol.* 54:35-100); the scarecrow (SCR) family (Di Laurenzio et al. (1996) *Cell* 86:423-433); the GF14 family (Wu et al. (1997) *Plant Physiol.* 114:1421-1431 ); the polycomb (PCOMB) family (Kennison (1995) *Annu. Rev. Genet.* 29:289-303); the teosinte branched (TEO) family (Luo et al. (1996) *Nature* 383:794-799; the ABl3 family (Giraudat et al. (1992) *Plant Cell* 4:1251 -1261); the triple helix (TH) family (Dehesh et al. (1990) *Science* 250:1397-1399); the EIL family (Chao et al. (1997) *Cell* 89:1133-44); the AT-HOOK family (Reeves and Nissen (1990) *Journal of Biological Chemistry* 265:8573-8582); the S1 FA family (Zhou et al. (1995) *Nucleic Acids Res.* 23:1165-1169); the bZIPT2 family (Lu and Ferl (1995) *Plant Physiol.* 109:723); the YABBY family (Bowman et al. (1999) *Development* 126:2387-96); the PAZ family (Bohmert et al. (1998) *EMBO J.* 17:170-80); a family of miscellaneous (MISC) transcription factors including the DPBF family (Kim et al. (1997) *Plant J.* 11:1237-1251) and the SPF1 family (Ishiguro and Nakamura (1994) *Mol. Gen. Genet.* 244:563-571); the golden (GLD) family (Hall et al. (1998) *Plant Cell* 10:925-936), and the TUBBY family (Boggin et al, (1999) Science 286:2119-2125)

In particular, the TFs that we have discovered that are implicated in flowering time or vernalization include members of the MADS transcription factor family, the MYB family, the WRKY family, the HLH/MYC family, GLD family, AT-HOOK family, the CAAT family, the bZIP family, and members of zinc coordinating protein families (Z-Dof, Z-CLDSH and Z-CH2H2). In fact we have identified the first members of the WRKY, CAAT, bZIP, AT-HOOK and HLH/MYC families that are associated with flowering time modification in plants: G192 and G190 (WRKY), G486 (CAAT), G562 (bZIP), G1073 (AT-HOOK) and G592 (HLH/MYC).

The polynucleotides and polypeptides are provided in the Sequence Listing and are tabulated in Figure 1. Figure 1 identifies a SEQ ID No., its corresponding GID number, whether the sequence is a polynucleotide or a polypeptide sequence, and indicates the conserved domains. We have also identified domains or fragments derived from each of the sequences in the Sequence Listing. The fragments can be from any region of the sequence, can be of any length up to the length of the sequence, and can be as short as six residues for protein and 18 nucleotides for DNA. Exemplary fragments of the DNA sequences are as follows: 1-50, 51-100, 101-200, 201-218, 218-300, 301-450 and 450-600; and exemplary fragments of proteins are as follows 1-50, 51-100, 101-200, 201-206, 206-250, 251-300. For DNA sequences, the numbers may be measured from either 5' or 3' end of the DNA. For the protein sequences the fragment location is determined from the N-terminus or C-terminus of the protein and may include adjacent amino acid sequences, such as for example for SEQ ID No. 2 an additional 10, 20, 40, 60 or 100 amino acids in either N-terminal or C-terminal direction of the described fragments.

The identified polypeptide fragments may be linked to fragments or sequences derived from other transcription factors so as to generate additional novel sequences, such as by employing the methods described in Short, PCT publication WO9827230, entitled "Methods and Compositions for Polypeptide Engineering" or in Patten et al., PCT publication WO9923236, entitled "Method of DNA Shuffling" or in Minshull and Stemmer, US Patent No. 5,837,458. Alternatively, the identified fragment may be linked to a transcription activation domain. A transcription activation domain assists in initiating transcription from a DNA binding site. A common feature of some activation domains is that they are designed to form amphiphilic alpha helices with excess positive or negative charge (Giniger and Ptashne (1987) Nature 330:670-672, Gill and Ptashne (1987) Cell 51:121-126, Estruch et al (1994) Nucl. Acids Res. 22:3983-3989). Examples include the transcription activation region of VP16 or GAL4 ( Moore et al. (1998) Proc. Natl. Acad. Sci. USA 95: 376-381; and Aoyama et al. (1995) Plant Cell 7:1773-1785), peptides derived from bacterial sequences (Ma and Ptashne (1987) Cell 51; 113-119) and synthetic peptides (Giniger and Ptashne, supra).

The isolated polynucleotides and polypeptides may be used to modify plant development, physiology or biochemistry such that the modified plants have a trait advantage over wild type plants. The identified polynucleotide fragments are also useful as nucleic acid probes and primers. A nucleic acid probe is useful in hybridization protocols, including protocols for microarray experiments. Primers may be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR) or other nucleic-acid amplification methods. See Sambrook et al., *Molecular Cloning. A Laboratory Manual,* Ed. 2, Cold Spring Harbor Laboratory Press, New York (1989) and Ausubel et al. (eds) *Current Protocols in Molecular Biology,* John Wiley & Sons (1998).

### 2. Identification of Homologous Sequences (Homologs)

Homologous sequences to those provided in the Sequence Listing derived from *Arabidopsis thaliana* or from other plants may be used to modify a plant trait. Homologous sequences may be derived from any plant including monocots and dicots and in particular agriculturally important plant species, including but not limited to, crops such as soybean, wheat, corn, potato, cotton, rice, oilseed rape (including canola), sunflower, alfalfa, sugarcane and turf; or fruits and vegetables, such as banana, blackberry, blueberry, strawberry, and raspberry, cantaloupe, carrot, cauliflower, coffee, cucumber, eggplant, grapes, honeydew, lettuce, mango, melon, onion, papaya, peas, peppers, pineapple, spinach, squash, sweet corn, tobacco, tomato, watermelon, rosaceous fruits (such as apple, peach, pear, cherry and plum) and vegetable brassicas (such as broccoli, cabbage, cauliflower, brussel sprouts and kohlrabi). Other crops, fruits and vegetables whose phenotype may be changed include barley, currant, avocado, citrus fruits such as oranges, lemons, grapefruit and tangerines, artichoke, cherries, nuts such as the walnut and peanut, endive, leek, roots, such as arrowroot, beet, cassava, turnip, radish, yam, sweet potato and beans. The homologs may also be derived from woody species, such pine, poplar and eucalyptus.

Substitutions, deletions and insertions introduced into the sequences provided in the Sequence Listing are also envisioned by the invention. Such sequence modifications can be engineered into a sequence by site-directed mutagenesis (Wu (ed.) *Meth. Enzymol.* (1993) vol. 217, Academic Press). Amino acid substitutions are typically of single residues; insertions usually will be on the order of about from 1 to 10 amino acid residues; and deletions will range about from 1 to 30 residues. In preferred embodiments, deletions or insertions are made in adjacent pairs, e.g., a deletion of two residues or insertion of two residues. Substitutions, deletions, insertions or any combination thereof may be combined to arrive at a sequence. The mutations that are made in the polynucleotide encoding the transcription factor should not place the sequence out of reading frame and should not create complementary regions that could produce secondary mRNA structure.

Substitutions are those in which at least one residue in the amino acid sequence has been removed and a different residue inserted in its place. Such substitutions may be conservative with little effect on the function of the gene, for example by substituting alanines for serines, arginines for lysines, glutamate for aspartate and the like. The substitutions which are not conservative are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

Additionally, the term "homologous sequence" may encompass a polypeptide sequence that is modified by chemical or enzymatic means. The homologous sequence may be a sequence modified by lipids, sugars, peptides, organic or inorganic compounds, by the use of modified amino acids or the like. Protein modification techniques are illustrated in Ausubel et al. (eds) *Current Protocols in Molecular Biology,* John Wiley & Sons (1998).

Homologous sequences also may mean two sequences having a substantial percentage of sequence identity after alignment as determined by using sequence analysis programs for database searching and sequence alignment and comparison available, for example, from the Wisconsin Package Version 10.0, such as BLAST, FASTA, PILEUP, FINDPATTERNS or the like (GCG, Madision, WI). Public sequence databases such as GenBank, EMBL, Swiss-Prot and PIR or private sequence databases such as PhytoSeq (Incyte Pharmaceuticals, Palo Alto, CA) may be searched. Alignment of sequences for comparison may be conducted by the local homology algorithm of Smith and Waterman (1981) *Adv. Appl. Math.* 2:482, by the homology alignment algorithm of Needleman and Wunsch (1970) *J. Mol. Biol.* 48:443, by the search for similarity method of Pearson and Lipman (1988) *Proc. Natl. Acad. Sci. U.S.A.* 85: 2444, by computerized implementations of these algorithms. After alignment, sequence comparisons between two (or more) polynucleotides or polypeptides are typically performed by comparing sequences of the two sequences over a comparison window to identify and compare local regions of sequence similarity. The comparison window may be a segment of at least about 20 contiguous positions, usually about 50 to about 200, more usually about 100 to about 150 contiguous positions. A description of the method is provided in Ausubel et al. (eds) (1999) *Current Protocols in Molecular Biology,* John Wiley & Sons.

Transcription factors that are homologs of the disclosed sequences will typically share at least 40% amino acid sequence identity. More closely related TFs may share at least 50%, 60%, 65%, 70%, 75% or 80% sequence identity with the disclosed sequences. Factors that are most closely related to the disclosed sequences share at least 85%, 90% or 95% sequence identity. At the nucleotide level, the sequences will typically share at least 40% nucleotide sequence identity, preferably at least 50%, 60%, 70% or 80% sequence identity, and more preferably 85%, 90%, 95% or 97% sequence identity. The degeneracy of the genetic code enables major variations in the nucleotide sequence of a polynucleotide while maintaining the amino acid sequence of the encoded protein.

One way to identify whether two nucleic acid molecules are closely related is that the two molecules hybridize to each other under stringent conditions. Generally, stringent conditions are selected to be about 5°C to 20°C lower than the thermal melting point (Tm) for the specific sequence at a defined ionic strength and pH. The Tₘ is the temperature (under defined ionic strength and pH) at which 50% of the target sequence hybridizes to a perfectly matched probe. Conditions for nucleic acid hybridization and calculation of stringencies can be found in Sambrook et al. (1989) *Molecular Cloning. A Laboratory Manual,* Ed. 2, Cold Spring Harbor Laboratory Press, New York and Tijssen (1993) *Laboratory Techniques in Biochemistry and Molecular Biology--Hybridization with Nucleic Acid Probes* Part I, Elsevier, New York. Nucleic acid molecules that hybridize under stringent conditions will typically hybridize to a probe based on either the entire cDNA or selected portions of the cDNA under wash conditions of 0.2x SSC to 2.0 x SSC, 0.1 % SDS at 50-65° C, for example 0.2 x SSC, 0.1 % SDS at 65° C. For detecting less closely related homologs washes may be performed at 50° C.

For conventional hybridization the hybridization probe is conjugated with a detectable label such as a radioactive label, and the probe is preferably of at least 20 nucleotides in length. As is well known in the art, increasing the length of hybridization probes tends to give enhanced specificity. The labeled probe derived from the *Arabidopsis* nucleotide sequence may be hybridized to a plant cDNA or genomic library and the hybridization signal detected using means known in the art. The hybridizing colony or plaque (depending on the type of library used) is then purified and the cloned sequence contained in that colony or plaque isolated and characterized. Homologs may also be identified by PCR-based techniques, such as inverse PCR or RACE, using degenerate primers. See Ausubel et al. (eds) (1998) *Current Protocols in Molecular Biology,* John W iley & Sons.

TF homologs may alternatively be obtained by immunoscreening an expression library. With the provision herein of the disclosed TF nucleic acid sequences, the polypeptide may be expressed and purified in a heterologous expression system (e.g., *E. coli)* and used to raise antibodies (monoclonal or polyclonal) specific for the TF. Antibodies may also be raised against synthetic peptides derived from TF amino acid sequences. Methods of raising antibodies are well known in the art and are described in Harlow and Lane (1988) *Antibodies: A Laboratory Manual,* Cold Spring Harbor Laboratory, New York. Such antibodies can then be used to screen an expression library produced from the plant from which it is desired to clone the TF homolog, using the methods described above. The selected cDNAs may be confirmed by sequencing and biological activity.

### 3. Altered Expression of Transcription Factors

Any of the identified sequences may be incorporated into a cassette or vector for expression in plants. A number of expression vectors suitable for stable transformation of plant cells or for the establishment of transgenic plants have been described including those described in Weissbach and Weissbach, (1989) *Methods for Plant Molecular Biology,* Academic Press, and Gelvin et al., (1990) *Plant Molecular Biology Manual,* Kluwer Academic Publishers. Specific examples include those derived from a Ti plasmid of *Agrobacterium tumefaciens,* as well as those disclosed by Herrera-Estrella, L., et al., (1983) *Nature* 303: 209, Bevan, M., *Nucl. Acids Res.* (1984) 12: 8711-8721, Klee, H. J., (1985) *Bio*/*Technology* 3: 637-642, for dicotyledonous plants. Ti-derived plasmids can be transferred into both monocotonous and docotyledonous species using Agrobacterium-mediated transformation (Ishida et al (1996) *Nat. Biotechnol.* 14:745-50; Barton et al. (1983) *Cell* 32:1033-1043).

Alternatively, non-Ti vectors can be used to transfer the DNA into plants and cells by using free DNA delivery techniques. Such methods may involve, for example, the use of liposomes, electroporation, microprojectile bombardment, silicon carbide wiskers, and viruses. By using these methods transgenic plants such as wheat, rice (Christou, P., (1991) *Bio*/*Technology 9:*957-962) and corn (Gordon-Kamm, W., (1990) *Plant Cell2:* 603-618) can be produced. An immature embryo can also be a good target tissue for monocots for direct DNA delivery techniques by using the particle gun (Weeks, T. et al., (1993) *Plant Physiol.* 102: 1077-1084; Vasil, V., (1993) *Bio*/*Technology* 10: 667-674; Wan, Y. and Lemeaux, P., (1994) *Plant Physiol.* 104: 37-48, and for Agrobacterium-mediated DNA transfer (Ishida et al., (1996) *Nature Biotech.* 14: 745-750).

Typically, plant transformation vectors include one or more cloned plant coding sequences (genomic or cDNA) under the transcriptional control of 5' and 3' regulatory sequences and a dominant selectable marker. Such plant transformation vectors typically also contain a promoter (e.g., a regulatory region controlling inducible or constitutive, environmentally-or developmentally-regulated, or cell- or tissue-specific expression), a transcription initiation start site, an RNA processing signal (such as intron splice sites), a transcription termination site, and/or a polyadenylation signal.

Examples of constitutive plant promoters which may be useful for expressing the TF sequence include: the cauliflower mosaic virus (CaMV) 35S promoter, which confers constitutive, high-level expression in most plant tissues *(see, e.g.,* Odel et al., (1985) *Nature* 313:810); the nopaline synthase promoter (An et al., (1988) *Plant Physiol.* 88:547); and the octopine synthase promoter (Fromm et al., (1989) *Plant Cell* 1: 977).

A variety of plant gene promoters that regulate gene expression in response to environmental, hormonal, chemical, developmental signals, and in a tissue-active manner can be used for expression of the TFs in plants, as illustrated by seed-specific promoters (such as the napin, phaseolin or DC3 promoter described in US Pat. No. 5,773,697), root-specific promoters, such as those disclosed in US Patent Nos. 5,618,988, 5,837,848 and 5,905,186; fruit-specific promoters that are active during fruit ripening (such as the dru 1 promoter (US Pat. No. 5,783,393), or the 2A11 promoter (US Pat. No. 4,943,674) and the tomato polygalacturonase promoter (Bird et al. (1988) *Plant Mol. Biol.* 11:651), root-specific promoters, such as those disclosed in US Patent Nos. 5,618,988, 5,837,848 and 5,905,186, pollen-active promoters such as PTA29, PTA26 and PTA13 (US Pat. No. 5,792,929), promoters active in vascular tissue (Ringli and Keller (1998) *Plant Mol. Biol.* 37:977-988), flower-specific (Kaiser et al, (1995) *Plant Mol. Biol.* 28:231-243), pollen (Baerson et al. (1994) *Plant Mol. Biol.* 26:1947-1959), carpels (Ohl et al. (1990) *Plant Cell* 2:837-848), pollen and ovules (Baerson et al. (1993) *Plant Mol. BioL* 22:255-267), auxin-inducible promoters (such as that described in van der Kop et al (1999) *Plant Mol. Biol.* 39:979-990 or Baumann et al. (1999) *Plant Cell* 11:323-334), cytokinin-inducible promoter (Guevara-Garcia (1998) *Plant Mol.* Biol. 38:743-753), promoters responsive to gibberellin (Shi et al. (1998) *Plant Mol. Biol.* 38:1053-1060, Willmott et al. (1998) 38:817-825) and the like. Additional promoters are those that elicit expression in response to heat (Ainley, et al. (1993) *Plant Mol. BioL* 22: 13-23), light (e.g., the pea rbcS-3A promoter, Kuhlemeier et al., (1989) *Plant Cell* 1:471, and the maize rbcS promoter, Schaffner and Sheen, (1991) Plant *Cell* 3: 997); wounding *(e.g., wunl,* Siebertz et al., (1989) *Plant Cell* 1: 961); pathogen resistance, and chemicals such as methyl jasmonate or salicylic acid.(Gatz et al., (1997) *Plant Mol. Biol.* 48: 89-108). In addition, the timing of the expression can be controlled by using promoters such as those acting at late seed development (Odell et al. (1994) *Plant Physiol.* 106:447-458).

Plant expression vectors may also include RNA processing signals that may be positioned within, upstream or downstream of the coding sequence. In addition, the expression vectors may include additional regulatory sequences from the 3'-untranslated region of plant genes, *e.g.,* a 3' terminator region to increase mRNA stability of the mRNA, such as the PI-II terminator region of potato or the octopine or nopaline synthase 3' terminator regions.

Finally, as noted above, plant expression vectors may also include dominant selectable marker genes to allow for the ready selection of transformants. Such genes include those encoding antibiotic resistance genes (e.g., resistance to hygromycin, kanamycin, bleomycin, G418, streptomycin or spectinomycin) and herbicide resistance genes (e.g., phosphinothricin acetyltransferase).

A reduction of TF expression in a transgenic plant to modifiy a plant trait may be obtained by introducing into plants antisense constructs based on the TF cDNA. For antisense suppression, the TF cDNA is arranged in reverse orientation relative to the promoter sequence in the expression vector. The introduced sequence need not be the full length TF cDNA or gene, and need not be identical to the TF cDNA or a gene found in the plant type to be transformed. Generally, however, where the introduced sequence is of shorter length, a higher degree of homology to the native TF sequence will be needed for effective antisense suppression. Preferably, the introduced antisense sequence in the vector will be at least 30 nucleotides in length, and improved antisense suppression will typically be observed as the length of the antisense sequence increases. Preferably, the length of the antisense sequence in the vector will be greater than 100 nucleotides. Transcription of an antisense construct as described results in the production of RNA molecules that are the reverse complement of mRNA molecules transcribed from the endogenous TF gene in the plant cell. Suppression of endogenous TF gene expression can also be achieved using a ribozyme. Ribozymes are synthetic RNA molecules that possess highly specific endoribonuclease activity. The production and use of ribozymes are disclosed in U.S. Patent No. 4,987,071 to Cech and U.S. Patent No. 5,543,508 to Haselhoff. The inclusion of ribozyme sequences within antisense RNAs may be used to confer RNA cleaving activity on the antisense RNA, such that endogenous mRNA molecules that bind to the antisense RNA are cleaved, which in turn leads to an enhanced antisense inhibition of endogenous gene expression.

Vectors in which RNA encoded by the TF cDNA (or variants thereof) is over-expressed may also be used to obtain co-suppression of the endogenous TF gene in the manner described in U.S. Patent No. 5,231,020 to Jorgensen. Such co-suppression (also termed sense suppression) does not require that the entire TF cDNA be introduced into the plant cells, nor does it require that the introduced sequence be exactly identical to the endogenous TF gene. However, as with antisense suppression, the suppressive efficiency will be enhanced as (1) the introduced sequence is lengthened and (2) the sequence similarity between the introduced sequence and the endogenous TF gene is increased.

Vectors expressing an untranslatable form of the TF mRNA may also be used to suppress the expression of endogenous TF activity to modify a trait. Methods for producing such constructs are described in U.S. Patent No. 5,583,021 to Dougherty et al. Preferably, such constructs are made by introducing a premature stop codon into the TF gene. Alternatively, a plant trait may be modified by gene silencing using double-strand RNA (Sharp (1999) *Genes and Development* 13: 139-141). This approach, whereby a vector is prepared in which a cDNA or gene is arranged in duplicated fashion and is capable of generating upon expression a double stranded RNA molecule with a hairpin structure. This procedure has been used to modify gene activity in plants (Chuang and Meyerowitz (1999) *Proc. Natl. Acad.* Sci. 97:4985-9490).

Another method for abolishing the expression of a gene is by insertion mutagenesis using the T-DNA of *Agrobacterium tumefaciens.* After generating the insertion mutants, the mutants can be screened to identify those containing the insertion in a TF gene. Mutants containing a single mutation event at the desired gene may be crossed to generate homozygous plants for the mutation (Koncz et al. (1992) *Methods in Arabidopsis Research.* World Scientific).

A plant trait may also be modified by using the cre-lox system (for example, as described in US Pat. No. 5,658,772). A plant genome may be modified to include first and second lox sites that are then contacted with a Cre recombinase. If the lox sites are in the same orientation, the intervening DNA sequence between the two sites is excised. If the lox sites are in the opposite orientation, the intervening sequence is inverted.

The polynucleotides and polypeptides of this invention may also be expressed in a plant in the absence of an expression cassette by manipulating the activity or expression level of the endogenous gene by other means. For example, by ectopically expressing a gene by T-DNA activation tagging (Ichikawa et al., (1997) *Nature* 390 698-701, Kakimoto et al., (1996) *Science* 274: 982-985). This method entails transforming a plant with a gene tag containing multiple transcriptional enhancers and once the tag has inserted into the genome, expression of a flanking gene coding sequence becomes deregulated. In another example, the transcriptional machinery in a plant may be modified so as to increase transcription levels of a polynucleotide of the invention (See PCT Publications WO9606166 and WO 9853057 which describe the modification of the DNA binding specificity of zinc finger proteins by changing particular amino acids in the DNA binding motif).

The transgenic plant may also comprise the machinery necessary for expressing or altering the activity of a polypeptide encoded by an endogenous gene, for example by altering the phosphorylation state of the polypeptide to maintain it in an activated state.

### 4. Transgenic Plants with Modified TF Expression

Once an expression cassette comprising a polynucleotide encoding a TF gene of this invention has been constructed, standard techniques may be used to introduce the polynucleotide into a plant in order to modify a trait of the plant. The plant may be any higher plant, including gymnosperms, monocotyledonous and dicotyledenous plants. Suitable protocols are available for *Leguminosae* (alfalfa, soybean, clover, etc.), *Umbelliferae* (carrot, celery, parsnip), *Cruciferae* (cabbage, radish, rapeseed, broccoli, etc.), *Curcurbitaceae* (melons and cucumber), *Gramineae* (wheat, corn, rice, barley, millet, etc.), *Solanaceae* (potato, tomato, tobacco, peppers, etc.), and various other crops. See protocols described in Ammirato et al. (1984) *Handbook* of *Plant Cell Culture ―Crop Species.* Macmillan Publ. Co. Shimamoto et al. (1989) *Nature* 338:274-276; Fromm et al. (1990) *Bio*/*Technology 8:833-*839; and Vasil et al. (1990) *Bio*/*Technology* 8:429-434.

Transformation and regeneration of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods may include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and *Agrobacterium tumeficiens* mediated transformation. Transformation means introducing a nucleotide sequence in a plant in a manner to cause stable or transient expression of the sequence.

Successful examples of the modification of plant characteristics by transformation with cloned sequences which serve to illustrate the current knowledge in this field of technology, and which are herein incorporated by reference, include: U.S. Patent Nos. 5,571,706; 5,677,175; 5,510,471; 5,750,386; 5,597,945; 5,589,615; 5,750,871; 5,268,526; 5,780,708; 5,538,880; 5,773,269; 5,736,369 and 5,610,042.

Following transformation, plants are preferably selected using a dominant selectable marker incorporated into the transformation vector. Typically, such a marker will confer antibiotic or herbicide resistance on the transformed plants, and selection of transformants can be accomplished by exposing the plants to appropriate concentrations of the antibiotic or herbicide.

After transformed plants are selected and grown to maturity, those plants showing a modified trait are identified. The modifed trait may be any of those traits described above. Additionally, to confirm that the modified trait is due to changes in expression levels or activity of the polypeptide or polynucleotide of the invention may be determined by analyzing mRNA expression using Northern blots, RT-PCR or microarrays, or protein expression using immunoblots or Western blots or gel shift assays.

### 5. Commercial Applications of the Polynucleotides and Polypeptides

Specific applications for the genes of the present invention relate to their potential roles in plant flowering time or the vernalization response. Most modern crop varieties are the result of extensive breeding programs and many generations of backcrossing may be required to introduce desired traits. Systems that accelerate flowering could have valuable applications in such programs since they allow much faster generation times. Additionally, in some instances, a faster generation time might allow additional harvests of a crop to be made within a given growing season. With the advent of transformation systems for tree species such as oil palm, aspen, pine and eucalyptus, forest biotechnology is a growing area of interest.

Also, in species such as sugarbeet where the vegetative parts of the plants constitute the crop and the reproductive tissues are discarded, it would be advantageous to delay or prevent flowering. Extending vegetative development could bring about large increases in yields.

Furthermore, by regulating the expression of flowering-time controlling genes, using inducible promoters, flowering could potentially be triggered as desired (for example, by application of a chemical inducer). This would allow, for example, flowering to be synchronized across a crop and facilitate more efficient harvesting. Such inducible systems could be used to tune the flowering of crop varieties to different latitudes. At present, species such as soybean and cotton are available as a series of maturity groups that are suitable for different latitudes on the basis of their flowering time (which is governed by day-length). A system in which flowering could be chemically controlled would allow a single high-yielding northern maturity group to be grown at any latitude. In southern regions such plants could be grown for longer, thereby increasing yields, before flowering was induced. In more northern areas, the induction would be used to ensure that the crop flowers prior to the first winter frosts. Currently, the existence of a series of maturity groups for different latitudes represents a major barrier to the introduction of new valuable traits.

For many crop species, high yielding winter-varieties can only be grown in temperate regions where the winter season is prolonged and cold enough to elicit a vernalization response. Altered expression of the genes of the invention could compensate for a vernalization treatment in late-flowering *Arabidopsis* ecotypes. Similar effects might be achieved in crop plants. Winter varieties of wheat, for instance, which over-express G157 (or the wheat ortholog) might then be grown in areas like Southern California which would otherwise be too warm to allow effective vernalization. A second application for this system is in cherry *(Prunus).* Locally grown cherries are unavailable in the early Californian spring since the winters are too warm for vernalization to occur.

A further application exists in strawberry *(Fragaria).* Strawberry has a well-defined perennial cycle of flower initiation, dormancy, chilling, crop growth and runner production. In temperate European countries, the plants flower in early spring, and fruit is produced in May or June. Following fruiting, runners are generated that carry plantlets which take root. The plants then remain dormant all through the late summer and autumn. Flowering cannot be repeated until the following spring after the plants have received a winter cold treatment. A system, which bypasses this vernalization requirement, might permit a second autumn crop of strawberries to be harvested in addition to the spring crop.

Finally, in addition to the direct applications of the genes themselves, their regulatory regions could also be of value. If the promoters of these genes are responsive to low temperatures they could be incorporated into expression systems for regulation of genes that confer tolerance to freezing. Such genes would then be up regulated specifically at the time required, thereby minimizing any toxic effects that result from their constitutive expression.

### 6. Other Utility of the Polypeptide and Polynucleotides

A transcription factor coding provided by the present invention may also be used to identify exogenous or endogenous molecules that may affect expression of the transcription factors and may affect flowering time. These molecules may include organic or inorganic compounds.

For example, the method may entail first placing the molecule in contact with a plant or plant cell. The molecule may be introduced by topical administration, such as spraying or soaking of a plant, and then the molecule's effect on the expression or activity of the TF polypeptide or the expression of the polynucleotide monitored. Changes in the expression of the TF polypeptide may be monitored by use of polyclonal or monoclonal antibodies, gel electrophoresis or the like. Changes in the expression of the corresponding polynucleotide sequence may be detected by use of microarrays, Northerns or any other technique for monitoring changes in mRNA expression. These techniques are exemplified in Ausubel et al. (eds) *Current Protocols in Molecular Biology,* John Wiley & Sons (1998). Such changes in the expression levels may be correlated with modified plant traits and thus identified molecules may be useful for soaking or spraying on fruit, vegetable and grain crops to modify traits in plants.

The transcription factors may also be employed to identify promoter sequences with which they may interact. After identifying a promoter sequence, interactions between the transcription factor and the promoter sequence may be modified by changing specific nucleotides in the promoter sequence or specific amino acids in the transcription factor that interact with the promoter sequence to alter a plant trait. Typically, transcription factor DNA binding sites are identified by gel shift assays. After identifying the promoter regions, the promoter region sequences may be employed in double-stranded DNA arrays to identify molecules that affect the interactions of the TFs with their promoters (Bulyk et al. (1999) *Nature Biotechnology* 17:573-577).

The identified transcription factors are also useful to identify proteins that modify the activity of the transcription factor. Such modification may occur by covalent modification, such as by phosphorylation, or by protein-protein (homo or-heteropolymer) interactions. Any method suitable for detecting protein-protein interactions may be employed. Among the methods that may be employed are co-immunoprecipitation, cross-linking and co-purification through gradients or chromatographic columns, and the two-hybrid yeast system.

The two-hybrid system detects protein interactions in vivo and is described in Chien, et al., (1991), *Proc. Natl. Acad. Sci. USA,* 88, 9578-9582 and is commercially available from Clontech (Palo Alto, Calif.). In such a system, plasmids are constructed that encode two hybrid proteins: one consists of the DNA-binding domain of a transcription activator protein fused to the TF polypeptide and the other consists of the transcription activator protein's activation domain fused to an unknown protein that is encoded by a cDNA that has been recombined into the plasmid as part of a cDNA library. The DNA-binding domain fusion plasmid and the cDNA library are transformed into a strain of the yeast *Saccharomyces cerevisiae* that contains a reporter gene (e.g., IacZ) whose regulatory region contains the transcription activator's binding site. Either hybrid protein alone cannot activate transcription of the reporter gene. Interaction of the two hybrid proteins reconstitutes the functional activator protein and results in expression of the reporter gene, which is detected by an assay for the reporter gene product. Then, the library plasmids responsible for reporter gene expression are isolated and sequenced to identify the proteins encoded by the library, plasmids. After identifying proteins that interact with the transcription factors, assays for compounds that interfere with the TF protein-protein interactions may be preformed.

The following examples are intended to illustrate but not limit the present invention.

### EXAMPLES

### Methods

All experiments were performed using *Arabidopsis* of ecotype Columbia except where otherwise indicated. The Stockholm (CS6863) and Pitztal (CS6832) lines were supplied by the ABRC at Ohio State University. In all experiments, seeds were sterilized by a 2 minute ethanol treatment followed by 30 minutes in 30% bleach / 0.01% Tween and five washes in distilled water. Seeds were sown to MS agar in 0.1% agarose and stratified for 3-5 days at 4 °C, before transfer to growth rooms with a temperature of 20-25 °C. MS media was supplemented with 50mg/l kanamycin for selection of transformed plants. Plants were transplanted to soil after 7 days of growth on plates. For vernalization treatments, seeds were sown to MS agar plates, sealed with micropore tape, and placed in a 4°C cold room with low light levels for 6-8 weeks. The plates were then transferred to the growth rooms alongside plates containing freshly sown non-vernalized controls. Whole vegetative seedlings were harvested for gene expression analysis at 6 to 9 days after transfer. Rosette leaves were counted when a visible inflorescence of approximately 3 cm was apparent. Rosette and total leaf number on the progeny stem are tightly correlated with the timing of flowering (Koornneef et al (1991) *Mol. Gen. Genet* 229:57-66.

### Example I. Full Length Gene Identification and Cloning

For the following examples, G157 refers to SEQ ID Nos 1 and 2, G859 refers to SEQ ID Nos. 3-8, G1842 refers to SEQ ID Nos. 9-16, G1843 refers to SEQ ID Nos. 17 and 18, G1844 refers to SEQ ID Nos. 19-22, G861 refers to SEQ ID Nos. 23-26 and FLC or G1759 refers to SEQ ID Nos. 27, 28.

Putative transcription factor sequences (genomic or ESTs) related to known transcription factors were identified in the *Arabidopsis thaliana* GenBank database using the tblastn sequence analysis program using default parameters and a P-value cutoff threshold of -4 or-5 or lower, depending on the length of the query sequence. Putative transcription factor sequence hits were then screened to identify those containing particular sequence strings. If the sequence hits contained such sequence strings, the sequences were confirmed as transcription factors.

For example, we identified a MADS box gene G157 within BAC F22K20 (GenBank accession AC002291) from Chromosome 1 that was predicted to encode a protein related to FLC. An 872bp cDNA clone for G157 was identified among clones isolated from a library derived from leaf mRNA. The encoded protein was 196 amino acids in length, and shared 62% overall amino acid sequence identity with FLC, and 82% identity within the MADS DNA binding domain.

G157 is also related to G859, G1842, G1843, and G1844 that map together as a tightly linked cluster, at the bottom of chromosome V, that occupies approximately 22 kb and spans three adjacent clones, MXK3, F1505, and MQN23 (GenBank accession numbers AB019236, AB026633, and AB013395, respectively). G859, G1842, G1843, and G1844 are all arranged in the same orientation. G859, G1842, G1843, and G1844 were likely created by a duplication event; this could have allowed their divergence into different aspects of gene regulation. Their physical proximity suggests that they may act as a unit controlled via common regulatory elements.

The pair-wise comparisons of the 57 amino acid MADS domains of FLC, G157, G859, G1842, G1843, and G1844 are displayed in Table 1. The table shows percent amino acid sequence identity and, in parentheses, the sequence identity percentages when conservative amino acid substitutions are considered. The MADS domains of the proteins encoded by G859, G1842, G1843, and G1844 are highly conserved with those of FLC and G157: these proteins share from 75% to 91 % of amino acid sequence identity, depending on the pair-wise comparison as shown below. When conservative amino substitutions are made, the MADS domains of these proteins are 88%-99% identical to each other (shown in parentheses).

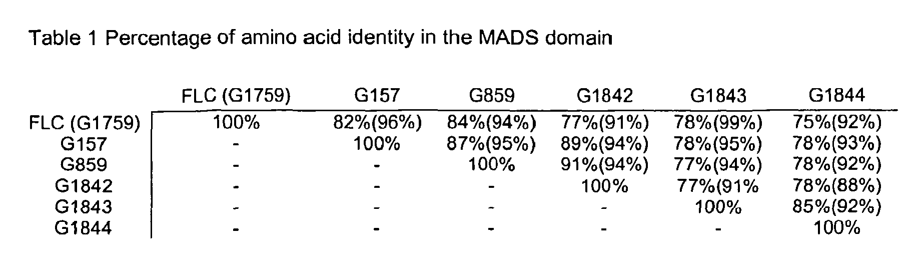

Amino acid residue 30 of FLC and by G157, G859, G1842, G1843, and G1844 is an acidic residue (E or D) whereas, in all other *Arabidopsis* MADS domain proteins so far identified, that position is occupied by a positively charged lysine residue. The crystal structure of the human SRF MADS domain bound to DNA has shown that lysine residue (which is also conserved in yeast MCM1 and human MEF2A proteins) to contact the phosphate backbone of the DNA target site (Pellegrini *et al.,* (1995) Nature 376:490-498). That amino acid difference could therefore confer DNA binding properties to FLC and by G157, G859, G1842, G1843, and G1844 distinct from other *Arabidopsis* MADS domain proteins. Therefore, MADS domain proteins with an acidic residue at position 30 may be particularly useful in modifying plant flowering time and vernalization response.

The transcripts from these genes were analyzed by 3' RACE (Rapid Amplification cDNA Ends) and corresponding cDNAs were isolated by RT-PCR from mixed samples of *Arabidopsis* tissue (Columbia ecotype). During this analysis, it was found that G859, G1842 and G1844 transcripts exist in multiple alternatively spliced forms.

### Example II. Flowering Time Associated Genes

Reverse transcriptase PCR was done using gene specific primers within the coding region for each sequence identified. Where possible, the primers were designed near the 3' region of each coding sequence initially identified.

Total RNA was isolated from plant tissue tissue and extracted using CTAB. Once extracted total RNA was normalized in concentration across all the tissue types to ensure that the PCR reaction for each tissue received the same amount of cDNA template using the 28S band as reference. Poly A+ was purified using a modified protocol from the Qiagen Oligotex kit batch protocol. cDNA was synthesized using standard protocols. After the first strand cDNA synthesis, primers for Actin 2 were used to normalize the concentration of cDNA across the tissue types. Actin 2 is found to be constitutively expressed in fairly equal levels across the Arabidopsis tissue types.

For RT PCR, cDNA template was mixed with corresponding primers and Taq polymerase. Each reaction consisted of 0.2 ul cDNA template, 2ul 10X Tricine buffer, and 16.8 ul water, 5pmol Primer 1, 5pmol Primer 2, 0.3 ul Taq polymerase, 200uM dNTPs and 8.6 ul water.

The 96 well plate was covered with microfilm and set in the Thermocycler to start the following reaction cycle. Step1 93° C for 3 mins, Step 2 93° C for 30 sec, Step 3 60-65° C for 1 min, Step 4 72° C for 2 mins,. Steps 2, 3 and 4 were repeated for 20-35 cycles. Step 5 72° C for 5 mins and Step 6 4° C. The PCR plate was sometimes placed back in the thermocycler to amplify more products for 5-15 more cycles to identify genes that have very low expression. The reaction cycle was as follows: Step 2 93° C for 30 sec, Step 3 65° C for 1 min, and Step 4 72° C for 2 ins, repeated for 8 cycles, and Step 4 4° C.

Eight microliters of PCR product and 1.5 ul of loading dye were loaded on a 1.2% agarose gel for analysis between 21 and 36 cycles. Expression levels of specific transcripts were considered low if they were only detectable after 35 cycles of PCR. Expression levels were considered medium or high depending on the levels of transcript compared with observed transcript levels for actin2.

As an example, to assess G157 mRNA levels in G157 plants, PCR was carried out over 25 cycles using primers 5'-GGCATAACCCTTATCGGAGATTTGAAGC-3' (SEQ ID No. 57) and 5'-ACACAAACTCTGATCTTGTCTCCGAAGG-3' (SEQ ID No. 58). To assess mRNA levels in different tissues extracted from wild type plants, 25 or 30 cycles of PCR were performed using primers 5'-GCATAACCCTTATCGGAGATTTGAAGCCAT-3' (SEQ ID No. 59) and 5'-AACATTCCTCTCTCATCATCTGTTGCCAGC-3' (SEQ ID No. 60). PCR for FLC was performed either with primers 5'-AACGCTTAGTATCTCCGGCGACTTGAAC-3' (SEQ ID No. 51) and 5'-CTCACACGAATAAGGTACAAAGTTCATC-3' (SEQ ID No. 62) over 35 cycles, or 5'-TTAGTATCTCCGGCGACTTGAACCCAAACC-3' (SEQ ID No. 63) and 5'-AGATTCTCAACAAGCTTCAACATGAGTTCG-3' (SEQ ID No. 64) over 30 cycles. Primer specificity was verified by sequencing RT-PCR products. Samples were standardized via 20-25 cycles of PCR with actin primers.

### Example III. Construction of Expression Vectors

The sequence was amplified from a genomic or cDNA library using primers specific to sequences upstream and downstream of the coding region. The expression vector was pMEN20 or pMEN65, which are both derived from pMON316 (Sanders et al, (1987) *Nucleic Acids Research* 15:1543-58) and contain the CaMV 35S promoter to express transgenes. To clone the sequence into the vector, both pMEN20 and the amplified DNA fragment were digested separately with Sall and Notl restriction enzymes at 37° C for 2 hours. The digestion products were subject to electrophoresis in a 0.8% agarose gel and visualized by ethidium bromide staining. The DNA fragments containing the sequence and the linearized plasmid were excised and purified by using a Qiaquick gel extraction kit (Qiagen, CA). The fragments of interest were ligated at a ratio of 3:1 (vector to insert). Ligation reactions using T4 DNA ligase (New England Biolabs, MA) were carried out at 16° C for 16 hours. The ligated DNAs were transformed into competent cells of the *E. coli strain* DH5alpha by using the heat shock method. The transformations were plated on LB plates containing 50 mg/l spectinomycin (Sigma).

Individual colonies were grown overnight in five milliliters of LB broth containing 50 mg/l spectinomycin at 37° C. Plasmid DNA was purified by using Qiaquick Mini Prep kits (Qiagen, CA).

### Example IV. Transformation of Agrobacterium with the Expression Vector

After the plasmid vector containing the gene was constructed, the vector was used to transform *Agrobacterium tumefaciens* cells expressing the gene products. The stock of *Agrobacterium tumefaciens* cells for transformation were made as described by Nagel et al. *FEMS Microbiol Letts* 67: 325-328 (1990). *Agrobacterium* strain GV3101 was grown in 250 ml LB medium (Sigma) overnight at 28°C with shaking until an absorbance (A₆₀₀) of 0.5 ― 1.0 was reached. Cells were harvested by centrifugation at 4,000 x g for 15 min at 4° C. Cells were then resuspended in 250 µl chilled buffer (1 mM HEPES, pH adjusted to 7.0 with KOH). Cells were centrifuged again as described above and resuspended in 125 µl chilled buffer. Cells were then centrifuged and resuspended two more times in the same HEPES buffer as described above at a volume of 100 µl and 750 µl, respectively. Resuspended cells were then distributed into 40 µl aliquots, quickly frozen in liquid nitrogen, and stored at -80° C.

*Agrobacterium* cells were transformed with plasmids prepared as described above following the protocol described by Nagel et al. *FEMS Microbiol Letts* 67: 325-328 (1990). For each DNA construct to be transformed, 50 ― 100 ng DNA (generally resuspended in 10 mM Tris-HCI, 1 mM EDTA, pH 8.0) was mixed with 40 µl of *Agrobacterium* cells. The DNA/cell mixture was then transferred to a chilled cuvette with a 2mm electrode gap and subject to a 2.5 kV charge dissipated at 25 µF and 200 µF using a Gene Pulser II apparatus (Bio-Rad). After electroporation, cells were immediately resuspended in 1.0 ml LB and allowed to recover without antibiotic selection for 2 - 4 hours at 28° C in a shaking incubator. After recovery, cells were plated onto selective medium of LB broth containing 100 µg/ml spectinomycin (Sigma) and incubated for 24-48 hours at 28° C. Single colonies were then picked and inoculated in fresh medium. The integrity of the plasmid construct was verified by PCR amplification and sequence analysis.

### Example V. Transformation of Arabidopsis Plants with Agrobacterium tumefaciens with Expression Vector

After transformation of *Agrobacterium tumefaciens* with plasmid vectors containing the gene, single *Agrobacterium* colonies were identified, propagated, and used to transform *Arabidopsis* plants. Briefly, 500 ml cultures of LB medium containing 50 mg/l spectinomycin were inoculated with the colonies and grown at 28° C with shaking for 2 days until an absorbance (A₆₀₀) of > 2.0 is reached. Cells were then harvested by centrifugation at 4,000 x g for 10 min, and resuspended in infiltration medium (1/2 X Murashige and Skoog salts (Sigma), 1 X Gamborg's B-5 vitamins (Sigma), 5.0% (w/v) sucrose (Sigma), 0.044 µM benzylamino purine (Sigma), 200 µl/L Silwet L-77 (Lehle Seeds) until an absorbance (A₆ₒₒ) of 0.8 was reached.

Prior to transformation, *Arabidopsis thaliana* seeds (ecotype Columbia) were sown at a density of ~10 plants per 4" pot onto Pro-Mix BX potting medium (Hummert International) covered with fiberglass mesh (18 mm X 16 mm). Plants were grown under continuous illumination (50-75 µE/m²/sec) at 22-23° C with 65-70% relative humidity. After about 4 weeks, primary inflorescence stems (bolts) are cut off to encourage growth of multiple secondary bolts. After flowering of the mature secondary bolts, plants were prepared for transformation by removal of all siliques and opened flowers.

The pots were then immersed upside down in the mixture of *Agrobacterium* infiltration medium as described above for 30 sec, and placed on their sides to allow draining into a 1' x 2' flat surface covered with plastic wrap. After 24 h, the plastic wrap was removed and pots are turned upright. The immersion procedure was repeated one week later, for a total of two immersions per pot. Seeds were then collected from each transformation pot and analyzed following the protocol described below.

### Example VI. Identification of Arabidopsis Primary Transformants

Seeds collected from the transformation pots were sterilized essentially as follows. Seeds were dispersed into in a solution containing 0.1% (v/v) Triton X-100 (Sigma) and sterile H₂O and washed by shaking the suspension for 20 min. The wash solution was then drained and replaced with fresh wash solution to wash the seeds for 20 min with shaking. After removal of the second wash solution, a solution containing 0.1% (v/v) Triton X-100 and 70% ethanol (Equistar) was added to the seeds and the suspension was shaken for 5 min. After removal of the ethanol/detergent solution, a solution containing 0.1 % (v/v) Triton X-100 and 30% (v/v) bleach (Clorox) was added to the seeds, and the suspension was shaken for 10 min. After removal of the bleach/detergent solution, seeds were then washed five times in sterile distilled H₂O. The seeds were stored in the last wash water at 4° C for 2 days in the dark before being plated onto antibiotic selection medium (1 X Murashige and Skoog salts (pH adjusted to 5.7 with 1 M KOH), 1 X Gamborg's B-5 vitamins, 0.9% phytagar (Life , Technologies), and 50 mg/l kanamycin). Seeds were germinated under continuous illumination (50-75 µE/m²/sec) at 22-23° C. After 7-10 days of growth under these conditions, kanamycin resistant primary transformants (T₁ generation) were visible and obtained. These seedlings were transferred first to fresh selection plates where the seedlings continued to grow for 3-5 more days, and then to soil (Pro-Mix BX potting medium).
Primary transformants are self-crossed and progeny seeds (T2) collected. T2 progeny seeds were germinated on kanamycin as described above and kanamycin resistant seedlings were selected, transferred to soil and analyzed.

### Example VII. Analysis of transgenic Arabidopsis plants

In a first experiment, G157 plants (ie plants expressing the G157 transgene) were grown in 12 hours light. 31 of 40 lines flowered earlier than control plants transformed with a control vector. Mean rosette leaf number of early T1 lines was 12.4+/-0.8 whereas control lines had 27+/-1.2 rosette leaves. 2 of 40 T1 plants flowered at the same time as controls and 7 of 40 lines were late flowering and produced visible inflorescences 2 to 3 weeks after wild type.

In further experiments, plants were grown under conditions of 24 hours light at 20-25 °C. Under these conditions, the non-transformed control plants produced a mean total of 14.3+/-0.7 leaves on the primary shoots prior to flower bud initiation. Flower buds were first visible on these plants at a mean of 21.1+/-0.5 days after sowing (error values represent standard error of the mean to which 95% confidence limits have been attached). For G859, 14/19 T1 plants were early flowering (mean leaf total of 6.4+/-0.7, flower buds visible at 12.9+/-0.7days after sowing), 3/19 were wild type, and 2/19 were slightly late flowering compared to wild type (mean total of 19 leaves, flower buds visible at 27 days). RT expression studies revealed that the late flowering individuals possessed the highest levels of transgene expression. These results strongly parallel those obtained for G157. For G1842, 7/10 T1 flowered early (mean total of 7.9+/-0.6 leaves, flower buds visible at 13.9+/-1.0 days), and 3/10 plants were wild type. Overexpression studies were also performed with cDNAs encoding shortened splice variants of G1842. For G1842.2 (encodes a 185 amino acid splice variant), 15/18 T1 plants flowered early (mean total of 6.9+/-0.9 leaves, flower buds visible at 14.5+/-0.6 days) and 3/18 were wild type. For G1842.6 (encodes a 77 amino acid splice variant), 8/10 T1 plants flowered early mean total of 6.8+/-1.6 leaves, flower buds visible at 13.9+/-0.9 days) and 2/10 were wild type. For G1842.7 (encodes a 118 amino acid splice variant) 8/10 T1 plants flowered early (accurate leaf counts not made) and 2/10 were wild type. Thus, the G1842 splice variants produced comparable effects to the full-length cDNA clone when over-expressed. For G1843, 7/11 flowered early (mean total of 6.4+/-0.5 leaves, flower buds visible at 16.0+/-1.6 days) and 2/11 had a wild type flowering time. The G1843 T1 plants, however, were dwarfed and showed retarded development of some organs. This suggests that G1843 has unpredicted toxic effects when over-expressed. For G1844, 6/10 T1 plants flowered early (mean total of 6.8+/-1.7 leaves, flower buds visible at 14.7+/-1.3 days) and 4/10 plants were wild type. Overexpression studies were also performed with a cDNA encoding a shortened splice variant of G1844. For overexpression of G1844.2 (encodes a 184 amino acid splice variant), 6/19 T1 plants flowered early (mean total of 7.8+/-1.7 leaves, flower buds visible at 15.7+/-1.3 days) and 13/19 were wild type). The over-expression data for G859, G1842, G1843, and G1844 support the hypothesis that they have a role in the control of flowering time.

RT-PCR was performed on materials from G157 plants using G157 specific primers at approximately 25 cycles. The highest levels of G157 expression were detected in late flowering individual plants or in samples from pooled seedlings that contained late flowering individuals. Plants that showed only moderate or low levels of overexpression compared to wild type were slightly early flowering or normal.

To test whether an increase in G157 could affect flowering time in late flowering ecotypes of Arabidopsis, we overexpressed G157 in the late flowering ecotypes Stockholm and Piztal. In this experiment, 32 primary transformants from each ecotype were grown interspersed with controls under continuous light conditions. In both ecotypes, around 50% of the transformants flowered earlier than controls, and in some transformants the time to flowering was halved. As was observed with Columbia G157 plants, a minority of Pitztal and Stockholm transformants were clearly later flowering compared to controls.

A correlation between G157 transgene expression and flowering time was also observed in G157 Stockholm and Pitztal T1 plants. RT-PCR was performed with two early and two late flowering lines in each background. Again, the late flowering lines contained the higher levels of G157 expression. Thus, the factor appears to affect flowering time in a quantitative manner; a modest level of overexpression triggers early flowering, whereas a larger increase delays flowering.

In conclusion, over-expression of G157 or any of the related genes modifies flowering time in plants: a modest level of over-expression triggers early flowering, whereas a larger increase delays flowering.

Using similar or identical methodologies described in the examples above, further Arabidopsis genes were identified whose altered expression was correlated with delayed or accelerated flowering. These genes are tabulated in Table 2 with their Sequence Listing Nos., and their effects on flowering time.

**Table 2. Further Arabidopsis genes for manipulating flowering time**

| SEQ ID Nos. | Gene | observations |
|---|---|---|
| 23, 24 | G861 | early or late flowering |
| 25, 26 | G861.1 | early or late flowering |
| 29, 30 | G192 | late flowering |
| 31 , 32 | G234 | late flowering |
| 33, 34 | G361 | late flowering |
| 35, 36 | G486 | late flowering |
| 37, 38 | G748 | late flowering |
| 39, 40 | G994 | late flowering |
| 41, 42 | G1335 | late flowering |
| 43, 44 | G562 | late flowering |
| 45, 46 | G736 | late flowering |
| 47,48 | G1073 | late flowering |
| 49,50 | G1435 | late flowering |
| 51,52 | G180 | early flowering |
| 53, 54 | G592 | early flowering |
| 55, 56 | G208 | early lowering |

The vernalization response was also investigated. Late flowering vernalization responsive ecotypes and mutants have high steady state levels of FLC transcript, which decrease during the promotion of flowering by vernalization (Michaels and Amasino, (1999) *Plant Cell* 11:949-956; Sheldon et al., (1999) *Plant Cell* 11:445-458; Sheldon et al., (2000) *Proc. Natl. Acad. Sci.* 97: 3735-3758). In contrast to FLC, G157 transcript levels show no consistent correlation with the vernalization response in the late flowering Stockholm and Pitztal ecotypes. Additionally we found that over-expression of G157 did not influence FLC levels. The effects of vernalization on expression of G861, G859, G1842, G1843, and G1844 were also examined. Germinating seeds of Columbia, Pitztal, Stockholm, *constans-1,* and fca-9 were vernalized on MS agar plates in a 4°C cold room for 8 weeks, and then transferred to a continuous light growth room. Total tissues from the vernalized seedlings, and freshly sown non-vernalized controls were harvested at 9 days after the transfer. RT-PCR was performed for FLC, G157, G859, G1842, G1843, G1844, and G861, and actin. Compared to FLC and G157, none of the genes showed a clear consistent decline upon vernalization in the five different sample sets. However, G1844 displayed a converse pattern of expression to FLC: G1844 levels consistently increased on vernalization. This is particularly significant as it directly implicates G1844 in control of the vernalization response. Thus G1844 likely activates flowering and has an opposing role to FLC.

To explore whether overexpression of G157 produces comparable effects on vernalization, batches of wild type Pitztal and Stockholm seedlings were cold treated for 6 weeks at 4°C, then grown amongst a second selection of G157 T1 Pitztal, G157 T1 Stockholm and non-vernalized wild type plants. As expected, vernalization markedly and uniformly reduced flowering time in both Pitztal and Stockholm wild type plants. Amongst the G157 Stockholm lines, the earliest flowering T1 group (8/23 lines) was indistinguishable from vernalized plants. For Pitztal, however, the early flowering T1 plants were on average marginally later than the vernalized plants. Therefore, overexpression of G157 can substantially reduce the requirement for vernalization in late flowering ecotypes.

Additionally, we observed that the late flowering of G157 lines is independent of FLC expression and does not respond to vernalization. However, the late flowering G157 plants are responsive to photoperiod. In an experiment conducted under short day conditions of 8 hours of light, we obtained a number of G157 Columbia T1 plants that flowered up to a month later than wild type controls (data not shown). To confirm that the late flowering effects caused by G157 overexpression were independent of *FLC* transcription, we tested whether late flowering G157 Columbia plants were responsive to vernalization. No significant change in flowering time was noted: in continuous light conditions, vernalized T2 plants of line 4 had a total of 31.3 +/- 1.8 leaves compared to 30.1 +/- 1.3 when non-vernalized. Control *fca* plants verified that the treatment was effective: vernalized plants flowered after only 10.3 +/- 0.9 leaves compared to more than 40 leaves for the non-vernalized controls. Thus, the late flowering phenotype caused by G157 could not be overcome by vernalization, as would be expected if the delay occurred independently of changes in FLC expression

### Example IX. Identification of Homologous Sequences

Homologs from plant species other than Arabidopsis were identified using database sequence search tools, such as the Basic Local Alignment Search Tool (BLAST) (Altschul et al. (1990) J. *Mol. Biol.* 215:403-410; and Altschul et al. (1997) *Nucl. Acid* Res. 25: 3389-3402). The tblastx sequence analysis programs were employed using the BLOSUM-62 scoring matrix (Henikoff, S. and Henikoff, J. G. (1992) *Proc. Natl. Acad. Sci.* USA 89: 10915-10919).

The entire NCBI Genbank database was filtered for sequences from all plants except Arabidopsis thaliana by selecting all entries in the NCBI Genbank database associated with NCBI taxonomic ID 33090 (Viridiplantae; all plants) and excluding entries associated with taxonomic ID 3701 (Arabidopsis thaliana). These sequences were compared to sequences representing genes of SEQ IDs 1-56 on 9/26/2000 using the Washington University TBLASTX algorithm (version 2.Oa19MP). For each gene of SEQ IDs 1-56, individual comparisons were ordered by probability score (P-value), where the score reflects the probability that a particular alignment occurred by chance. For example, a score of 3.6e-40 is 3.6 x 10⁻⁴⁰. For up to ten species, the gene with the lowest P-value (and therefore the most likely homolog) is listed in Figure 2.

In addition to P-values, comparisons were also scored by percentage identity. Percentage identity reflects the degree to which two segments of DNA or protein are identical over a particular length. The ranges of percent identity between the non-Arabidopsis genes shown in Figure 2 and the Arabidopsis genes in the sequence listing are: SEQ ID No. 1: 54%-67%; SEQ ID Nos. 3,5,7: 37%-47%; SEQ ID Nos. 9,11,13,15: 54%-62%; SEQ ID No. 17: 62%-71%; SEQ ID Nos. 19, 21: 50%-67%; SEQ ID Nos. 23,25: 75%-91%; SEQ ID No. 27: 46%-69%; SEQ ID No. 29: 44%-90%; SEQ ID No. 31: 57-89%; SEQ ID No. 33: 37%-79%; SEQ ID No. 35: 50%-71 %; SEQ ID No. 37: 39%-63%; SEQ ID No. 39: 58%-70%; SEQ ID No. 41: 45%-73%; SEQ ID No. 43: 42%-84%; SEQ ID No. 45: 47%-81%; SEQ ID No. 47: 31%-71%; SEQ ID No. 49: 40%-67%; SEQ ID No. 51: 69%-51%; SEQ ID No. 53: 43%-86%; and SEQ ID No. 55: 79%-89%.

Arabidopsis homologs of genes in Table 2 were also identifed using BLAST. These genes are found in the following Arabidopsis BAC sequences, identified by their Genbank sequence NID numbers: 2827698 (G234 homolog), 3241917 (G748 homolog), 2618604 (G994 homolog), 6598548 (G1335 homolog), 7340331 (G736 homolog), 6523051 (G1435 homolog), 6598491 (G208 homolog) and 3172156 (G208 homolog).

All references (publications and patents) are incorporated herein by reference in their entirety for all purposes.

Although the invention has been described with reference to the embodiments and examples above, it should be understood that various modifications can be made without departing from the spirit of the invention. Accordingly, the invention is limited only by the following claims.

**Figure 1**

| SEQ ID No. | Gene | cDNA or | conserved doman |
|---|---|---|---|
| 1 | G157 | cDNA | |
| 2 | G157 | protein | 2-57 |
| 3 | G859 | cDNA | |
| 4 | G859 | protein | 2-57 |
| 5 | G859.1 | cDNA | |
| 6 | G859.1 | protein | 2-57 |
| 7 | G859.2 | cDNA | |
| 8 | G859.2 | protein | 2-57 |
| 9 | G1842 | cDNA | |
| 10 | G1842 | protein | 2-57 |
| 11 | G1842.2 | cDNA | |
| 12 | G1842.2 | protein | 2-57 |
| 13 | G1842.6 | cDNA | |
| 14 | G1842.6 | protein | 2-57 |
| 15 | G1842.7 | cDNA | |
| 16 | G1842.7 | protein | 2-57 |
| 17 | G1843 | cDNA | |
| 18 | G1843 | protein | 2-57 |
| 19 | G1844 | cDNA | |
| 20 | G1844 | protein | 2-57 |
| 21 | G1844.2 | cDNA | |
| 22 | G1844.2 | protein | 2-57 |
| 23 | G861 | cDNA | |
| 24 | G861 | protein | 2-57 |
| 25 | G861.1 | cDNA | |
| 26 | G861.1 | protein | 2-57 |
| 27 | G1759 | cDNA | |
| 28 | G1759 | protein | 2-57 |
| 29 | G192 | cDNA | |
| 30 | G192 | protein | 128-185 |
| 31 | G234 | cDNA | |
| 32 | G234 | protein | 14-115 |
| 33 | G361 | cDNA | |
| 34 | G361 | protein | 43-63 |
| 35 | G486 | cDNA | |
| 36 | G486 | protein | 5-66 |
| 37 | G748 | cDNA | |
| 38 | G748 | protein | 112-140 |
| 39 | G994 | cDNA | |
| 40 | G994 | protein | 14-123 |
| 41 | G1335 | cDNA | |
| 42 | G1335 | protein | 24-43, 131-144, 185-203 |
| 43 | G562 | cDNA | |
| 44 | G562 | protein | 253-315 |
| 45 | G736 | cDNA | |
| 46 | G736 | protein | 54-111 |
| 47 | G1073 | cDNA | |
| 48 | G1073 | protein | 33-42, 78-175 |
| 49 | G1435 | cDNA | |
| 50 | G1435 | protein | 146-194 |
| 51 | G180 | cDNA | |
| 52 | G180 | protein | 118-174 |
| 53 | G592 | cDNA | |
| 54 | G592 | protein | 290-342 |
| 55 | G208 | cDNA | |
| 56 | G208 | protein | 14-116 |

**Figure 2A**

| SEQ IDs | Gene Ids | Genbank NID | P-value | Species |
|---|---|---|---|---|
| 1 | G157 | 6530836 | 3.10E-22 | Lycopersicon esculentum |
| 1 | G157 | 5606765 | 5.50E-14 | Glycine max |
| 1 | G157 | 6826955 | 1.20E-13 | Zea mays |
| 1 | G157 | 6536942 | 6.00E-13 | Medicago truncatula |
| 1 | G157 | 8707754 | 1.40E-12 | Hordeum vulgare |
| 1 | G157 | 2293891 | 1.40E-12 | Petunia x hybrida |
| 1 | G157 | 19870 | 1.40E-12 | Nicotiana tabacum |
| 1 | G157 | 7628118 | 3.70E-12 | Gossypium arboreum |
| 1 | G157 | 5050220 | 3.80E-12 | Gossypium hirsutum |
| 1 | G157 | 9414215 | 4.50E-12 | Triticum aestivum |
| 3,5,7 | G859 | 6530836 | 1.40E-34 | Lycopersicon esculentum |
| 3,5,7 | G859 | 5777903 | 4.70E-30 | Malus domestica |
| 3,5,7 | G859 | 9367312 | 7.10E-30 | Hordeum vulgare |
| 3,5,7 | G859 | 6467973 | 3.60E-29 | Dendrobium grex Madame Thong-IN |
| 3,5,7 | G859 | 4204233 | 1.20E-28 | Lolium temulentum |
| 3,5,7 | G859 | 939784 | 2.50E-28 | Zea mays |
| 3,5,7 | G859 | 6651032 | 3.10E-28 | Capsicum annuum |
| 3,5,7 | G859 | 1483227 | 4.60E-28 | Betula pendula |
| 3,5,7 | G859 | 5295983 | 8.70E-28 | Oryza sativa |
| 3,5,7 | G859 | 5070137 | 1.10E-27 | Nicotiana sylvestris |
| 9,11,13,15 | G1842 | 6530836 | 5.90E-19 | Lycopersicon esculentum |
| 9,11,13,15 | G1842 | 5606765 | 8.00E-15 | Glycine max |
| 9,11,13,15 | G 1842 | 6826955 | 1.20E-12 | Zea mays |
| 9,11,13,15 | G1842 | 4979250 | 1.50E-11 | Oryza sativa |
| 9,11,13,15 | G1842 | 6536942 | 1.50E-11 | Medicago truncatula |
| 9,11,13,15 | G1842 | 7501504 | 4.OOE-11 | Gossypium arboreum |
| 9,11,13,15 | G1842 | 9444818 | 4.70E-11 | Triticum aestivum |
| 9,11,13,15 | G1842 | 5859176 | 5.40E-11 | Pinus taeda |
| 9,11,13,15 | G1842 | 5777905 | 6.80E-11 | Malus domestica |
| 9,11,13,15 | G1842 | 6647105 | 6.80E-11 | Mesembryanthemum crystallinum |
| 17 | G1843 | 8707754 | 6.60E-15 | Hordeum vulgare |
| 17 | G1843 | 5606765 | 1.10E-14 | Glycine max |
| 17 | G1843 | 4387730 | 1.50E-14 | Lycopersicon esculentum |
| 17 | G1843 | 3646325 | 1.60E-14 | Malus domestica |
| 17 | G1843 | 7625048 | 1.60E-14 | Gossypium arboreum |
| 17 | G1843 | 5050220 | 1.80E-14 | Gossypium hirsutum |
| 17 | G1843 | 9429009 | 3.00E-14 | Triticum aestivum |
| 17 | G1843 | 7145381 | 6.30E-14 | Zea mays |
| 17 | G1843 | 3824730 | 8.20E-14 | Oryza sativa |
| 17 | G1843 | 4528048 | 2.30E-13 | Citrus unshiu |
| 19,21 | G1844 | 5606765 | 1.70E-14 | Glycine max |
| 19,21 | G1844 | 8707754 | 3.30E-13 | Hordeum vulgare |
| 19,21 | G1844 | 9429009 | 4.40E-13 | Triticum aestivum |
| 19,21 | G1844 | 4979250 | 1.00E-12 | Oryza sativa |
| 19,21 | G1844 | 7628118 | 1.10E-12 | Gossypium arboreum |
| 19,21 | G1844 | 5050220 | 1.20E-12 | Gossypium hirsutum |
| 19,21 | G1844 | 6530836 | 1.40E-12 | Lycopersicon esculentum |
| 19,21 | G1844 | 6918768 | 1.70E-12 | Zea mays |
| 19,21 | G1844 | 6536942 | 3.50E-12 | Medicago truncatula |
| 19,21 | G1844 | 2252481 | 3.70E-12 | Ceratopteris richardii |
| 23,25 | G861 | 5601313 | 8.20E-49 | Lycopersicon esculentum |
| 23,25 | G861 | 2735763 | 1.50E-37 | Solanum tuberosum |
| 23,25 | G861 | 6652755 | 5.40E-37 | Paulownia kawakamii |

**Figure 2B**

| SEQ IDs | Gene Ids | Genbank NID | P-value | Species |
|---|---|---|---|---|
| 23,25 | G861 | 9367233 | 3.30E-33 | Hordeum vulgare |
| 23,25 | G861 | 7672990 | 8.90E-29 | Canavalia lineata |
| 23,25 | G861 | 3986688 | 5.20E-26 | Cichorium intybus |
| 23,25 | G861 | 7552197 | 2.40E-25 | Sorghum bicolor |
| 23,25 | G861 | 5295977 | 4.90E-24 | Oryza sativa |
| 23,25 | G861 | 9194959 | 3.60E-19 | Medicago truncatula |
| 23,25 | G861 | 3855425 | 4.40E-19 | Populus tremula x Populus tremuloides |
| 27 | G1759 | 5606765 | 4.60E-16 | Glycine max |
| 27 | G1759 | 7647685 | 4.10E-15 | Lycopersicon esculentum |
| 27 | G1759 | 4979250 | 2.70E-14 | Oryza sativa |
| 27 | G1759 | 8707754 | 6.30E-14 | Hordeum vulgare |
| 27 | G1759 | 5777905 | 6.80E-14 | Malus domestica |
| 27 | G1759 | 7626240 | 1.10E-13 | Gossypium arboreum |
| 27 | G1759 | 5047371 | 1.10E-13 | Gossypium hirsutum |
| 27 | G1759 | 6918768 | 1.20E-13 | Zea mays |
| 27 | G1759 | 8574456 | 1.30E-13 | Capsicum annuum |
| 27 | G1759 | 8216956 | 1.30E-13 | Cucumis sativus |
| 29 | G192 | 7284340 | 3.60E-40 | Glycine max |
| 29 | G192 | 7779802 | 1.10E-39 | Lotus japonicus |
| 29 | G192 | 9361307 | 9.40E-28 | Triticum aestivum |
| 29 | G192 | 7340336 | 8.10E-24 | Oryza sativa |
| 29 | G192 | 6529152 | 4.70E-23 | Lycopersicon esculentum |
| 29 | G192 | 7206269 | 2.90E-22 | Medicago truncatula |
| 29 | G192 | 4886128 | 4.50E-15 | Zea mays |
| 29 | G192 | 8706346 | 4.70E-13 | Hordeum vulgare |
| 29 | G192 | 9302479 | 8.80E-13 | Sorghum bicolor |
| 29 | G192 | 3326241 | 2.40E-12 | Gossypium hirsutum |
| 31 | G234 | 9193243 | 7.50E-60 | Medicago truncatula |
| 31 | G234 | 9264511 | 3.30E-57 | Glycine max |
| 31 | G234 | 7412424 | 3.60E-49 | Lycopersicon esculentum |
| 31 | G234 | 8335078 | 2.60E-48 | Oryza sativa |
| 31 | G234 | 7218651 | 1.00E-42 | Sorghum bicolor |
| 31 | G234 | 9364630 | 9.90E-40 | Triticum aestivum |
| 31 | G234 | 6079814 | 5.10E-36 | Gossypium arboreum |
| 31 | G234 | 9252441 | 5.40E-35 | Solanum tuberosum |
| 31 | G234 | 5860031 | 1.00E-33 | Pinus taeda |
| 31 | G234 | 5050757 | 2.60E-33 | Gossypium hirsutum |
| 33 | G361 | 7561045 | 2.30E-21 | Medicago truncatula |
| 33 | G361 | 9307604 | 1.20E-17 | Sorghum bicolor |
| 33 | G361 | 4119050 | 1.70E-13 | Oryza sativa |
| 33 | G361 | 8175037 | 7.30E-13 | Hordeum vulgare |
| 33 | G361 | 8329902 | 5.30E-09 | Mesembryanthemum crystallinum |
| 33 | G361 | 6534259 | 1.20E-08 | Lycopersicon esculentum |
| 33 | G361 | 7283798 | 1.30E-08 | Glycine max |
| 33 | G361 | 3854369 | 5.50E-08 | Populus tremula x Populus tremuloides |
| 33 | G361 | 9365078 | 1.70E-07 | Triticum aestivum |
| 33 | G361 | 5268965 | 0.00023 | Zea mays |
| 35 | G486 | 6845875 | 3.10E-36 | Glycine max |
| 35 | G486 | 8172030 | 4.20E-29 | Medicago truncatula |
| 35 | G486 | 9416562 | 6.40E-29 | Triticum aestivum |
| 35 | G486 | 5050127 | 4.90E-28 | Gossypium hirsutum |
| 35 | G486 | 7628400 | 6.10E-28 | Gossypium arboreum |
| 35 | G486 | 7781090 | 2.10E-27 | Lotus japonicus |

**Figure 2C**

| SEQ IDs | Gene Ids | Genbank NID | P-value | Species |
|---|---|---|---|---|
| 35 | G486 | 22379 | 3.40E-27 | Zea mays |
| 35 | G486 | 9441376 | 4.80E-27 | Chlamydomonas reinhardtii |
| 35 | G486 | 7409616 | 1.30E-26 | Lycopersicon esculentum |
| 35 | G486 | 8071558 | 1.40E-26 | Solanum tuberosum |
| 37 | G748 | 853689 | 5.60E-87 | Cucurbita maxima |
| 37 | G748 | 7242897 | 3.10E-59 | Oryza sativa |
| 37 | G748 | 5888560 | 9.70E-46 | Lycopersicon esculentum |
| 37 | G748 | 6341666 | 4.50E-38 | Glycine max |
| 37 | G748 | 9190140 | 2.90E-35 | Medicago truncatula |
| 37 | G748 | 7535776 | 4.00E-33 | Sorghum bicolor |
| 37 | G748 | 9419494 | 1.70E-31 | Hordeum vulgare |
| 37 | G748 | 9410157 | 8.20E-29 | Triticum aestivum |
| 37 | G748 | 3929324 | 3.50E-25 | Dendrobium grex Madame Thong-IN |
| 37 | G748 | 6020953 | 7.30E-21 | Zea mays |
| 39 | G994 | 6651291 | 1.50E-55 | Pimpinella brachycarpa |
| 39 | G994 | 7561750 | 5.60E-51 | Medicago truncatula |
| 39 | G994 | 5268844 | 2.10E-50 | Zea mays |
| 39 | G994 | 1430845 | 3.10E-50 | Lycopersicon esculentum |
| 39 | G994 | 1945282 | 5.40E-49 | Oryza sativa |
| 39 | G994 | 22637 | 1.40E-46 | Physcomitrella patens |
| 39 | G994 | 7626566 | 4.40E-44 | Gossypium arboreum |
| 39 | G994 | 2921339 | 4.50E-44 | Gossypium hirsutum |
| 39 | G994 | 7590249 | 3.60E-43 | Glycine max |
| 39 | G994 | 20562 | 6.30E-43 | Petunia x hybrida |
| 41 | G1335 | 19742 | 8.40E-63 | Nicotiana sylvestris |
| 41 | G1335 | 5398738 | 1.20E-59 | Zea mays |
| 41 | G1335 | 9361467 | 1.40E-50 | Triticum aestivum |
| 41 | G1335 | 8330366 | 1.60E-48 | Mesembryanthemum crystallinum |
| 41 | G1335 | 8174823 | 7.50E-43 | Hordeum vulgare |
| 41 | G1335 | 6696628 | 8.00E-42 | Pinus taeda |
| 41 | G1335 | 7721100 | 1.20E-39 | Lotus japonicus |
| 41 | G1335 | 7502173 | 2.60E-37 | Gossypium arboreum |
| 41 | G1335 | 1817176 | 5.60E-36 | Pinus radiata |
| 41 | G1335 | 7550978 | 3.30E-35 | Sorghum bicolor |
| 43 | G562 | 1399004 | 6.60E-142 | Brassica napus |
| 43 | G562 | 5381310 | 6.80E-53 | Catharanthus roseus |
| 43 | G562 | 169958 | 3.80E-45 | Glycine max |
| 43 | G562 | 2879779 | 3.60E-43 | Spinacia oleracea |
| 43 | G562 | 7565950 | 2.10E-41 | Medicago truncatula |
| 43 | G562 | 728627 | 4.50E-41 | Nicotiana tabacum |
| 43 | G562 | 1155053 | 2.30E-40 | Phaseolus vulgaris |
| 43 | G562 | 1498300 | 5.70E-40 | Petroselinum crispum |
| 43 | G562 | 5046889 | 6.70E-34 | Gossypium hirsutum |
| 43 | G562 | 8328888 | 2.60E-25 | Mesembryanthemum crystallinum |
| 45 | G736 | 7409627 | 1.40E-37 | Lycopersicon esculentum |
| 45 | G736 | 9197391 | 5.60E-32 | Medicago truncatula |
| 45 | G736 | 9419494 | 4.70E-27 | Hordeum vulgare |
| 45 | G736 | 7328718 | 1.30E-25 | Oryza sativa |
| 45 | G736 | 9410157 | 1.80E-25 | Triticum aestivum |
| 45 | G736 | 853689 | 5.20E-25 | Cucurbita maxima |
| 45 | G736 | 7535776 | 6.60E-25 | Sorghum bicolor |
| 45 | G736 | 3929324 | 4.70E-21 | Dendrobium grex Madame Thong-IN |
| 45 | G736 | 2393774 | 9.60E-20 | Zea mays |

**Figure 2D**

| SEQ IDs | Gene Ids | Genbank NID | P-value | Species |
|---|---|---|---|---|
| 45 | G736 | 7624398 | 1.10E-19 | Gossypium arboreum |
| 47 | G1073 | 7718401 | 2.20E-55 | Medicago truncatula |
| 47 | G1073 | 6846994 | 2.50E-44 | Glycine max |
| 47 | G1073 | 7615218 | 1.60E-42 | Lotus japonicus |
| 47 | G1073 | 7333102 | 2.70E-34 | Lycopersicon esculentum |
| 47 | G1073 | 9445090 | 3.40E-25 | Triticum aestivum |
| 47 | G1073 | 9252370 | 2.20E-24 | Solanum tuberosum |
| 47 | G1073 | 5042437 | 4.60E-21 | Oryza sativa |
| 47 | G1073 | 7536402 | 5.30E-20 | Sorghum bicolor |
| 47 | G1073 | 2213535 | 7.30E-19 | Pisum sativum |
| 47 | G1073 | 7624850 | 2.10E-18 | Gossypium arboreum |
| 49 | G1435 | 9203811 | 3.70E-37 | Glycine max |
| 49 | G1435 | 9430136 | 4.10E-35 | Lycopersicon esculentum |
| 49 | G1435 | 8904354 | 4.30E-32 | Hordeum vulgare |
| 49 | G1435 | 5050706 | 3.30E-26 | Gossypium hirsutum |
| 49 | G1435 | 7614196 | 6.40E-19 | Lotus japonicus |
| 49 | G1435 | 7551484 | 1.00E-18 | Sorghum bicolor |
| 49 | G1435 | 6916552 | 7.20E-12 | Lycopersicon pennellii |
| 49 | G1435 | 2443007 | 5.50E-11 | Oryza sativa |
| 49 | G1435 | 9255229 | 1.30E-10 | Zea mays |
| 49 | G1435 | 7766737 | 2.80E-10 | Medicago truncatula |
| 51 | G180 | 8468047 | 1.90E-35 | Oryza sativa |
| 51 | G180 | 7559831 | 1.20E-24 | Medicago truncatula |
| 51 | G180 | 5272716 | 9.90E-24 | Lycopersicon esculentum |
| 51 | G180 | 9187621 | 3.30E-23 | Solanum tuberosum |
| 51 | G180 | 6566312 | 1.30E-22 | Glycine max |
| 51 | G180 | 9304207 | 1.30E-21 | Sorghum bicolor |
| 51 | G180 | 7721184 | 1.30E-20 | Lotus japonicus |
| 51 | G180 | 9444636 | 3.10E-19 | Triticum aestivum |
| 51 | G180 | 3220212 | 5.20E-19 | Gossypium hirsutum |
| 51 | G180 | 1159876 | 8.00E-19 | Avena fatua |
| 53 | G592 | 7924069 | 7.10E-27 | Glycine max |
| 53 | G592 | 5896650 | 1.10E-22 | Lycopersicon esculentum |
| 53 | G592 | 6279773 | 1.10E-17 | Lycopersicon pennellii |
| 53 | G592 | 9364330 | 1.20E-14 | Triticum aestivum |
| 53 | G592 | 6166282 | 5.40E-14 | Pinus taeda |
| 53 | G592 | 8367093 | 1.60E-12 | Zea mays |
| 53 | G592 | 9301543 | 6.60E-11 | Sorghum bicolor |
| 53 | G592 | 7562632 | 2.80E-10 | Medicago truncatula |
| 53 | G592 | 702652 | 5.80E-05 | Oryza sativa |
| 53 | G592 | 7322923 | 0.094 | Lycopersicon hirsutum |
| 55 | G208 | 437326 | 2.80E-65 | Gossypium hirsutum |
| 55 | G208 | 7765706 | 4.40E-64 | Medicago truncatula |
| 55 | G208 | 5269878 | 5.80E-64 | Lycopersicon esculentum |
| 55 | G208 | 19054 | 6.90E-63 | Hordeum vulgare |
| 55 | G208 | 2605616 | 1.00E-62 | Oryza sativa |
| 55 | G208 | 7626566 | 3.50E-62 | Gossypium arboreum |
| 55 | G208 | 6667606 | 4.10E-62 | Glycine max |
| 55 | G208 | 517492 | 1.80E-60 | Zea mays |
| 55 | G208 | 9302672 | 2.40E-57 | Sorghum bicolor |
| 55 | G208 | 5860031 | 1.30E-54 | Pinus taeda |

A transgenic plant comprising a recombinant polynucleotide comprising a nucleotide sequence encoding a polypeptide comprising at least 6 consecutive amino acids of a sequence selected from the group consisting of SEQ ID Nos. 2N, where N=1-28 but excluding SEQ ID No. 28, wherein said transgenic plant has (I) a modified flowering time compared with another plant lacking the recombinant polynucleotide or (ii) a modified vernalization requirement compared with another plant lacking the recombinant polynucleotide.

The transgenic plant as defined above, wherein the nucleotide sequence encodes a polypeptide comprising a conserved domain selected from the group consisting of conserved domains of SEQ ID Nos. 2N, where N=1-28.

The transgenic plant as defined above, wherein the recombinant polynucleotide further comprises a promoter operably linked to said nucleotide sequence.

The transgenic plant as defined above wherein said promoter is constitutive or inducible or tissue-active.

The transgenic plant as defined above, wherein said recombinant polynucleotide encodes a polypeptide comprising a conserved domain having greater than an 84% sequence identity to a sequence selected from the group consisting of SEQ ID Nos. 2N, where N=1-28.

A method for altering the flowering time or vernalization requirement of a plant, said method comprising (a) transforming a plant with a recombinant polynucleotide comprising a nucleotide sequence encoding a polypeptide comprising at least 6 consecutive amino acids of a sequence selected from the group consisting of SEQ ID Nos. 2N, where N=1-28 but excluding SEQ ID No. 28, (b) selecting said transformed plants; and (c) identifying a transformed plant having an altered flowering time.

The method as defined above, wherein the nucleotide sequence encodes a polypeptide comprising a conserved domain selected from the group consisting of conserved domains of SEQ ID Nos. 2N, where N=1-28.

The method as defined above, wherein the recombinant polynucleotide further comprises a promoter operably linked to said nucleotide sequence.

The method as defined above, wherein said promoter is constitutive or inducible or tissue-active.

The method as defined above, wherein said recombinant polynucleotide encodes a polypeptide comprising a conserved domain having greater than an 84% sequence identity to a sequence selected from the group consisting of SEQ ID Nos. 2N, where N=1-28.

A method for altering the flowering time or vernalization requirement of a plant, said method comprising (a) transforming the plant with a recombinant polynucleotide comprising a nucleotide sequence comprising at least 18 consecutive nucleotides of a sequence selected from the group consisting of SEQ lD Nos. 2N-1, where N= 1-28, but excluding SEQ ID No. 27; and (b) selecting said transformed plant.

The method as defined above, wherein said recombinant polynucleotide encodes a polypeptide comprising a conserved domain having greater than an 84% sequence identity to a sequence selected from the group consisting of SEQ ID Nos. 2N, where N=1-28.

A method for altering a plant's flowering time or vernalization requirement, said method comprising (a) providing a database sequence; (b) comparing said database sequence with a polypeptide selected from SEQ ID Nos. 2N, where N=1-28; (c) selecting a database sequence that meets selected sequence criteria; and (d) transforming said selected database sequence in the plant,

The method as defiend above, werein said recombinant polynucleotide encodes a polypeptide comprising a conserved domain having greater than an 84% sequence identity to a sequence selected from the group consisting of SEQ ID Nos. 2N, where N=1-28.

A method for altering a plant's flowering time or vernalization requirement, said method comprising (a) providing a database sequence; (b) comparing said database sequence with a polynucleotide selected from SEQ ID Nos. 2N-1, where N= 1-28; (c) selecting a database sequence that meets selected sequence criteria; and (d) transforming said selected database sequence in the plant.

The method as defined above wherein said recombinant polynucleotide encodes a polypeptide comprising a conserved domain having greater than an 84% sequence identity to a sequence selected from the group consisting of SEQ ID Nos. 2N, where N=1-28.

## Claims

1. A polynucleotide sequence encoding a transcription factor polypeptide having an amino acid sequence comprising a conserved domain that has at least 85% sequence identity to a conserved domain of amino acid residues 24-43 of SEQ ID NO: 42, wherein said transcription factor polypeptide confers late flowering in a plant as compared to a flowering time of a wild-type plant.

2. Use of the transcription factor polypeptide of claim 1 for delaying flowering time of a plant as compared to the flowering time of a wild-type plant of the same species.

3. A polynucleotide sequence encoding a transcription factor polypeptide having an amino acid sequence comprising a conserved domain that has at least 85% sequence identity to a conserved domain of amino acid residues 24-43 of SEQ ID NO: 42, wherein said transcription factor polypeptide confers increased yield of a plant as compared to a yield from a wild-type plant.

4. Use of the transcription factor polypeptide of claim 3 for increasing yield of a plant as compared to a yield of a wild-type plant of the same species.

5. A method for increasing yield of a plant, the method comprising the steps of: (a) transforming a first plant with a nucleic acid construct comprising a polynucleotide sequence;
wherein the polynucleotide sequence is operably linked to a regulatory sequence;
wherein the polynucleotide sequence encodes a transcription factor polypeptide which comprises a conserved domain that has at least 85% sequence identity to a conserved domain of amino acid residues 24-43 of SEQ ID NO: 42; and (b) growing a transgenic plant from a cell or seed derived the first plant, wherein the transcription factor polypeptide is expressed in the transgenic plant at higher levels than a wild-type plant, and the levels of expression are sufficient to delay flowering and increase yield of the transgenic plant relative to the wild-type plant.

6. A method according to claim 5, wherein the regulatory sequence is a constitutive, inducible or tissue-specific promoter.

7. A transgenic plant transformed with a nucleic acid construct comprising a regulatory sequence and a transcription factor polynucleotide sequence;
wherein the transcription factor polynucleotide sequence encodes a transcription factor polypeptide that has a conserved domain with at least 85% sequence identity to a conserved domain of amino acid residues 24-43 of SEQ ID NO: 42.

8. The transgenic plant of claim 7, wherein the transcription factor polypeptide is expressed in the transgenic plant at higher levels in the transgenic plant than a wild-type plant, and the expression levels are sufficient to delay flowering of the transgenic plant relative to a wild-type plant.

9. The transgenic plant of claim 7, wherein the transcription factor polypeptide is expressed in the transgenic plant at higher levels in the transgenic plant than a wild-type plant, and the expression levels are sufficient to increase yield of the transgenic plant relative to a wild-type plant.

10. The transgenic plant of claim 7, wherein the regulatory sequence is a constitutive, inducible or tissue-specific promoter.
